# EUROPEAN PATENT APPLICATION

(11) **EP 3 656 400 A1**
(43) Date of publication of application: **27.05.2020**
(21) Application number: 18836127.3
(22) Date of filing: 19.07.2018
(51) Int. Cl.: A61K 45/06, A61K 31/196, A61K 31/341, A61K 31/403, A61K 31/407, A61K 31/5415, A61K 31/63, A61K 39/395, A61P 31/00, A61P 35/00, A61P 43/00, C07K 16/28

(54) **COMBINATION USE OF INHIBITOR TARGETING PD-1/PD-L1 AND COX-2 INHIBITOR**

(30) Priority: 20.07.2017 JP 2017140891; 01.02.2018 JP 2018016074
(71) Applicant: National University Corporation Hokkaido University, Sapporo-shi, Hokkaido 060-0808 (JP); Fuso Pharmaceutical Industries, Ltd., Osaka-shi, Osaka 541-0045 (JP)
(72) Inventor: KONNAI, Satoru, Sapporo-shi Hokkaido 060-0808 (JP); OHASHI, Kazuhiko, Sapporo-shi Hokkaido 060-0808 (JP); MURATA, Shiro, Sapporo-shi Hokkaido 060-0808 (JP); OKAGAWA, Tomohiro, Sapporo-shi Hokkaido 060-0808 (JP); MAEKAWA, Naoya, Sapporo-shi Hokkaido 060-0808 (JP); NISHIMORI, Asami, Sapporo-shi Hokkaido 060-0808 (JP); GOTO, Shinya, Sapporo-shi Hokkaido 060-0808 (JP); SUZUKI, Yasuhiko, Sapporo-shi Hokkaido 060-0808 (JP); NAKAJIMA, Chie, Sapporo-shi Hokkaido 060-0808 (JP); SAJIKI, Yamato, Sapporo-shi Hokkaido 060-0808 (JP)
(74) Representative: Hoffmann Eitle
(86) International application number: PCT/JP2018/027041
(87) International publication number: WO 2019/017420

(57) **Abstract**

The present invention provides a novel therapeutic strategy using an inhibitor targeting PD-1/PD-L1. A pharmaceutical composition which comprises a COX-2 inhibitor and is administered before, after or simultaneously with the administration of an inhibitor targeting PD-1/PD-L1. A potentiator for the immunostimulatory effect of an inhibitor targeting PD-1/PD-L1, which comprises a COX-2 inhibitor.

## Description

### TECHNICAL FIELD

The present invention relates to combined use of an inhibitor targeting PD-1/PD-L1 and a COX-2 inhibitor.

### BACKGROUND ART

The interaction between PD-1 and PD-L1 is one of the major molecular mechanisms through which tumors and infections evade immune responses. It has been reported that inhibition of the above interaction by using an antibody which specifically binds to either of those molecules can produce antitumor effects and anti-pathogenic effects (Non-Patent Documents Nos. 1 to 5).

### PRIOR ART LITERATURE

### Non-Patent Documents

Non-Patent Document No. 1: Bramer J, Reckamp K, et al: Nivolumab versus Docetaxel in Advanced Nonsquamous Non-Small-Cell Lung Cancer. N Engl J Med, 373:1627-1639, 2015.
Non-Patent Document No. 2: Hamanishi J, Mandai M, Ikeda T, et al: Safety and Antitumor Activity of Anti-PD-1 Antibody, Nivolumab, in Patients with Platinum-Resistant Ovarian Cancer. J Clin Oncol, 33:4015-4022, 2015.
Non-Patent Document No. 3: Motzer RJ, Escudier B, McDermott DF, et al: Nivolumab versus Everolimus in Advanced Renal-Cell Carcinoma. N Engl J Med, 373:1803-1813, 2015.
Non-Patent Document No. 4: Barber DL, Wherry EJ, Masopust D, et al: Restoring function in exhausted CD8 T cells during chronic viral infection. Nature, 439:682-687, 2006.
Non-Patent Document No. 5: Velu V, Titanji K, Zhu B, et al: Enhancing SIV-specific immunity in vivo by PD-1 blockade. Nature 458:206-210, 2009.

### DISCLOSURE OF THE INVENTION

### PROBLEM FOR SOLUTION BY THE INVENTION

It is an object of the present invention to provide a novel therapeutic strategy using inhibitors targeting PD-1/PD-L1.

### MEANS TO SOLVE THE PROBLEM

Toward establishment of a novel control method for canine tumors and bovine infections, the present inventors have confirmed in *in vitro* tests an immunostimulatory effect induced by COX-2 inhibitors and enhancement of that effect when such inhibitors are used in combination with anti-PD-Ll antibody. The present invention has been achieved based on these findings.

A summary of the present invention is as described below.
(1) A pharmaceutical composition which comprises a COX-2 inhibitor and is administered before, after or simultaneously with the administration of an inhibitor targeting PD-1/PD-L1.
(2) The pharmaceutical composition of (1) above, wherein the inhibitor targeting PD-1/PD-L1 is an antibody.
(3) The pharmaceutical composition of (1) or (2) above, wherein the antibody is at least one antibody selected from the group consisting of anti-PD-1 antibody and anti-PD-Ll antibody.
(4) The pharmaceutical composition of any one of (1) to (3) above, wherein the COX-2 inhibitor is at least one compound selected from the group consisting of meloxicam, piroxicam, celecoxib, firocoxib, robenacoxib, carprofen and etodolac.
(5) The pharmaceutical composition of any one of (1) to (4) above for use in prevention and/or treatment of cancer and/or infection.
(6) The pharmaceutical composition of any one of (1) to (5) above, wherein the inhibitor targeting PD-1/PD-L1 and the COX-2 inhibitor are administered separately.
(7) The pharmaceutical composition of any one of (1) to (5) above, which is a combination drug comprising the inhibitor targeting PD-1/PD-L1 and the COX-2 inhibitor.
(8) A potentiator for the immunostimulatory effect of an inhibitor targeting PD-1/PD-L1, which comprises a COX-2 inhibitor.
(9) A method of preventing and/or treating cancer and/or infection, comprising administering to a human or animal subject a pharmaceutically effective amount of a COX-2 inhibitor before, after or simultaneously with the administration of an inhibitor targeting PD-1/PD-L1.
(10) Use of a COX-2 inhibitor for preventing and/or treating cancer and/or infection, wherein the COX-2 inhibitor is administered before, after or simultaneously with the administration of an inhibitor targeting PD-1/PD-L1.
(11) Use of a COX-2 inhibitor for use in a method of preventing and/or treating cancer and/or infection, wherein the COX-2 inhibitor is administered before, after or simultaneously with the administration of an inhibitor targeting PD-1/PD-L1.

### EFFECT OF THE INVENTION

Immunostimulatory effect is enhanced by combined use of an inhibitor targeting PD-1/PD-L1 and a COX-2 inhibitor.

The present specification encompasses the contents disclosed in the specifications and/or drawings of Japanese Patent Application Nos. 2017-140891 and No. 2018-016074 based on which the present patent application claims priority.

### BRIEF DESCRIPTION OF THE DRAWINGS

[Fig. 1] PGE₂ production from canine tumor cell lines CMeC, LMeC, CMM-1, CMM-2 (these four are derived from melanoma) and HM-POS (derived from osteosarcoma). PGE₂ production tended to be high in CMM-1 and HM-POS.
[Fig. 2] *COX2* expression levels in canine tumor cell lines CMeC, LMeC, CMM-1, CMM-2 (these four are derived from melanoma) and HM-POS (derived from osteosarcoma). Consistent with PGE₂ production, *COX2* expression levels were high in CMM-1 and HM-POS.
[Fig. 3] Effect of PGE₂ on canine peripheral blood mononuclear cells (PBMCs). Canine PBMCs were cultured under stimulation for 3 days in the presence of a superantigen SEB and anti-CD28 antibody. Then, IL-2 and IFN-γ concentrations in the resultant culture supernatant were determined by ELISA. PGE₂ inhibited production of IL-2 and IFN-γ from canine PBMCs.
[Fig. 4] PGE₂ production inhibitory effect of COX-2 inhibitor upon canine tumor cell lines. Meloxicam showed a tendency to decrease PGE₂ production from canine tumor cell lines CMM-1 (derived from melanoma) and HM-POS (derived from osteosarcoma).
[Fig. 5] PGE₂ production inhibitory effect of COX-2 inhibitor upon canine PBMCs. Meloxicam decreased the amount of PGE₂ produced from canine PBMCs cultured for 3 days in the presence of a superantigen SEB and anti-CD28 antibody for stimulation.
[Fig. 6] Canine immune cell activating effect of COX-2 inhibitor. Canine PBMCs were cultured for 3 days in the presence of a superantigen SEB and anti-CD28 antibody to stimulate canine lymphocytes. Then, IL-2 concentration in the resultant culture supernatant was determined by ELISA. Meloxicam increased the IL-2 production from canine PBMCs.
[Fig. 7] Canine immune cell activating effect by combined use of anti-PD-Ll antibody and COX-2 inhibitor. Canine PBMCs were cultured for 3 days in the presence of a superantigen SEB and anti-CD28 antibody to stimulate canine lymphocytes. Then, IL-2 concentration in the resultant culture supernatant was determined by ELISA. Although anti-PD-L1 antibody taken alone increased the IL-2 production from canine PBMCs, the IL-2 production was further increased when meloxicam was used in combination with the antibody.
[Fig. 8] Inhibition of the binding of recombinant canine PD-L1 to recombinant canine PD-1. The binding of canine PD-L1-Ig to canine PD-1-Ig was detected on ELISA plates. The optical density (O.D.) without addition of antibody was taken as 100%. O.D. at each antibody concentration was shown as relative value. Among rat anti-bovine PD-L1 monoclonal antibodies 4G12 (Rat IgG2a (κ)), 5A2 (Rat IgG1 (κ)) and 6G7 (Rat IgM (κ)) which showed cross-reaction with canine PD-L1, clones 4G12 and 6G7 exhibited a high binding inhibition capacity.
[Fig. 9] Schematic drawings of pDC6 vector and a rat-canine chimeric anti-PD-Ll antibody.
[Fig. 10] Expression and purification of rat-canine chimeric anti-PD-Ll antibodies c4G12 and c6G7. SDS-PAGE was performed under non-reducing conditions, followed by visualization of bands by CBB staining. a: purification with protein A alone. b: a + gel filtration chromatography.

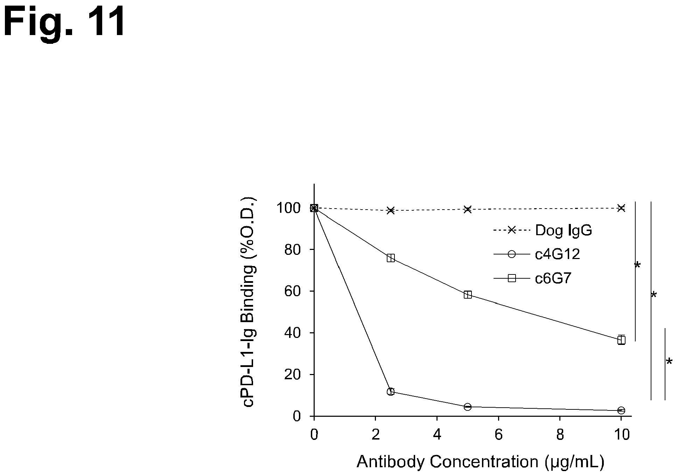

[Fig. 11] PD-1/PD-L1 binding inhibition activities of rat-canine chimeric anti-PD-L1 antibodies c4G12 and c6G7.
[Fig. 12] Establishment of cell clones capable of high expression of rat-canine chimeric anti-PD-Ll antibody c4G12.
[Fig. 13] SDS-PAGE images of rat-canine chimeric anti-PD-Ll antibody c4G12. Rat anti-bovine PD-L1 antibody 4G12 and rat-canine chimeric anti-PD-Ll antibody c4G12 were electrophoresed under reducing conditions and non-reducing conditions, followed by visualization of bands by CBB staining. Under reducing conditions, a band of antibody's heavy chain was detected at around 50 kDa and a band of antibody's light chain at around 25 kDa. No bands other than the bands of interest were detected.
[Fig. 14] Inhibitory activities of rat anti-bovine PD-L1 antibody 4G12 and rat-canine chimeric anti-PD-Ll antibody c4G12 against canine PD-1/PD-L1 binding and CD80/PD-L1 binding. Rat anti-bovine PD-L1 monoclonal antibody 4G12 and rat-canine chimeric anti-PD-Ll antibody c4G12 reduced the amounts of binding of PD-L1-Ig to canine PD-1-Ig and CD80-Ig. No change due to chimerization of the antibody was observed in binding inhibition activity
[Fig. 15] Canine immune cell activation effect by rat-canine chimeric anti-PD-Ll antibody c4G12. Canine PBMCs were cultured under stimulation for 3 days, followed by determination of IL-2 and IFN-γ concentrations in the supernatant by ELISA. Further, nucleic acid analogue EdU was added to the culture medium at day 2 of the culture under stimulation, followed by determination of the EdU uptake by flow cytometry. Rat-canine chimeric anti-PD-Ll antibody c4G12 increased the production of IL-2 and IFN-γ from canine PBMCs and enhanced proliferation of CD4⁺ and CD8⁺ lymphocytes.
[Fig. 16] Expression of PD-L1 in oral melanoma (A) and undifferentiated sarcoma (B)

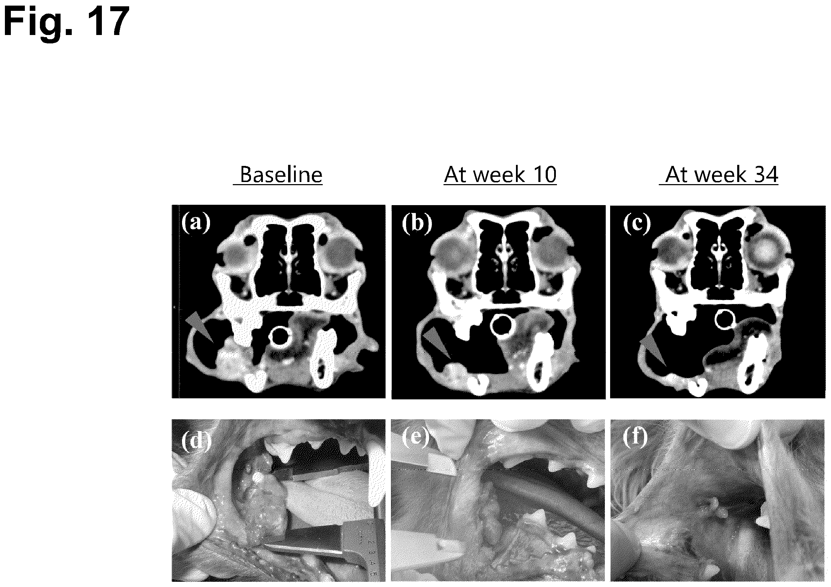

[Fig. 17] CT images and appearances of tumor in a test of treatment by administering rat-canine chimeric anti-PD-Ll antibody c4G12 to a dog with oral melanoma. (a,d) Before the start of the treatment, (b,e) at week 10 of the treatment, and (c,f) at week 34 of the treatment. A remarkable antitumor effect was recognized upon five administrations of the antibody (at week 10 from the start of the treatment). At week 34, a further reduction of tumor was confirmed.
[Fig. 18] Time-dependent changes in the longest diameter of the tumor in the dog with oral melanoma shown in Fig. 17. Reduction by 30% or more compared to the baseline longest diameter was regarded as partial response (PR).
[Fig. 19] CT images in a test of treatment by administering rat-canine chimeric anti-PD-L1 antibody c4G12 to a dog with undifferentiated sarcoma. (a,c) Before the start of the treatment, (b,d) at week 3 of the treatment. A remarkable reduction of tumor was recognized upon two administrations of the antibody.
[Fig. 20] CT images in a test of treatment by administering rat-canine chimeric anti-PD-L1 antibody c4G12 to dogs with oral melanoma (pulmonary metastatic cases). (a,d,g) Before the start of the treatment, (b,e,h) at week 6 of the treatment, and (c,f,i) at week 18 of the treatment. A plurality of pulmonary metastatic lesions disappeared upon nine administrations of the antibody.
[Fig. 21] Time-dependent changes in the proportion survival of dogs with oral melanoma after the occurrence of pulmonary metastasis. In the antibody administration group, the survival duration may have been prolonged compared to the control group.
[Fig. 22] CDR1, CDR2 and CDR3 regions in the light chain variable region and the heavy chain variable region of rat anti-bovine PD-L1 antibody 4G12 are illustrated.
[Fig. 23] Effect of PGE₂ on bovine T cell responses.
[Fig. 24] Effect of PGE₂ on expression of immune-related genes in bovine PBMCs.
[Fig. 25] Effect of PGE₂ on expression of PD-L1 in bovine PBMCs.
[Fig. 26] Immunostimulatory effect of COX-2 inhibitor in bovine PBMCs.
[Fig. 27] Kinetic analyses of PGE₂ in cattle infected with *M*. *avium* subsp. *paratuberculosis.*
[Fig. 28] Changes in PD-L1 expression level by antigen stimulation in cattle infected with *M*. *avium* subsp. *paratuberculosis.*
[Fig. 29] Analyses of expression of PGE₂, EP2 and PD-L1 in *M*. *avium* subsp. *paratuberculosis-infected* lesions.
[Fig. 30] Activation of *M*. *avium* subsp. *paratuberculosis-specific* immune responses by COX-2 inhibitor.
[Fig. 31] Immunostimulatory effect of rat anti-bovine PD-L1 antibody in cattle infected with *M*. *avium* subsp. *paratuberculosis.*
[Fig. 32] Combined effect of COX-2 inhibitor and rat anti-bovine PD-L1 antibody on activation of *M*. *avium* subsp. *paratuberculosis-specific* immune responses.
[Fig. 33] Combined effect of COX-2 inhibitor and rat-bovine chimeric anti-bovine PD-L1 antibody on activation of *M*. *avium* subsp. *paratuberculosis-specific* immune responses.
[Fig. 34] Kinetic analyses of PGE₂ in BLV-infected cattle.
[Fig. 35] Expression analyses of *COX-2* and *EP4* in BLV-infected cattle.
[Fig. 36] Changes in PGE₂ production by antigen stimulation in BLV-infected cattle.
[Fig. 37] Effect of PGE₂ on BLV provirus in PBMCs derived from BLV-infected cattle.
[Fig. 38] Changes in PD-L1 expression by antigen stimulation in BLV-infected cattle.
[Fig. 39] Activation of BLV-specific immune responses by COX-2 inhibitor.
[Fig. 40] Antiviral effect of COX-2 inhibitor in BLV-infected cattle.
[Fig. 41] Combined effect of COX-2 inhibitor and rat anti-bovine PD-L1 antibody on activation of BLV-specific immune responses.
[Fig. 42] Combined effect of COX-2 inhibitor and rat-bovine chimeric anti-bovine PD-L1 antibody on activation of BLV-specific immune responses.
[Fig. 43] Antiviral effect from combined use of COX-2 inhibitor and rat-bovine chimeric anti-bovine PD-L1 antibody in BLV-infected cattle.
[Fig. 44] Kinetic analyses of PGE₂ in *Mycoplasma bovis*-infected cattle.
[Fig. 45] Correlation between plasma PGE₂ and indicators of immunosuppression in *Mycoplasma bovis*-infected cattle.
[Fig. 46] Expression analyses of *COX-2* and *EP4* in *Mycoplasma bovis*-infected cattle.
[Fig. 47] Combined immunostimulatory effect of COX-2 inhibitor and rat anti-bovine PD-L1 antibody in *Mycoplasma bovis*-infected cattle.
[Fig. 48] The amino acid sequence of rat-bovine chimeric anti-bovine PD-L1 antibody ch4G12. CDR1, CDR2 and CDR3 regions in the light chain variable region and the heavy chain variable region of rat anti-bovine PD-L1 antibody 4G12 are shown. Further, amino acids introduced as mutations to bovine IgG1 (CH2 domain) are also shown (amino acid numbers and mutations: 250 E→P, 251 L→V, 252 P→7A, 253 G→deletion, 347 A→S, 348 P→S).
[Fig. 49] Schematic drawings of pDC6 vector and rat-bovine chimeric anti-bovine PD-L1 antibody ch4G12.
[Fig. 50] Confirmation of the purity of purified rat-bovine chimeric anti-bovine PD-L1 antibody ch4G12.
[Fig. 51] Binding specificity of rat-bovine chimeric anti-bovine PD-L1 antibody ch4G12.
[Fig. 52] Inhibitory activity of rat-bovine chimeric anti-bovine PD-L1 antibody ch4G12 against bovine PD-1/PD-L1 binding (the test results of inhibition against binding of bovine PD-L1 expressing cells and soluble bovine PD-1).
[Fig. 53] Inhibitory activity of rat-bovine chimeric anti-bovine PD-L1 antibody ch4G12 against bovine PD-1/PD-L1 binding (the test results of inhibition against binding of bovine PD-1 expressing cells and soluble bovine PD-L1).
[Fig. 54] Responsivity of rat-bovine chimeric anti-bovine PD-L1 antibody ch4G12 to BLV antigen (in terms of cell proliferation).
[Fig. 55] Responsivity of rat-bovine chimeric anti-bovine PD-L1 antibody ch4G12 to BLV antigen (in terms of IFN-γ production).
[Fig. 56] The proliferation response of T cells against BLV antigen in a calf experimentally infected with BLV through administration of rat-bovine chimeric anti-bovine PD-L1 antibody ch4G12.
[Fig. 57] Changes in BLV provirus loads in the calf experimentally infected with BLV through administration of rat-bovine chimeric anti-bovine PD-L1 antibody ch4G12.

### BEST MODES FOR CARRYING OUT THE INVENTION

Hereinbelow, the present invention will be described in detail.

The present invention provides a pharmaceutical composition which comprises a COX-2 inhibitor and is administered before, after or simultaneously with the administration of an inhibitor targeting PD-1/PD-L1.

Cyclooxygenase 2 (COX-2) is an enzyme involved in a process of biosynthesizing prostanoids including prostaglandin E2 (PGE₂). In contrast to COX-1 that is expressed constitutively, expression of COX-2 is induced by stimulation from cytokines, growth factors, etc. in inflammatory tissues. High expression of COX-2 has been reported in various tumors and infections, and it is believed that COX-2 is involved in the growth and pathogenesis of tumor cells and infected cells. Since PGE₂ inhibits, in particular, the effector function of cytotoxic T-cells via receptors EP2 and EP4, PGE₂ has recently been attracting attention as a humoral factor constituting an immunosuppressive tumor microenvironment. On the other hand, COX-2 inhibitors are expected to decrease PGE₂ production to thereby reduce the suppression upon immune cells. In mouse models, enhancement of antitumor effect and antiviral effect by combined use of a COX-2 inhibitor (such as celecoxib) and an inhibitor targeting PD-1/PD-L1 has been recognized.

COX-2 inhibitor may be an agent that selectively inhibits COX-2. Specific examples of COX-2 inhibitor include, but are not limited to, meloxicam, piroxicam, celecoxib, firocoxib, robenacoxib, carprofen and etodolac.

PD-1 (Programmed cell death-1) is a membrane protein expressed in activated T cells and B cells. Its ligand PD-L1 is expressed in various cells such as antigen-presenting cells (monocytes, dendritic cells, etc.) and cancer cells. PD-1 and PD-L1 work as inhibitory factors which inhibit T cell activation. Certain types of cancer cells and virus-infected cells escape from host immune surveillance by expressing the ligand of PD-1 to thereby inhibit T cell activation.

As inhibitors targeting PD-1/PD-L1, substances which specifically bind to PD-1 or PD-L1 may be given. Such substances include, but are not limited to, proteins, polypeptides, oligopeptides, nucleic acids (including natural-type and artificial nucleic acids), low molecular weight organic compounds, inorganic compounds, cell extracts, and extracts from animals, plants, soils or the like. These substances may be either natural or synthetic products. Preferable inhibitors targeting PD-1/PD-L1 are antibodies. More preferably, antibodies such as anti-PD-1 antibody and anti-PD-Ll antibody may be given. Any type of antibody may be used as long as it has an inhibitory activity targeting PD-1/PD-L1. The antibody may be any of polyclonal antibody, monoclonal antibody, chimeric antibody, single chain antibody, humanized antibody or human-type antibody. Methods for preparing such antibodies are known. The antibody may be derived from any organisms such as human, mouse, rat, rabbit, goat, guinea pig, dog or cattle. As used herein, the term "antibody" is a concept encompassing antibodies of smaller molecular sizes such as Fab, F(ab)'₂, ScFv, Diabody, V_{H}, V_{L}, Sc(Fv)₂, Bispecific sc(Fv)₂, Minibody, scFv-Fc monomer or scFv-Fc dimer.

As an example of anti-PD-Ll antibody, one comprising (a) a light chain comprising a light chain variable region containing CDR1 having the amino acid sequence of QSLLYSENQKDY (SEQ ID NO: 37), CDR2 having the amino acid sequence of WAT and CDR3 having the amino acid sequence of GQYLVYPFT (SEQ ID NO: 38) and a light chain constant region of an antibody of an animal other than rat; and (b) a heavy chain comprising a heavy chain variable region containing CDR1 having the amino acid sequence of GYTFTSNF (SEQ ID NO: 39), CDR2 having the amino acid sequence of IYPEYGNT (SEQ ID NO: 40) and CDR3 having the amino acid sequence of ASEEAVISLVY (SEQ ID NO: 41) and a heavy chain constant region of an antibody of an animal other than rat may be given.

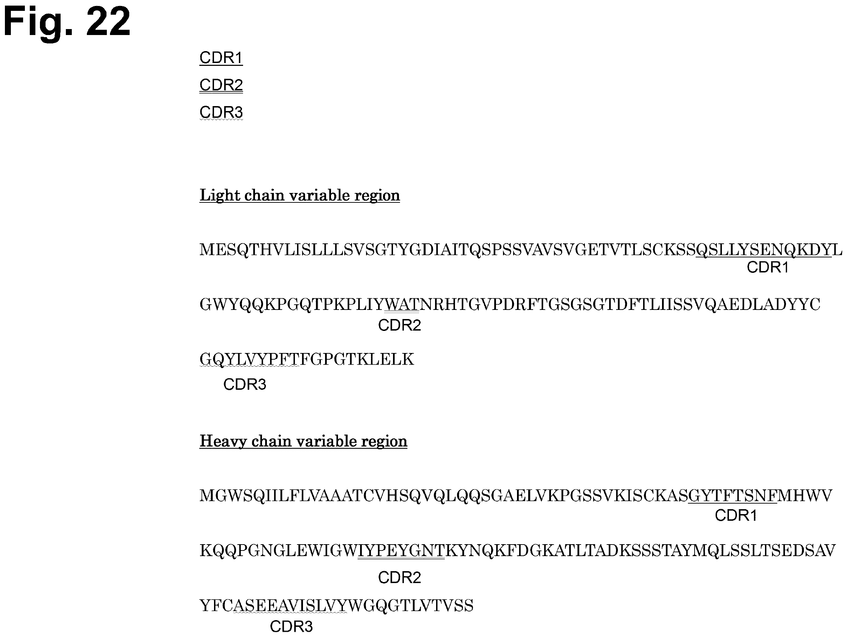

CDR1, CDR2 and CDR3 in the light chain variable region (VL) of rat anti-bovine PD-L1 antibody 4G12 are a region comprising the amino acid sequence of QSLLYSENQKDY (SEQ ID NO: 37), a region comprising the amino acid sequence of WAT and a region comprising the amino acid sequence of GQYLVYPFT (SEQ ID NO: 38), respectively (see Fig. 22).

Further, CDR1, CDR2 and CDR3 in the heavy chain variable region (VH) of rat anti-bovine PD-L1 antibody 4G12 are a region comprising the amino acid sequence of GYTFTSNF (SEQ ID NO: 39), a region comprising the amino acid sequence of IYPEYGNT (SEQ ID NO: 40) and a region comprising the amino acid sequence of ASEEAVISLVY (SEQ ID NO: 41), respectively (see Fig. 22).

In the amino acid sequences of QSLLYSENQKDY (SEQ ID NO: 37), WAT and GQYLVYPFT (SEQ ID NO: 38), as well as the amino acid sequences of GYTFTSNF (SEQ ID NO: 39), IYPEYGNT (SEQ ID NO: 40) and ASEEAVISLVY (SEQ ID NO: 41), one, two, three, four or five amino acids may be deleted, substituted or added.

In the above-described anti-PD-L1 antibody, VL and VH thereof may be derived from rat. For example, VL thereof may be the VL of a rat anti-bovine PD-L1 antibody, and VH thereof may be the VH of the rat anti-bovine PD-L1 antibody.

The amino acid sequence of the VL and the amino acid sequence of the VH of the rat anti-bovine PD-L1 antibody are shown in SEQ ID NOS: 1 and 2, respectively. The amino acid sequences as shown in SEQ ID NOS: 1 and 2 may have deletion(s), substitution(s) or addition(s) of one or several (e.g., up to five, about 10 at the most) amino acids. Even when such mutations have been introduced, the resulting amino acid sequences are capable of having the function as VL or VH of the PD-L1 antibody.

The CL and CH of an antibody of an animal other than rat may be derived from an animal which produces a PD-L1 that cross-reacts with rat anti-bovine PD-L1 antibody 4G12.

There are two types of immunoglobulin light chain, which are called Kappa chain (κ) and Lambda chain (λ). In the above-described anti-PD-L1 antibody, the light chain constant region (CL) of an antibody of an animal other than rat may have the amino acid sequence of the constant region of either Kappa chain or Lambda chain. However, the relative abundance of Lambda chain is higher in ovine, feline, canine, equine and bovine, and that of Kappa chain is higher in mouse, rat, human and porcine. Since a chain with a higher relative abundance is considered to be preferable, an ovine, feline, canine, equine or bovine antibody preferably has the amino acid sequence of the constant region of Lambda chain whereas a mouse, rat, human or porcine antibody preferably has the amino acid sequence of the constant region of Kappa chain.

The heavy chain constant region (CH) of an antibody of an animal other than rat may have the amino acid sequence of the constant region of an immunoglobulin equivalent to human IgG4. Immunoglobulin heavy chain is classified into γ chain, µ chain, α chain, δ chain and ε chain depending on the difference in constant region. According to the type of heavy chain present, five classes (isotypes) of immunoglobulin are formed; they are IgG, IgM, IgA, IgD and IgE.

Immunoglobulin G (IgG) accounts for 70-75% of human immunoglobulins and is the most abundantly found monomeric antibody in plasma. IgG has a four-chain structure consisting of two light chains and two heavy chains. Human IgG1, IgG2 and IgG4 have molecular weights of about 146,000, whereas human IgG3 has a long hinge region that connects Fab region and Fc region and has a larger molecular weight of 170,000. Human IgG1 accounts for about 65%, human IgG2 about 25%, human IgG3 about 7%, and human IgG4 about 3% of human IgG. They are uniformly distributed inside and outside of blood vessels. Having a strong affinity for Fc receptors and complement factors on effector cell surfaces, human IgG1 induces antibody-dependent cell cytotoxicity (ADCC) and also activates complements to induce complement-dependent cell cytotoxicity (CDC). Human IgG2 and IgG4 are low at ADCC and CDC activities because their affinity for Fc receptors and complement factors is low.

Immunoglobulin M (IgM), which accounts for about 10% of human immunoglobulins, is a pentameric antibody consisting of five basic four-chain structures joined together. It has a molecular weight of 970,000. Usually occurring only in blood, IgM is produced against infectious microorganisms and takes charge of early stage immunity.

Immunoglobulin A (IgA) accounts for 10-15% of human immunoglobulins. It has a molecular weight of 160,000. Secreted IgA is a dimeric antibody consisting of two IgA molecules joined together. IgA1 is found in serum, nasal discharge, saliva and breast milk. In intestinal juice, IgA2 is found abundantly.

Immunoglobulin D (IgD) is a monomeric antibody accounting for no more than 1% of human immunoglobulins. IgD is found on B cell surfaces and involved in induction of antibody production.

Immunoglobulin E (IgE) is a monomeric antibody that occurs in an extremely small amount, accounting for only 0.001% or less of human immunoglobulins. Immunoglobulin E is considered to be involved in immune response to parasites but in advanced countries where parasites are rare, IgE is largely involved in bronchial asthma and allergy among other things.

With respect to canine, sequences of IgG-A (equivalent to human IgG2), IgG-B (equivalent to human IgG1), IgG-C (equivalent to human IgG3) and IgG-D (equivalent to human IgG4) have been identified as the heavy chain of IgG. In the above-described anti-PD-L1 antibody, an IgG's heavy chain constant region with neither ADCC activity nor CDC activity is preferable (IgG4 in human). In the case where the constant region of an immunoglobulin equivalent to human IgG4 has not been identified, one may use a constant region that has lost both ADCC activity and CDC activity as a result of introducing mutations into the relevant region of an immunoglobulin equivalent to human IgG1.

In bovine, the constant region of an immunoglobulin equivalent to human IgG4 has not been identified, so mutations may be added at the relevant region of an immunoglobulin equivalent to human IgG1 and the resultant constant region then used. As one example, the amino acid sequence of the CH of a bovine antibody (IgG1 chain, GenBank: X62916) having mutations introduced into CH2 domain and a nucleotide sequence for such amino acid sequence (after codon optimization) are shown in SEQ ID NOS: 102 and 103, respectively.

When an animal other than rat is canine or bovine, an anti-PD-Ll antibody is more preferable in which (i) the CL of a canine or bovine antibody has the amino acid sequence of the constant region of Lambda chain and (ii) the CH of the canine or bovine antibody has the amino acid sequence of the constant region of an immunoglobulin equivalent to human IgG4.

The above-described anti-PD-Ll antibody encompasses rat-canine chimeric antibodies, caninized antibodies, rat-bovine chimeric antibodies and bovinized antibodies. However, animals are not limited to canine and bovine and may be exemplified by human, porcine, simian, mouse, feline, equine, goat, sheep, water buffalo, rabbit, hamster, guinea pig, bovine and the like.

For example, the anti-PD-Ll antibody described above may be an anti-PD-Ll antibody in which the CL of a canine antibody has the amino acid sequence as shown in SEQ ID NO: 3 and the CH of the canine antibody has the amino acid sequence as shown in SEQ ID NO: 4; or an anti-PD-Ll antibody in which the CL of a bovine antibody has the amino acid sequence as shown in SEQ ID NO: 100 and the CH of the bovine antibody has the amino acid sequence as shown in SEQ ID NO: 102.

The amino acid sequences as shown in SEQ ID NOS: 3 and 4 as well as SEQ ID NOS: 100 and 102 may have deletion(s), substitution(s) or addition(s) of one or several (e.g., up to five, about 10 at the most) amino acids. Even when such mutations have been introduced, the resulting amino acid sequences are capable of having the function as CL or CH of the PD-L1 antibody.

The above-described anti-PD-Ll antibody may have a four-chain structure comprising two light chains and two heavy chains.

The above-described anti-PD-Ll antibody may be prepared as described below. Briefly, an artificial gene is synthesized which comprises (i) the identified variable region sequences of a rat anti-bovine PD-L1 antibody and (ii) the constant region sequences of an antibody of an animal other than rat (e.g., canine or bovine) (preferably, human IgG4 antibody or antibody equivalent to human IgG4 antibody). The resultant gene is inserted into a vector (e.g., plasmid), which is then introduced into a host cell (e.g., mammal cell such as CHO cell). The host cell is cultured, and the antibody of interest is collected from the resultant culture.

The amino acid sequence and the nucleotide sequence of the VL of the rat anti-bovine PD-L1 antibody identified by the present inventors are shown in SEQ ID NOS: 1 and 5, respectively. Further, nucleotide sequences after codon optimization are shown in SEQ ID NOS: 15 and 112.

The amino acid sequence and the nucleotide sequence of the VH of the rat anti-bovine PD-L1 antibody identified by the present inventors are shown in SEQ ID NOS: 2 and 6, respectively. Further, nucleotide sequences after codon optimization are shown in SEQ ID NOS: 16 and 113.

The amino acid sequence and the nucleotide sequence of the CL (Lambda chain, GenBank: E02824.1) of a canine antibody are shown in SEQ ID NOS: 3 and 7, respectively. Further, the nucleotide sequence after codon optimization is shown in SEQ ID NO: 17.

The amino acid sequence and the nucleotide sequence of the CH (IgG-D chain, GenBank: AF354267.1) of the canine antibody are shown in SEQ ID NOS: 4 and 8, respectively. Further, the nucleotide sequence after codon optimization is shown in SEQ ID NO: 18.

Further, SEQ ID NO: 9 shows the amino acid sequence of a chimeric light chain comprising the VL of the rat anti-bovine PD-L1 antibody and the CL (Lambda chain, GenBank: E02824.1) of the canine antibody. The nucleotide sequence (after codon optimization) of the chimeric light chain comprising the VL of the rat anti-bovine PD-L1 antibody and the CL (Lambda chain, GenBank: E02824.1) of the canine antibody is shown in SEQ ID NO: 19.

SEQ ID NO: 10 shows the amino acid sequence of a chimeric heavy chain comprising the VH of the rat anti-bovine PD-L1 antibody and the CH (IgG-D chain, GenBank: AF354267.1) of the canine antibody. The nucleotide sequence (after codon optimization) of the chimeric heavy chain comprising the VH of the rat anti-bovine PD-L1 antibody and the CH (IgG-D chain, GenBank: AF354267.1) of the canine antibody is shown in SEQ ID NO: 20.

The amino acid sequence and the nucleotide sequence of the CL (Lambda chain, GenBank: X62917) of a bovine antibody are shown in SEQ ID NOS: 100 and 101, respectively. Further, the nucleotide sequence after codon optimization is shown in SEQ ID NO: 114.

The amino acid sequence and the nucleotide sequence (after codon optimization) of the CH (IgG1 chain, modified from GenBank: X62916) of the bovine antibody are shown in SEQ ID NOS: 102 and 103, respectively.

Further, SEQ ID NO: 115 shows the amino acid sequence of a chimeric light chain comprising the VL of the rat anti-bovine PD-L1 antibody and the CL (Lambda chain, GenBank: X62917) of the bovine antibody. The nucleotide sequence (after codon optimization) of the chimeric light chain comprising the VL of the rat anti-bovine PD-L1 antibody and the CL (Lambda chain, GenBank: X62917) of the bovine antibody is shown in SEQ ID NO: 117.

SEQ ID NO: 116 shows the amino acid sequence of a chimeric heavy chain comprising the VH of the rat anti-bovine PD-L1 antibody and the CH (IgG1 chain, modified from GenBank: X62916) of the bovine antibody. The nucleotide sequence (after codon optimization) of the chimeric heavy chain comprising the VH of the rat anti-bovine PD-L1 antibody and the CH (IgG1 chain, modified from GenBank: X62916) of the bovine antibody is shown in SEQ ID NO: 118.

Amino acid sequences and nucleotide sequences of CLs and CHs for various animals other than rat, canine and bovine may be obtained from known databases for use in the present invention.

Amino acid sequences and nucleotide sequences of CLs and CHs for canine, ovine, porcine, water buffalo, human and bovine are summarized in the table below.

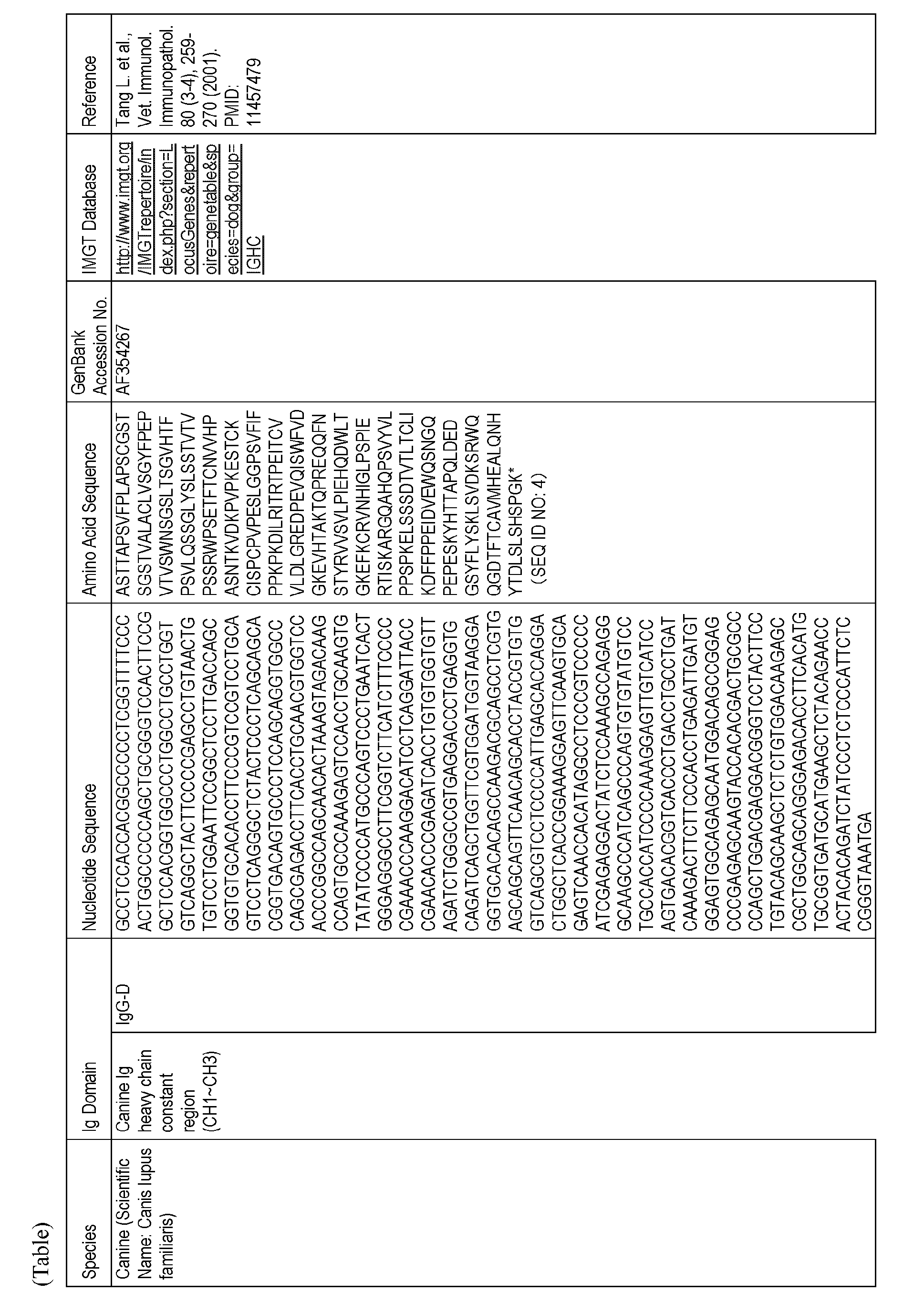

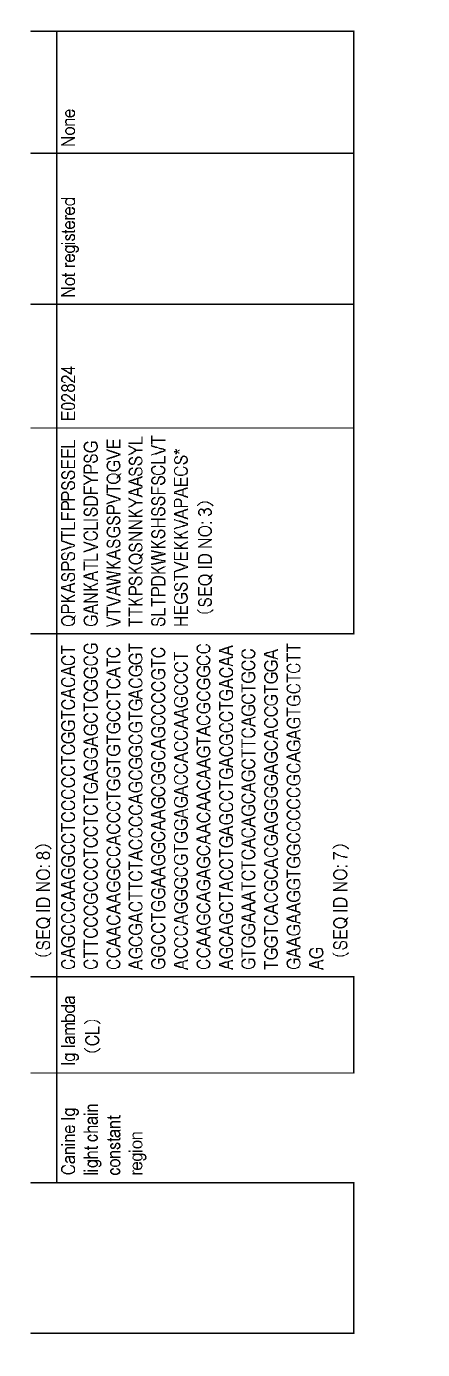

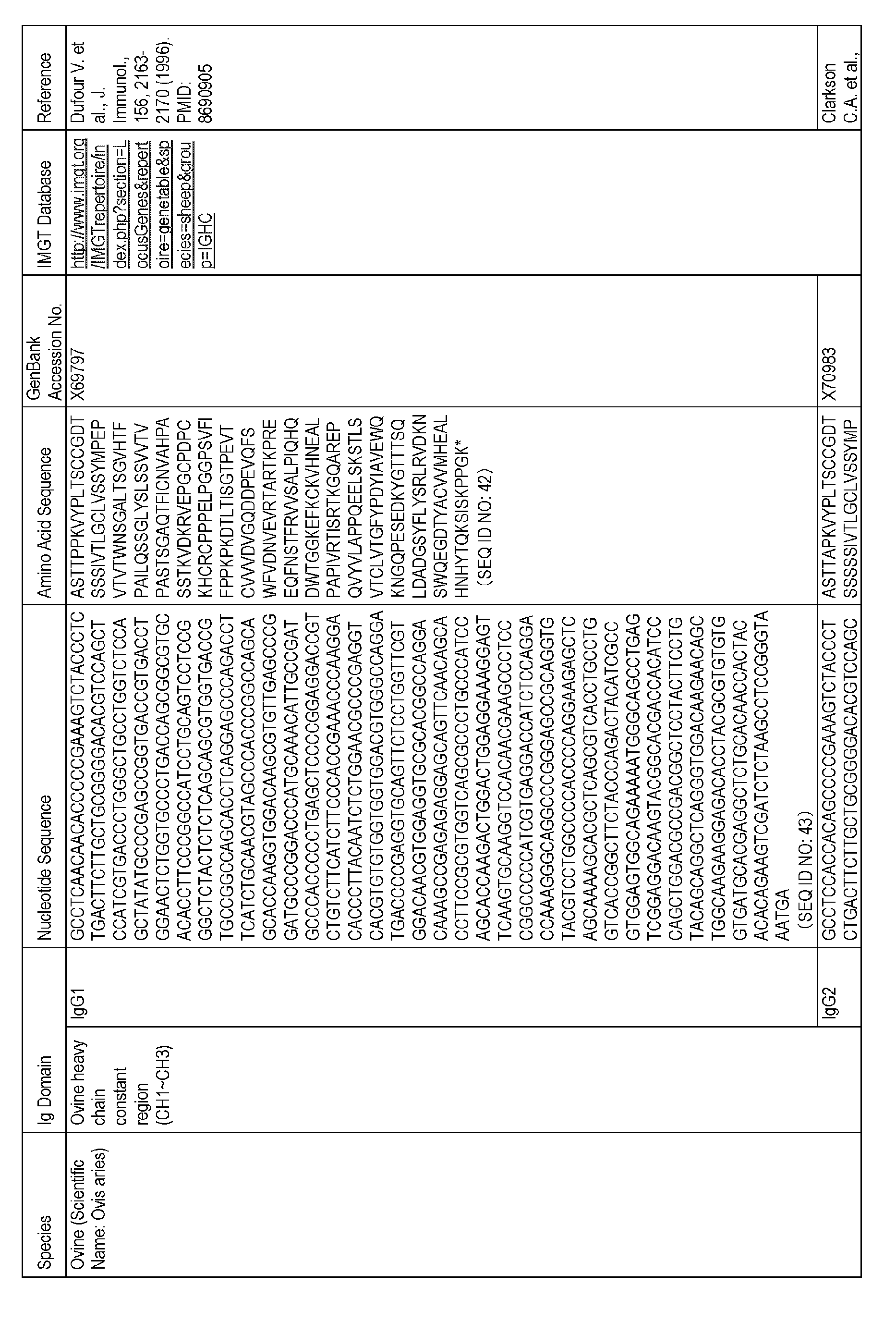

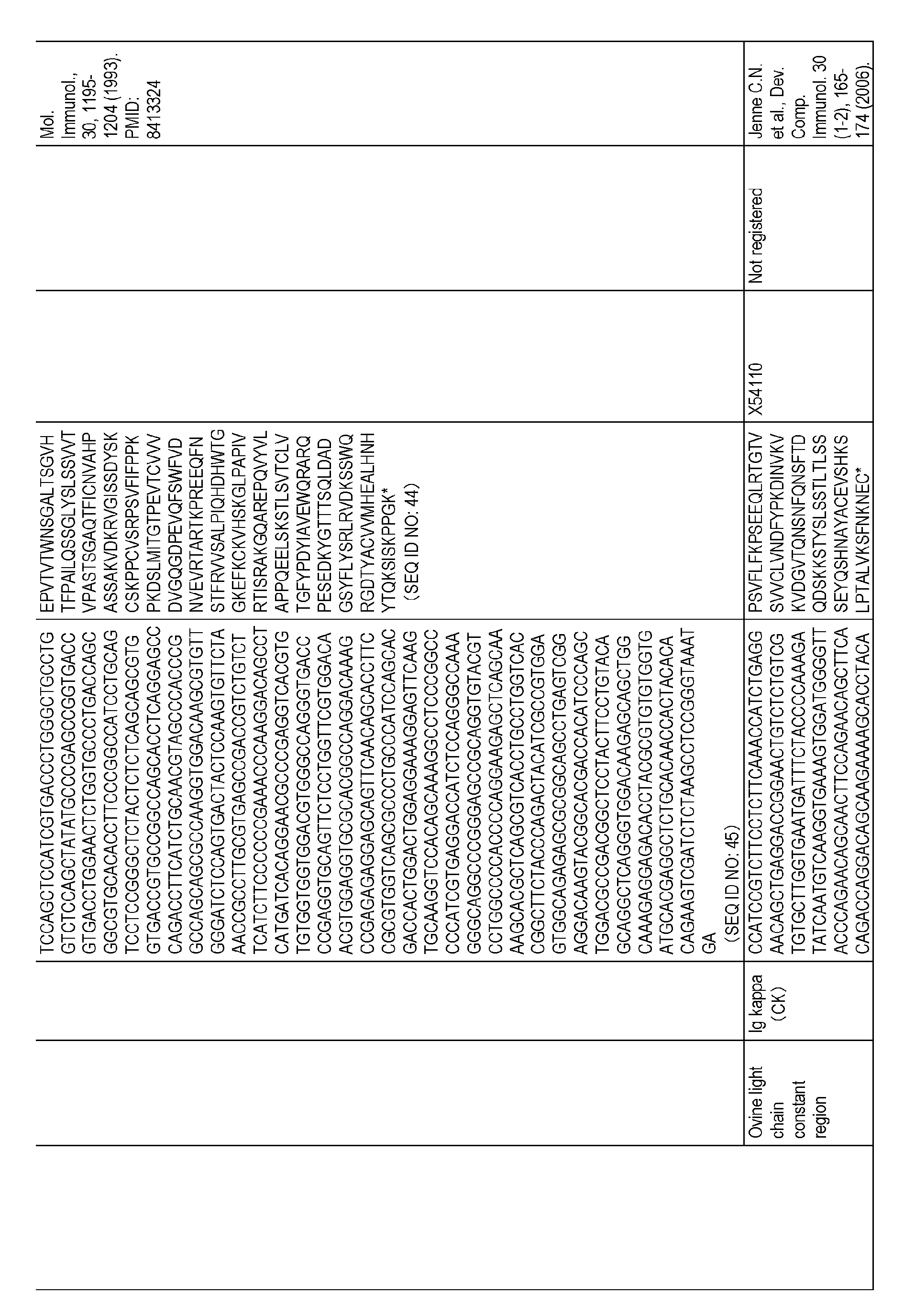

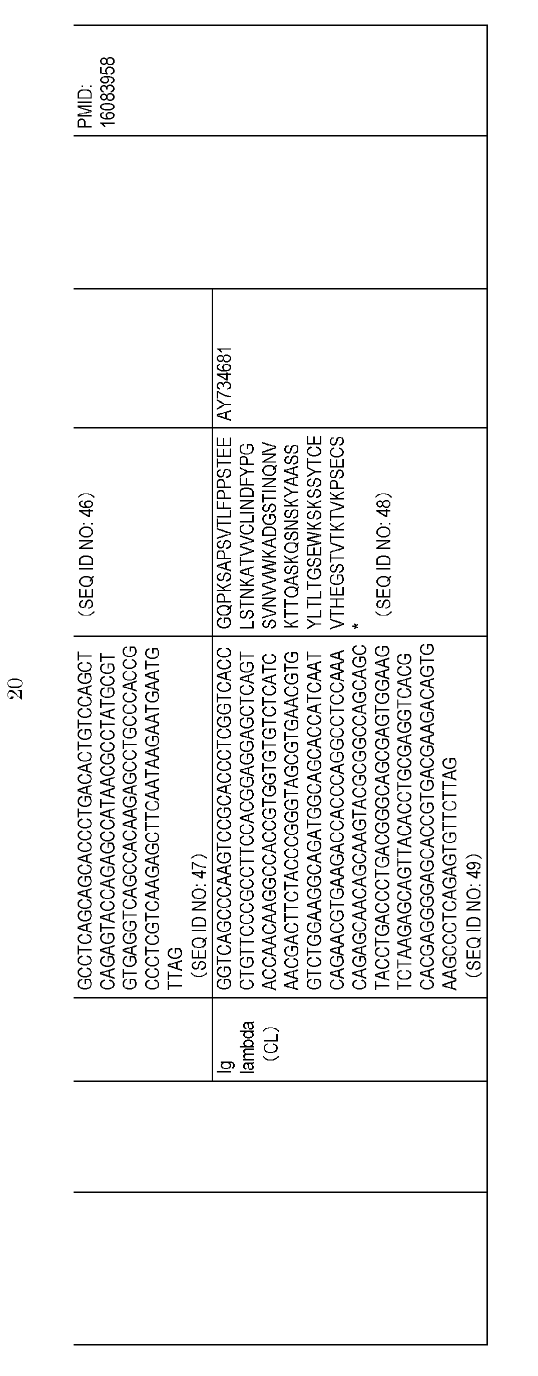

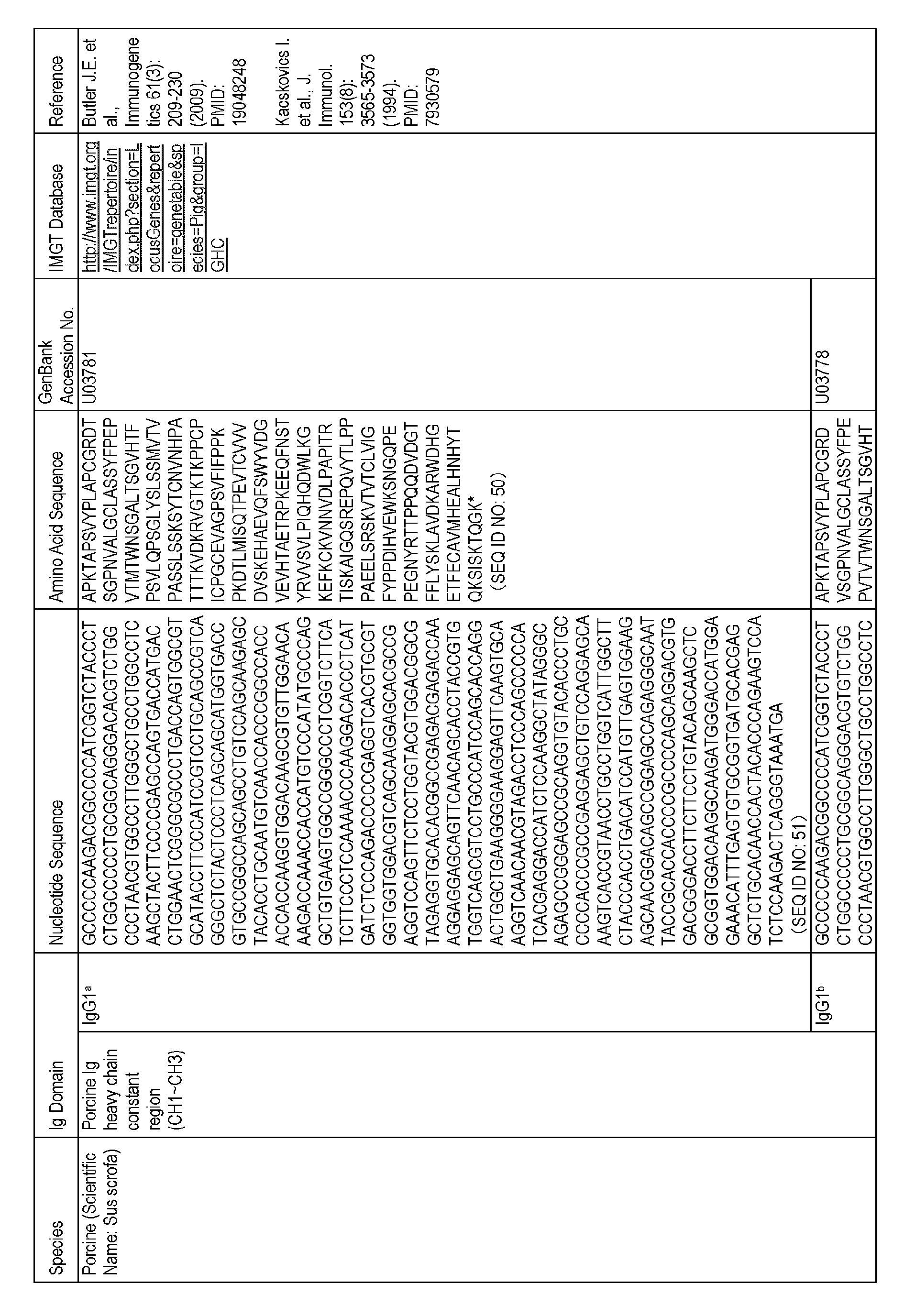

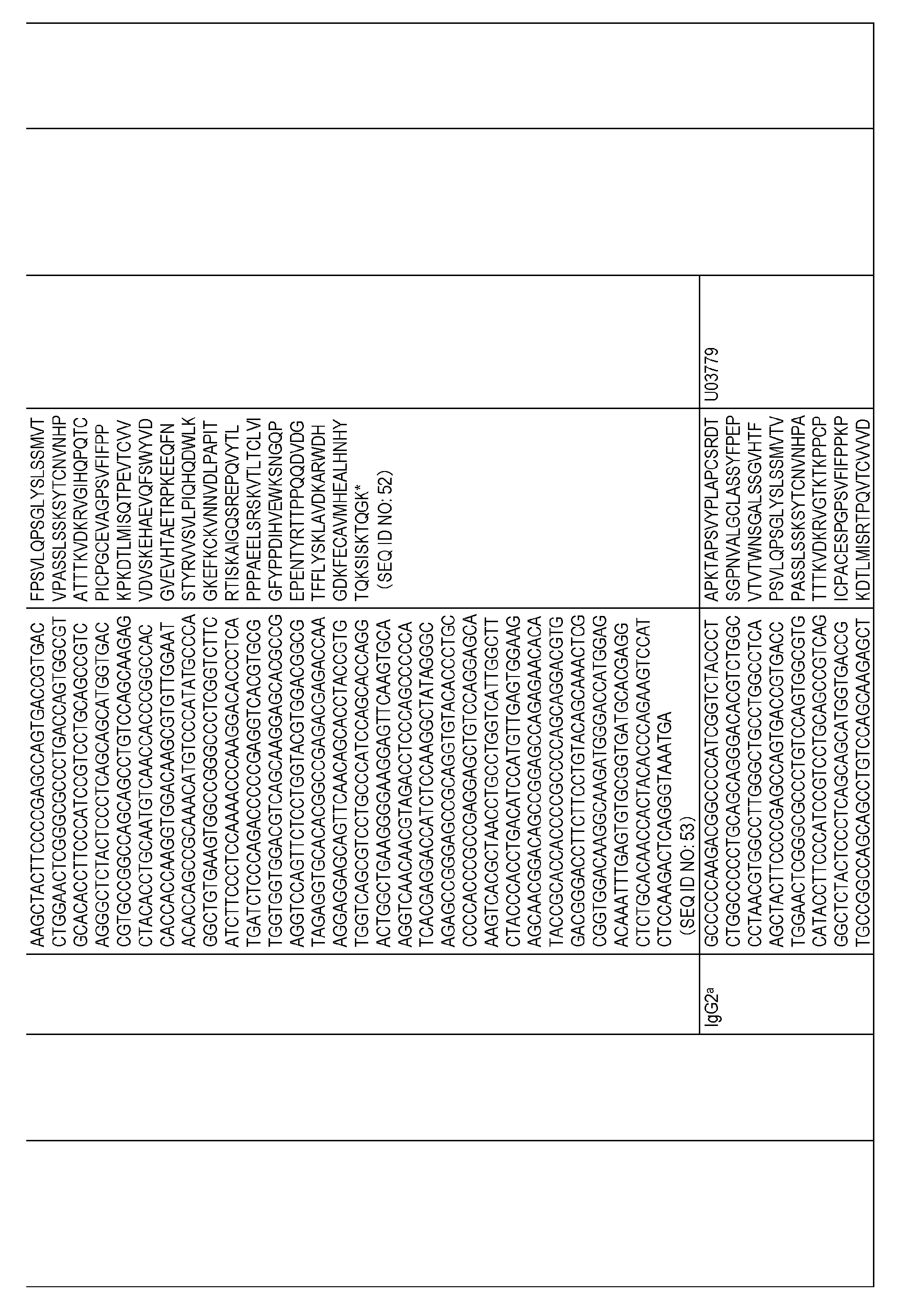

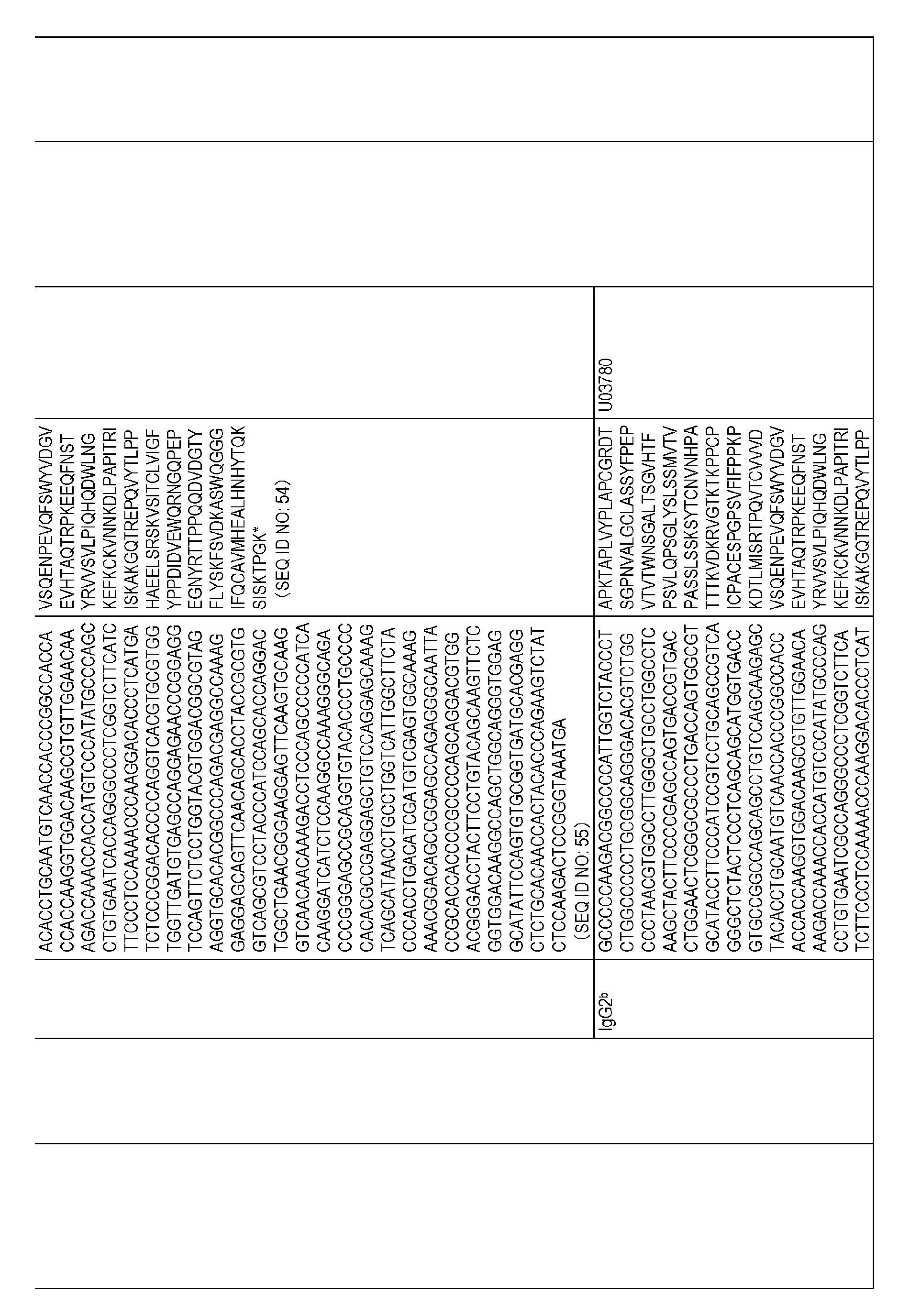

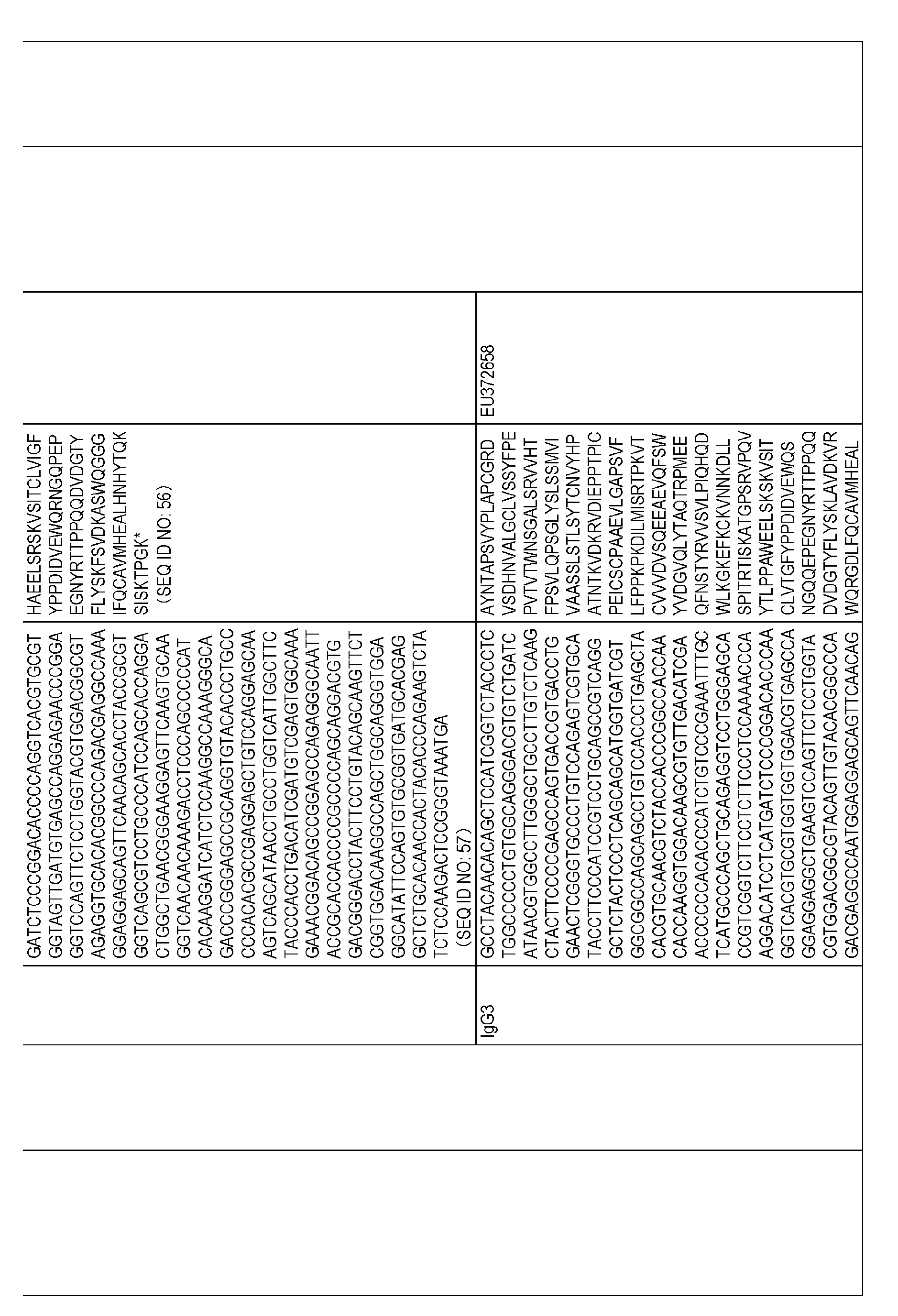

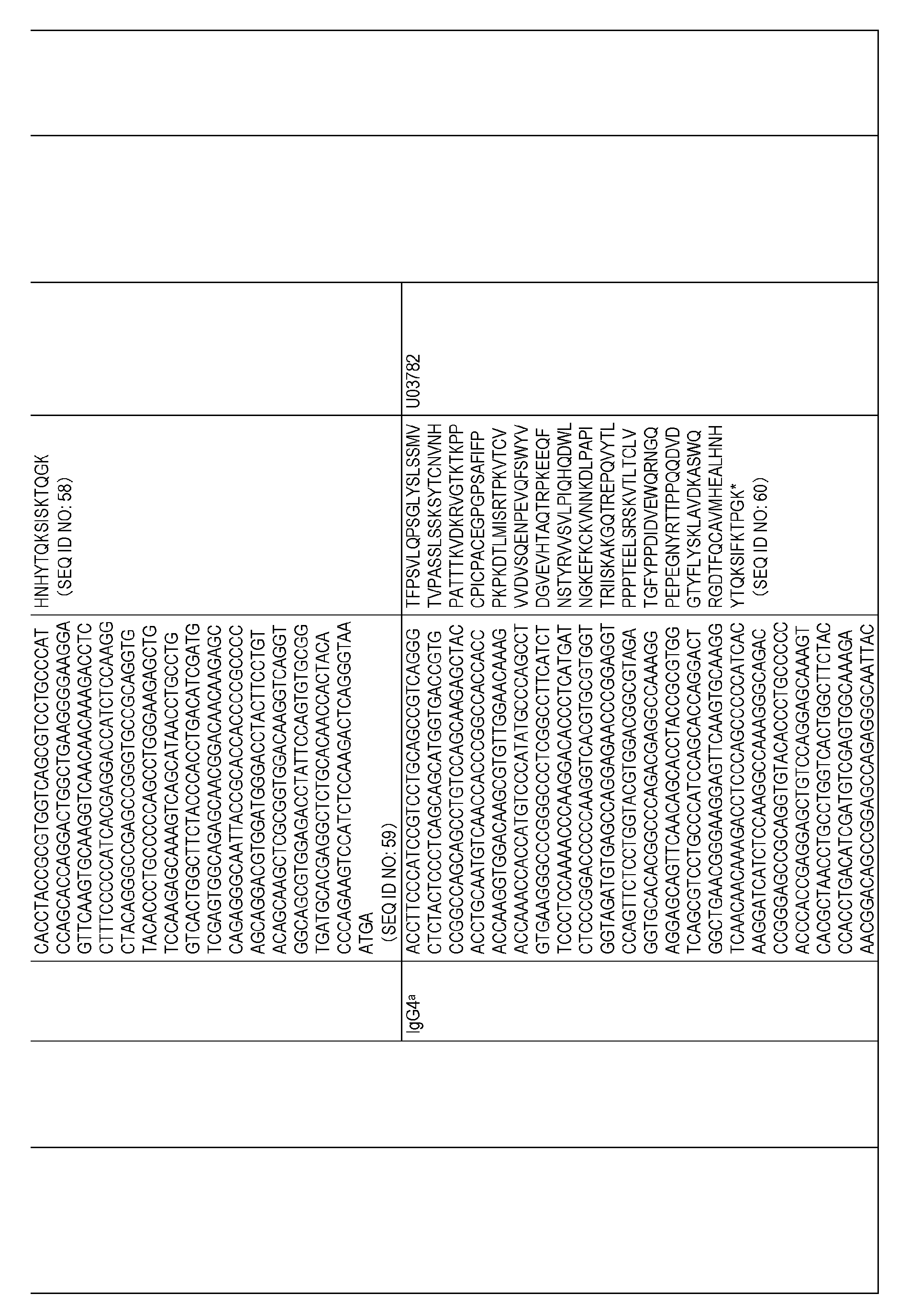

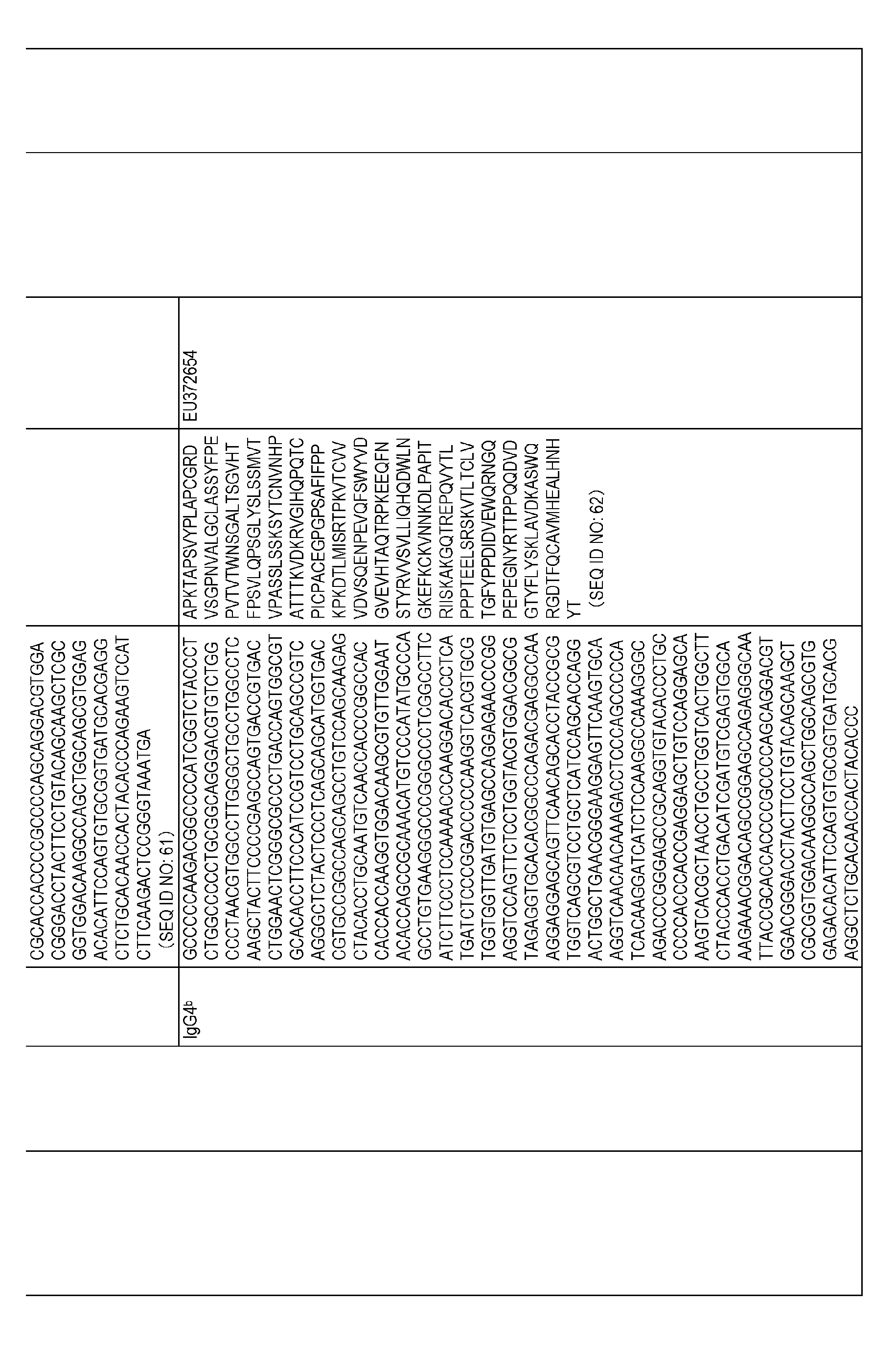

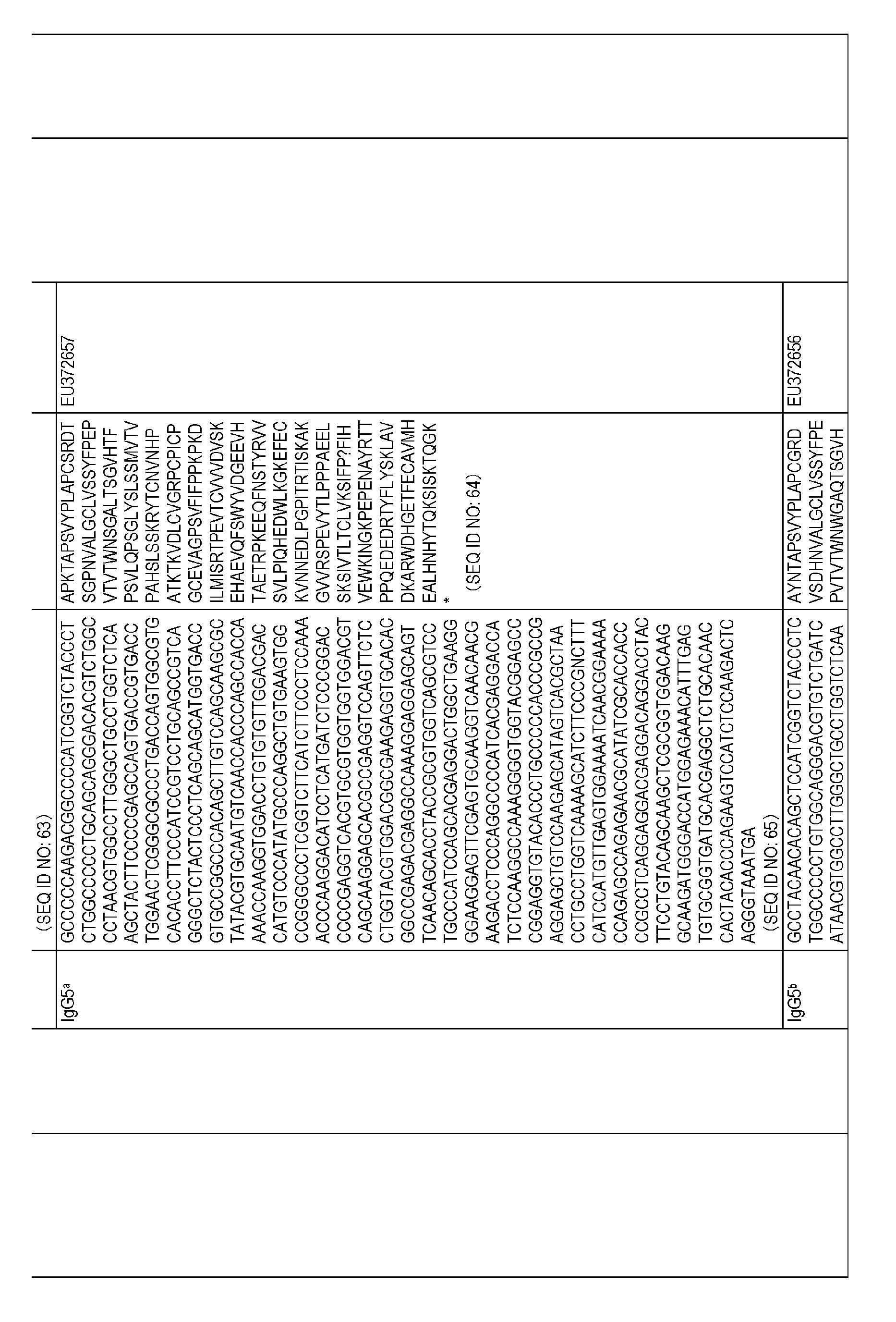

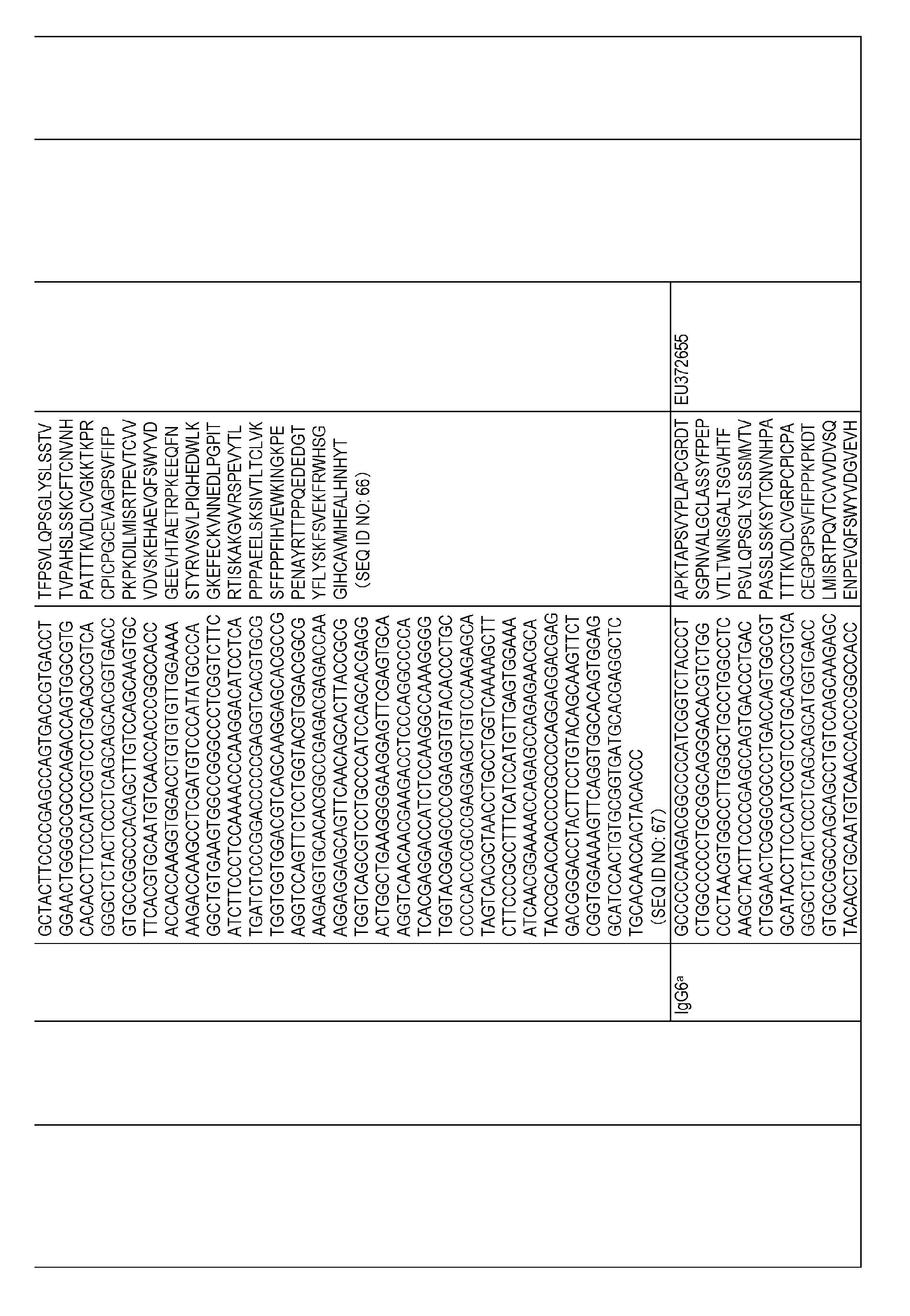

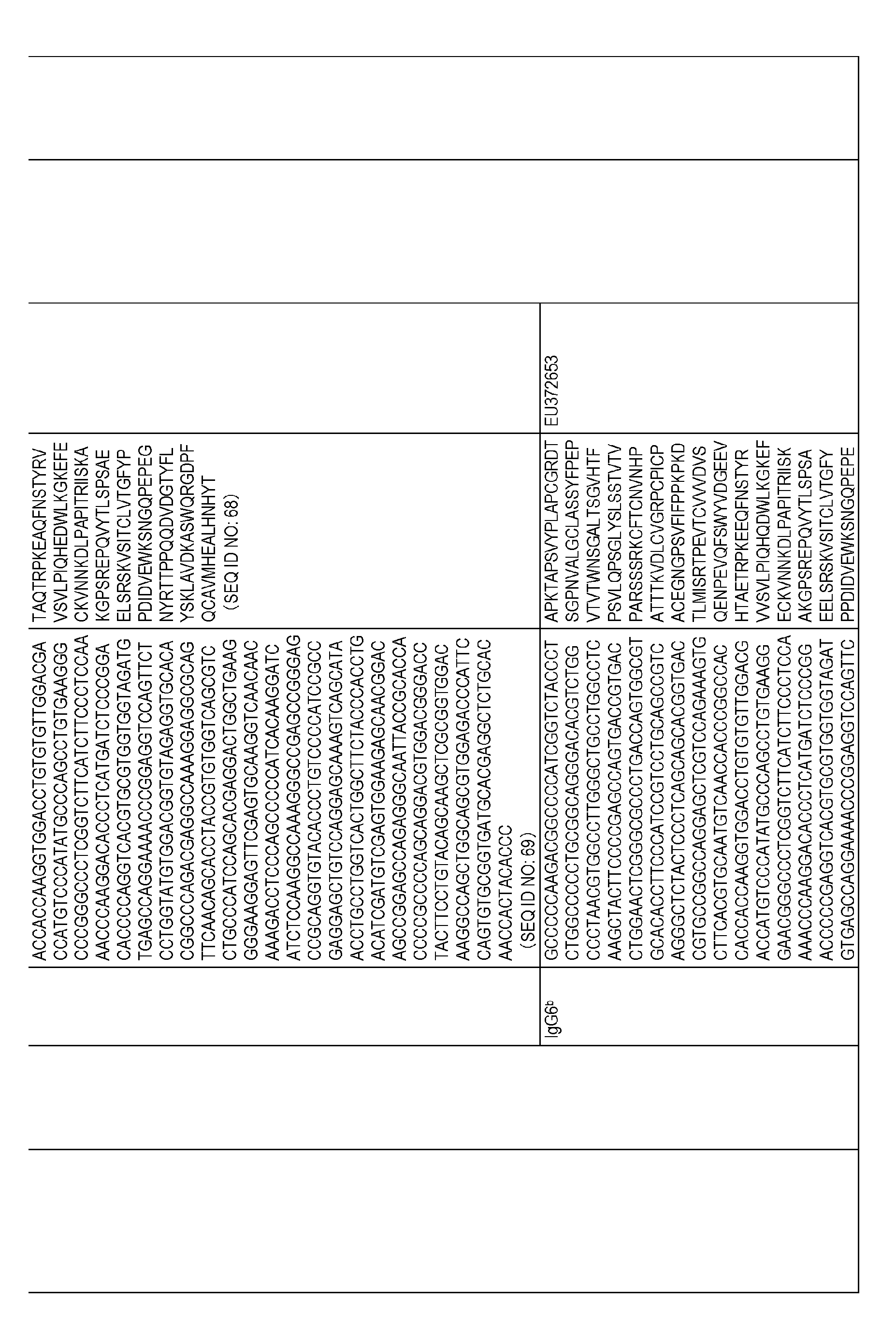

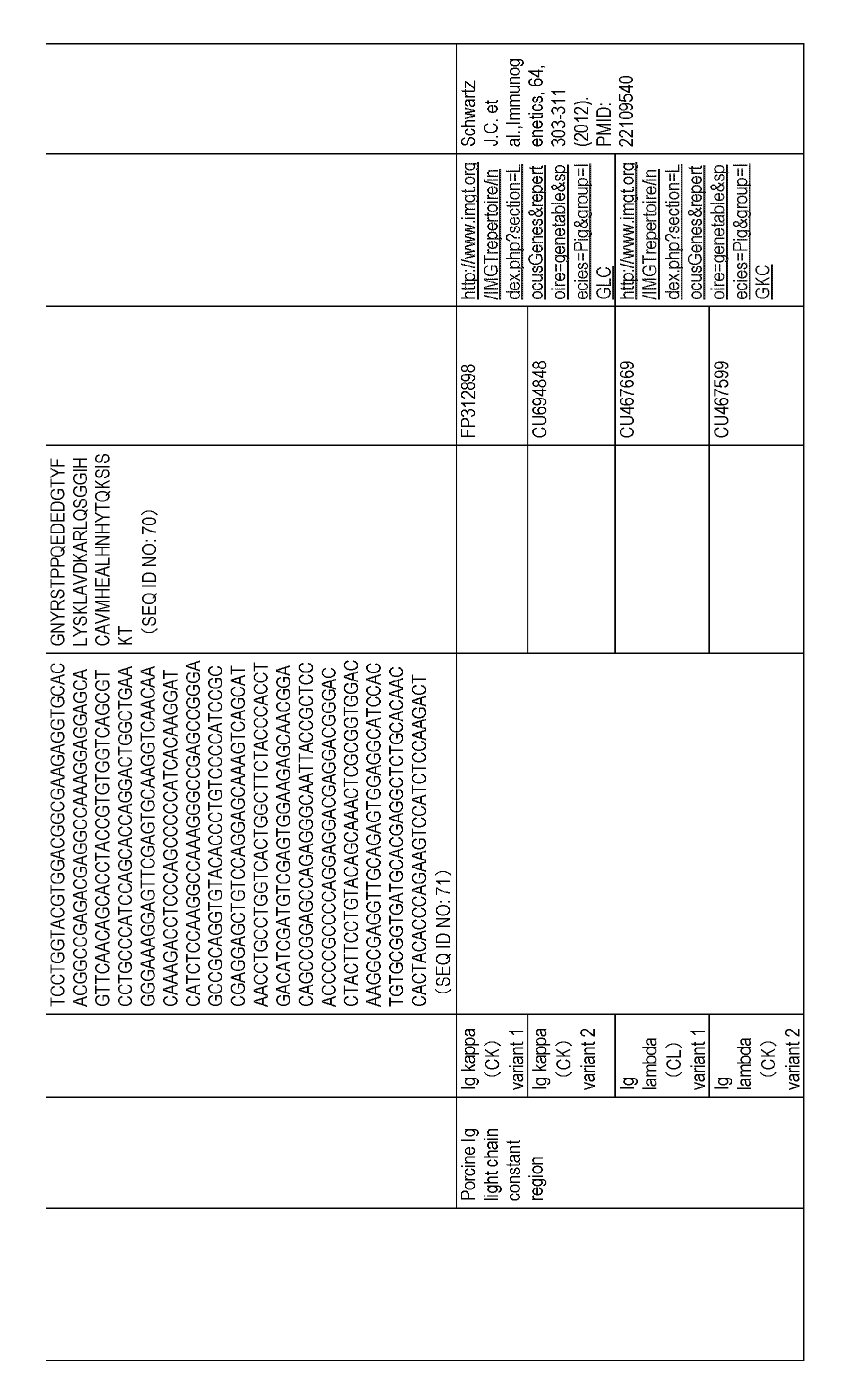

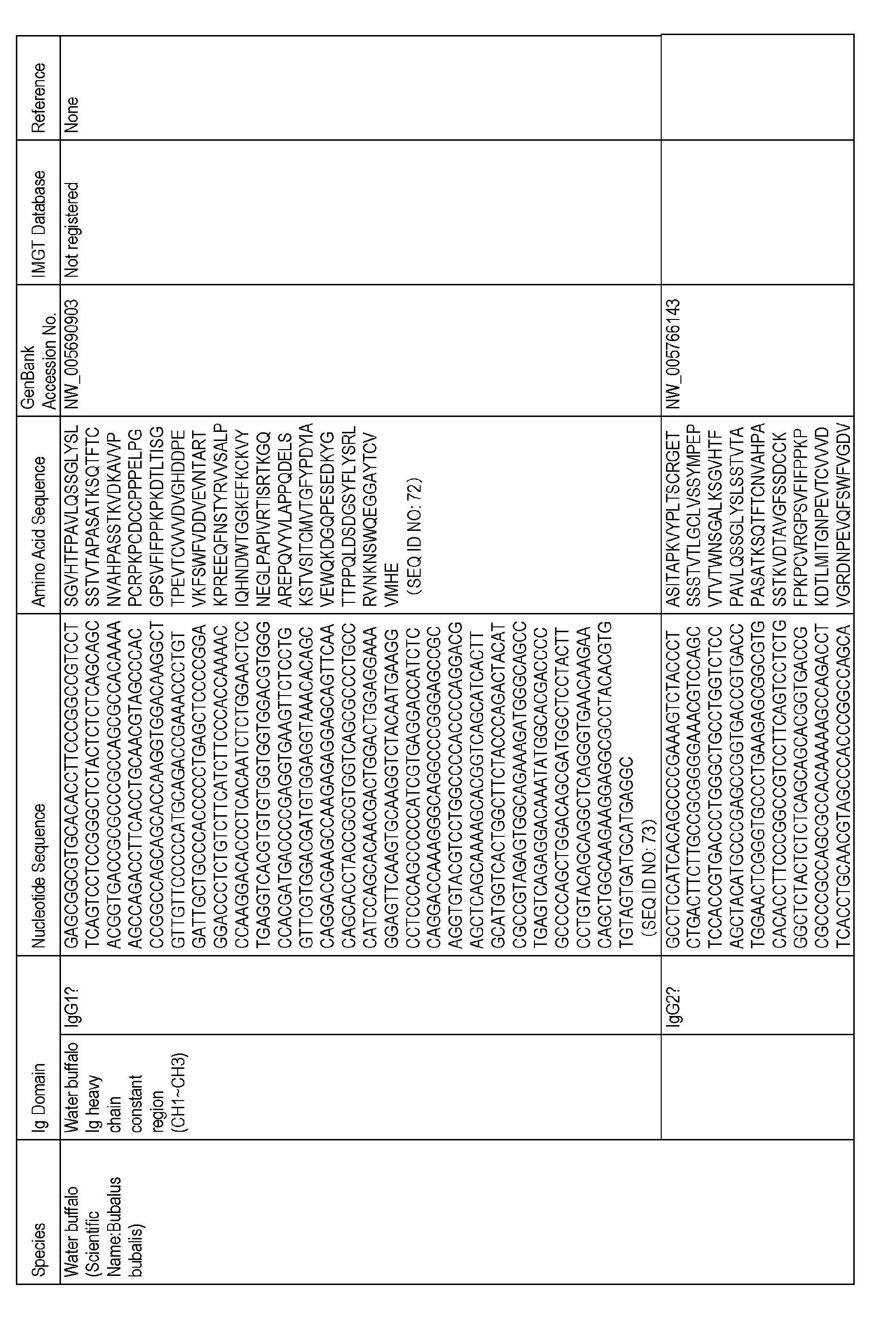

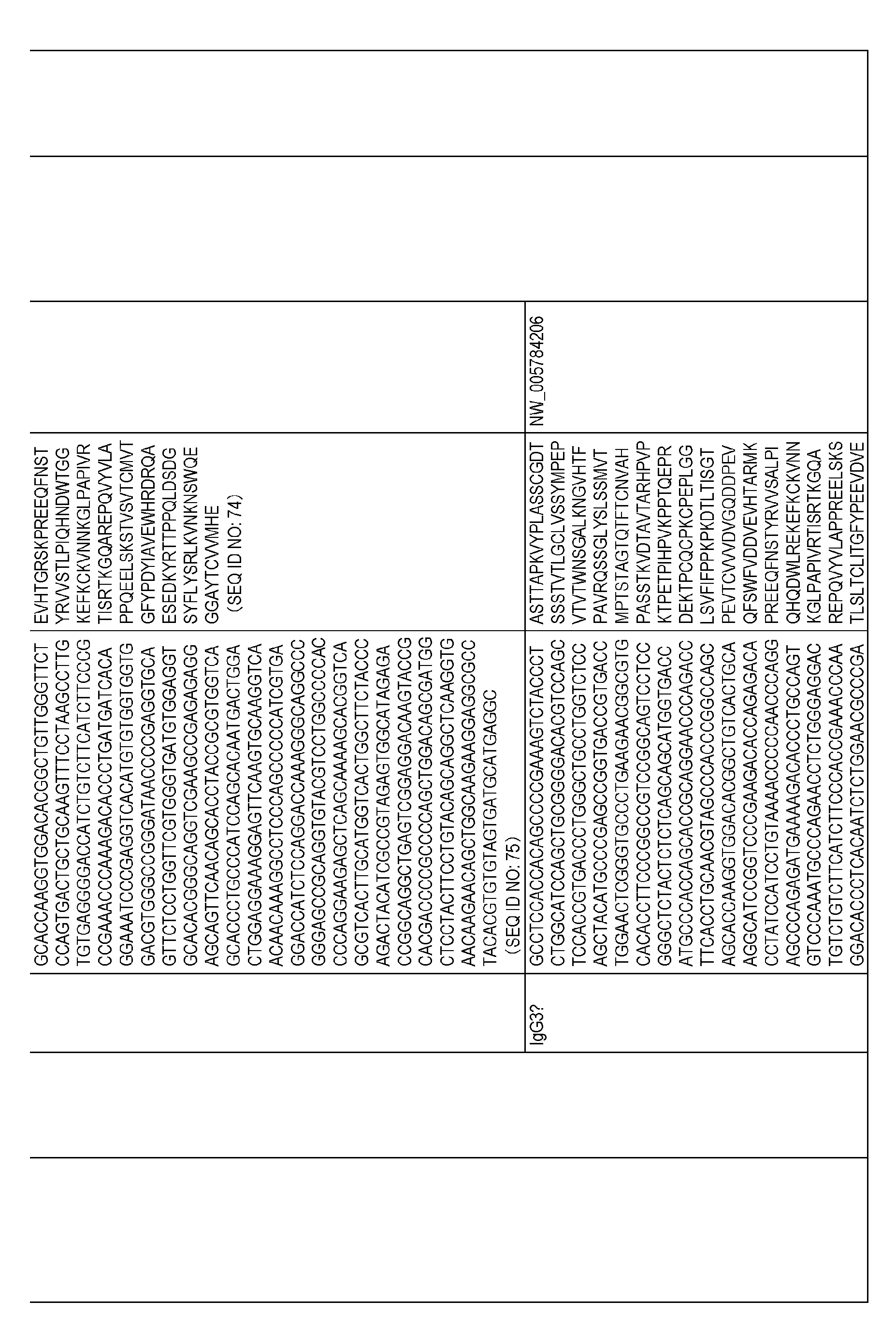

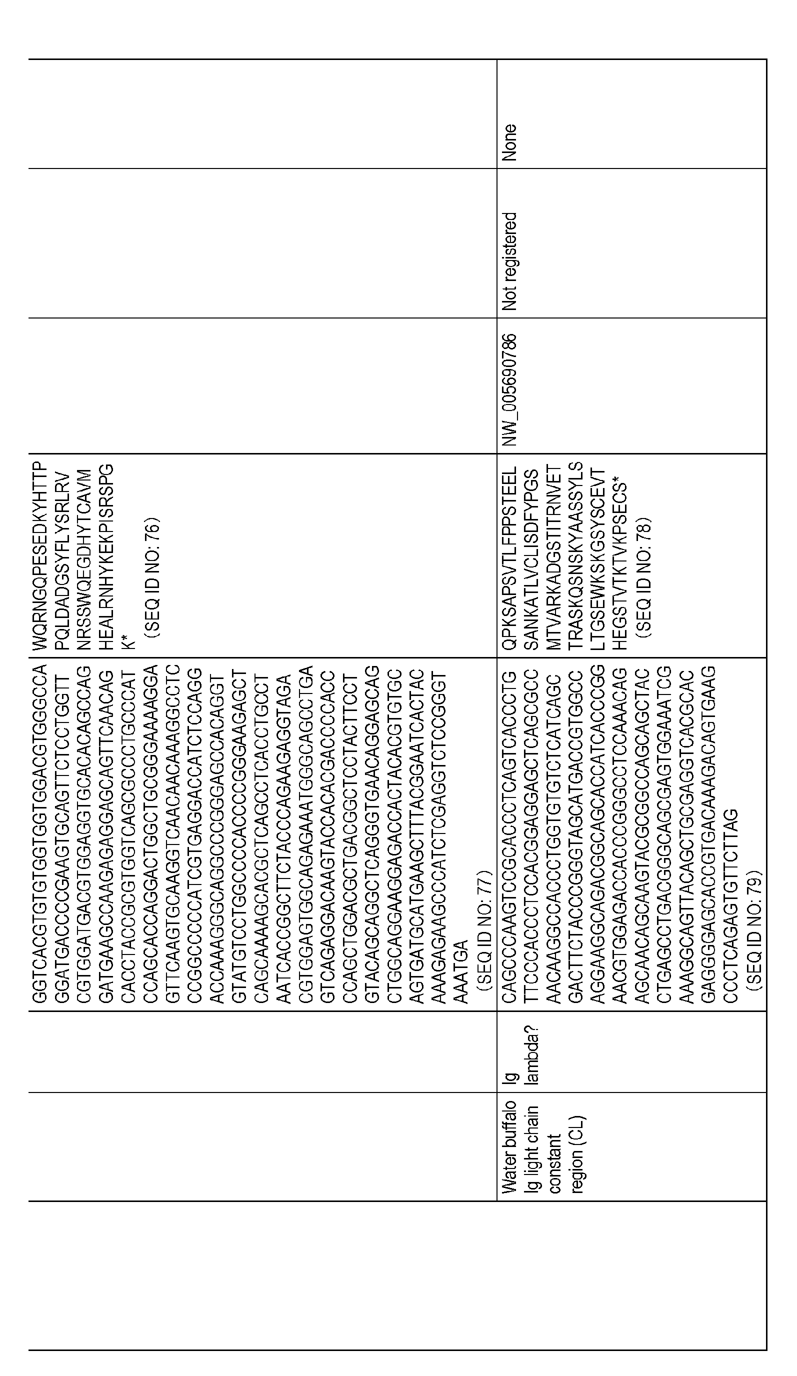

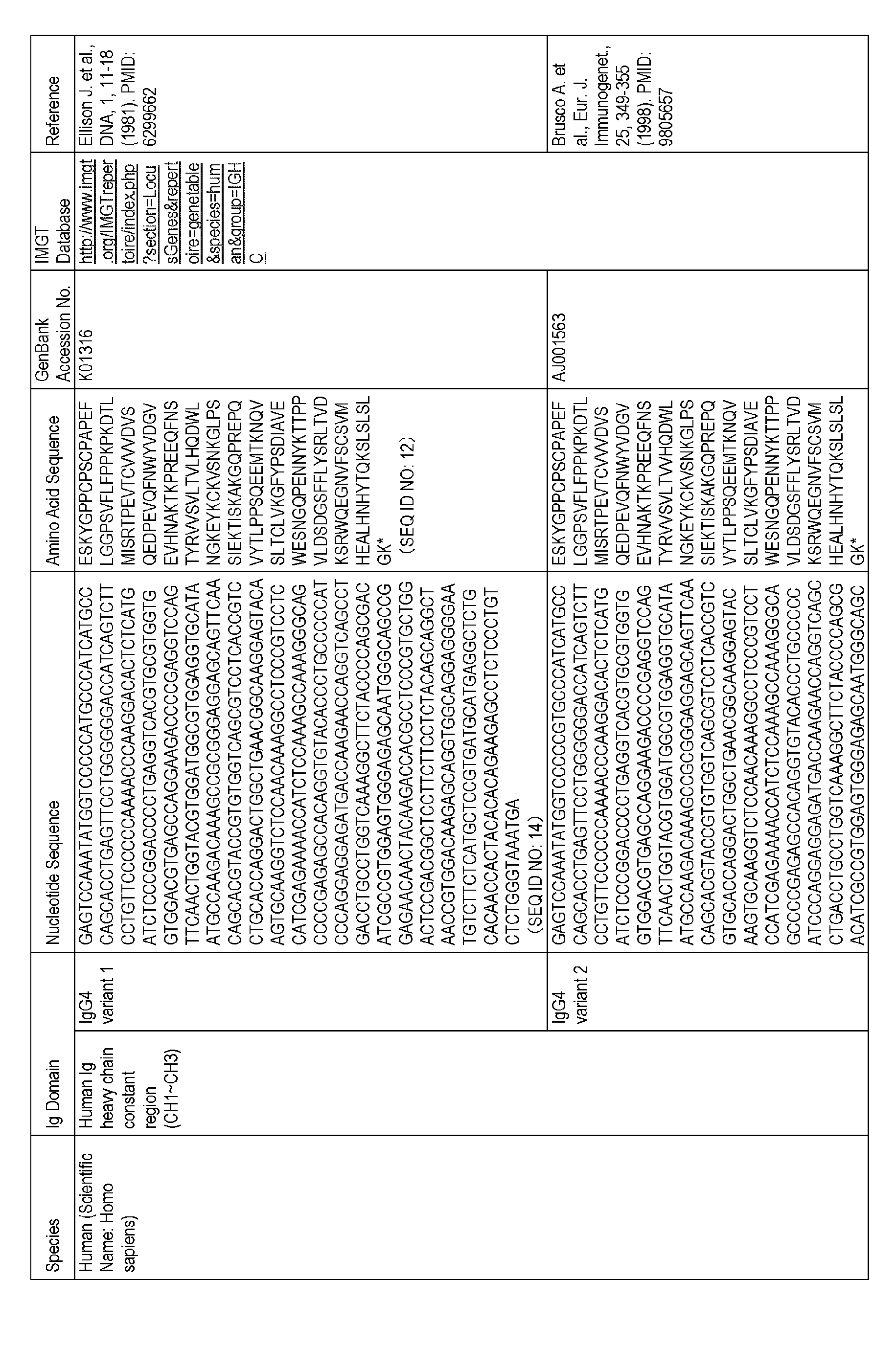

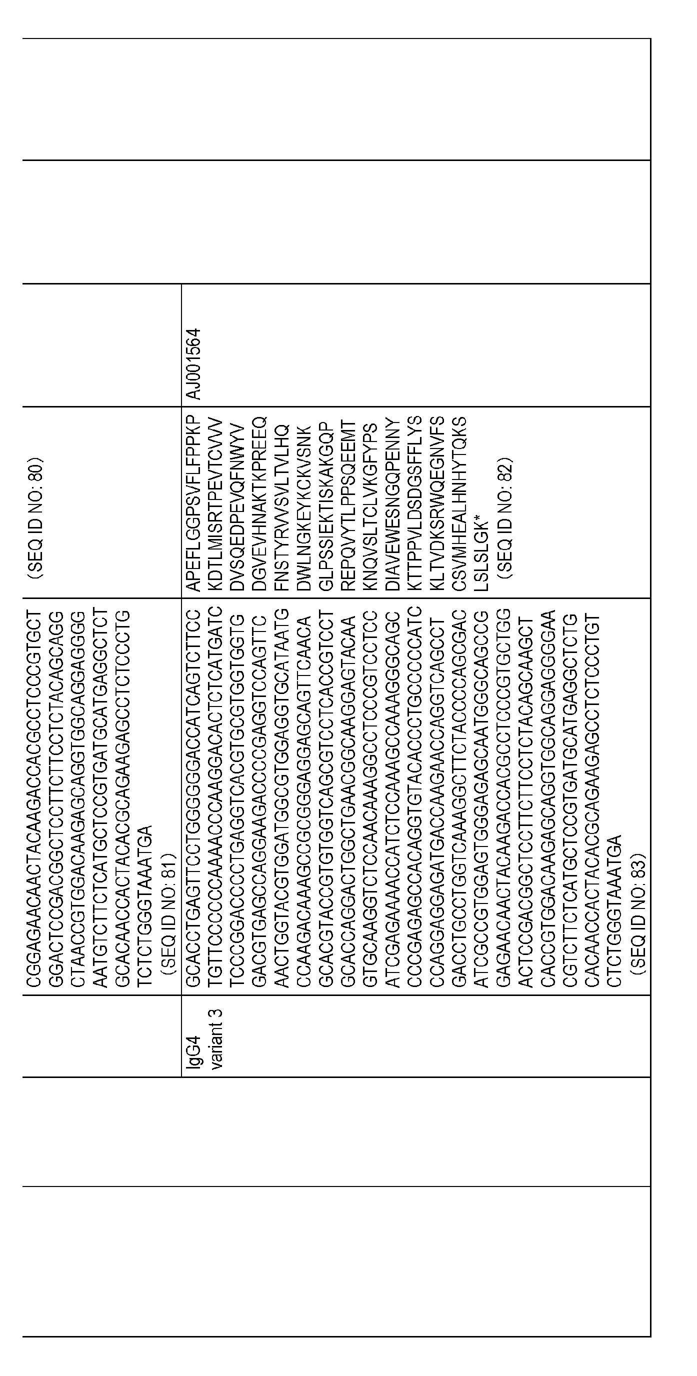

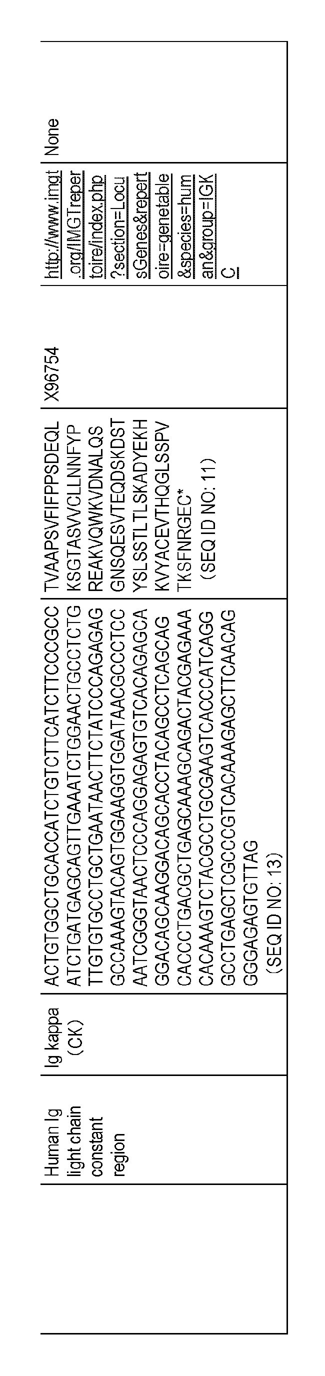

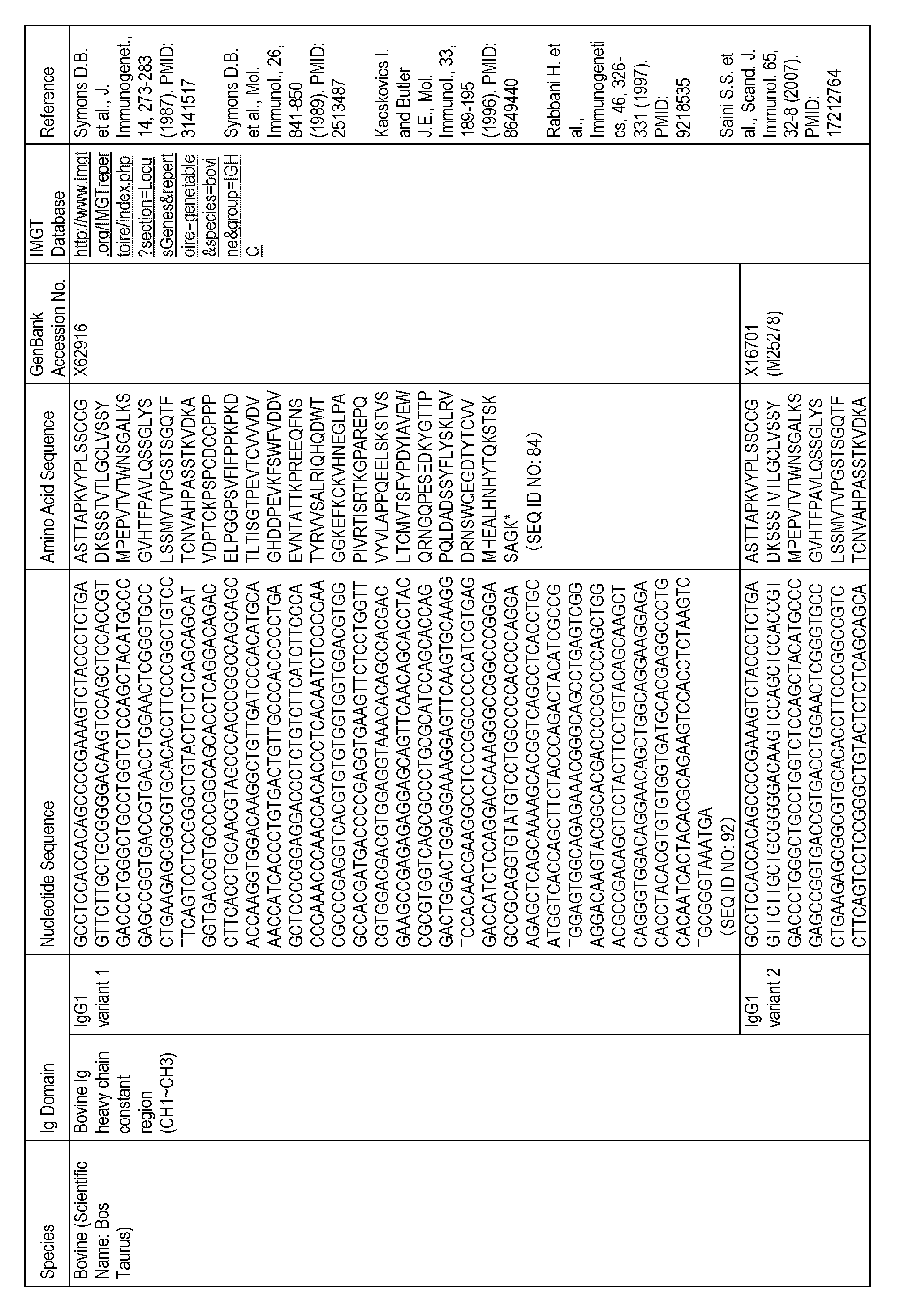

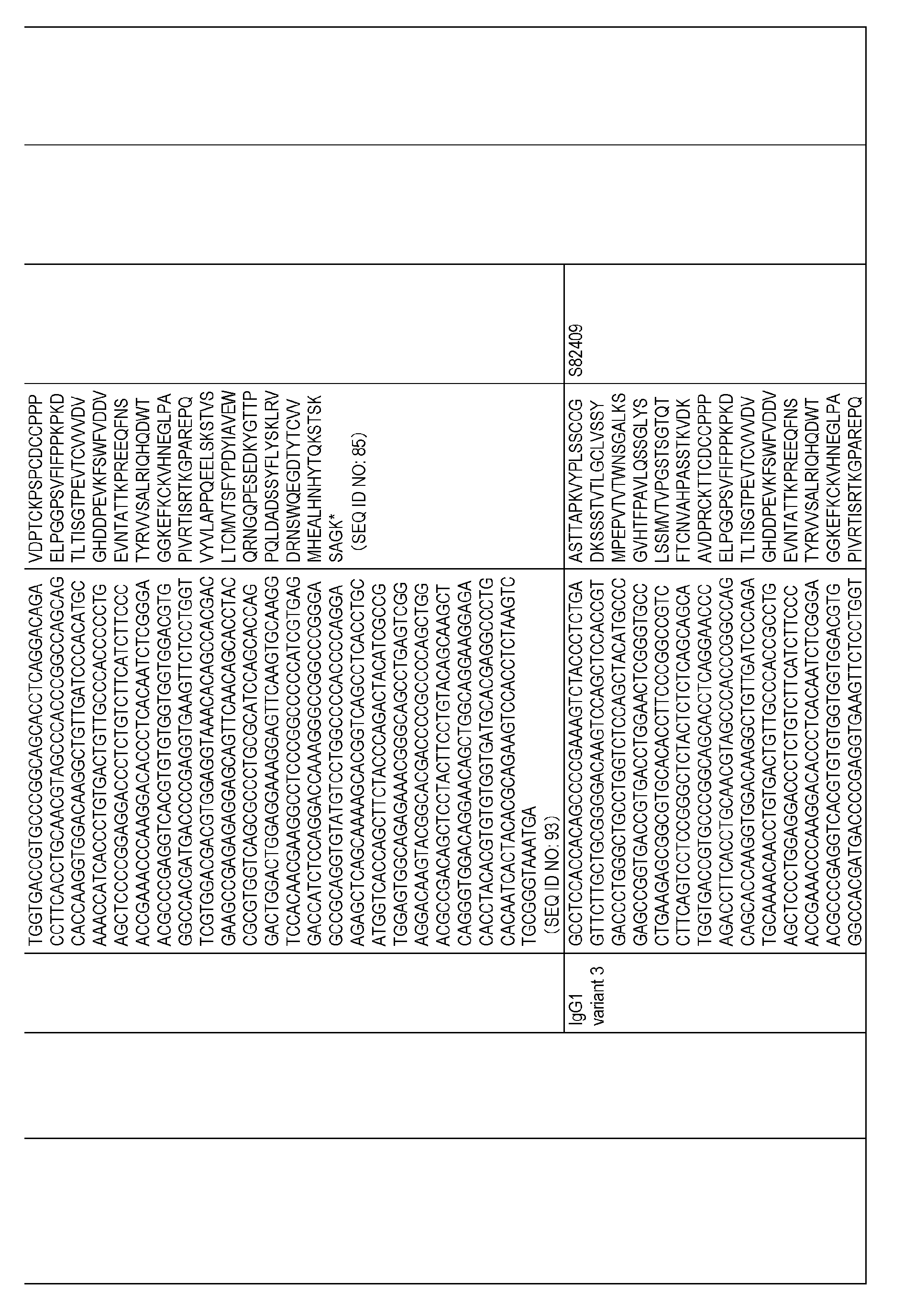

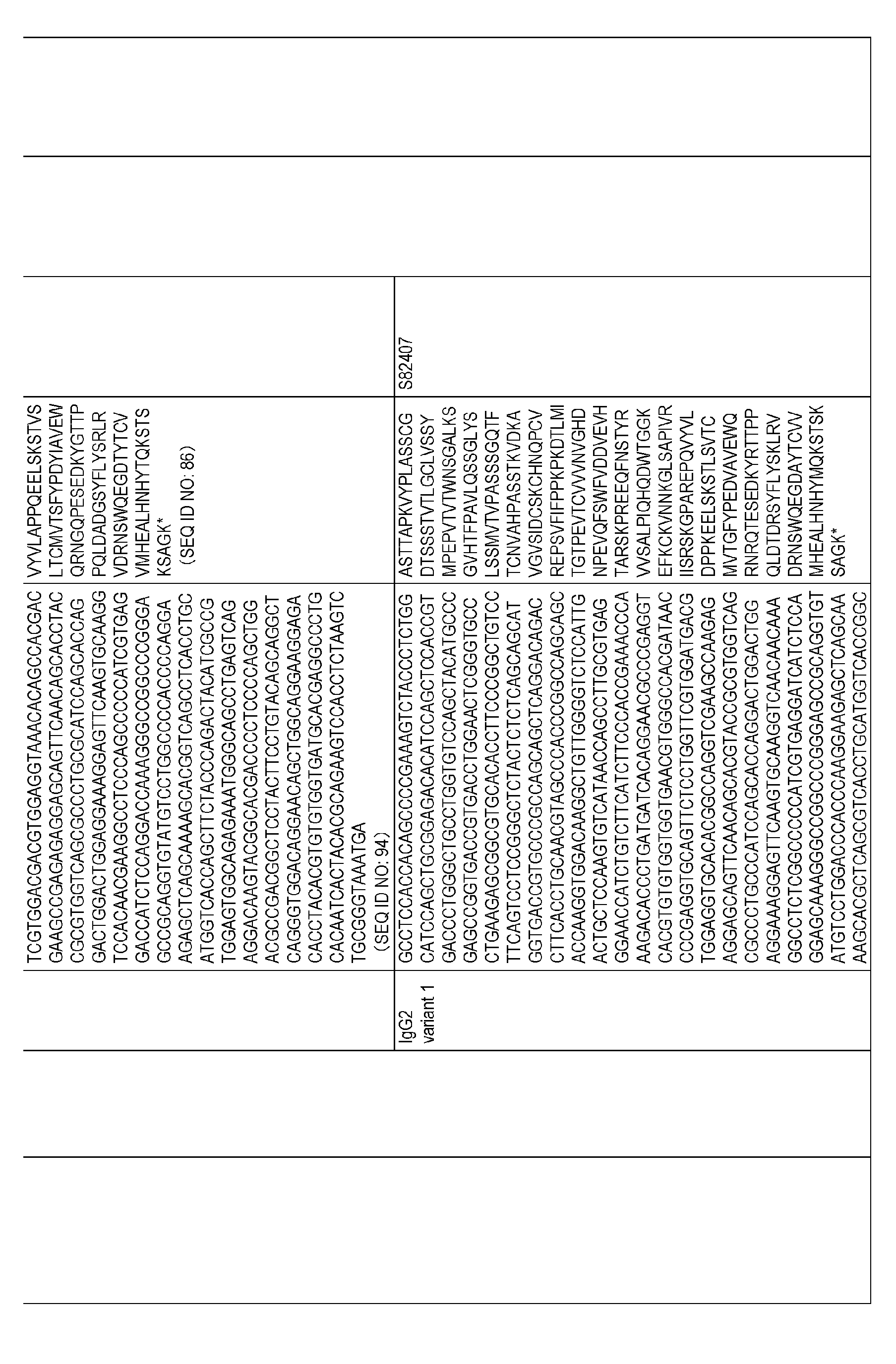

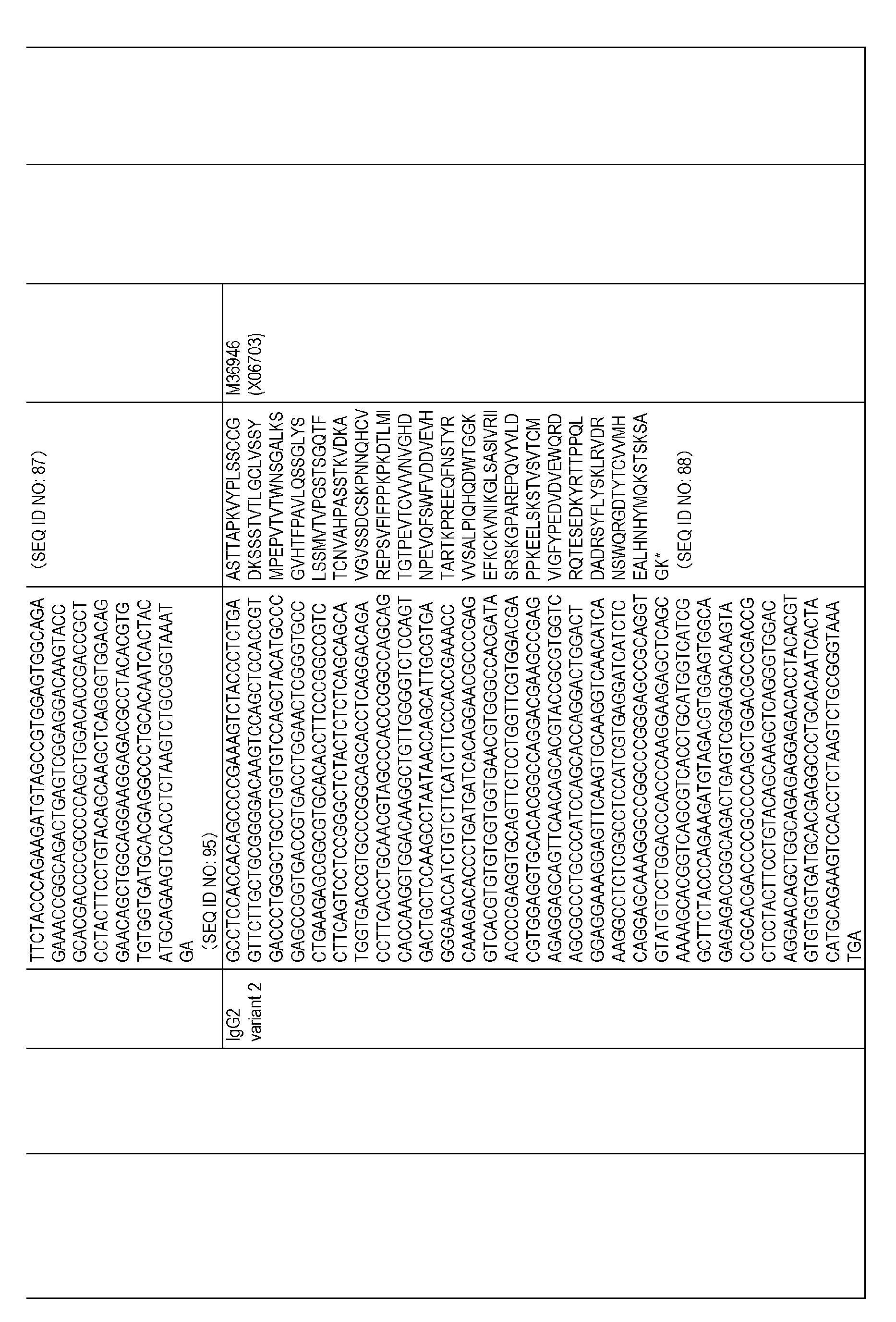

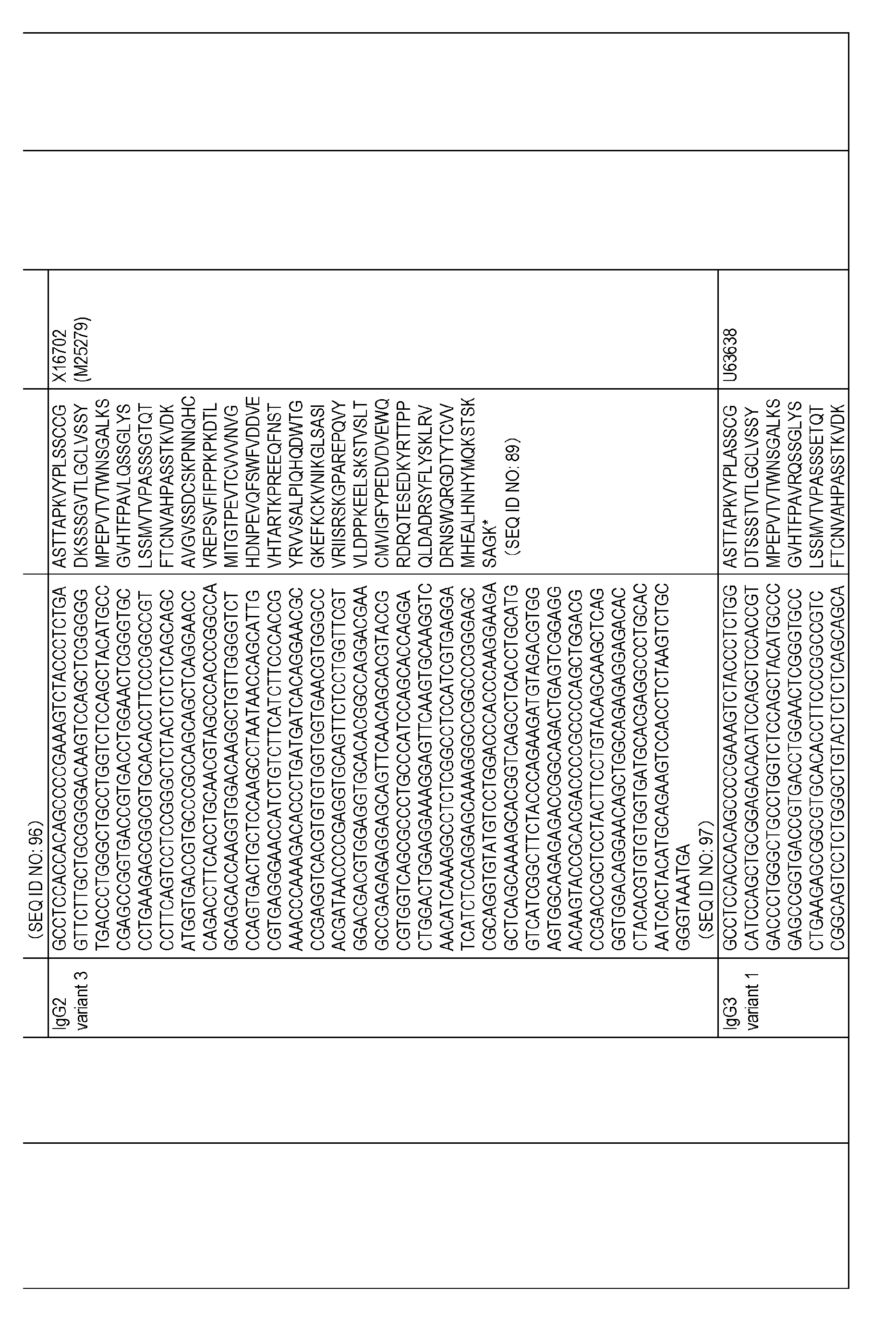

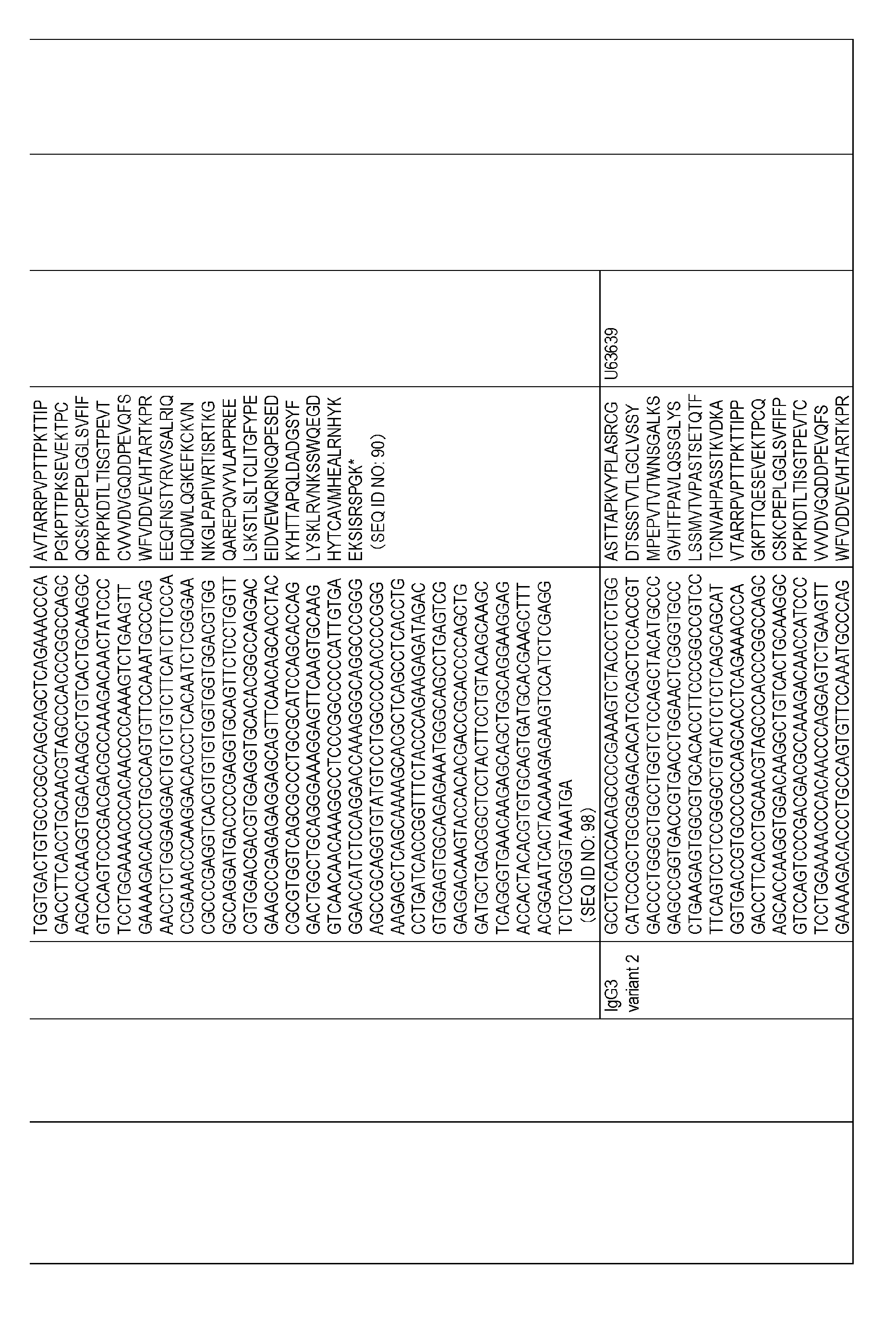

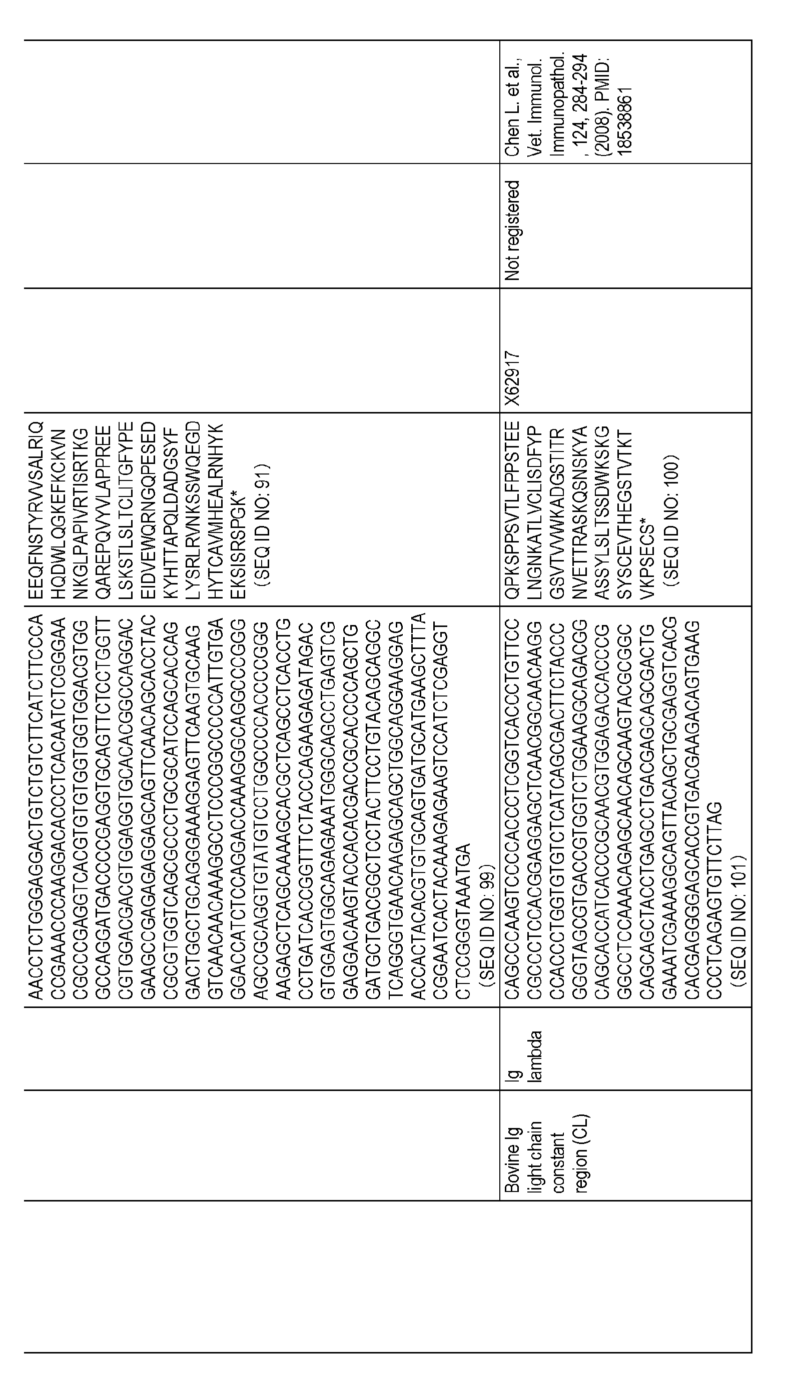

Although the constant region of wild-type human IgG1 has ADCC activity and CDC activity, it is known that these activities can be reduced by introducing amino acid substitutions and deletions into specific sites. In the case of animals other than human where the constant region of an immunoglobulin equivalent to human IgG4 has not been identified, mutations may be introduced into the relevant region of an immunoglobulin equivalent to human IgG1 so that the resultant constant region with reduced ADCC activity and CDC activity can be used.

The amino acid sequences as shown in SEQ ID NOS: 4, 3, 42, 44, 46, 48, 50, 52, 54, 56, 58, 60, 62, 64, 66, 68, 70, 72, 74, 76, 78, 12, 80, 82, 84-91, 100, 102 and 11 may have deletion(s), substitution(s) or addition(s) of one or several (e.g., up to five, about 10 at the most) amino acids.

The pharmaceutical composition of the present invention may be used for prevention and/or treatment of cancer and/or infection. Examples of cancer and/or infection include, but are not limited to, neoplastic diseases (e.g., malignant melanoma, lung cancer, gastric cancer, renal cancer, breast cancer, bladder cancer, esophageal cancer, ovarian cancer and the like), leukemia, Johne's disease, anaplasmosis, bacterial mastitis, mycotic mastitis, mycoplasma infections (such as mycoplasma mastitis, mycoplasma pneumonia or the like), tuberculosis, *Theileria orientalis* infection, cryptosporidiosis, coccidiosis, trypanosomiasis and leishmaniasis.

The pharmaceutical composition of the present invention comprises a COX-2 inhibitor and is administered before, after or simultaneously with the administration of an inhibitor targeting PD-1/PD-L1.

In the pharmaceutical composition of the present invention, an inhibitor targeting PD-1/PD-L1 and a COX-2 inhibitor may be used in combination or may be formulated as a single dosage.

When an inhibitor targeting PD-1/PD-L1 and a COX-2 inhibitor are used in combination, the inhibitor targeting PD-1/PD-L1 and the COX-2 inhibitor may be administered separately.

When an inhibitor targeting PD-1/PD-L1 and a COX-2 inhibitor are formulated as a single dosage, a combination drug containing the inhibitor targeting PD-1/PD-L1 and the COX-2 inhibitor may be prepared.

The pharmaceutical composition of the present invention can be administered to human or animal subjects systemically or locally by an oral or parenteral route.

The inhibitor targeting PD-1/PD-L1 may be dissolved in buffers such as PBS, physiological saline or sterile water, optionally filter- or otherwise sterilized before being administered to animal subjects (including human) by injection. To the solution of inhibitors targeting PD-1/PD-L1, additives such as coloring agents, emulsifiers, suspending agents, surfactants, solubilizers, stabilizers, preservatives, antioxidants, buffers, isotonizing agents, pH adjusters and the like may be added. As routes of administration, intravenous, intramuscular, intraperitoneal, subcutaneous or intradermal administration may be selected. Transnasal or oral administration may also be selected.

The content of the inhibitor targeting PD-1/PD-L1 in a preparation varies with the type of the preparation and is usually 1-100% by weight, preferably 50-100% by weight. Such a preparation may be formulated into a unit dosage form.

The dose and the number of times and frequency of administration of the inhibitor targeting PD-1/PD-L1 (e.g., PD-L1 antibody) may vary with the symptoms, age and body weight of the human or animal subject, the method of administration, dosage form and so on. For example, 0.1-100 mg/kg body weight, preferably 1-10 mg/kg body weight, may usually be administered per adult at least once at a frequency that enables obtainment of the desired effect.

A COX-2 inhibitor may be contained in a preparation comprising an inhibitor targeting PD-1/PD-L1. Alternatively, the COX-2 inhibitor either alone or in admixture with an excipient or carrier may be formulated into tablets, capsules, powders, granules, liquids, syrups, aerosols, suppositories, injections or the like. The excipient or carrier may be of any type that is routinely used in the art and pharmaceutically acceptable, with their type and composition being appropriately changed. As a liquid carrier, for example, water, plant oil or the like may be used. As a solid carrier, saccharides such as lactose, sucrose or glucose, starches such as potato starch or corn starch, cellulose derivatives such as microcrystalline cellulose, and the like may be used. Lubricants such as magnesium stearate, binders such as gelatin or hydroxypropyl cellulose, and disintegrants such as carboxymethyl cellulose, and the like may be added. What is more, antioxidants, coloring agents, flavoring agents, preservatives, and the like may also be added.

The COX-2 inhibitor may be administered via various routes such as oral, transnasal, rectal, transdermal, subcutaneous, intravenous or intramuscular route.

The content of the COX-2 inhibitor in a preparation varies with the type of the preparation and is usually 1-100% by weight, preferably 50-100% by weight. In the case of a liquid, for example, the content of the COX-2 inhibitor in the preparation is preferably 1-100% by weight. In the case of a capsule, tablet, granule or powder, the content of the COX-2 inhibitor in the preparation is usually 10-100% by weight, preferably 50-100% by weight, with the balance being the carrier. The preparation may be formulated into a unit dosage form.

The dose and the number of times and frequency of administration of the COX-2 inhibitor may vary with the symptoms, age and body weight of the animal or human subject, the method of administration, dosage form and so on. For example, in terms of the amount of the active ingredient, 0.05 to 20 mg (or ml)/kg body weight may usually be administered per adult at least once at a frequency that enables confirmation of the desired effect.

The ratio (in mass) of inhibitor targeting PD-1/PD-L1 to COX-2 inhibitor is appropriately from 1:100 to 1000:1, preferably from 1:10 to 100:1.

The present invention provides a method of preventing and/or treating cancer and/or infection, comprising administering to a human or animal subject a pharmaceutically effective amount of a COX-2 inhibitor before, after or simultaneously with the administration of an inhibitor targeting PD-1/PD-L1.

Further, the present invention provides use of a COX-2 inhibitor for preventing and/or treating cancer and/or infection, wherein the COX-2 inhibitor is administered before, after or simultaneously with the administration of an inhibitor targeting PD-1/PD-L1.

Still further, the present invention provides use of a COX-2 inhibitor for use in a method of preventing and/or treating cancer and/or infection, wherein the COX-2 inhibitor is administered before, after or simultaneously with the administration of an inhibitor targeting PD-1/PD-L1.

The immunostimulatory effect of an inhibitor targeting PD-1/PD-L1 can be enhanced by using a COX-2 inhibitor in combination. Therefore, the present invention provides a potentiator for the immunostimulatory effect of an inhibitor targeting PD-1/PD-L1, which comprises a COX-2 inhibitor.

The potentiator_may be used in combination with an inhibitor targeting PD-1/PD-L1 or formulated together with an inhibitor targeting PD-1/PD-L1 into a combination drug. Combined use of an inhibitor targeting PD-1/PD-L1 and a COX-2 inhibitor, as well as formulating them together as a single dosage are as described above. The potentiator_may be used as an experimental reagent in addition to its application as a pharmaceutical.

### EXAMPLES

Hereinbelow, the present invention will be described in more detail with reference to the following Examples. However, the present invention is not limited to these Examples.

### [Example 1]

### Examination of Combined Effect of Anti-PD-L1 Antibody and COX-2 Inhibitor in Dogs

### 1. Introduction

The interaction between PD-1 and PD-L1 is one of the major molecular mechanisms through which tumors evade immune responses. It has been reported that inhibition of the above interaction by using an antibody which specifically binds to either of those molecules can produce antitumor effects. In the subject Example, toward establishment of a novel control method for canine tumors, the present inventors have confirmed in *in vitro* tests an immunostimulatory effect induced by a COX-2 inhibitor and enhancement of that effect when the inhibitor is used in combination with anti-PD-L1 antibody.

### 2. Materials and Methods, as well as Experimental Results

### 2.1. PGE₂ Production from Canine Tumor Cell Lines

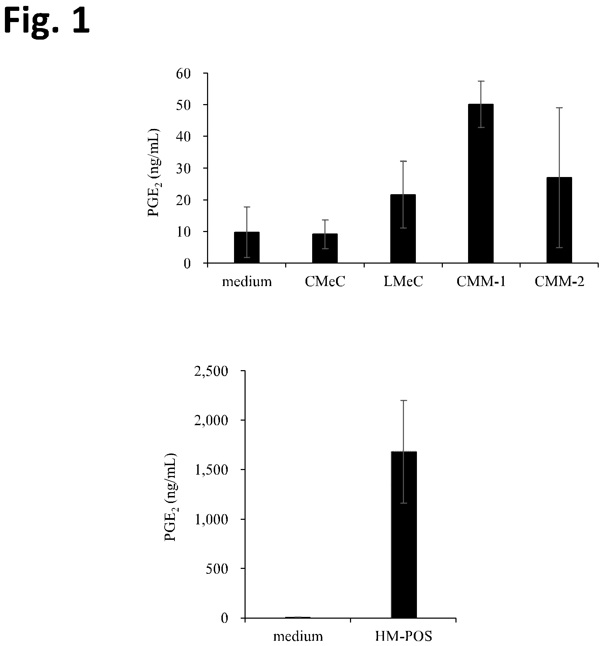

Canine melanoma-derived cell lines of CMeC and LMeC (Ohashi E, Inoue K, Kagechika H, Hong SH, Nakagawa T, et al: Effect of natural and synthetic retinoids on the proliferation and differentiation of three canine melanoma cell lines. J Vet Med Sci 64: 169-172, 2002) as well as CMM-1 and CMM-2 (Ohashi E, Hong SH, Takahashi T, Nakagawa T, Mochizuki M, et al.: Effect of retinoids on growth inhibition of two canine melanoma cell lines. J Vet Med Sci 63: 83-86, 2001) were cultured in RPMI 1640 medium (Sigma) supplemented with 2-mercaptoethanol 2x10⁻⁵ M, 10% inactivated fetal bovine serum (Valley Biomedical), antibiotics (streptomycin 100 µg/ml, penicillin 100 U/ml) (Invitrogen) and 2 mM L-glutamine (Invitrogen) at 37°C in the presence of 5% CO₂. A canine osteosarcoma-derived cell line HM-POS (Barroga EF, Kadosawa T, Okumura M, Fujinaga T: Establishment and characterization of the growth and pulmonary metastasis of a highly lung metastasizing cell line from canine osteosarcoma in nude mice. J Vet Med Sci 61: 361-367, 1999) was cultured in Dulbecco's Modified Eagle Medium (D-MEM; Invitrogen) supplemented with 10% inactivated fetal bovine serum (Valley Biomedical), antibiotics (streptomycin 100 µg/ml, penicillin 100 U/ml) (Invitrogen) and 2 mM L-glutamine (Invitrogen) at 37°C in the presence of 5% CO₂. Cells adjusted to a density of 5x10⁵ cells/mL were cultured for 24 hours. The amount of PGE₂ in the culture supernatant was quantified with Prostaglandin E2 Express EIA Kit (Cayman Chemical). As a result, CMM-1 and HM-POS showed a relatively high PGE₂ production (Fig. 1).

### 2.2. COX2 Expression in Canine Tumor Cell Lines

From the canine tumor-derived cell lines cultured as described in section 2.1. of Materials and Methods, RNA was extracted with TRI reagent (Molecular Research Center) and the concentration thereof was measured with NanoDrop8000 (Thermo Scientific). RNA samples were stored at -80°C until use in experiments.

To 1 µg of the thus obtained RNA, DNase I Reaction buffer and 1 U DNase I Amplification Grade (Invitrogen) were added. Then, deionized distilled water was added to make a 10 µl solution, which was subjected to DNase I treatment at room temperature for 15 min. Subsequently, 25 nmol ethylenediaminetetraacetic acid (EDTA) was added and the resultant mixture was treated at 65°C for 10 min. Then, 200 pmol oligo-dT primer was added to the reaction mixture which was treated at 65°C for 5 min. Thereafter, reverse transcription reaction solution [PrimeScript Buffer (TaKaRa), 7.5 nmol dNTPs, 20 U RNase Plus RNase inhibitor (Promega), 100 U PrimeScript RTase (TaKaRa)] was added to give a final volume of 20 µl. Reverse transcription reaction was carried out at 42°C for 60 min to thereby synthesize a single-stranded cDNA.

Primers (canine COX2 rt F and canine COX2 rt R; canine HPRT1 rt F and canine HPRT1 rt R) were designed based on the nucleotide sequences of canine *COX2* (NM_001003354.1) and canine *HPRT1* (AY283372.1) registered at the National Center for Biotechnology Information (NCBI), and real-time PCR was performed. Using 1 µl of the cDNA of each tumor-derived cell line as a template, real-time PCR was performed with LightCycler480 System II (Roche) in a PCR reaction mixture containing 0.3 µl each of primers canine COX2 rt F and canine COX2 rt R or canine HPRT1 rt F and canine HPRT1 rt R (each of which had been adjusted to a concentration of 10 pmol/µl), 5 µl of SYBR Premix DimerEraser (TaKaRa) and 3.4 µl of DDW under the conditions described below. Primer (canine COX2 rt F): AAGCTTCGATTGACCAGAGCAG (SEQ ID NO: 108) Primer (canine COX2 rt R): TCACCATAAAGGGCCTCCAAC (SEQ ID NO: 109) Primer (canine UPRT1 rt F): TGGCGTCGTGATTAGTGATGA (SEQ ID NO: 110) Primer (canine UPRT1 rt R): CAGAGGGCTACGATGTGATGG (SEQ ID NO: 111)

### (PCR Reaction Conditions)

1. Pre incubation 95°C for 30 sec
2. Quantification 50 cycles each consisting of the following 3 steps:
   I. Thermal denaturation 95°C for 5 sec
   II. Annealing 58°C for 30 sec
   III. Extension 72°C for 30 sec
3. Melting curve
   I. 95°C for 1 sec
   II. 65°C for 15 sec
   III. 95°C continue
4. Cooling 40°C

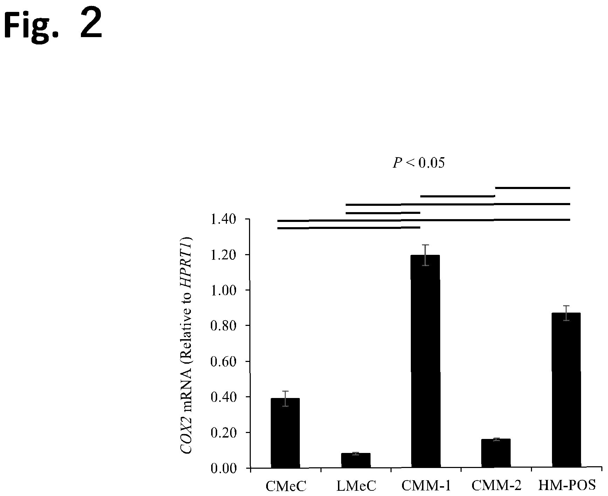

The resultant *COX2* mRNA expression level was divided by the expression level of internal control gene *HPRT1* mRNA, and the thus obtained value was taken as *COX2* expression level. As it turned out, *COX2* expression level was high in CMM-1 and HM-POS, consistent with the results of PGE₂ production in culture supernatant (Fig. 2; Tukey's multiple comparison test; P<0.05).

### 2.3. Effect of PGE₂ on Cytokine Production from Canine Peripheral Blood Mononuclear Cells

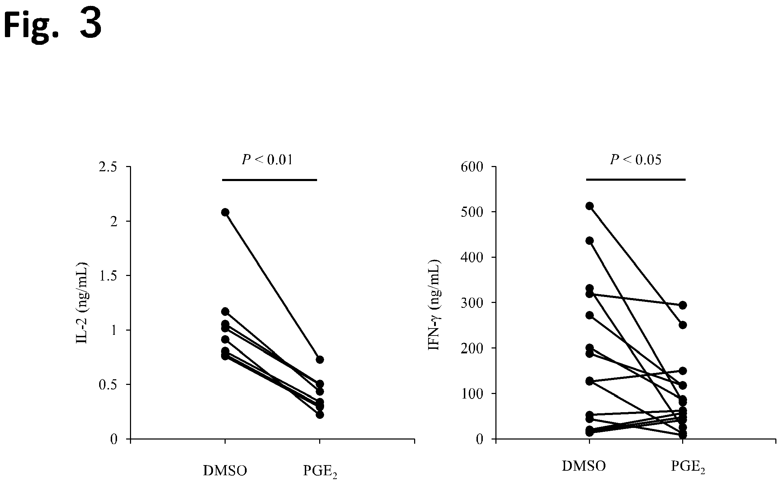

Peripheral blood mononuclear cells (PBMCs) were isolated from heparinized canine peripheral blood samples collected from healthy beagles and mixed breed dogs by density gradient centrifugation using Percoll (GE Healthcare). The resultant PBMCs were cultured in RPMI 1640 medium (Sigma), supplemented with 10% inactivated fetal bovine serum (Valley Biomedical), antibiotics (streptomycin 100 µg/ml, penicillin 100 U/ml) (Invitrogen) and 2 mM L-glutamine (Invitrogen) and further supplemented with 5 µg/ml of Staphylococcal Enterotoxin B (SEB) (Sigma) and 1 µg/ml of Anti-Canine CD28 (eBioscience), at 37°C in the presence of 5% CO₂ for 3 days. Production of interleukin 2 (IL-2) and interferon γ (IFN-γ) into the culture supernatant upon addition of prostaglandin E2 (Cayman Chemical) at a final concentration of 2.5 µM was measured with Canine IL-2 DuoSet ELISA (R&D systems) and Canine IFN-gamma DuoSet ELISA (R&D systems). PGE₂ significantly decreased IL-2 and IFN-γ productions from canine PBMCs (Fig. 3; Wilcoxon signed-rank test; P<0.01 and P<0.05).

### 2.4. PGE₂ Production Inhibitory Effect of COX-2 Inhibitor

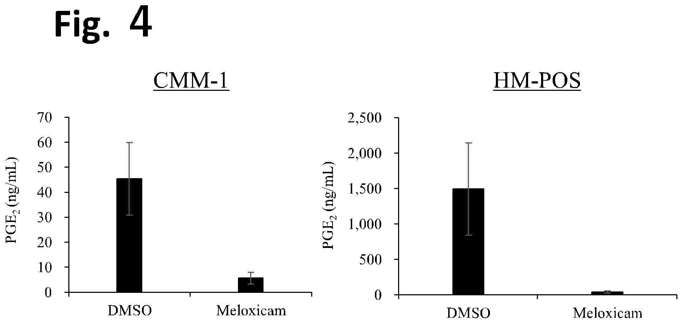

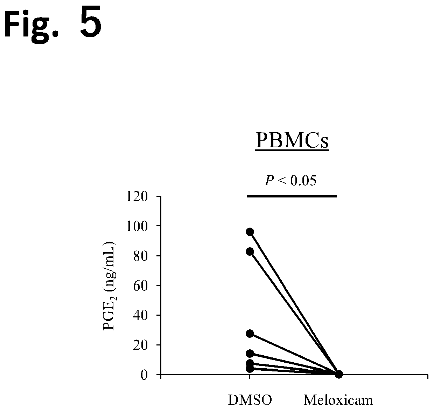

Canine tumor cell lines CMM-1 and HM-POS were cultured as described in section 2.1 of Materials and Methods, and meloxicam (Sigma) was added to give a final concentration of 5 µM. PGE₂ production from each tumor cell line was quantified by ELISA. PGE₂ production showed a tendency to decrease as a result of addition of meloxicam (Fig. 4). Further, canine PBMCs were cultured as described in section 2.3 of Materials and Methods, and meloxicam (Sigma) was added to give a final concentration of 5 µM. As a result, PGE₂ production from PBMCs decreased significantly (Fig. 5; Wilcoxon signed-rank test; P<0.05).

### 2.5. Effect of COX-2 Inhibitor on Cytokine Production from Canine Peripheral Blood Mononuclear Cells

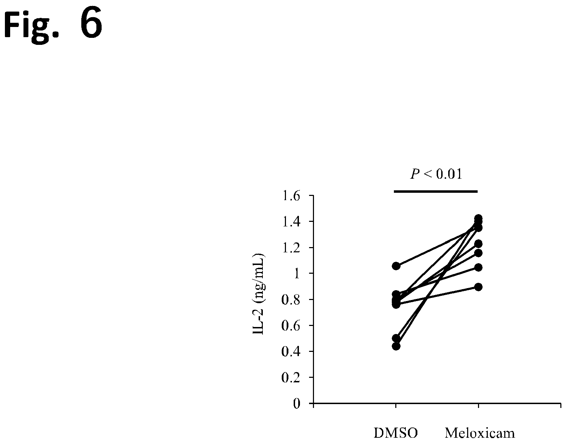

Canine PBMCs were cultured as described in section 2.3 of Materials and Methods, and meloxicam (Sigma) was added to give a final concentration of 5 µM. Then, IL-2 concentration in the culture supernatant was quantified by ELISA. IL-2 production from canine PBMCs was increased significantly as a result of addition of meloxicam (Fig. 6; Wilcoxon signed-rank test; P<0.01).

### 2.6. Enhancement of Canine PBMC Activation Effect by Combined Use of Anti-PD-Ll Antibody and COX-2 Inhibitor

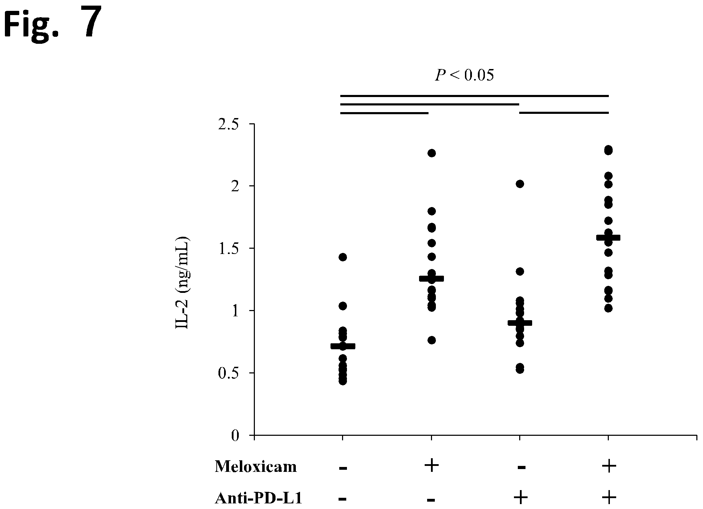

Canine PBMCs were cultured as described in section 2.3 of Materials and Methods. To the resultant PBMCs, rat-canine chimeric anti-PD-Ll antibody c4G12 (Maekawa et al., data in submission; see Reference Example 1 described below) and meloxicam (Sigma) were added to give final concentrations of 20 µg/mL and 5 µM, respectively. Subsequently, IL-2 concentration in the culture supernatant was quantified by ELISA. Although the PD-L1 antibody taken alone increased IL-2 production, combined use of meloxicam further increased IL-2 production (Fig. 7; Steel-Dwass test; P<0.05).

### [Reference Example 1]

### Rat-Canine Chimeric Anti-PD-Ll Antibody

### 1. Introduction

Programmed cell death 1 (PD-1), an immunoinhibitory receptor, and its ligand programmed cell death ligand 1 (PD-L1) are molecules identified by Prof. Tasuku Honjo et al., Kyoto University, as factors which inhibit excessive immune response and are deeply involved in immunotolerance. Recently, it has been elucidated that these molecules are also involved in immunosuppression in tumors. In the subject Example, for the purpose of establishing a novel therapy for canine neoplastic diseases, a chimeric antibody gene was prepared in which a variable region gene of a rat anti-bovine PD-L1 monoclonal antibody (4G12) capable of inhibiting the binding of canine PD-1 to PD-L1 was linked to a constant region gene of a canine immunoglobulin (IgG4). The resultant chimeric antibody gene was introduced into Chinese hamster ovary cells (CHO cells), which were cultured to produce a rat-canine chimeric anti-PD-Ll antibody c4G12. The effect of this chimeric antibody was confirmed *in vitro* and *in vivo.*

### 2. Materials and Methods

### 2.1 Bovine PD-L1 Monoclonal Antibody Producing Cells

The nucleotide sequence of bovine PD-L1 was identified (Ikebuchi R, Konnai S, Shirai T, Sunden Y, Murata S, Onuma M, Ohashi K. Vet Res. 2011 Sep 26;42:103). Based on the sequence information, a recombinant bovine PD-L1 was prepared. Rat was immunized with this recombinant protein to prepare a rat anti-bovine PD-L1 antibody (Ikebuchi R, Konnai S, Okagawa T, Yokoyama K, Nakajima C, Suzuki Y, Murata S, Ohashi K. Immunology. 2014 Aug;142(4):551-61; Clone 4G12 which would later serve as the variable region of the canine chimeric antibody of interest is described in this article.)

### 2.2 Identification of Full-Length Canine PD-1 and PD-L1 Genes

To determine the full lengths of canine PD-1 and PD-L1 cDNAs, PCR primers were first designed based on the putative nucleotide sequences of canine PD-1 and PD-L1 already registered at The National Center for Biotechnology Information (NCBI) (GenBank accession number; XM_543338 and XM_541302). Briefly, primers to amplify the inner sequence of the open reading frame (ORF) of each gene were designed (cPD-1 inner F and R, cPD-L1 inner F and R), and PCR was performed. For the amplified products, nucleotide sequences were determined with a capillary sequencer according to conventional methods. Further, to determine the nucleotide sequences of full-length PD-1 and PD-L1 cDNA, primers (cPD-1 5' GSP and 3' GSP; cPD-L1 5' GSP and 3'GSP) were designed based on the canine PD-1 and PD-L1 cDNA sequences determined above. 5'-RACE and 3'-RACE were then performed using the 5'-RACE system for rapid amplification of cDNA ends and 3'-RACE system for rapid amplification of cDNA ends (Invitrogen), respectively. The resultant gene fragments of interest were sequenced as described (Maekawa N, Konnai S, Ikebuchi R, Okagawa T, Adachi M, Takagi S, Kagawa Y, Nakajima C, Suzuki Y, Murata S, Ohashi K. PLoS One. 2014 Jun 10;9(6):e98415). Primer (cPD-1 inner F): AGGATGGCTCCTAGACTCCC (SEQ ID NO: 21) Primer (cPD-1 inner R): AGACGATGGTGGCATACTCG (SEQ ID NO: 22) Primer (cPD-L1 inner F): ATGAGAATGTTTAGTGTCTT (SEQ ID NO: 23) Primer (cPD-L1 inner R): TTATGTCTCTTCAAATTGTATATC (SEQ ID NO: 24) Primer (cPD-1 5'GSP): GTTGATCTGTGTGTTG (SEQ ID NO: 25) Primer (cPD-1 3'GSP): CGGGACTTCCACATGAGCAT (SEQ ID NO: 26) Primer (cPD-L1 5'GSP): TTTTAGACAGAAAGTGA(SEQ ID NO: 27) Primer (cPD-L1 3'GSP): GACCAGCTCTTCTTGGGGAA(SEQ ID NO: 28)

### 2.3 Construction of Canine PD-1 and PD-L1 Expressing COS-7 Cells

For preparing canine PD-1-EGFP and PD-L1-EGFP expression plasmids, PCR was performed using a synthesized beagle PBMC-derived cDNA as a template and primers designed by adding *Xho*I and *Bam*HI recognition sites (PD-1) and *Bgl*II and *Eco*RI recognition sites (PD-L1) on the 5' side (cPD-1-EGFP F and R; cPD-L1-EGFP F and R). The resultant PCR products were digested with *Xho*I (Takara) and *Bam*HI (Takara) (PD-1) and with *Bgl*II (New England Biolabs) and *Eco*RI (Takara) (PD-L1), and then purified with FastGene Gel/PCR Extraction Kit (NIPPON Genetics), followed by cloning into pEGFP-N2 vector (Clontech) treated with restriction enzymes in the same manner. The resultant expression plasmids of interest were extracted with QIAGEN Plasmid Midi kit (Qiagen) and stored at -30°C until use in experiments. Hereinafter, the thus prepared expression plasmids are designated as pEGFP-N2-cPD-1 and pEGFP-N2-cPD-L 1. Primer (cPD-1-EGFP F): CCGCTCGAGATGGGGAGCCGGCGGGGGCC (SEQ ID NO: 29) Primer (cPD-1-EGFP R): CGCGGATCCTGAGGGGCCACAGGCCGGGTC (SEQ ID NO: 30) Primer (cPD-L1-EGFP F): GAAGATCTATGAGAATGTTTAGTGTC (SEQ ID NO: 31) Primer (cPD-L1-EGFP R): GGAATTCTGTCTCTTCAAATTGTATATC (SEQ ID NO: 32)

COS-7 cells were subcultured at a density of 5x10⁴ cells/cm² in 6-well plates, and then cultured overnight in RPMI 1640 medium containing 10% inactivated fetal bovine serum and 0.01% L-glutamine at 37°C in the presence of 5% CO₂. The pEGFP-N2-cPD-1, pEGFP-N2-cPD-L1 or pEGFP-N2 (negative control) was introduced into COS-7 cells at 0.4 µg/cm² using Lipofectamine 2000 (Invitrogen). The cells were cultured for 48 hours (cPD-1-EGFP expressing cell and cPD-L1-EGFP expressing cell). In order to confirm the expression of canine PD-1 and PD-L1 in the thus prepared expressing cells, intracellular localization of enhanced green fluorescent protein (EGFP) was visualized with an inverted confocal laser microscope LSM700 (ZEISS) (Maekawa N, Konnai S, Ikebuchi R, Okagawa T, Adachi M, Takagi S, Kagawa Y, Nakajima C, Suzuki Y, Murata S, Ohashi K. PLoS One. 2014 Jun 10; 9(6):e98415).

### 2.4 Construction of Recombinant Canine PD-1, PD-L1 and CD80

In order to amplify the extracellular regions of canine PD-1, PD-L1 and CD80 estimated from their putative amino acid sequences, primers were designed. Briefly, primers having an *Nhel* or *Eco*RV recognition sequence (PD-1 and PD-L1) added on the 5' side (cPD-1-Ig F and R; cPD-L1-Ig F and R) or having an *Eco*RV or *Kpn*I (CD80) recognition sequence added on the 5' side (cCD80-Ig F and R) were designed. PCR was performed using a synthesized beagle PBMC-derived cDNA as a template. The PCR products were digested with *Nhel* (Takara) and *Eco*RV (Takara) or with *Eco*RV (Takara) and *Kpn*I (New England Biolabs) and purified with FastGene Gel/PCR Extraction Kit (NIPPON Genetics). The thus purified DNAs were individually cloned into pCXN2.1-Rabbit IgG Fc vector (Niwa et al., 1991; Zettlmeissl et al.,1990; kindly provided by Dr. T. Yokomizo, Juntendo University Graduate School of Medicine, and modified in the inventors' laboratory) treated with restriction enzymes in the same manner. The expression plasmids were purified with QIAGEN Plasmid Midi kit (Qiagen) and stored at -30°C until use in experiments. Hereinafter, the thus prepared expression plasmids are designated as pCXN2.1-cPD-1-Ig, pCXN2.1-cPD-L1-Ig and pCXN2.1-cCD80-Ig, respectively. Primer (cPD-1-Ig F): CGCGGCTAGCATGGGGAGCCGGCGGGGGCC (SEQ ID NO: 33) Primer (cPD-1-Ig R): CGCGGATATCCAGCCCCTGCAACTGGCCGC (SEQ ID NO: 34) Primer (cPD-L1-Ig F): CGCGGCTAGCATGAGAATGTTTAGTGTCTT (SEQ ID NO: 35) Primer (cPD-L1-Ig R): CGCGGATATCAGTCCTCTCACTTGCTGGAA (SEQ ID NO: 36) Primer (cCD80-Ig F): CGCGGATATCATGGATTACACAGCGAAGTG (SEQ ID NO: 104) Primer (cCD80-Ig R): CGGGGTACCCCAGAGCTGTTGCTGGTTAT (SEQ ID NO: 105)

These expression vectors were individually transfected into Expi293F cells (Life Technologies) to obtain a culture supernatant containing a recombinant Ig fusion protein. The recombinant protein produced was purified from the supernatant with Ab Capcher Extra (Protein A mutant; ProteNova). After buffer exchange with phosphate-buffered physiological saline (PBS; pH 7.4) using PD-MidiTrap G-25 (GE Healthcare), each recombinant protein was stored at -30°C until use in experiments (cPD-1-Ig, cPD-L1-Ig and cCD80-Ig). The concentration of each protein was measured with Pierce BCA Protein Assay Kit (Thermo Fisher Scientific) before use in subsequent experiments.

### 2.5 Identification of Rat Anti-bovine PD-L1 Monoclonal Antibody Showing Cross-reactivity with Canine PD-L 1

In order to identify rat anti-bovine PD-L1 monoclonal antibody showing cross-reactivity with canine PD-L1, flow cytometry was performed using the anti-bovine PD-L1 antibody prepared in 2.1 above. The anti-bovine PD-L1 antibody (10 µg/ml) was reacted with 2x10⁵⁻1x10⁶ cells at room temperature for 30 min. After washing, the anti-bovine PD-L1 antibody was detected with allophycocyanine-labeled anti-rat Ig goat antibody (Beckman Coulter). FACS Verse (Becton, Dickinson and Company) was used for analysis. As negative controls, rat IgG2a (κ) isotype control (BD Biosciences), rat IgG1 (κ) isotype control (BD Biosciences) and rat IgM (κ) isotype control (BD Biosciences) were used. For every washing operation and dilution of antibodies, 10% inactivated goat serum-supplemented PBS was used (Maekawa N, Konnai S, Ikebuchi R, Okagawa T, Adachi M, Takagi S, Kagawa Y, Nakajima C, Suzuki Y, Murata S, Ohashi K. PLoS One. 2014 Jun 10;9(6):e98415 which is an article describing the use of three bovine PD-L1 monoclonal antibodies: 4G12 (Rat IgG2a (κ)), 5A2 (Rat IgG1 (κ)) and 6G7 (Rat IgM (κ)).

### 2.6 Selection Test of Variable Region for Establishment of Rat-Canine Chimeric Anti-PD-Ll Antibody

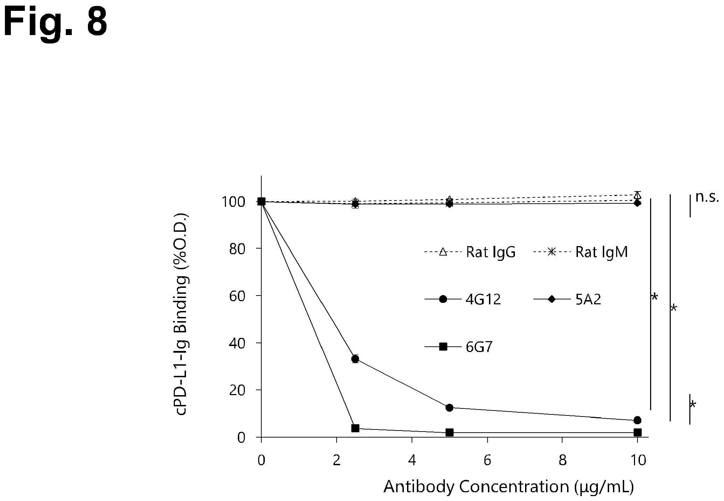

Out of 10 clones of rat anti-bovine PD-L1 monoclonal antibody which showed cross-reactivity with canine PD-L1, 4G12 (Rat IgG2a (κ)), 5A2 (Rat IgG1 (κ)) and 6G7 (Rat IgM (κ)) were selected and check was made to see whether these antibodies would inhibit canine PD-1/PD-L1 binding. Briefly, canine PD-1-Ig (prepared in 2.4 above) was immobilized on flat bottomed 96-well plates and blocked with 1% BSAand 0.05% Tween 20-containing PBS. Canine PD-L1-Ig (prepared in 2.4 above) was biotinylated using Lightning-Link Biotin Conjugation Kit (Innova Bioscience) and reacted with various concentrations (0, 2.5, 5 and 10 µg/ml) of rat anti-bovine PD-L1 antibodies 4G12, 5A2 and 6G7 at 37°C for 30 min, followed by addition to the 96-well plates. The binding of cPD-L1-Ig to cPD-1-Ig was measured by color reaction using Neutravidin-HRP (Thermo Fisher Scientific) and TMB one component substrate (Bethyl Laboratories). As a result, rat anti-bovine PD-L1 monoclonal antibodies 4G12 and 6G7 showed a good inhibitory activity against canine PD-1/PD-L1 binding, whereas 5A2 showed no binding inhibitory activity (Fig. 8).

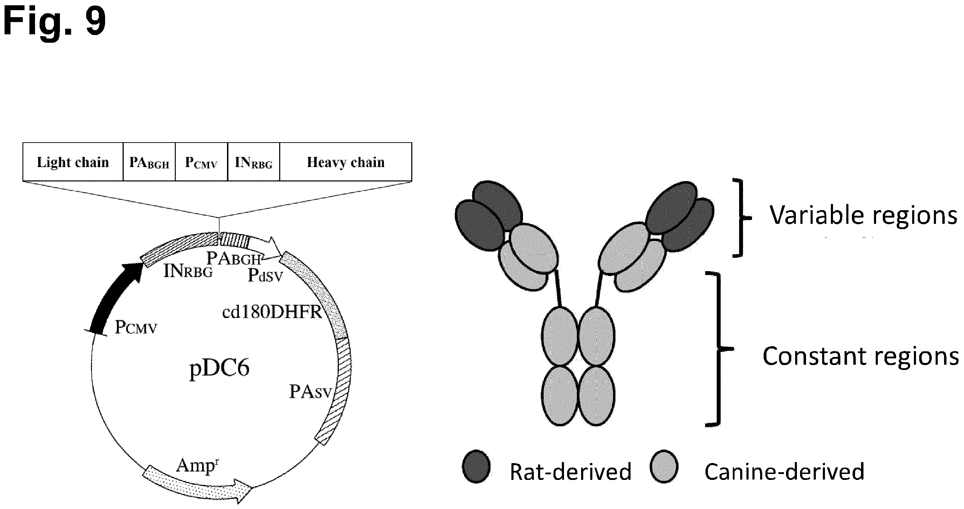

### 2.7 Preparation of Rat-Canine Chimeric Anti-PD-Ll Antibody Expressing Vector (Fig. 9)

Using rat anti-bovine PD-L1 monoclonal antibodies 4G12 and 6G7 which showed a good inhibitory activity against canine PD-1/PD-L1 binding (Fig. 1) as the variable region, two types of rat-canine chimeric anti-PD-L1 antibodies were established.

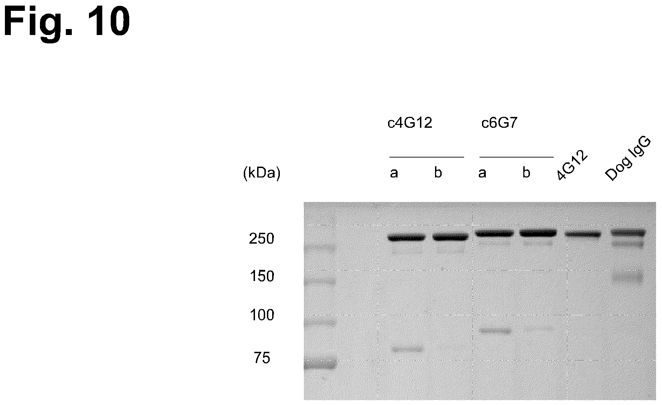

Briefly, heavy chain and light chain variable region genes were identified from hybridomas producing rat anti-bovine PD-L1 monoclonal antibodies 4G12 and 6G7. Further, the heavy chain and light chain variable region genes of the above rat antibodies were linked to the constant region of heavy chain IgG4 and the constant region of light chain Lambda of a known canine antibody, respectively, to prepare nucleotide sequences, followed by codon optimization (SEQ ID NOS: 9 and 10 (amino acid sequences), SEQ ID NOS: 19 and 20 (nucleotide sequences after codon optimization). Then, synthesis of genes was performed so that *Not*I restriction enzyme recognition site, KOZAK sequence, chimeric antibody's light chain sequence, poly-A addition signal sequence (PABGH), promoter sequence (PCMV), *Sac*I restriction enzyme recognition site, intron sequence (INRBG), KOZAK sequence, chimeric antibody's heavy chain sequence and *Xbal* restriction enzyme recognition site would be located in this order. The synthesized gene strands were individually incorporated into the cloning site (*Not*I and *Xbal* restriction enzyme recognition sequences downstream of PCMV and between INRBG and PABGH) of expression vector pDC6 (kindly provided by Prof. S. Suzuki, Hokkaido University Research Center for Zoonosis Control) using restriction enzyme recognition sequences so that the above-listed sequences would be located in the above-mentioned order (Fig. 9). Thus, rat-canine chimeric anti-PD-Ll antibody expressing vectors were constructed. Each of the expression vectors was transfected into Expi293F cells (Life Technologies) to obtain a culture supernatant containing a chimeric antibody. The chimeric antibody was purified from the supernatant with Ab Capcher Extra (Protein A mutant; ProteNova) and further purified by gel filtration chromatography. SDS-PAGE was performed under non-reducing conditions using 10% acrylamide gel. Bands were stained with Quick-CBB kit (Wako Pure Chemical) and decolorized in distilled water. Although contaminant proteins were observed after protein A purification alone, a highly purified antibody could be obtained by performing gel filtration chromatography (Fig. 10). It was confirmed by flow cytometry that the resultant purified antibodies specifically bound to canine PD-L1 expressing cells (data not shown). When the inhibitory activity of the two chimeric antibodies against canine PD-1/PD-L1 binding was examined by the method described in 2.6 above, rat-canine chimeric anti-PD-Ll antibody c4G12 showed a binding inhibitory activity similar to that of its original rat anti-bovine PD-L1 monoclonal antibody 4G12, whereas binding inhibition capacity was clearly attenuated in rat-canine chimeric anti-PD-L1 antibody c6G7 (Fig. 4) Therefore, rat-canine chimeric anti-PD-Ll antibody c4G12 was selected as a therapeutic antibody, which incorporated the variable region sequences of rat anti-bovine PD-L1 monoclonal antibody 4G12 (SEQ ID NOS: 2 and 1 (amino acid sequences) and SEQ ID NOS: 16 and 15 (nucleotide sequences after codon optimization)). The amino acid sequence and the nucleotide sequence (after codon optimization) of the light chain of c4G12 are shown in SEQ ID NOS: 9 and 19, and the amino acid sequence and the nucleotide sequence (after codon optimization) of the heavy chain of c4G12 are shown in SEQ ID NOS: 10 and 20.

### 2.8 Expression of Rat-Canine Chimeric Anti-PD-Ll Antibody c4G12

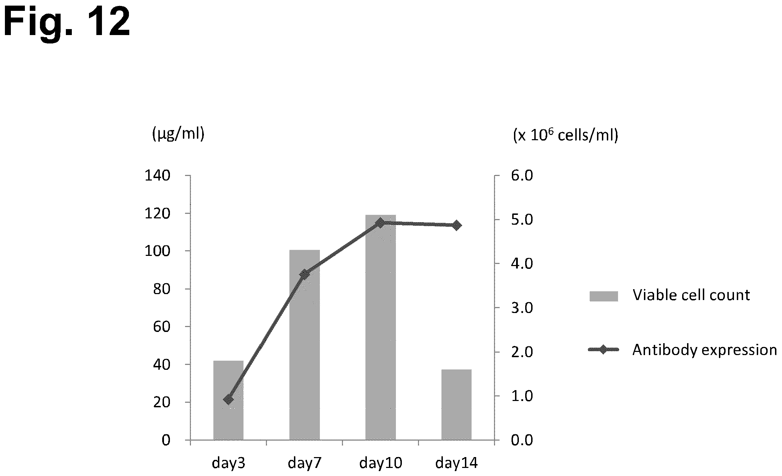

Rat-canine chimeric anti-PD-Ll antibody c4G12 expressing vector pDC6 as used in 2.7 above was transfected into CHO-DG44 cells (CHO-DG44(dfhr⁻/⁻)) which were dihydrofolate reductase deficient cells and high expression clones were selected by dot blotting. Further, gene amplification treatment was performed by adding load on cells in a medium containing 60 nM methotrexate (Mtx). Cells stably expressing rat-canine chimeric anti-PD-Ll antibody c4G12 (clone name: 4.3F1) after gene amplification were transferred to Mtx-free Opti-CHO medium and cultured under shaking for 14 days (125 rpm, 37°C, 5% CO₂). Cell survival rate was calculated by trypan blue staining (Fig. 12). Chimeric antibody production in the culture supernatant was measured by ELISA (Fig. 12). The culture supernatant at day 14 was centrifuged at 10,000 g for 10 min to remove cells, then passed through a 0.22 µm filter before the process proceeded to purification steps for the antibody.

It should be noted that by exchanging the medium with Dynamis medium and doing appropriate feeding, antibody production was improved about two-fold compared to the conventional production (data not shown).

### 2.9 Purification of Rat-Canine Chimeric Anti-PD-Ll Antibody c4G12

The culture supernatant provided as described above was purified with Ab Capcher Extra (ProteNova). An open column method was used for binding to resin; PBS pH 7.4 was used as equilibration buffer and wash buffer. As elution buffer, IgG Elution Buffer (Thermo Scientific) was used. As neutralization buffer, 1 M Tris was used. The purified antibody was concentrated and buffer-exchanged with PBS by ultrafiltration using Amicon Ultra-15 (50 kDa, Millipore). The resultant antibody was passed through a 0.22 µm filter for use in respective experiments.

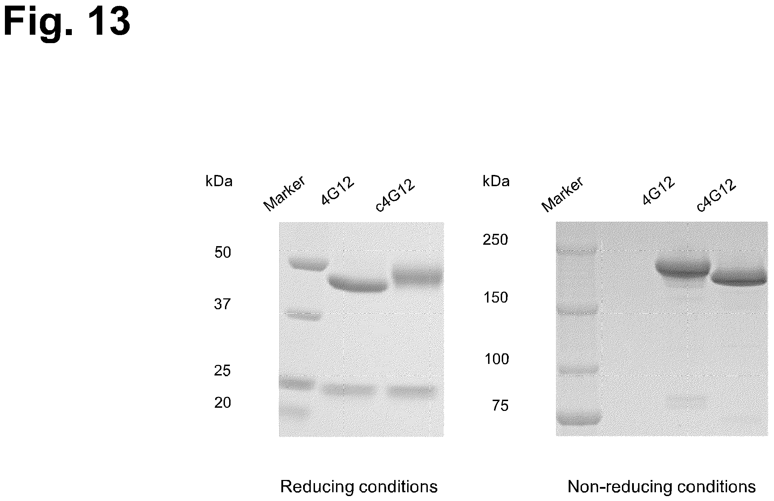

### 2.10 Confirmation of Purification of Rat-Canine Chimeric Anti-PD-Ll Antibody c4G12 (Fig. 13)

In order to confirm the purity of the purified antibody, antibody proteins were detected by SDS-PAGE and CBB staining. Using SuperSep Ace 5-20% (Wako) gradient gel, rat anti-bovine PD-L1 monoclonal antibody 4G12 and rat-canine chimeric anti-PD-Ll antibody c4G12 were electrophoresed under reducing conditions and non-reducing conditions. Bands were stained with Quick-CBB kit (Wako) and decolored in distilled water. Bands were observed at positions of molecular weights corresponding to antibodies. No bands of contaminant proteins were recognized visually.

### 2.11 Measurement of Binding Avidities to cPD-L1-His of Rat Anti-Bovine PD-L1 Monoclonal Antibody 4G12 and Rat-Canine Chimeric Anti-PD-Ll Antibody c4G12

In order to amplify the extracellular region of canine PD-L1 estimated from its putative amino acid sequence, primers were designed. Briefly, a primer having an *Nhel* recognition sequence added on the 5' side (cPD-L1-His F) and a primer having an *Eco*RV recognition sequence and 6xHis tag sequence added on the 5' side (cPD-L1-His R) were designed. PCR was performed using a synthesized beagle PBMC-derived cDNA as a template. The PCR products were digested with *Nhel* (Takara) and *Eco*RV (Takara) and purified with FastGene Gel/PCR Extraction Kit (NIPPON Genetics). The thus purified DNA was cloned into pCXN2.1 vector (Niwa et al., 1991; kindly provided by Dr. T. Yokomizo, Juntendo University Graduate School of Medicine) treated with restriction enzymes in the same manner. The expression plasmids were purified with QIAGEN Plasmid Midi kit (Qiagen) and stored at -30°C until use in experiments. Hereinafter, the thus prepared expression plasmid is designated as pCXN2.1-cPD-L1-His. Primer (cPD-L1-His F): CGCGGCTAGCATGAGAATGTTTAGTGTCTT (SEQ ID NO: 106) Primer (cPD-L1-His R): CGCGGATATCTTAATGGTGATGGTGATGGTGAGTCCTCTCACTTGCTGG (SEQ ID NO: 107)

The expression vector was transfected into Expi293F cells (Life Technologies) to obtain a culture supernatant containing a recombinant protein. The recombinant protein produced was purified from the supernatant using TALON Metal Affinity Resin (Clontech), and the buffer was exchanged with PBS using Amicon Ultra-4 Ultracel-3 (Merck Millipore). The thus obtained recombinant protein was stored at 4°C until use in experiments (cPD-L1-His). The protein concentration was measured with Pierce BCA Protein Assay Kit (Thermo Fisher Scientific) for use in subsequent experiments.

Using a biomolecular interaction analyzer (Biacore X100), the binding avidities to cPD-L1-His of rat anti-bovine PD-L1 monoclonal antibody 4G12 and rat-canine chimeric anti-PD-L1 antibody c4G12 were assessed. Briefly, anti-histidine antibody was fixed on CM5 censor chip, followed by capturing of cPD-L1-His. Subsequently, monoclonal antibodies were added as analyte to observe specific binding. Both antibodies exhibited specific binding and their avidities were almost comparable (Table 1). Further, the binding avidities of canine PD-1-Ig and CD80-Ig to cPD-L1-His were measured in the same manner and found to be clearly lower than that of rat-canine chimeric anti- PD-L1 antibody c4G12 (Table 1).

**Table 1. Binding Avidity of Each Antibody and Recombinant Protein to Canine PD-L1-His**

| | ka (× 10⁶/Ms) | kd (× 10⁻³/5) | KD (nM) |
|---|---|---|---|
| 4G12 | 2.42 ± 0.10 | 4.54 ± 0.19 | 1.88 ± 0.06 |
| c4G 12 | 3.14 ± 0.19 | 7.19 ± 0.26 | 2.30 ± 0.07 |
| cPD-1 | | | 25.4 ± 4.89 |
| cCD80 | | | 24.3 ± 0.89 |

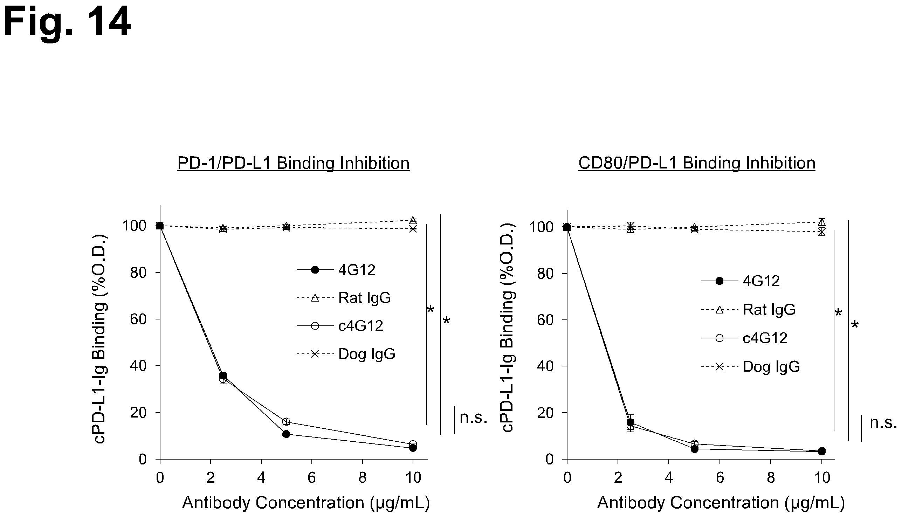

### 2.12 Inhibitory Activity of Rat-Canine Chimeric Anti-PD-Ll Antibody c4G12 against Canine PD-1/PD-L1 Binding and CD80/PD-L1 Binding (Fig. 14)

Using the canine PD-1-Ig, PD-L1-Ig and CD80-Ig (described above), anti-PD-Ll antibody was tested for its ability to inhibit canine PD-1/PD-L1 binding and CD80/PD-L1 binding. Briefly, canine PD-1-Ig or CD80-Ig was immobilized on flat-bottom 96-well plates. Canine PD-L1-Ig was reacted with various concentrations (0, 2.5,5 and 10 µg/ml) of rat anti-bovine PD-L1 antibody 4G12 or rat-canine chimeric anti-PD-Ll antibody c4G12 according to the same procedures as described in 2.6 above, and the binding of canine PD-L1-Ig was assessed. No change in binding inhibition activity was observed due to the chimerization of antibody.

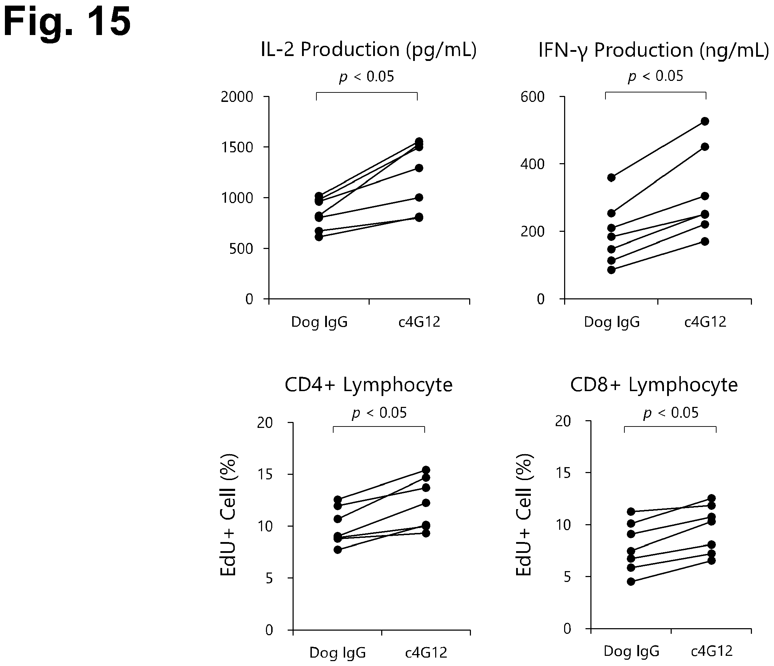

### 2.13. Canine Immune Cell Activating Effect of Rat-Canine Chimeric Anti-PD-Ll Antibody c4G12 (Fig. 15)

Canine PBMCs were cultured under stimulation with a superantigen Staphylococcal Enterotoxin B (SEB) for three days, and changes in cytokine production by addition of rat-canine chimeric anti-PD-Ll antibody c4G12 were measured by ELISA using Duoset ELISA canine IL-2 or IFN-γ (R&D systems). Rat-canine chimeric anti-PD-Ll antibody c4G12 increased the production of IL-2 and IFN-γ from canine PBMCs. Further, nucleic acid analogue EdU was added to the culture medium at day 2 of the culture under SEB stimulation. Two hours later, uptake of EdU was measured by flow cytometry using Click-iT Plus EdU flow cytometry assay kit (Life Technologies). As a result, EdU uptake in canine CD4⁺ and CD8⁺ lymphocytes was enhanced by addition of rat-canine chimeric anti-PD-Ll antibody c4G12, indicating an elevated cell proliferation capacity.

### 2.14 Selection of Tumor-Affected Dogs to be used in Canine Inoculation Test

Since the subject treatment is expected to manifest a higher efficacy when PD-L1 is being expressed in tumors, PD-L1 expression analysis at the tumor site of dogs was performed by immunohistochemical staining. Briefly, tumor tissue samples fixed with formaldehyde and embedded in paraffin were sliced into 4 µm thick sections with a microtome, attached to and dried on silane-coated slide glass (Matsunami Glass) and deparaffinized with xylene/alcohol. While the resultant sections were soaked in citrate buffer [citric acid (Wako Pure Chemical) 0.37 g, trisodium citrate dihydrate (Kishida Chemical) 2.4 g, distilled water 1000 ml], antigen retrieval treatment was performed for 10 min with microwave, followed by staining using a Nichirei automatic immuno-staining device. As pretreatment, sample sections were soaked in 0.3% hydrogen peroxide-containing methanol solution at room temperature for 15 min and washed with PBS. Then, anti-bovine PD-L1 monoclonal antibody was added and reaction was conducted at room temperature for 30 min. After washing with PBS, histofine simple stain MAX-PO (Rat) (Nichirei Bioscience) was added and reaction was carried at room temperature for 30 min, followed by coloring with 3,3'-diaminobenzidine tetrahydrocholride and observation with a light microscope. Dogs with oral melanoma or undifferentiated sarcoma in which tumor cells were PD-L1 positive were used in the following inoculation test (clinical trial). Anti-bovine PD-L1 monoclonal antibody was established from a rat anti-bovine PD-L1 monoclonal antibody producing hybridoma (Ikebuchi R, Konnai S, Okagawa T, Yokoyama K, Nakajima C, Suzuki Y, Murata S, Ohashi K. Immunology. 2014 Aug;142(4):551-61).

### 2.15 Inoculation Test on Dogs

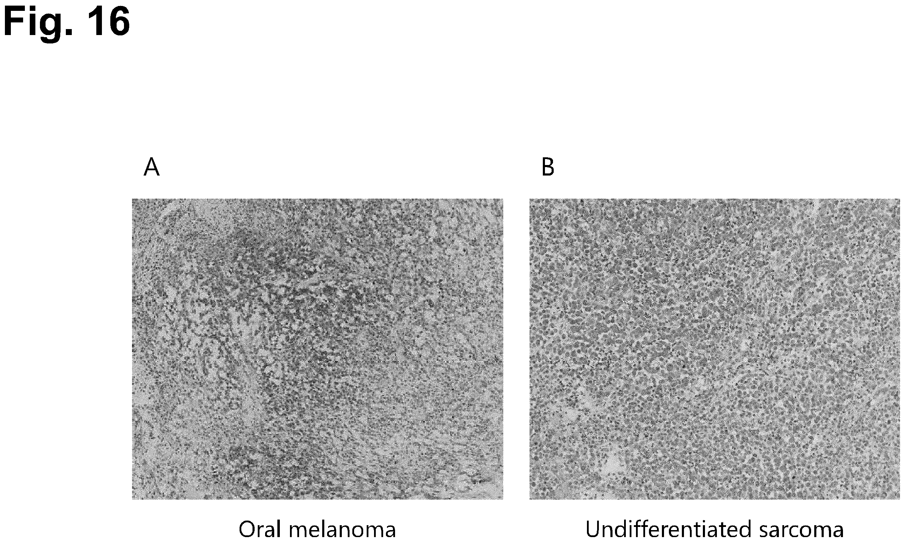

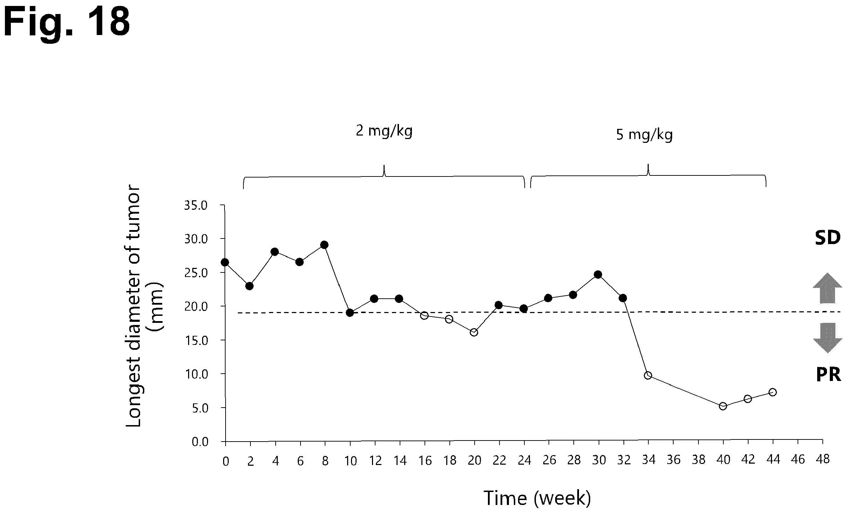

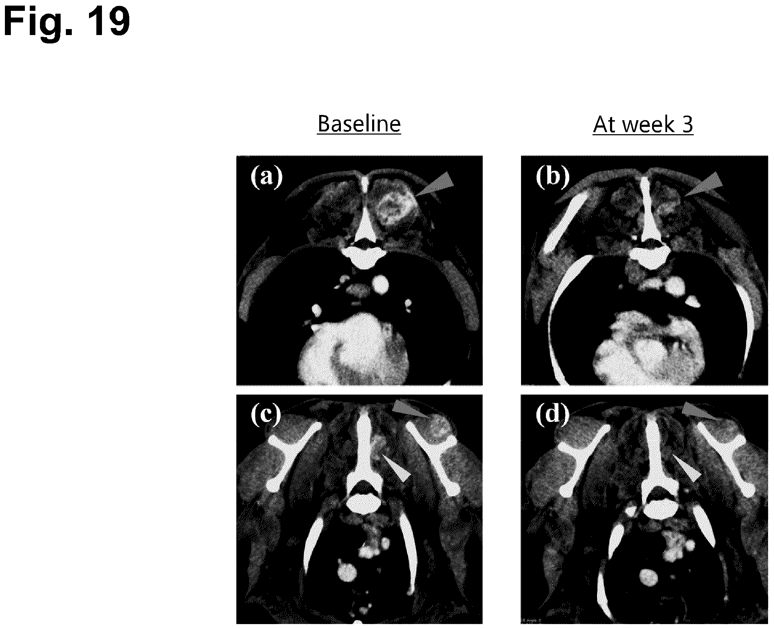

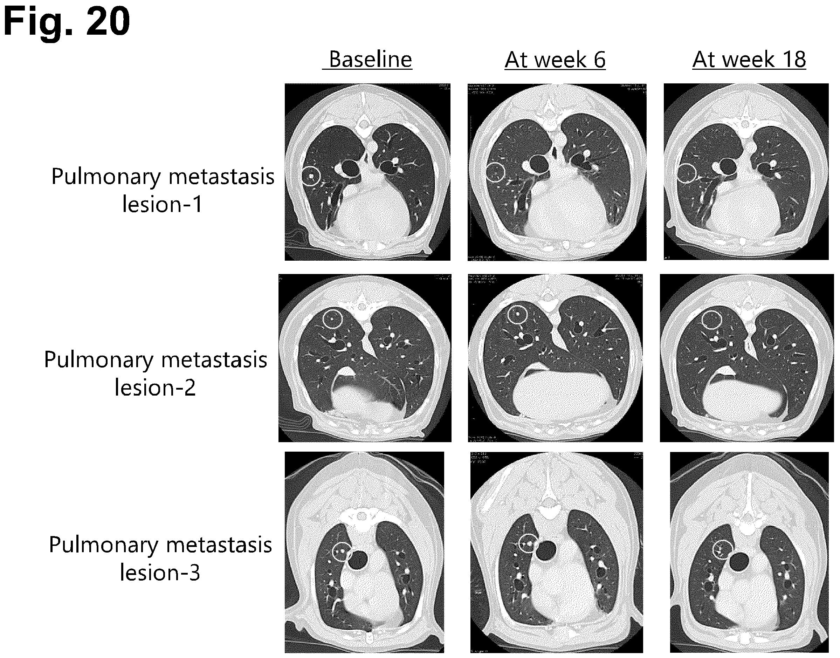

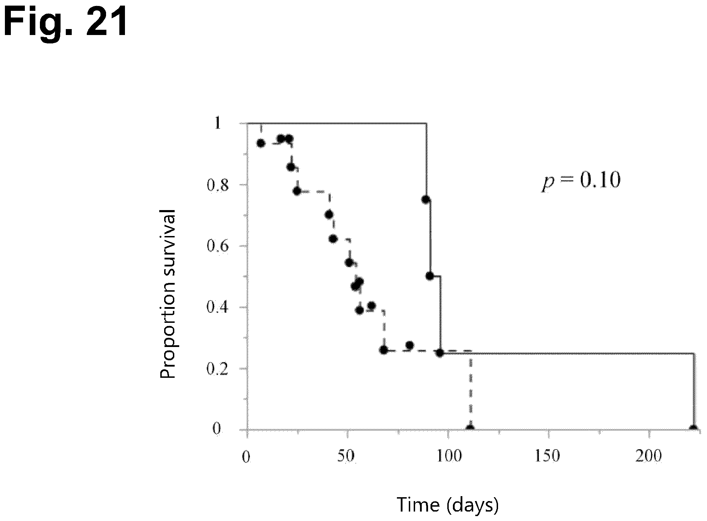

With respect to the rat-canine chimeric anti-PD-Ll antibody c4G12 to be inoculated into dogs in the clinical trial, the culture supernatant obtained by the procedures described in 2.8 above was purified by affinity chromatography using MabSelect SuRe LX (GE Healthcare) and then by hydroxyapatite chromatography using BioScale CHT20-I prepacked column (Bio-Rad) in order to remove contaminants and polymeric proteins. Aggregate-containing fractions were further purified by anion exchange chromatography using HiScreen Q-Sepharose HP prepacked column (GE Healthcare).
(1) Safety Test: The established rat-canine chimeric anti-PD-Ll antibody c4G12 was administered intravenously into a dog (beagle, spayed female, 13-year-old, about 10 kg in body weight) at 2 mg/kg, every 2 weeks, 3 times in total. There was observed no anaphylaxis or other adverse effects that would cause any trouble in clinical trials. (2) Clinical Trial 1: The established rat-canine chimeric anti-PD-Ll antibody c4G12 was administered intravenously into a PD-L1 positive dog with relapsed oral melanoma (Fig. 16A) (miniature dachshund, male, 11-year-old, about 7.5 kg in body weight) at 2 mg/kg or 5 mg/kg, every 2 weeks, 22 times in total. At week 10 after the start of treatment, a remarkable reduction in tumor size was recognized. At week 34 after the start of treatment, a still further reduction was confirmed (Fig. 10). During the observation period of 44 weeks, no metastases to lymph nodes or the lung were observed. When 30% or more reduction in the longest diameter of tumor compared to that at the baseline is defined as PR (partial response), the criterion of PR was satisfied at weeks 16-20 and at week 34 and thereafter (Fig. 18).
(3) Clinical Trial 2: Rat-canine chimeric anti-PD-Ll antibody c4G12 was administered intravenously into a dog with undifferentiated sarcoma whose primary lesion was PD-L1 positive (Fig. 16B) and who had a plurality of metastatic lesions in muscles throughout the body (west highland white terrier, castrated male, 12-year-old, about 8 kg in body weight) at 5 mg/kg, every 2 weeks, 2 times in total. At week 3 from the start of treatment, a clear regression of tumor was recognized (Fig. 19).
(4) Clinical Trial 3: Rat-canine chimeric anti-PD-Ll antibody c4G12 was administered intravenously into a dog with oral melanoma whose primary lesion had been removed by surgery (beagle, spayed female, 11-year-old, about 10 kg in body weight) at 2 mg/kg or 5 mg/kg, every 2 weeks, 9 times in total. At week 18 after the start of treatment, a plurality of pulmonary metastatic lesions disappeared (Fig. 20),
(5) Clinical Trial 4: Rat-canine chimeric anti-PD-Ll antibody c4G12 was administered intravenously into 4 dogs with oral melanoma with pulmonary metastasis at 2 mg/kg or 5 mg/kg, every 2 weeks. Although no clear reduction in tumor size was observed during the observation period, the duration of the treated dogs' survival after confirmation of pulmonary metastasis tended to be longer than that of a control group (antibody not administered, historical control group: n=15) (Fig. 21). Therefore, the survival duration may have been extended by antibody administration.

### 2.16 CDR Analysis of Anti-PD-L 1 Antibody

The complementarity-determining regions (CDRs) of rat anti-bovine PD-L1 antibody 4G12 were determined using NCBI IGBLAST (http://www.ncbi.nlm.nih.gov/igblast/). The results are shown in Fig. 22.

### [Example 2]

### Examination of Combined Effect of Anti-Bovine PD-L1 Antibody and COX-2 Inhibitor in Cattle with Johne's Disease

### 1. Introduction

The interaction between PD-1 and PD-L1 is one of the major molecular mechanisms through which pathogens evade immune responses. It has been reported that inhibition of the above interaction by using an antibody which specifically binds to either of those molecules can produce anti-pathogenic effects. In the subject Example, toward establishment of a novel control method against Johne's disease, the present inventors have confirmed in *in vitro* tests an immunostimulatory effect induced by a COX-2 inhibitor and enhancement of that effect when the inhibitor is used in combination with anti-bovine PD-L1 antibody.

### 2. Materials and Methods, as well as Experimental Results

### 2.1. Examination of Immunosuppressive Effects of PGE₂

In order to examine the immunosuppressive effects of PGE₂ in cattle, the present inventors evaluated how PBMCs derived from uninfected cattle stimulated with anti-CD3 monoclonal antibody and anti-CD28 monoclonal antibody changed in proliferation capacity and cytokine production capacity as well as in expression levels of cytokine and transcription factor genes and PD-L1 in the presence of PGE₂.

### (1) Changes in Cell Proliferation Capacity Induced by PGE₂

Peripheral blood mononuclear cells (PBMCs) derived from cattle not infected with *Mycobacterium avium* subsp. *paratuberculosis* (hereinafter, referred to as "uninfected cattle") were seeded in 96-well plates (Corning) at 4x10⁵ cells/well. The cells were cultured in RPMI 1640 medium (Sigma-Aldrich) supplemented with 10% inactivated fetal bovine serum (Thermo Fisher Scientific), antibiotics (streptomycin 100 µg/ml, penicillin 100 U/ml) (Thermo Fisher Scientific) and 2 mM L-glutamine (Thermo Fisher Scientific) for 3 days at 37°C in the presence of 5% CO₂. The PBMCs were labeled with Carboxyfluorescein Diacetate Succinimidyl Ester (CFSE, Invitrogen). To the medium, 10-fold serially diluted PGE₂ (from 2.5 nM to 2,500 nM) (Cayman Chemical) or, as a negative control, phosphate buffered physiological saline (PBS, pH 7.2, Wako Pure Chemical) was added to give a total volume of 200 µl. As stimulants for T cells, 1 µg/ml of anti-CD3 monoclonal antibody (MM1A; Washington State University Monoclonal Antibody Center) and 1 µg/ml of anti-CD28 monoclonal antibody (CC220; Bio-Rad) were added. After culturing, PBMCs were harvested and analyzed by flow cytometry. In order to prevent non-specific reactions, PBS supplemented with 10% inactivated goat serum (Thermo Fisher Scientific) was added to each well at 100 µl/well, and left stationary at room temperature for 15 min. After washing, Alexa Fluor 647-labeled anti-CD4 monoclonal antibody (CC30; Bio-Rad), peridinin-chlorophyll-protein complex/cyanin 5.5 (PerCp/Cy 5.5)-labeled anti-CD8 monoclonal antibody (CC63; Bio-Rad) and phycoerythrin/cyanin 7 (PE/Cy7)-labeled anti-IgM monoclonal antibody (IL-A30; Bio-Rad) were reacted at room temperature for 20 min. The anti-CD4 monoclonal antibody (CC30) was labeled with Alexa Fluor 647 using Zenon Mouse IgG1 labeling Kits (Thermo Fisher Scientific). The anti-CD8 monoclonal antibody (CC63) and the anti-IgM monoclonal antibody (IL-A30) were labeled with PerCp/Cy5.5 and PE/Cy7, respectively, using Lightning-Link Conjugation Kit (Innova Biosciences). After reaction, washing was performed twice. Then, cell proliferation was analyzed with FACS Verse (BD Biosciences) and FCS Express 4 (De Novo Software). For every washing operation and dilution of antibodies, PBS supplemented with 1% bovine serum albumin (Sigma Aldrich) was used.

### (2) Changes in Cytokine Production Induced by PGE₂

PBMCs derived from uninfected cattle were seeded in 96-well plates at 4x10⁵ cells/well and cultured in the same manner as described in (1) above for 3 days (Note: analysis of TNF-α production was performed only for the cells cultured under stimulation with 2,500 nM PGE₂). After 3 days, the culture supernatant was collected. IFN-γ production was measured with ELISA for Bovine IFN-γ (MABTECH), and TNF-α production was measured with Bovine TNF alpha Do-It-Yourself ELISA (Kingfisher Biotech). For the measurement, absorbance at 450 nm was measured using a microplate reader MTP-900 (Corona Electric).

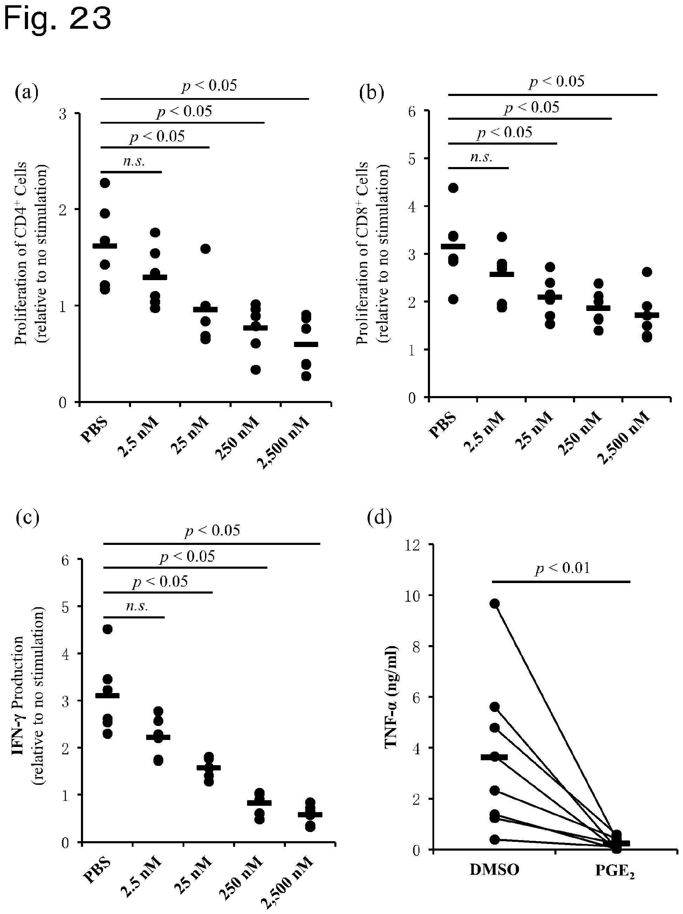

Experimental results of (1) and (2) above are shown in Fig. 23. In groups where PGE₂ was added at 25 nM, 250 nM and 2,500 nM, proliferation of CD4⁺ cells and CD8⁺ cells was significantly inhibited (Fig. 23a and b). Likewise, IFN-γ production was significantly inhibited in groups where PGE₂ was added at 25 nM, 250 nM and 2,500 nM (Fig. 23c). Further, in the group where PGE₂ was added at 2,500 nM, TNF-α production was also significantly inhibited (Fig. 23d). These results demonstrated that PGE₂ has immunosuppressive effects also in cattle.

### (3) Changes in mRNA Expression Levels of Cytokines, etc. Induced by PGE₂

PBMCs derived from uninfected cattle were seeded in 96-well plates at 1x10⁶ cells/well and cultured for 3 days in the presence of 2,500 nM PGE₂ or DMSO. Total cellular RNA was extracted from the thus cultured PBMCs using TRI reagent (Molecular Research Center), and cDNA was synthesized with PrimeScript Reverse Transcriptase (TaKaRa) and Oligo-dT primers. Using the synthesized cDNA as a template, real-time PCR was performed with LightCycler480 System II (Roche) in a 10 µl reaction solution containing SYBR Premix DimerEraser (TaKaRa) and 3 pmol each of the primers specific to individual genes. Then, changes in expression levels of individual genes were observed. Primer (boIL2 F): TTT TAC GTG CCC AAG GTT AA (SEQ ID NO: 119) Primer (boIL2 R): CGT TTA CTG TTG CAT CAT CA (SEQ ID NO: 120) Primer (boIL10 F): TGC TGG ATG ACT TTAAGG G (SEQ ID NO: 121) Primer (boIL10 R): AGG GCAGAAAGC GAT GAC A (SEQ ID NO: 122) Primer (boIFNγ F): ATA ACC AGG TCA TTC AAA GG (SEQ ID NO: 123) Primer (boIFNγ R): ATT CTG ACT TCT CTT CCG CT (SEQ ID NO: 124) Primer (boTNFα F): TAA CAA GCC AGT AGC CCA CG (SEQ ID NO: 125) Primer (boTNFα R):GCAAGG GCT CTT GAT GGC AGA(SEQ ID NO:126) Primer (boTGFβ1 F): CTG CTG AGG CTC AAG TTAAAA GTG (SEQ ID NO: 127) Primer (boTGFβ1 R): CAG CCG GTT GCT GAG GTA G (SEQ ID NO: 128) Primer (boFoxp3 F): CAC AAC CTG AGC CTG CAC AA (SEQ ID NO: 129) Primer (boFoxp3 R): TCT TGC GGAACT CAAACT CAT C (SEQ ID NO: 130) Primer (boSTAT3 F): ATG GAAACAACC AGT CGG TGA (SEQ ID NO: 131) Primer (boSTAT3 R): TTT CTG CAC ATA CTC CAT CGC T (SEQ ID NO: 132) Primer (boACTB F): TCT TCC AGC CTT CCT TCC TG (SEQ ID NO: 133) Primer (boACTB R): ACC GTG TTG GCG TAG AGG TC (SEQ ID NO: 134) Primer (boGAPDH F): GGC GTG AAC CAC GAG AAG TAT AA (SEQ ID NO: 135) Primer (boGAPDH R): CCC TCC ACG ATG CCAAAG T (SEQ ID NO: 136)

Reaction conditions of the real-time PCR were as described below.

| | |
|---|---|
| Thermal denaturation | 95°C for 5 sec (30 sec only for the first cycle) |
| Annealing | 60°C for 30 sec |
| Extension | 72°C for 30 sec |

After 45 cycles of thermal denaturation, annealing and extension, the temperature was raised from 65°C to 95°C at 0.1°C/sec for melting curve analysis. The melting temperatures of amplified products were measured to confirm specificity. For each sample, expression levels of genes *ACTB* and *GAPDH* were quantified as internal standards.

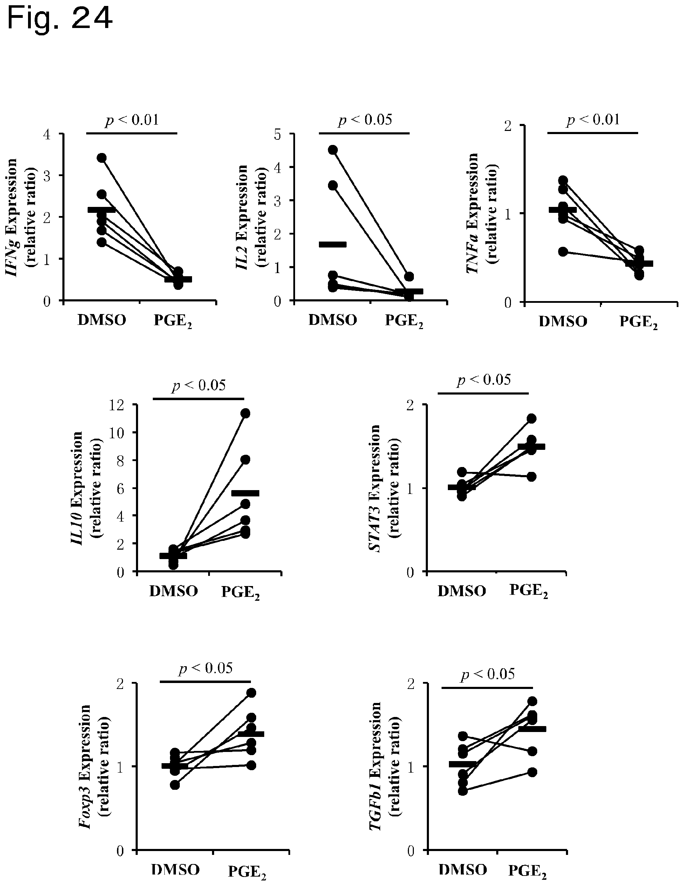

Experimental results of (3) are shown in Fig. 24. Addition of PGE₂ significantly decreased the expression levels of *IFNγ, IL-2* and *TNFα* in PBMCs. On the other hand, PGE₂ significantly increased the expression levels of *IL-10, STAT3, Foxp3* and *TGFβ1* in PBMCs.

### (4) Changes in PD-L1 Expression Induced by PGE₂

PBMCs derived from uninfected cattle were seeded in 96-well plates at 1x10⁶ cells/well and cultured for 24 hours under the same conditions as described in (1) above. Total cellular RNA was extracted from the cultured PBMCs, and cDNA was synthesized in the same manner as described in (3) above. Then, real-time PCR was performed using *PDL1* specific primers.
Primer (boPDL1 F): GGG GGT TTA CTG TTG CTT GA (SEQ ID NO: 137) Primer (boPDL1 R): GCC ACT CAG GAC TTG GTG AT (SEQ ID NO: 138) Further, expression of PD-L1 protein in the cultured PBMCs was analyzed by flow cytometry. Briefly, PBS supplemented with 10% inactivated goat serum (Thermo Fisher Scientific) was added to harvested PBMCs at 100 µl/well and left stationary at room temperature for 15 min. After washing, rat anti-bovine PD-L1 antibody (4G12; Rat IgG2a; Ikebuchi R, Konnai S, Okagawa T, Yokoyama K, Nakajima C, Suzuki Y, Murata S, Ohashi K. Immunology 2014 Aug.; 142(4):551-561) or rat IgG2a isotype control (BD Bioscience) was added and reaction was conducted at room temperature for 20 min. After washing twice, allophycocyanin (APC)-labeled anti-rat Ig antibody (Southern Biotech) was added and reaction was conducted at room temperature for 20 min. After washing twice, expression of PD-L1 protein was analyzed with FACS Verse (BD Biosciences) and FCS Express 4 (De Novo Software). For every washing operation and dilution of antibodies, PBS supplemented with 1% bovine serum albumin (Sigma Aldrich) was used.

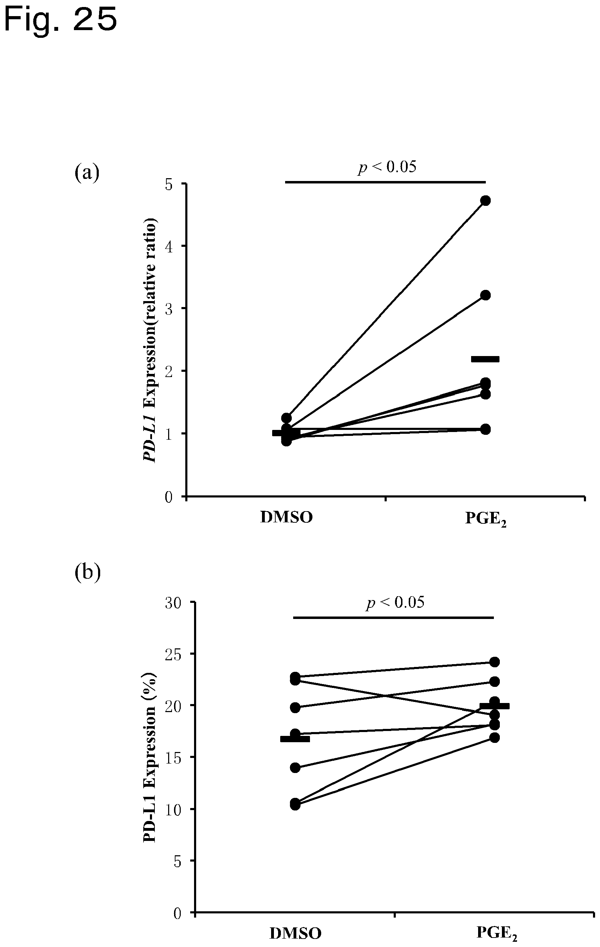

Experimental results of (3) are shown in Fig. 25. Expressions of *PD-L1* mRNA (Fig. 25a) and PD-L1 protein (Fig. 25b) were significantly increased in PBMCs derived from the uninfected cattle that were cultured in the presence of added PGE₂. These results show a possibility that PGE₂ affects PD-L1 expression also in cattle.

### 2.2. Examination of Immunostimulatory Effects of COX-2 Inhibitor in Cattle PBMCs

In order to examine immunostimulatory effects of COX-2 inhibitor in cattle, a COX-2 inhibitor meloxicam was added to a PBMC culture test under stimulation with anti-CD3 monoclonal antibody and anti-CD28 monoclonal antibody, followed by evaluation of the proliferation capacity and cytokine production capacity of PBMCs derived from uninfected cattle.

PBMCs derived from uninfected cattle were seeded in 96-well plates at 4x10⁵ cells/well and cultured for 3 days in the presence of 1,000 nM meloxicam (Sigma-Aldrich) or DMSO as a negative control. As stimulants for T cells, 1 µg/ml of anti-CD3 monoclonal antibody (MM1A; Washington State University Monoclonal Antibody Center) and 1 µg/ml of anti-CD28 monoclonal antibody (CC220; Bio-Rad) were added. After 3 days, cell proliferation capacity and cytokine production were evaluated in the same manner as described in (1) and (2) in section 2.1 above.

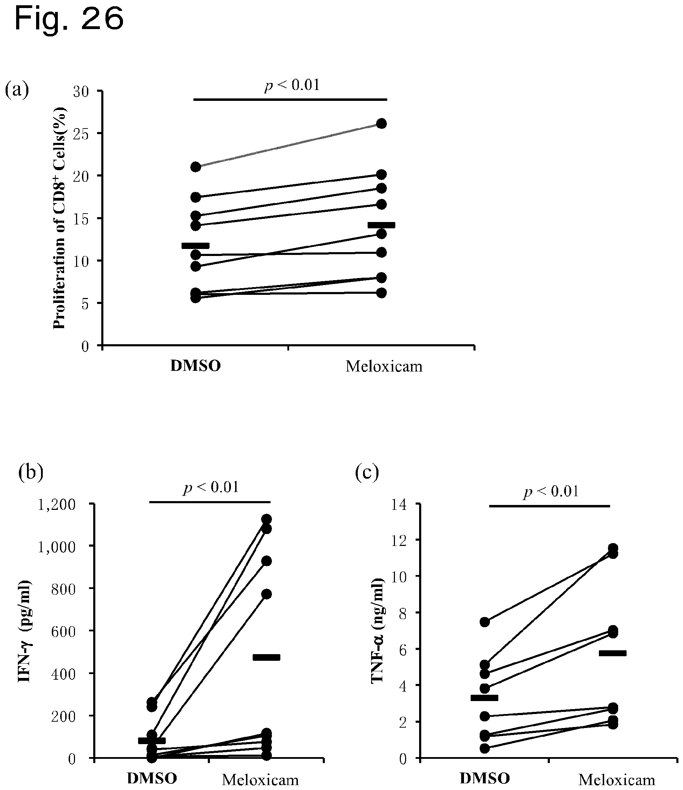

Experimental results are shown in Fig. 26. In the meloxicam-added group, proliferation rate of CD8⁺ cells was increased significantly (Fig. 26a), and so were the production of IFN-γ and TNF-α (Fig. 26b and 26c). These results demonstrated that COX-2 inhibitor has immunostimulatory effects also in cattle.

### 2.3. Kinetic Analysis of PGE₂ in Cattle infected with M. avium subsp. paratuberculosis

In order to elucidate the relation between bovine chronic infections and PGE₂, the present inventors performed kinetic analysis of PGE₂ in cattle infected with *M. avium* subsp. *paratuberculosis.*

### (1) Measurement of Serum PGE₂

First, PGE₂ contained in serum derived from cattle that developed Johne's disease from natural infection and PGE₂ contained in serum derived from uninfected cattle were quantified by ELISA. Briefly, the amount of PGE₂ contained in serum derived from cattle that naturally developed Johne's disease (kindly provided by Dr. Yasuyuki Mori, National Institute of Animal Health, National Agriculture and Food Research Organization) was measured with Prostaglandin E2 Express ELISA Kit (Cayman Chemical). For the measurement, absorbance at 450 nm was measured with a microplate reader MTP-900 (Corona Electric).

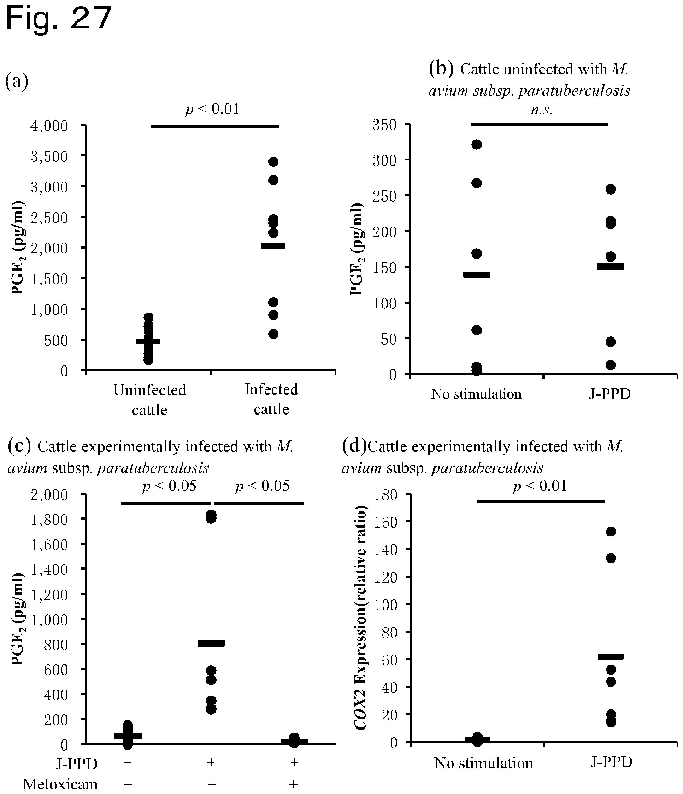

Experimental results of (1) are shown in Fig. 27a. Compared to uninfected cattle, the cattle manifesting Johne's disease showed a significant increase in serum PGE₂.

### (2) Changes in PGE₂ Production by M. avium subsp. paratuberculosis Antigen Stimulation

In order to confirm that PGE₂ production is promoted by *M. avium* subsp. *paratuberculosis* antigen, PBMCs derived from cattle experimentally infected with *M. avium* subsp. *paratuberculosis* and those derived from uninfected cattle were cultured with *M. avium* subsp. *paratuberculosis* antigen, and PGE₂ in culture supernatants was quantified by ELISA. Briefly, PBMCs derived from experimentally infected cattle and those from uninfected cattle were seeded in 96-well plates at 4x10⁵ cells/well and cultured for 5 days in the presence of 1 µg/ml of *M. avium* subsp. *paratuberculosis* antigen. As the *M. avium* subsp. *paratuberculosis* antigen, Johnin Purified Protein Derivative (J-PPD) was used. Further, in order to confirm that PGE₂ production by antigen stimulation is inhibited by COX-2 inhibitor, 1 µg/ml of J-PPD and 1000 nM meloxicam (Signa-Aldrich) were added to the medium. After 5 days, the culture supernatant was collected, and PGE₂ contained therein was quantified with Prostaglandin E2 Express ELISA Kit (Cayman Chemical).

Experimental results of (2) are shown in Fig 27b and c. Addition of J-PPD significantly promoted PGE₂ production from PBMCs in experimentally infected cattle (Fig. 27c). On the other hand, no significant difference was observed in uninfected cattle (Fig. 27b). It has been demonstrated that J-PPD-promoted PGE₂ production from experimentally infected cattle is a specific response to *M. avium* subsp. *paratuberculosis.* Further, PGE₂ production was inhibited significantly by culture with a COX-2 inhibitor added under the above-described conditions (Fig. 27c).

### (3) Changes in COX2 Expression by J-PPD Stimulation

PBMCs derived from cattle experimentally infected with *M. avium* subsp. *paratuberculosis* were seeded in 96-well plates at 1x10⁶ cells/well and cultured in the presence of J-PPD for 24 hours. After culturing, PBMCs were harvested and total cellular RNA was extracted as described above. Then, cDNA was synthesized. Using the synthesized cDNA as a template, real-time PCR was performed with *COX2* specific primers in the same manner as described above. Primer (boCOX2 F): ACG TTT TCT CGT GAA GCC CT (SEQ ID NO: 139) Primer (boCOX2 R): TCT ACC AGA AGG GCG GGA TA (SEQ ID NO: 140)

Experimental results of (3) are shown in Fig. 27d. *COX2* expression was increased significantly by J-PPD stimulation in cattle experimentally infected with *M. avium* subsp. *paratuberculosis.* These results suggested that *COX2* expression is increased by J-PPD stimulation in cattle experimentally infected with *M. avium* subsp. *paratuberculosis* and that this increase results in promoted PGE₂ production.

### 2.4. Changes in PD-L1 Expression by J-PPD Stimulation

Effects of J-PPD stimulation on PD-L1 expression in cattle infected with *M. avium* subsp. *paratuberculosis* were evaluated. PBMCs derived from cattle experimentally infected with *M. avium* subsp. *paratuberculosis* and those derived from uninfected cattle were seeded in 96-well plates at 1x10⁶ cells/well and cultured for 24 hours in the presence of J-PPD. Cultured PBMCs were harvested, and then PD-L1 expression on lymphocytes, CD4⁺ T cells, CD8⁺ T cells, IgM⁺ cells and CD14⁺ cells was analyzed by flow cytometry. Briefly, PBS supplemented with 10% inactivated goat serum (Thermo Fisher Scientific) was added to each well in a volume of 100 µl and cells were left stationary at room temperature for 15 min. After washing, rat anti-bovine PD-L1 antibody (4G12; Rat IgG2a; Ikebuchi R, Konnai S, Okagawa T, Yokoyama K, Nakajima C, Suzuki Y, Murata S, Ohashi K. Immunology 2014 Aug.; 142(4):551-561) or rat IgG2a isotype control (BD Bioscience) was added and reaction was conducted at room temperature for 20 min. After washing twice, secondary antibodies were added and reaction was conducted at room temperature for 20 min. As secondary antibodies, phycoerythrin (PE)-labeled anti-CD3 monoclonal antibody (MM1A; Washington State University Monoclonal Antibody Center), fluorescein isothiocyanate (FITC)-labeled anti-CD4 monoclonal antibody (CC8; Bio-Rad), PerCp/Cy 5.5-labeled anti-CD8 monoclonal antibody (CC63; Bio-Rad), PE/Cy7-labeled anti-IgM monoclonal antibody (IL-A30; Bio-Rad) and APC-labeled anti-rat Ig antibody (Southern Biotech) were used for analysis of PD-L1 expression on T cells and IgM⁺ cells. Anti-CD3 monoclonal antibody (MM1A) was labeled with PE using Zenon Mouse IgG1 labeling Kit. For analysis of PD-L1 expression on CD14⁺ cells, PerCp/Cy5.5-labeld anti-CD14 monoclonal antibody (CAM36A; Washington State University Monoclonal Antibody Center) and APC-labeled anti-rat Ig antibody (Southern Biotech) were used. Anti-CD14 monoclonal antibody (CAM36A) was labeled with PerCp/Cy5.5 using Lightning-Link Conjugation Kit. After reaction, washing was conducted twice. Then, PD-L1 expression was analyzed with FACS Verse (BD Biosciences) and FCS Express 4 (De Novo Software). For every washing operation and dilution of antibodies, PBS supplemented with 1% bovine serum albumin (Sigma Aldrich) was used.

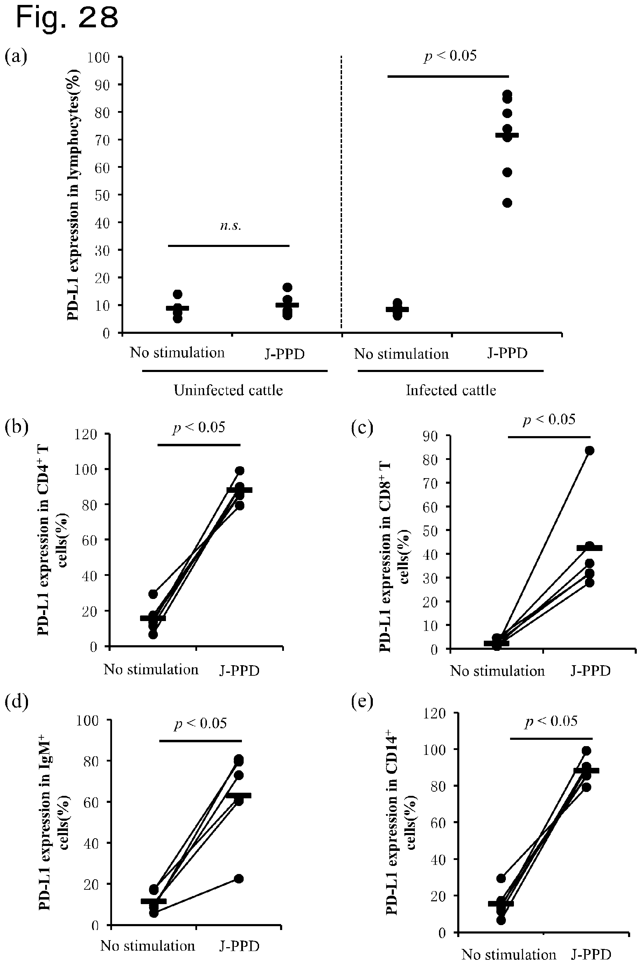

Experimental results are shown in Fig. 28. It was shown that PD-L1 expression rate is significantly increased in J-PPD-added groups of cattle experimentally infected with *M. avium* subsp. *paratuberculosis,* as compared to uninfected cattle (Fig. 28a). PD-L1 expression rates were also increased in CD4⁺ T cells, CD8⁺ T cells, IgM⁺ B cells and CD14⁺ cells in cattle infected with *M. avium* subsp. *paratuberculosis* (Fig. 28b-e).

### 2.5. Expression Analyses of PGE₂, EP2 and PD-L1 in Johne's Disease Lesions

Subsequently, the present inventors performed expression analyses of PGE₂, EP2 and PD-L1 in Johne's disease lesions by immunohistochemical staining. Briefly, ilium tissue blocks from cattle which naturally developed Johne's disease (#1, presenting clinical symptoms of Johne's disease such as diarrhea and severe emaciation), cattle experimentally infected with *M. avium* subsp. *paratuberculosis* (#65, clinical symptoms such as shedding of *M. avium* subsp. *paratuberculosis* and diarrhea were observed; Okagawa T, Konnai S, Nishimori A, Ikebuchi R, Mizorogi S, Nagata R, Kawaji S, Tanaka S, Kagawa Y, Murata S, Mori Y and Ohashi K., Infect Immun, 84:77-89, 2016) and uninfected control cattle (C#6) (those blocks were kindly provided by Dr. Yasuyuki Mori, National Institute of Animal Health, National Agriculture and Food Research Organization) were used for immunohistochemical staining. Samples fixed with 4% paraformaldehyde [paraformaldehyde 20 g, PBS (pH 7.4) 500 ml] and embedded in paraffin were sliced into 4 mm thick sections with a microtome, attached to and dried on silane-coated slide glass (Matsunami Glass) and deparaffinized with xylene/alcohol. While the resultant sections were soaked in citrate buffer (citric acid 0.37 g, trisodium citrate dihydrate 2.4 g, distilled water 1000 ml), antigen retrieval treatment was performed for 10 min with microwave, followed by staining using a Nichirei automatic immuno-staining device. As pretreatment, sample sections were soaked in 0.3% hydrogen peroxide-containing methanol solution at room temperature for 15 min and washed with PBS. Then, anti-PGE₂-polyclonal antibody (Abcam), anti-EP2 monoclonal antibody (EPR8030(B); Abcam) or rat anti-bovine PD-L1 monoclonal antibody (6C11-3A11; Rat IgG2a; Japanese Patent Application No. 2017-61389, Konnai S, Ohashi K, Murata S, Okagawa T, Nishimori A, Maekawa N, Takagi S, Kagawa Y, Suzuki S, Nakajima C, titled "Anti-PD-Ll Antibody for Detecting PD-L1") was added and reaction was conducted at room temperature for 30 min. After washing with PBS, histofine simple stain MAX-PO (Nichirei Bioscience) was added and reaction was carried out at room temperature for 30 min, followed by coloring with 3,3'-diaminobenzidine tetrahydrocholride and observation with a light microscope.

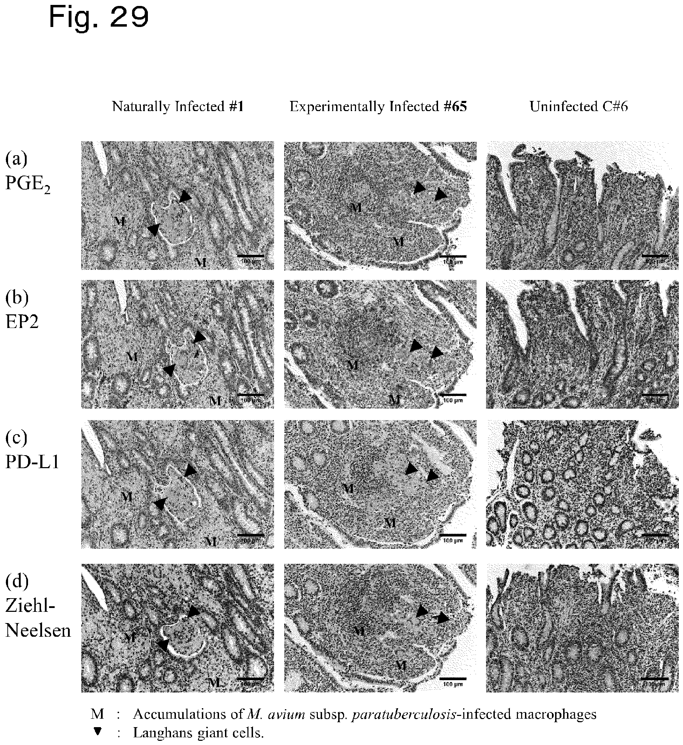

Experimental results are shown in Fig. 29. In ileal lesions of cattle (#1 cattle naturally developing johne's disease and #65 experimentally infected cattle) where *M. avium* subsp. *paratuberculosis* was confirmed by Ziehl-Neelsen staining, PGE₂, EP2 and PD-L1 were expressed strongly (Fig. 29a-d). On the other hand, in the ileum of uninfected cattle (C#6), the expression of EP2 was confirmed but PGE₂ and PD-L1 were hardly expressed (Fig. 29a-d). These results indicated a possibility that PGE₂ production and PD-L1 expression are enhanced in Johne's disease lesions.

### 2.6. Examination of Stimulatory Effects of COX-2 Inhibitor on M. avium subsp. paratuberculosis-Specific Immune Responses

In order to confirm that COX-2 inhibitor has stimulatory effects on *M. avium* subsp. *paratuberculosis-*specific immune responses, the present inventors cultured PBMCs derived from cattle experimentally infected with *M. avium* subsp. *paratuberculosis* in the presence of added meloxicam and J-PPD and evaluated their proliferation capacity and cytokine production capacity. Briefly, PBMCs derived from cattle experimentally infected with *M. avium* subsp. *paratuberculosis* were seeded in 96-well plates at 4x10⁵ cells/well and cultured for 5 days under stimulation in the presence of 1 µg/ml of J-PPD and 1000 nM meloxicam (Sigma-Aldrich). After culturing, cell proliferation capacity and cytokine production capacity were evaluated in the same manner as described above.

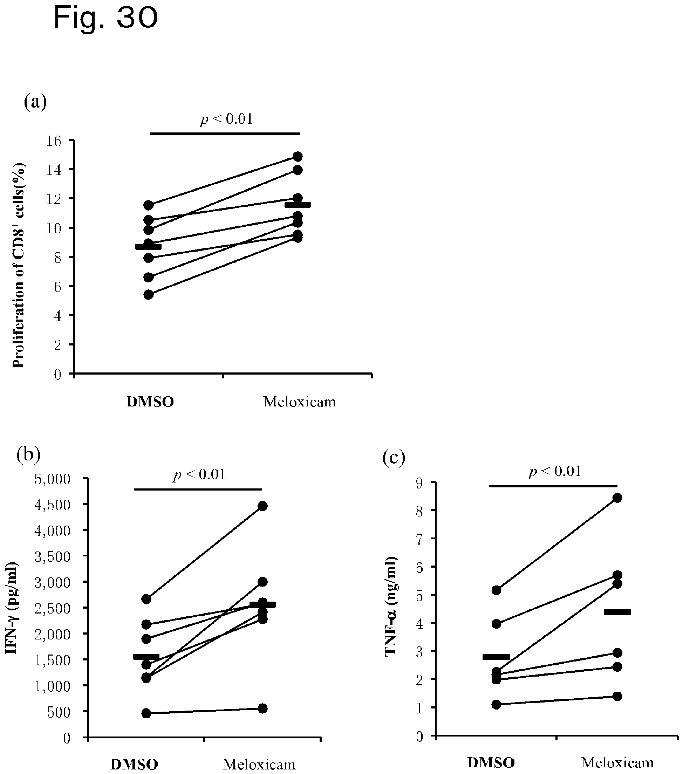

Experimental results are shown in Fig. 30. In cattle experimentally infected with *M. avium* subsp. *paratuberculosis*, a significant increase was observed in proliferation rate of CD8⁺ cells (Fig. 30a), IFN-γ production (Fig. 30b) and TNF-α production (Fig. 30c). These results demonstrated that COX-2 inhibitor has stimulatory effects on *M. avium* subsp. *paratuberculosis-*specific immune responses.

### 2.7. Examination of Immunostimulatory Effects of Rat Anti-Bovine PD-L1 Antibody in M. avium subsp. paratuberculosis-Infected Cattle

In order to confirm that rat anti-bovine PD-L1 antibody also has immunostimulatory effects in *M. avium* subsp. *paratuberculosis-*infected cattle, the present inventors performed a PBMC culture test under stimulation in the presence of rat anti-bovine PD-L1 antibody and evaluated J-PPD-specific T-cell responses. Briefly, PBMCs derived from cattle experimentally infected with *M. avium* subsp. *paratuberculosis* were seeded in 96-well plates at 4x10⁵ cells/well and cultured for 5 days under stimulation with 1 µg/ml of J-PPD. At the time of this stimulation culture, 1 µg/ml of rat anti-bovine PD-L1 antibody (4G12; Ikebuchi R, Konnai S, Okagawa T, Yokoyama K, Nakajima C, Suzuki Y, Murata S, Ohashi K. Immunology 2014 Aug.; 142(4):551-561) as a blocking antibody or the same amount of a rat serum-derived IgG (Sigma-Aldrich) as a negative control was added. After culturing, cell proliferation capacity and cytokine production were evaluated in the same manner as described above.

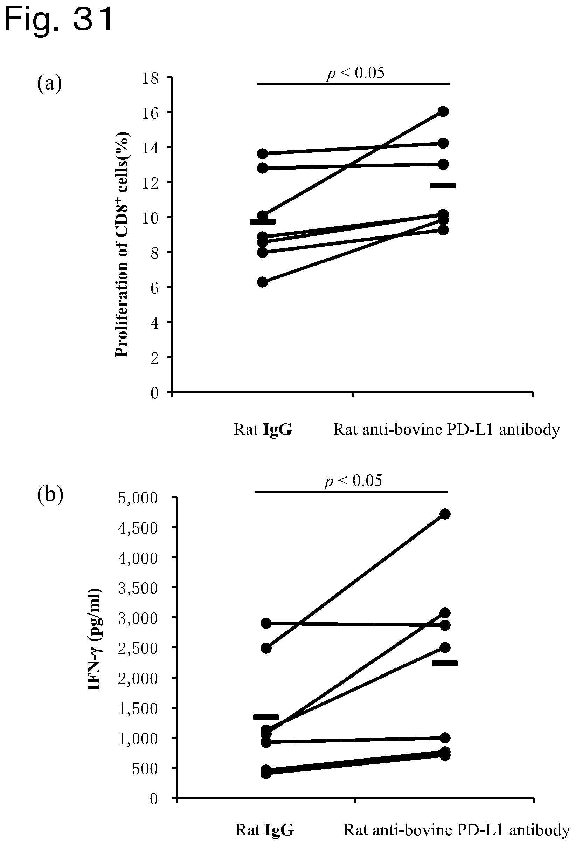

Experimental results are shown in Fig. 31. In the cattle experimentally infected with *M. avium* subsp. *paratuberculosis*, a significant increase was observed in the proliferation rate of CD8⁺ cells (Fig. 30a), IFN-γ production (Fig. 30b) and TNF-α production (Fig. 30c) as a result of the addition of rat anti-bovine PD-L1 antibody. These results indicated that PD-1/PD-L1 blockade has stimulatory effects on J-PPD-specific T-cell responses.

### 2.8. Examination of Combined Effects of COX-2 Inhibitor and Rat Anti-Bovine PD-L1 Antibody on Immunostimulation

Subsequently, the present inventors examined combined effects of COX-2 inhibitor and rat anti-bovine PD-L1 antibody on immunostimulation in cattle experimentally infected with *M. avium* subsp. *paratuberculosis.* Briefly, PBMCs derived from cattle experimentally infected with *M. avium* subsp. *paratuberculosis* were seeded in 96-well plated at 4x10⁵ cells/well and cultured in the presence of J-PPD or a negative control antigen for 5 days. As the negative control antigen, *Mycobacterium bovis* BCG strain-derived purified protein (B-PPD) was used. To the medium, 1,000 nM meloxicam (Sigma-Aldrich) and 1 µg/ml of rat anti-bovine PD-L1 antibody (4G12; Ikebuchi R, Konnai S, Okagawa T, Yokoyama K, Nakajima C, Suzuki Y, Murata S, Ohashi K. Immunology 2014 Aug.; 142(4):551-561) were added to make a total volume 200 µl. As a negative control for meloxicam, DMSO was used. As a negative control antibody, rat serum-derived IgG (Sigma-Aldrich) was used. After culturing, cell proliferation capacity and cytokine production were evaluated in the same manner as described above.

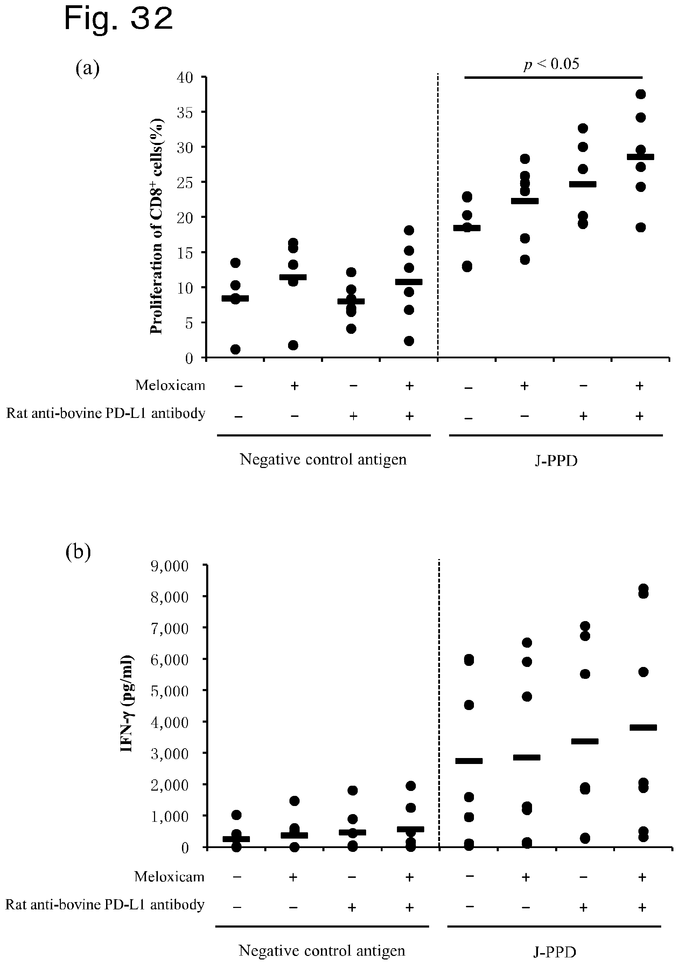

Experimental results are shown in Fig. 32. The proliferation rate of CD8⁺ cells was increased significantly in the groups where meloxicam and rat anti-bovine PD-L1 antibody had been added, as compared to the negative control groups (Fig. 32a). Since no such change was observed in the groups stimulated with the negative control antigen, a possibility was shown that combined use of COX-2 inhibitor and anti-bovine PD-L1 monoclonal antibody stimulates J-PPD-specific CD8⁺ cell responses (Fig. 32a). With respect to IFN-γ production, no significant change was observed whether J-PPD stimulation or negative control antigen stimulation was applied (Fig. 32b). These results showed a possibility that combined use of COX-2 inhibitor and rat anti-bovine PD-L1 antibody will produce a larger immunostimulatory effect in *M. avium* subsp. *paratuberculosis-*infected cattle than when the individual agents are used as single dosages.

### 2.9. Examination of Combined Effects of COX-2 Inhibitor and Rat-Bovine Chimeric Anti-PD-L1 Antibody on Immunostimulation

Finally, the present inventors examined combined effects of COX-2 inhibitor and rat-bovine chimeric anti-PD-L1 antibody on immunostimulation in cattle experimentally infected with *M. avium* subsp. *paratuberculosis.* Briefly, PBMCs derived from cattle experimentally infected with *M. avium* subsp. *paratuberculosis* were seeded in 96-well plated at 4x10⁵ cells/well and cultured in the presence of J-PPD or a negative control antigen for 5 days. As the negative control antigen, B-PPD was used. To the medium, 1,000 nM meloxicam (Sigma-Aldrich) and 1 µg/ml of rat- bovine chimeric anti-PD-L1 antibody (ch4G12; Japanese Patent Application No. 2016-159089, Konnai S, Ohashi K, Murata S, Okagawa T, Nishimori A, Maekawa N, Suzuki S, Nakajima C; Anti-PD-L1 Antibody for Cattle) were added to make a total volume 200 µl. As a negative control for meloxicam, DMSO was used. As a negative control antibody, bovine serum-derived IgG (Sigma-Aldrich) was used. After culturing, cell proliferation capacity and cytokine production were evaluated in the same manner as described above.

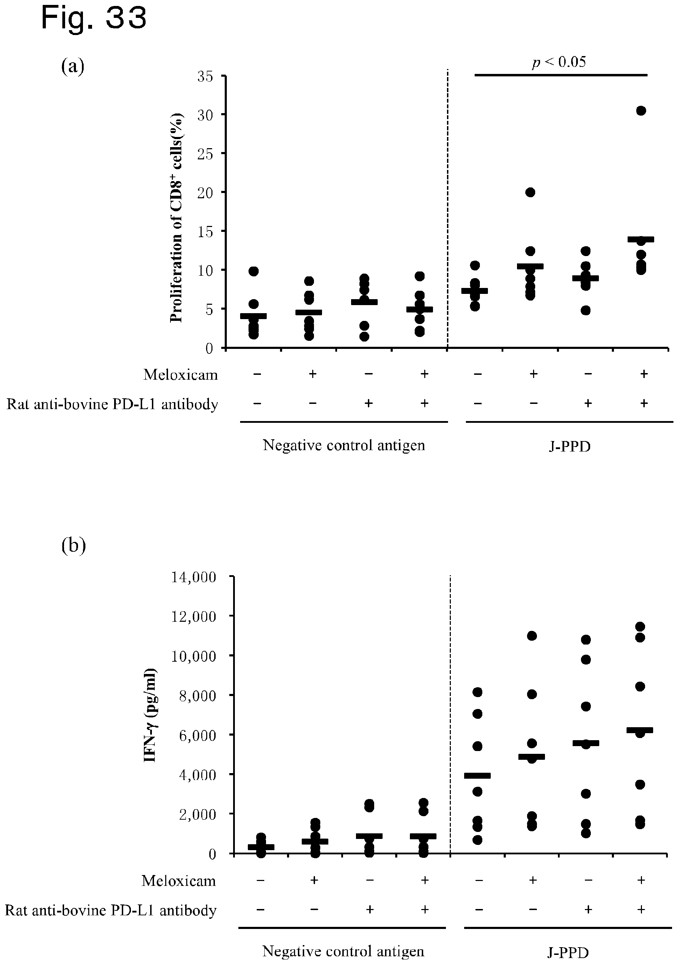

Experimental results are shown in Fig. 33. As a result of evaluation of combined effects in *M. avium* subsp. *paratuberculosis-*infected cattle, a possibility was shown that combined use of COX-2 inhibitor and rat-bovine chimeric anti-PD-L1 antibody stimulates J-PPD-specific CD8⁺ cell responses (Fig. 33a) as in the case of combined use with rat anti-bovine PD-L1 antibody. With respect to IFN-γ production, no significant change was observed whether J-PPD stimulation or negative control antigen stimulation was applied (Fig. 33b). From these results, it is understood that immunostimulatory effects by combined use of COX-2 inhibitor and PD-1/PD-L1 inhibitor were shown even when rat-bovine chimeric anti-PD-L1 antibody was used in *M. avium* subsp. *paratuberculosis-*infected cattle.

### [Example 3]

### Examination of Combined Effects of Anti-Bovine PD-L1 Antibody and COX-2 Inhibitor in Bovine Leukemia Virus-Infected Cattle

### 1. Introduction

The interaction between PD-1 and PD-L1 is one of the major molecular mechanisms through which pathogens evade immune responses. It has been reported that inhibition of the above interaction by using an antibody which specifically binds to either of those molecules can produce anti-pathogenic effects. In the subject Example, toward establishment of a novel control method against bovine leukemia virus (BLV) infection, the present inventors have confirmed in *in vitro* tests an immunostimulatory effect induced by a COX-2 inhibitor and enhancement of that effect when the inhibitor is used in combination with anti-bovine PD-L1 antibody.

### 2. Materials and Methods, as well as Experimental Results

### 2.1. Kinetic Analysis of PGE₂ in BLV-Infected Cattle

In order to elucidate the involvement of PGE₂ in the progression of pathology in BLV infection as a bovine chronic viral infection, the present inventors performed kinetic analysis of PGE₂ in BLV-infected cattle.

### (1) Measurement of Plasma PGE₂ and Analysis of Correlation with Other Indicators

First, the amount of PGE₂ contained in the plasma of BLV-infected cattle was quantified with Prostaglandin E2 Express ELISA Kit (Cayman Chemical). For the measurement, absorbance at 450 nm was measured using a microplate reader MTP-900 (Corona Electric). Further, correlation between the amount of plasma PGE₂ and the number of lymphocytes in peripheral blood or PD-L1 expression rate in IgM⁺ cells was examined. Briefly, PBMCs isolated from BLV-infected cattle were analyzed by flow cytometry to quantify the PD-L1 expression on IgM⁺ cells. First, in order to block non-specific reactions of antibody, PBS supplemented with 10% inactivated goat serum (Thermo Fisher Scientific) was added to each well in an amount of 100 µl and left stationary at room temperature for 15 min. After washing, rat anti-bovine PD-L1 antibody (4G12; Rat IgG2a; Ikebuchi R, Konnai S, Okagawa T, Yokoyama K, Nakajima C, Suzuki Y, Murata S, Ohashi K. Immunology 2014 Aug.; 142(4):551-561) or rat IgG2a isotype control (BD Bioscience) was added and reaction was conducted at room temperature for 20 min. After washing twice, PE/Cy7-labeled anti-IgM monoclonal antibody (IL-A30; Bio-Rad) and APC-labeled anti-rat Ig antibody (Southern Biotech) were added and reaction was conducted at room temperature for 20 min. Anti-IgM monoclonal antibody (IL-A30) was labeled with PE/Cy7 using Lightning-Link Conjugation Kit. After the reaction, washing was performed twice. Then, cells were analyzed with FACS Verse (BD Biosciences) and FCS Express 4 (De Novo Software). For every washing operation and dilution of antibodies, PBS supplemented with 1% bovine serum albumin (Sigma Aldrich) was used.

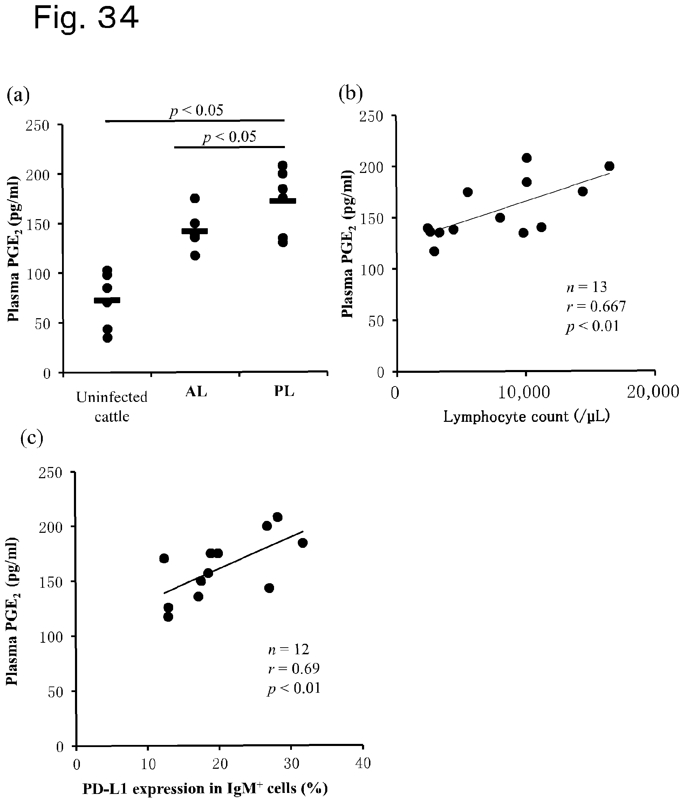

Experimental results of (1) are shown in Fig. 34. As the disease stage of BLV infection progressed (AL: aleukemic, asymptomatic; PL: persistent lymphocytosis), plasma PGE₂ increased significantly (Fig. 34a). As a result of examination of correlation between the number of lymphocytes in peripheral blood (an indicator of the disease progression in BLV infection) and plasma PGE₂, a positive correlation was observed (Fig. 34b). Further, as a result of examination of correlation between plasma PGE₂ and PD-L1 expression rate in IgM⁺ cells, a positive correlation was shown (Fig. 34c). These results suggested that PGE₂ is involved in the disease progression in BLV infection and the resultant immunosuppression.

### (2) Expression Analysis of COX2 and EP4 in BLV-Infected Cattle

For more detailed analysis, in addition to plasma PGE₂, expression levels of *COX2* (i.e., involved in PGE₂ synthesis) and the gene of EP4 (a PGE₂ receptor that transmits immunosuppressive signals) were quantified by real-time PCR. Briefly, total cellular RNA was extracted from PBMCs, CD4⁺ cells, CD8⁺ cells, CD14⁺ cells and CD21⁺ cells derived from BLV-infected cattle and uninfected cattle in the same manner as described in (3), section 2.1 of Example 2, and cDNA was synthesized. Using the synthesized cDNA as a template, real-time PCR was performed with *COX2*-specific primers (already described in (3), section 2.3 of Example 2) and *EP4*-specific primers in the same manner as described in Example 2.
Primer (boEP4 F): GTG ACC ATC GCC ACC TAC TT (SEQ ID NO: 141) Primer (boEP4 R): CTC ATC GCA CSG ATG ATG CT (SEQ ID NO: 142)

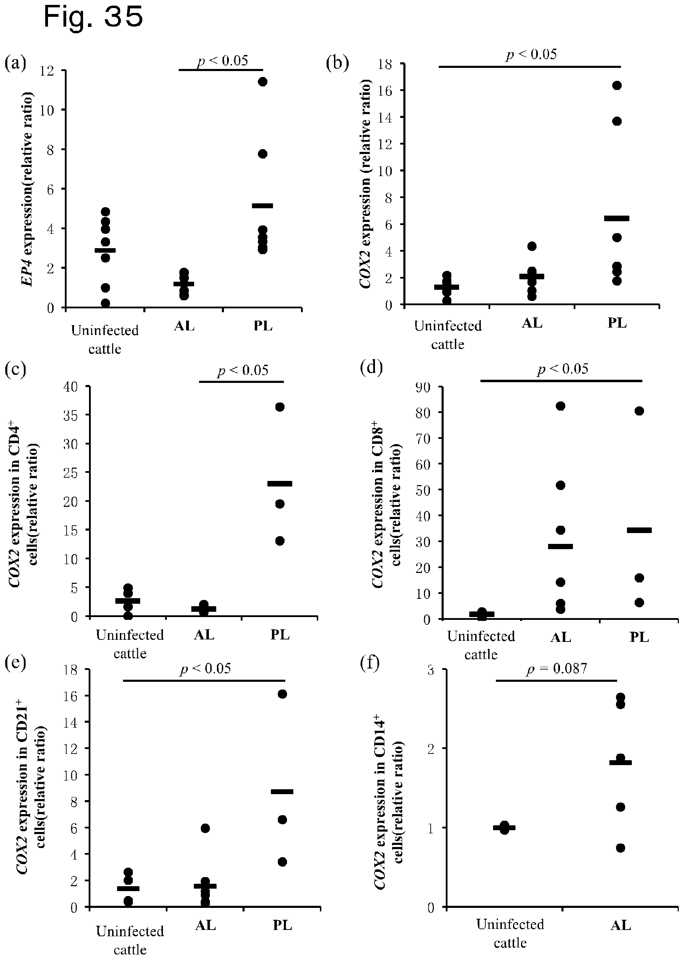

Experimental results of (2) are shown in Fig. 35. It was confirmed that *EP4* expression level increased in cattle with PL (an advanced stage of the disease) (hereinafter, referred to as "PL cattle") (Fig. 35a). The same is true for *COX2* expression levels, which also increased in PL cattle (Fig. 35b). In order to examine PGE₂ producing cells, *COX2* expression in CD4⁺, CD8⁺, CD14⁺ and CD21⁺ cells was quantified. As it turned out, *COX2* expression clearly increased in CD4⁺, CD8⁺ and CD21⁺ cells in PL cattle (Fig. 35c-e). With respect to CD14⁺ cells, evaluation using samples from PL cattle is yet to be performed, but *COX2* expression showed a tendency to increase in AL cattle compared to uninfected cattle (Fig. 35f). These results showed that *COX2* expression increases in various cell groups as the disease stage of BLV infection progresses.

### (3) Changes in PGE₂ Production by BLV Antigen Stimulation

It has been reported that *COX2* expression is increased by antigen stimulation in BLV infection (Pyeon D, Diaz FJ, Splitter GA. J Virol. 74:5740-5745, 2000). From this report, it is predicted that PGE₂ production by PBMCs derived from BLV-infected cattle will also be promoted by antigen stimulation. To verify this hypothesis, the present inventors cultured PBMCs with BLV antigen and quantified PGE₂ in the culture supernatant by ELISA. Briefly, PBMCs derived from BLV-infected cattle and those derived from uninfected cattle were seeded in 96-wel plates at 4x10⁵ cells/well and cultured in the presence of BLV antigen (final concentration 2%) for 6 days. As the BLV antigen, a culture supernatant of fetal lamb kidney cells persistently infected with BLV (FLK-BLV) was used after heat treatment at 65°C. In order to confirm that PGE₂ production by antigen stimulation is inhibited by COX-2 inhibitor, cells were also cultured under such conditions that both BLV antigen and 1,000 nM meloxicam (Sigma Aldrich) were added to the medium. After 6 days, culture supernatant was collected and PGE₂ contained in it was quantified with Prostaglandin E2 Express ELISA Kit (Cayman Chemical).

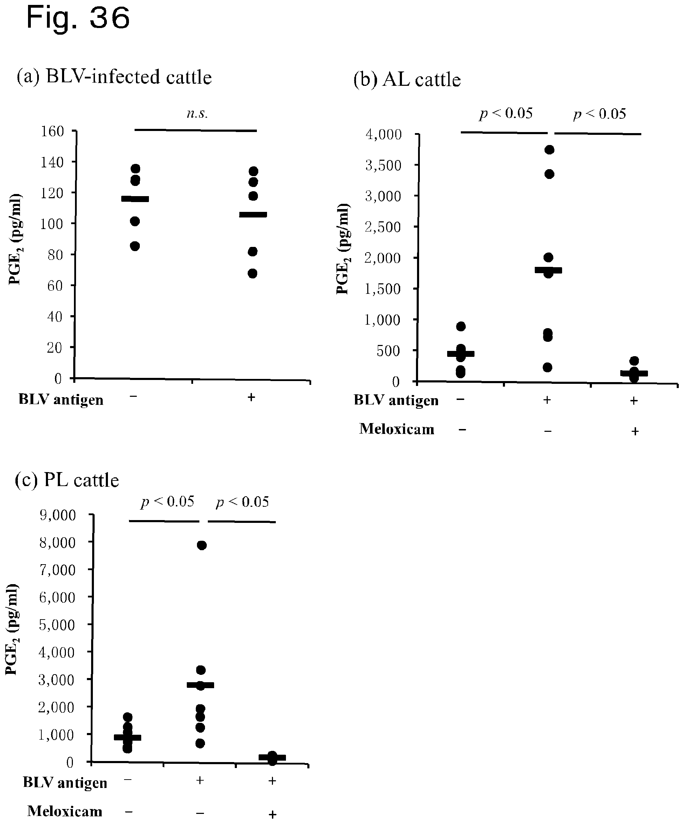

Experimental results of (3) are shown in Fig. 36. In BLV-infected cattle (AL: Fig. 36b; PL: Fig. 36c), PGE₂ production from PBMCs was shown to be significantly promoted by addition of BLV antigen (Fig. 36b, c). No significant differences were observed in uninfected cattle (Fig. 36a). Thus, it has been demonstrated that the induction of PGE₂ production stimulated by the BLV antigen in PBMCs derived from BLV-infected cattle is a response specific to BLV. Further, it was confirmed that PGE₂ production is significantly inhibited by culture in the presence of an added COX-2 inhibitor under the above-described conditions (Fig. 36b, c).

### (4) Effect of PGE₂ on BLV Provirus Load

In various chronic infections, a possibility has been suggested that PGE₂ promotes viral replication (Pyeon D, Diaz FJ, Splitter GA. J Virol. 74:5740-5745, 2000; Waris D, Siddiqui A. J Virol. 79:9725-9734, 2005). Then, in order to evaluate the effect of PGE₂ on viral replication in BLV infection, the present inventors cultured PBMCs with PGE₂ and quantified BLV provirus load by real-time PCR. Briefly, PBMCs derived from BLV-infected cattle were seeded in 96-well plates at 1x10⁶ cells/well and cultured in the presence of 2,500 nM PGE₂ or DMSO for 3 days. After culturing, DNA was extracted from harvested PBMCs with Wizard DNA Purification kit (Promega). The concentration of the extracted DNA was quantified by measuring the absorbance (260 nm) with Nanodrop 8000 Spectrophotometer (Thermo Fisher Scientific). For measuring BLV provirus load in PBMCs, real-time PCR was performed using Cycleave PCR Reaction Mix SP (TaKaRa) and Probe/Primer/Positive control for detecting bovine leukemia virus (TaKaRa). LightCycler480 System II (Roche Diagnosis) was used for measurement.

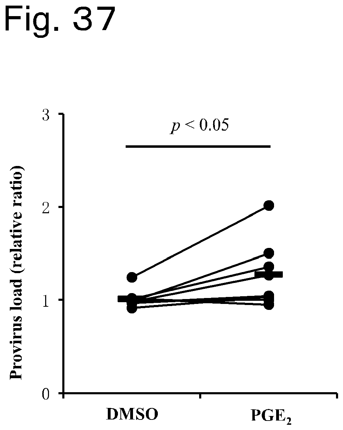

Experimental results of (4) are shown in Fig. 37. Provirus loads were shown to increase significantly in PGE₂-treated group (Fig. 37), suggesting a possibility that PGE₂ promotes BLV replication.

### 2.2. Changes in PD-L1 Expression by BLV Antigen Stimulation

Effects of BLV antigen stimulation on PD-L1 expression in BLV-infected cattle were evaluated. Briefly, PBMCs derived from BLV-infected and those from uninfected cattle were seeded in 96-well plates at 1x10⁶ cells/well and cultured in the presence of BLV antigen (final concentration 2%) for 24 hours. After culturing, PBMCs were harvested, and PD-L1 expression on lymphocytes, CD4⁺ T cells, CD8⁺ T cells, IgM⁺ cells and CD14⁺ cells was analyzed by flow cytometry in the same manner as described in section 2.4 of Example 2.

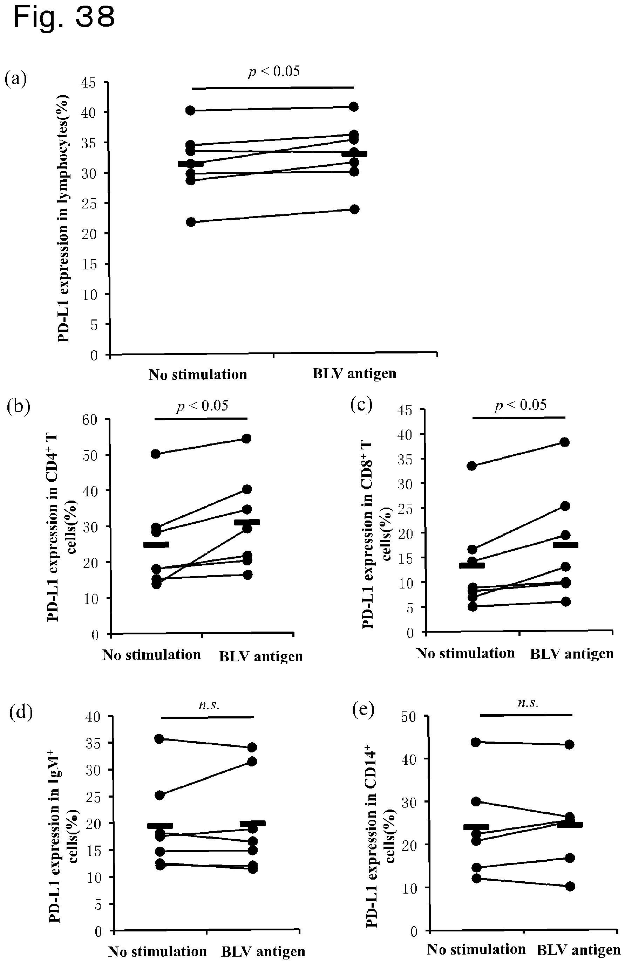

Experimental results of 2.2 are shown in Fig. 38. PD-L1 expression was shown to increase significantly in lymphocytes when PBMCs from BLV-infected cattle were stimulated with BLV antigen (Fig. 38a). Further, changes in PD-L1 expression in individual cell groups (CD4⁺ T cells, CD8⁺ T cells, IgM⁺ cells and CD14⁺ cells) were analyzed to reveal that PD-L1 expression was increased in CD4⁺ cells and CD8⁺ cells by BLV antigen stimulation (Fig. 38b-e).

### 2.3. Examination of Stimulatory Effects of COX-2 Inhibitor on BLV-Specific Immune Responses

In order to confirm that COX-2 inhibitor has stimulatory effects on BLV antigen-specific immune responses, the present inventors evaluated the proliferation capacity and cytokine production capacity of PBMCs that were cultured in the presence of meloxicam and BLV antigen. Briefly, PBMCs derived from BLV-infected cattle were seeded in 96-well plated at 4x10⁵ cells/well and cultured under stimulation in the presence of BLV antigen (final concentration 2%) and 1,000 nM meloxicam (Sigma-Aldrich) for 6 days. After culturing, cell proliferation capacity and cytokine production capacity were evaluated in the same manner as described in Example 2.

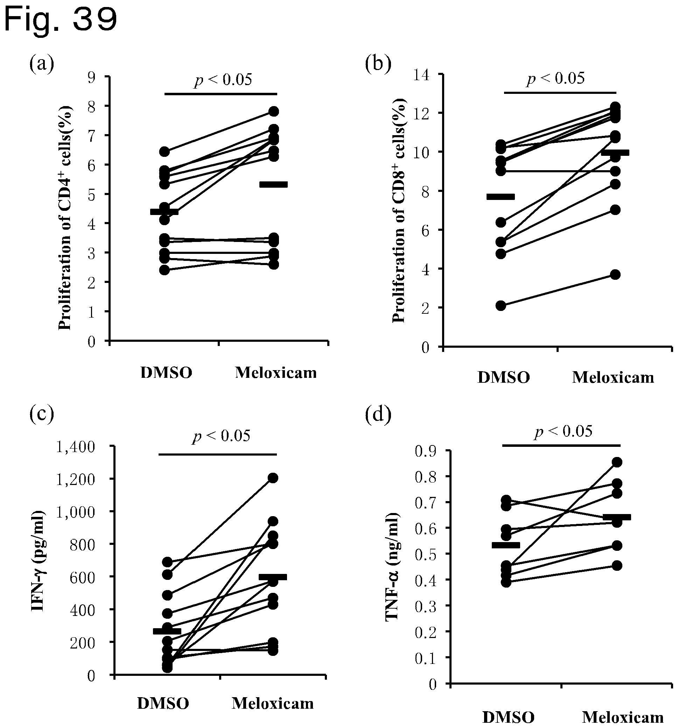

Experimental results are shown in Fig. 39. In the cattle experimentally infected with BLV, a significant increase was observed in proliferation rate of CD4⁺ cells (Fig. 39a), proliferation rate of CD8⁺ cells (Fig. 39b), IFN-γ production (Fig. 39c) and TNF-α production due to the addition of meloxicam. These results indicated that COX-2 inhibitor has stimulatory effects on BLV antigen-specific T cell responses.

### 2.3. Examination of Antiviral Effects of COX-2 Inhibitor in BLV-Infected Cattle

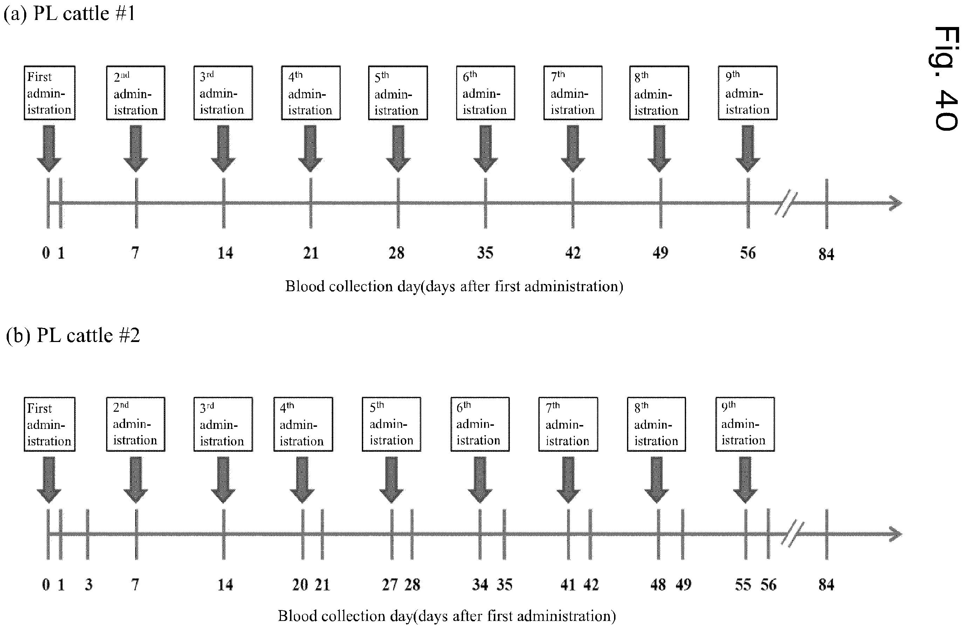

In order to examine the antiviral effects of COX-2 inhibitor *in vivo,* the present inventors conducted a clinical application test using BLV-infected cattle. Two individuals (#1 and #2) of PL cattle of Holstein species were used in the test. The body weight and age at the beginning of the test were 736 kg and 8 years and 1 month for #1 and 749 kg and 3 years and 7 months for #2. As a COX-2 inhibitor, Metacam™ 2% injection (hereinafter, referred to as "Metacam™"; Kyoritsu Seiyaku) was inoculated subcutaneously at 0.5 mg/kg. In addition to the first inoculation, individual #1 received inoculation 7, 14, 21, 28, 35, 42, 49 and 56 days after the first inoculation; and individual #2 received inoculation 7, 14, 20, 27, 34, 41, 48 and 55 days after the first inoculation (Fig. 40a, b). Blood collection was performed on each inoculation day, and 1 day and 84 days after the first inoculation for individual #1; and for individual #2, each inoculation day, the day after each inoculation, and 3 days and 84 days after the first inoculation (Fig. 40a, b). Blood collection on each inoculation day was conducted before Metacam™ inoculation. Using the collected blood samples, BLV provirus loads, serum PGE₂ concentrations and IFN-γ concentrations were quantified. Provirus loads were quantified in the same manner as described in (4), section 2.1 above. Serum PGE₂ concentrations and IFN-γ concentrations were measured with Prostaglandin E2 Express ELISA Kit (Cayman Chemical) and ELISA for Bovine IFN-γ (MABTECH), respectively.

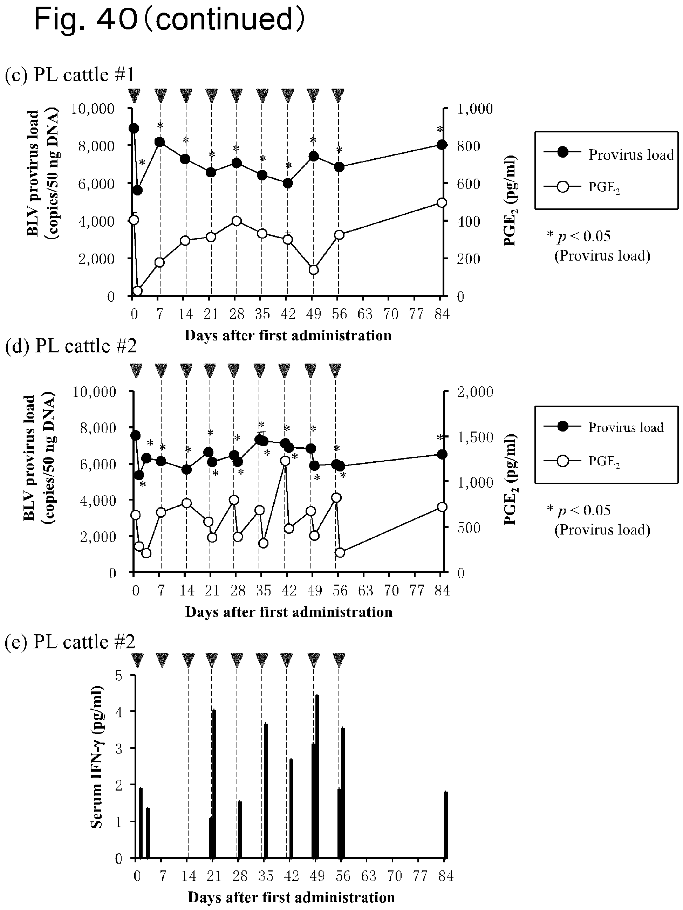

Experimental results are shown in Fig. 40. As a result of Metacam™ administration, BLV provirus load decreased significantly in both #1 and #2 (Fig. 40c, d). Serum PGE₂ concentration decreased on the day after Metacam™ administration, and provirus load also decreased on the day after Metacam™ administration (Fig. 40c, d). Further, in #2, serum IFN-γ concentration increased on the day after Metacam™ administration (Fig. 40e). In #1, serum IFN-γ concentration was below the measurement limit of ELISA. These results indicated that COX-2 inhibitor has antiviral effects in BLV-infected cattle *in vivo.*

### 2.4. Examination of Immunostimulatory Effects due to Combined Use of COX-2 Inhibitor and Rat Anti-Bovine PD-L1 Antibody

Provirus loads in BLV-infected cattle decreased upon administration of a COX-2 inhibitor (Fig. 40c, d). In order to obtain stronger antiviral effects, the present inventors examined immunostimulatory effects of combined use of COX-2 inhibitor and anti-bovine PD-L1 antibody in BLV-infected cattle. Briefly, PBMCs derived from BLV-infected cattle were seeded in 96-well plates at 4x10⁵ cells/well and cultured in the presence of BLV antigen or a negative control antigen for 6 days. As the negative control antigen, a culture supernatant of BLV-uninfected fetal lamb kidney cells (FLK) was used after heat treatment at 65°C. To the medium, 1,000 nM meloxicam (Sigma-Aldrich) and 1 µg/ml of rat anti-bovine PD-L1 antibody (4G12; Ikebuchi R, Konnai S, Okagawa T, Yokoyama K, Nakajima C, Suzuki Y, Murata S, Ohashi K. Immunology 2014 Aug.; 142(4):551-561) were added to make a total volume 200 µl. As a negative control for meloxicam, DMSO was used; and as a negative control antibody, rat serum derived IgG (Sigma-Aldrich) was used. After culturing, cell proliferation capacity and cytokine production capacity were evaluated in the same manner as described above.

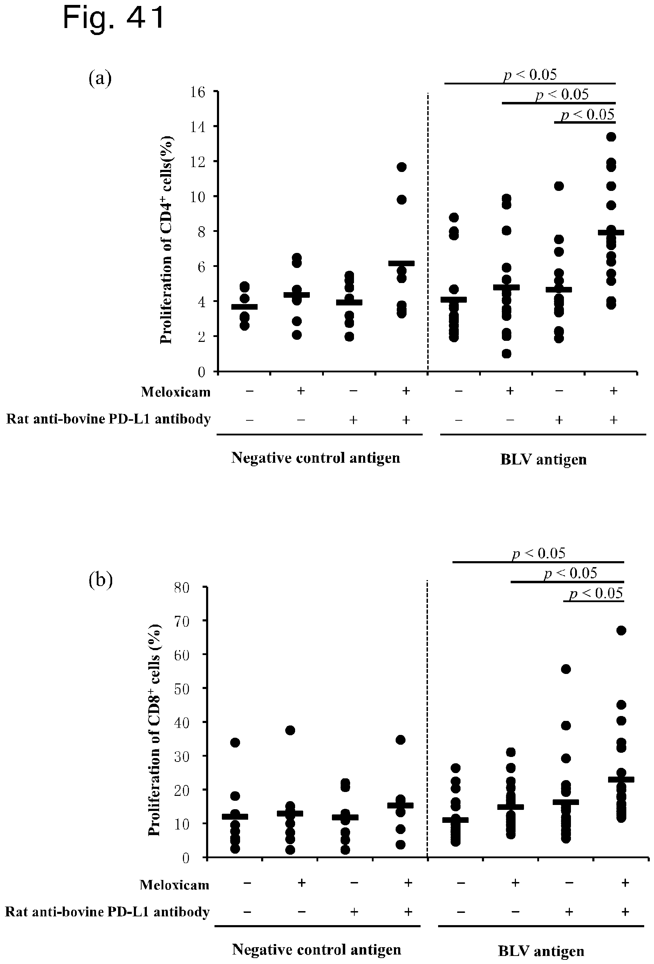

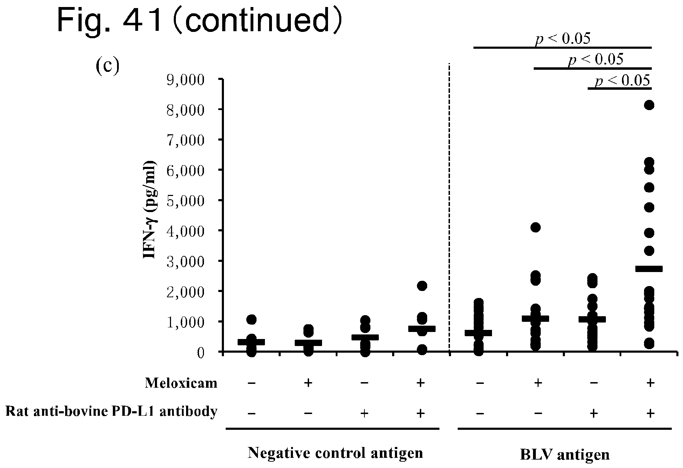

Experimental results are shown in Fig. 41. When PBMCs were stimulated with BLV antigen, the group in which both meloxicam and rat anti-bovine PD-L1 antibody were added showed significant increases in proliferation rate of CD4⁺ cells (Fig. 41a), proliferation rate of CD8⁺ cells (Fig. 41b) and IFN-γ production (Fig. 41c), as compared to the negative control group, the meloxicam alone group and the rat anti-bovine PD-L1 antibody alone group. No such changes were observed when PBMCs were stimulated with the negative control antigen. Therefore, it was suggested that BLV antigen-specific T cell responses are activated by combined use of COX-2 inhibitor and rat anti-bovine PD-L1 antibody (Fig. 41a-c). From these results, a possibility was shown that a greater immunostimulatory effect may be obtained from combined use of COX-2 inhibitor and rat anti-bovine PD-L1 antibody in BLV-infected cattle than when the inhibitor or the antibody is used alone.

### 2.5. Examination of Immunostimulatory Effects due to Combined Use of COX-2 Inhibitor and Rat-Bovine Chimeric Anti-PD-L1 Antibody

Finally, the present inventors examined immunostimulatory effects due to combined use of COX-2 inhibitor and rat-bovine chimeric anti-PD-Ll antibody in BLV-infected cattle. Briefly, PBMCs derived from BLV-infected cattle were seeded in 96-well plates at 4x10⁵ cells/well and cultured in the presence of BLV antigen or a negative control antigen for 6 days. As the negative control antigen, a culture supernatant of BLV-uninfected fetal lamb kidney cells (FLK) was used after heat treatment at 65°C. To the medium, 1,000 nM meloxicam (Sigma-Aldrich) and 1 µg/ml of rat-bovine chimeric anti-PD-Ll antibody (ch4G12; Japanese Patent Application No. 2016-159089, Konnai S, Ohashi K, Murata S, Okagawa T, Nishimori A, Maekawa N, Suzuki S, Nakajima C; Anti-PD-Ll Antibody for Cattle) were added to make a total volume of 200 µl. As a negative control for meloxicam, DMSO was used. As a negative control antibody, bovine serum-derived IgG (Sigma-Aldrich) was used. After culturing, cell proliferation capacity and cytokine production were evaluated in the same manner as described above.

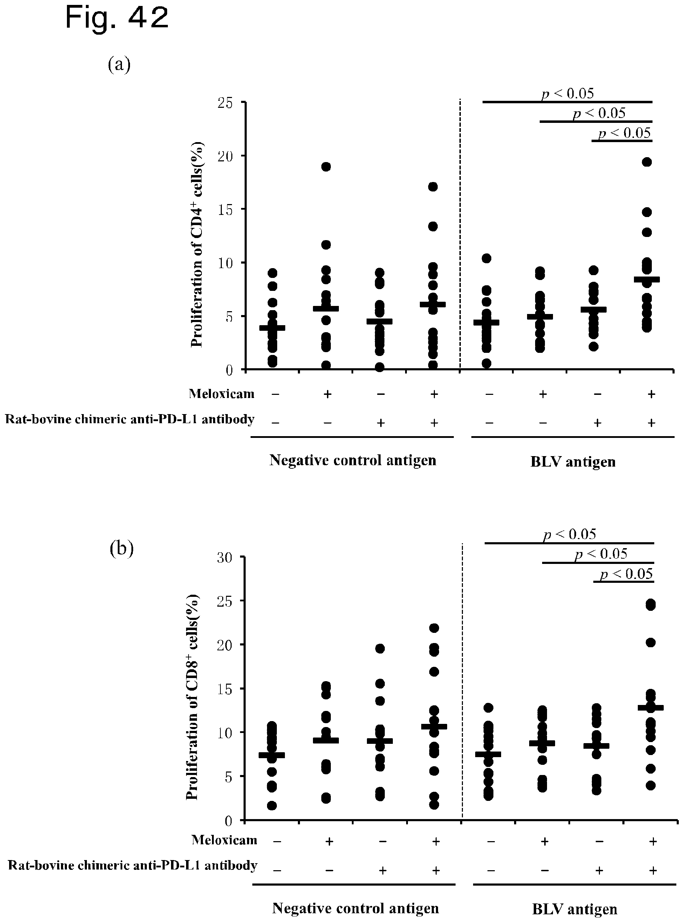

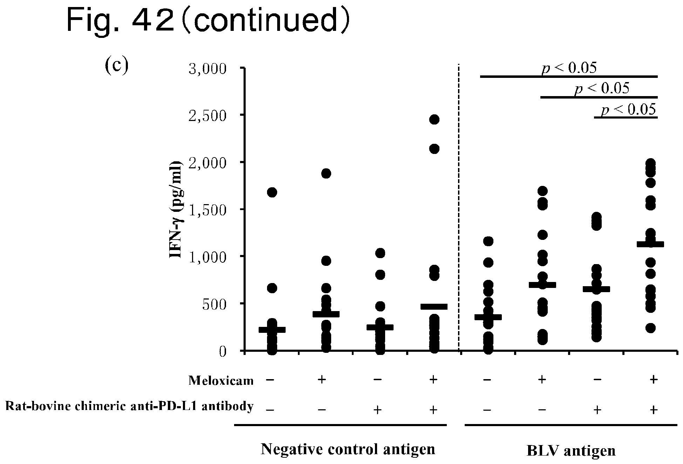

Experimental results are shown in Fig. 42. In BLV-infected cattle, BLV antigen-specific CD4⁺ cell response, CD8⁺ cell response and IFN-γ production were activated by combined use of COX-2 inhibitor and rat-bovine chimeric anti PD-L1 antibody (Fig. 42a-c), as seen in the case where rat anti-bovine PD-L1 antibody was used. Thus, in BLV-infected cattle, the immunostimulatory effect of combined use of COX-2 inhibitor and PD-1/PD-L1 inhibitor was also shown when rat-bovine chimeric anti-PD-Ll antibody was used.

### 2.6. Examination of In Vivo Antiviral Effect due to Combined Use of COX-2 Inhibitor and Rat-Bovine Chimeric Anti-PD-L1 Antibody

In order to examine the *in vivo* antiviral effect of combined use of COX-2 inhibitor and PD-1/PD-L1 inhibitor, the present inventors conducted a clinical application test using BLV-infected cattle. Two individuals (#1719 and #2702; Holstein species) of BLV-infected cattle with a high BLV provirus load were used in the test. The body weight and age at the beginning of the test were 799 kg and 7 years and 4 months for #1719 and 799 kg and 4 years and 3 months for #2702. As a COX-2 inhibitor, Metacam™ 2% injection (hereinafter, referred to as "Metacam™"; Kyoritsu Seiyaku) was inoculated subcutaneously at 0.5 mg/kg. As a PD-1/PD-L1 inhibitor, rat-bovine chimeric anti-PD-Ll antibody (ch4G12; WO2018/034225, Konnai S, Ohashi K, Murata S, Okagawa T, Nishimori A, Maekawa N, Suzuki S, Nakajima C; Anti-PD-L1 Antibody for Cattle) was administered intravenously at 1.0 mg/kg. In addition to the first inoculation, Metacam™ was inoculated 7 and 14 days after the first inoculation. Blood collection was performed 7 days before the antibody administration (at -7 day); at the antibody administration day; at day 1, day 3 and day 7 after the antibody administration; and once weekly from day 14 to day 58 after the antibody administration. Blood collection on antibody/Metacam™ administration day (at day 0) and Metacam™ administration days (at day 7 and day 14) was carried out before administration of antibody and Metacam™. Using the collected blood samples, BLV provirus loads were quantified. Provirus loads were quantified in the same manner as described in (4), section 2.1 above.

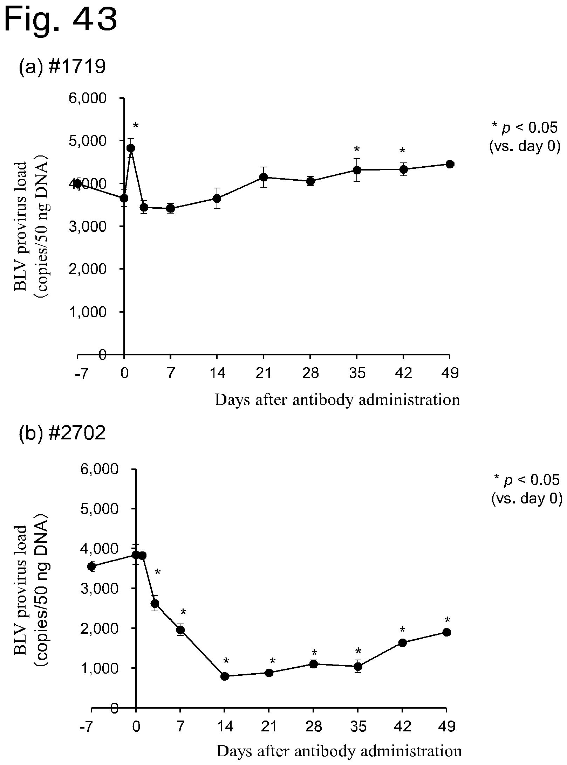

Experimental results are shown in Fig. 43. BLV provirus load on the antibody administration day (immediately before antibody administration) was 3,662 copies/50 ng DNA in #1719 and 3,846 copies/50 ng DNA in #2702. In #1719 which received rat-bovine chimeric anti-PD-L1 antibody alone, no decrease of BLV provirus load was recognized (Fig. 43a), whereas in #2702 which received a combination of Metacam™ and rat-bovine chimeric anti-PD-L1 antibody, significant decreases of BLV provirus load were recognized from day 3 to day 49 after administration (Fig. 43b). These results showed that *in vivo* antiviral effect is enhanced by combined use of COX-2 inhibitor and PD-1/PD-L1 inhibitor. Such combined effect will be obtained when BLV provirus load is about 3,846 copies/50 ng DNA or less. It should be noted here that this value is based on the BLV provirus load measured in the same manner as described in (4), section 2.1 above.

### [Example 4]

### Examination of Combined Effect of Anti-Bovine PD-L1 Antibody and COX-2 Inhibitor in Mycoplasma bovis-Infected Cattle

### 1. Introduction

The interaction between PD-1 and PD-L1 is one of the major molecular mechanisms through which pathogens evade immune responses. It has been reported that inhibition of the above interaction by using an antibody which specifically binds to either of those molecules can produce anti-pathogenic effects. In the subject Example, toward establishment of a novel control method against bovine mycoplasma infections caused by *Mycoplasma bovis*, the present inventors have confirmed in *in vitro* tests, an immunostimulatory effect induced by COX-2 inhibitor and enhancement of that effect when the inhibitor is used in combination with anti-bovine PD-L1 antibody.

### 2. Materials and Methods, as well as Experimental Results

### 2.1. Analysis of Serum PGE₂ in M. bovis-Infected Cattle

In order to elucidate the involvement of PGE₂ in the disease progression of bovine mycoplasmosis caused by *Mycoplasma bovis*, the present inventors performed kinetic analysis of PGE₂ in *M. bovis-*infected cattle. The amounts of PGE₂ contained in the serum of *M. bovis-*infected cattle and *M. bovis*-uninfected cattle (hereinafter, referred to as "uninfected cattle") were quantified with Prostaglandin E2 Express ELISA Kit (Cayman Chemical). For measurement, absorbance at 450 nm was measured using a microplate reader MTP-900 (Corona Electric).

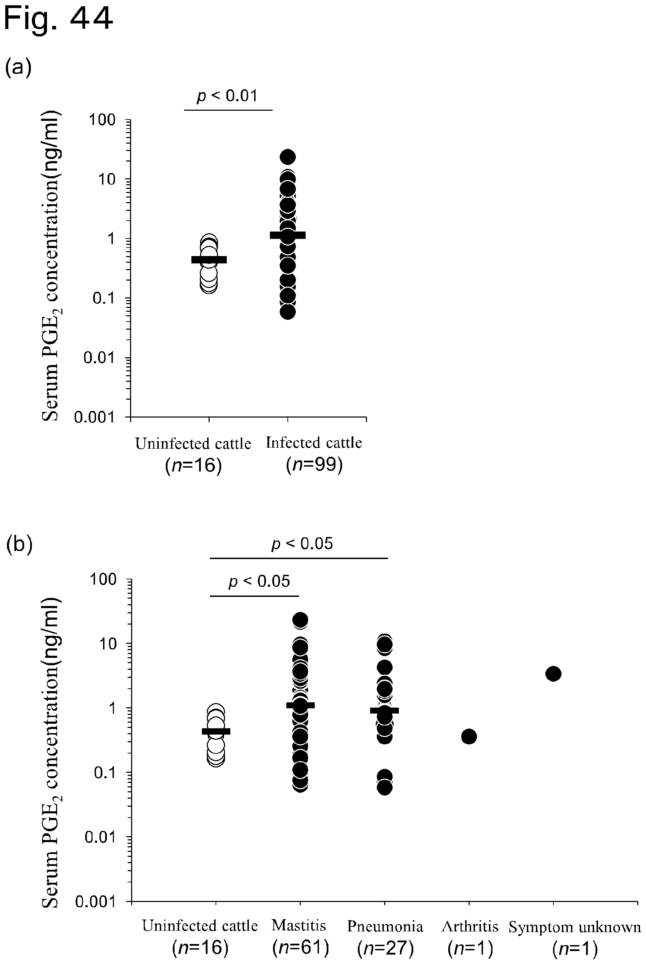

Experimental results are shown in Fig. 44. Serum PGE₂ was significantly higher in *M. bovis-*infected cattle than in uninfected cattle (Fig. 44a). Further, *M. bovis-*infected cattle were classified into groups by clinical symptom, and the concentrations of serum PGE₂ were compared between groups. Those individuals which presented mastitis and pneumonia due to *M. bovis* showed significantly high PGE₂ levels in serum as compared to the uninfected cattle (Fig. 44b). These results suggested a possibility that PGE₂ is involved in the disease progression of bovine mycoplasmosis.

### 2.2. Correlation Analysis between Plasma PGE₂ and Indicators of Immune Responses

Subsequently, the present inventors examined correlation between the amount of plasma PGE₂ in *M. bovis-*infected cattle and *M. bovis*-specific IFN-γ responses or PD-L1 expression rate in CD14⁺ cells. First, plasma was isolated from the blood of *M. bovis-*infected cattle, and the amount of PGE₂ contained in the plasma was measured as described in 2.1 above. Subsequently, peripheral blood mononuclear cells (PBMCs) isolated from the blood of *M. bovis-*infected cattle were suspended in RPMI 1640 medium (Sigma-Aldrich) supplemented with 10% inactivated fetal bovine serum (Thermo Fisher Scientific), antibiotics (streptomycin 100 µg/ml, penicillin 100 U/ml) (Thermo Fisher Scientific) and 2 mM L-glutamine (Thermo Fisher Scientific) and seeded in 96-well plates (Corning) at 4x10⁵ cells/well. Then, 1.5 µg/ml of *M. bovis* antigen was added and cells were cultured under stimulation at 37°C in the presence of 5% CO₂ for 5 days. As *M. bovis* antigen, *M. bovis* PG45 strain (ATCC 25523; kindly provided by Prof. Hidetoshi Higuchi, Rakuno Gakuen University) was used after heat treatment. After 5 days, culture supernatant was collected and IFN-γ production was measured with ELISA for Bovine IFN-γ (MABTECH). For the measurement, absorbance at 450 nm was measured using a microplate reader MTP-900 (Corona Electric).

Further, PD-L1 expression on CD14⁺ cells was measured by flow cytometry analysis of PBMCs derived from *M. bovis-*infected cattle. First, in order to block non-specific reactions of antibody, PBS supplemented with 10% inactivated goat serum (Thermo Fisher Scientific) was added to each well in an amount of 100 µl and left stationary at room temperature for 15 min. After washing, rat anti-bovine PD-L1 antibody (4G12; Rat IgG2a; Ikebuchi R, Konnai S, Okagawa T, Yokoyama K, Nakajima C, Suzuki Y, Murata S, Ohashi K. Immunology 2014 Aug.; 142(4):551-561) or rat IgG2a isotype control (BD Bioscience) was added and reaction was conducted at room temperature for 20 min. After washing twice, PerCp/Cy5.5-labeled anti-CD14 monoclonal antibody (CAM36A; Washington State University Monoclonal Antibody Center) and APC-labeled anti-rat Ig antibody (Southern Biotech) were reacted with cells. Anti-CD14 monoclonal antibody (CAM36A) was labeled with PerCp/Cy5.5 using Lightning-Link Conjugation Kit. After the reaction, washing was performed twice. Then, cells were analyzed with FACS Verse (BD Biosciences) and FCS Express 4 (De Novo Software). For every washing operation and dilution of antibodies, PBS supplemented with 1% bovine serum albumin (Sigma Aldrich) was used.

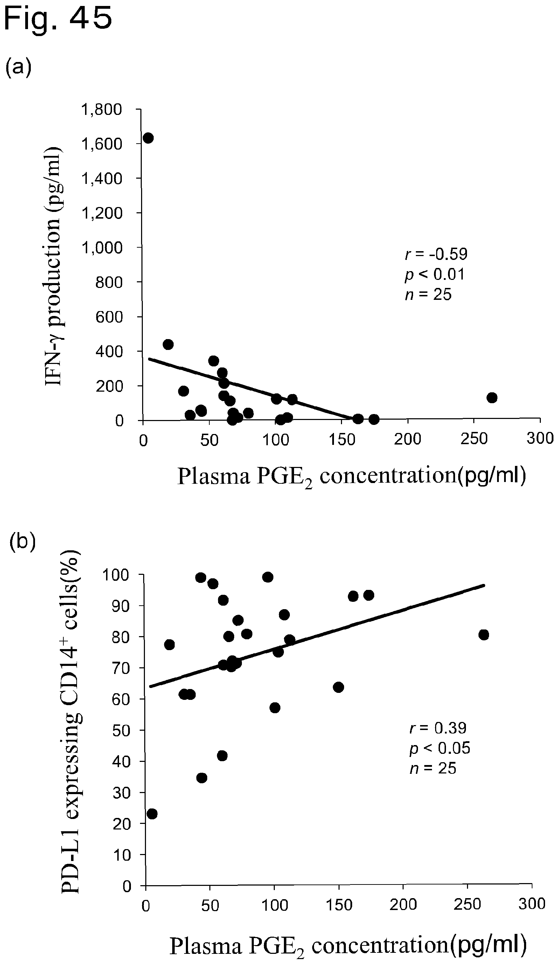

Experimental results are shown in Fig. 45. In *M. bovis-*infected cattle, a negative correlation was recognized between plasma PGE₂ and *M. bovis*-specific IFN-γ response (Fig. 45a). Further, positive correlation was observed between plasma PGE₂ and PD-L1 expression in CD14⁺ cells (Fig. 45b). These results suggested that PGE₂ is involved in immunosuppression resulting from bovine mycoplasmosis.

### 2.3. Expression Analysis of COX2 and EP4 in M. bovis-Infected Cattle

For more detailed analysis, expression levels of *COX2* (involved in PGE₂ synthesis) and the gene of EP4 (a PGE₂ receptor that transmits immunosuppressive signals) were quantified by real-time PCR. Briefly, total cellular RNA was extracted from PBMCs derived from *M. bovis-*infected cattle and those from uninfected cattle in the same manner as described in (3), section 2.1 of Example 2, and cDNA was synthesized. Using the synthesized cDNA as a template, real-time PCR was performed with *COX2*-specific primers (described in (3), section 2.3 of Example 2) and *EP4*-specific primers (described in (2), section 2.1 of Example 3) according to the method described in Example 2.

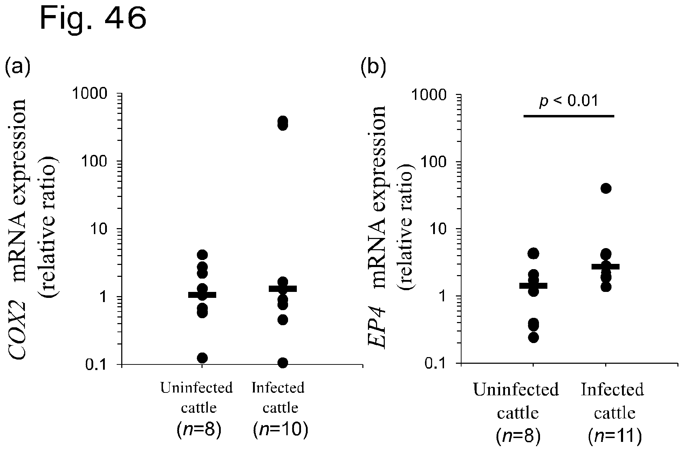

Experimental results are shown in Fig. 46. Although PBMCs from *M. bovis-*infected cattle showed no significant difference in *COX2* expression as compared to those from uninfected cattle (Fig. 46a), they showed significant increases in *EP4* expression (Fig. 46b).

### 2.4. Examination of Immunostimulatory Effects due to Combined Use of COX-2 Inhibitor and Rat Anti-Bovine PD-L1 Antibody in M. bovis-Infected Cattle

Finally, the present inventors examined immunostimulatory effects due to combined use of COX-2 inhibitor and anti-bovine PD-L1 antibody in *M. bovis-*infected cattle. Briefly, PBMCs derived from *M. bovis-*infected cattle were seeded in 96-well plates (Corning) at 4x10⁵ cells/well and cultured in the presence of 1.5 µg/ml of *M. bovis* antigen (as antigen-specific stimulant) or 2 µg/ml each of anti-CD3 monoclonal antibody (MM1A; Washington State University Monoclonal Antibody Center) and anti-CD28 monoclonal antibody (CC220; Bio-Rad) (as T cell stimulants) for 5 days. To the medium, 10 µM meloxicam (Sigma-Aldrich) and 10 µg/ml of rat anti-bovine PD-L1 antibody (4G12; Ikebuchi R, Konnai S, Okagawa T, Yokoyama K, Nakajima C, Suzuki Y, Murata S, Ohashi K. Immunology 2014 Aug.; 142(4):551-561) were added. As a negative control for meloxicam, DMSO was used; and as a negative control antibody, rat serum-derived IgG (Sigma-Aldrich) was used. After culturing, cell proliferation capacity and cytokine production were evaluated in the same manner as described above.

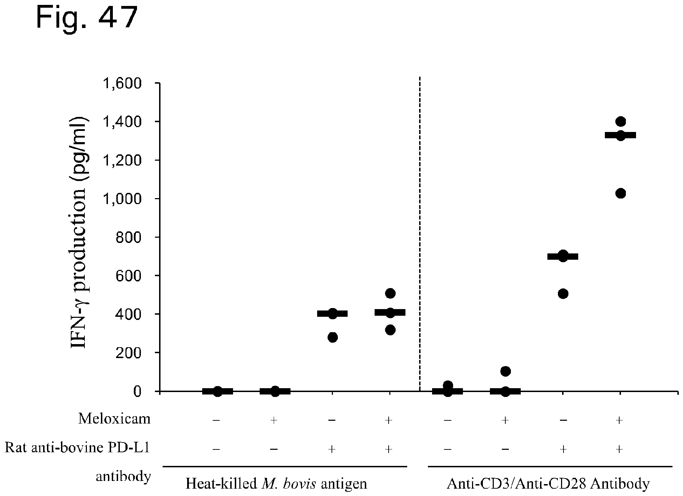

Experimental results are shown in Fig. 47. Under stimulation with *M. bovis* antigen, IFN-γ production tended to increase in the group which received rat anti-bovine PD-L1 antibody alone or in the group which received the combination of meloxicam and rat anti-bovine PD-L1 antibody, compared to the negative control group and the group that received meloxicam alone (Fig. 47). Under stimulation with anti-CD3 antibody and anti-CD28 antibody, IFN-γ production tended to increase in the group which received rat anti-bovine PD-L1 antibody alone, compared to the negative control group and the group that received meloxicam alone. In the group which received the combination of meloxicam and rat anti-bovine PD-L1 antibody, IFN-γ production was further enhanced although no significant difference was recognized (Fig. 47). These results suggested that immunostimulatory effect is more strongly induced in *M. bovis-*infected cattle by combined use of COX-2 inhibitor and rat anti-bovine PD-L1 antibody.

### [Reference Example 2]

### Rat-Bovine Chimeric Anti-PD-L1 Antibody

### 1. Introduction

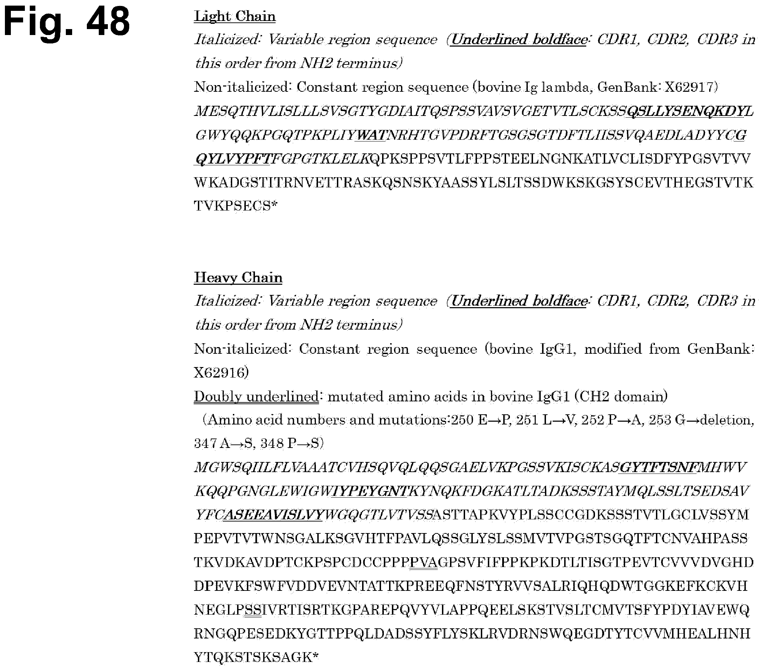

Programmed cell death 1 (PD-1), an immunoinhibitory receptor, and its ligand programmed cell death ligand 1 (PD-L1) are molecules identified by Prof. Tasuku Honjo et al., Kyoto University, as factors which inhibit excessive immune response and are deeply involved in immunotolerance. Recently, it has been elucidated that these molecules are also involved in immunosuppression in tumors. In the subject Example, for the purpose of establishing a novel therapy for bovine infections, the present inventors have prepared a chimeric antibody gene by linking the variable region gene of rat anti-bovine PD-L1 monoclonal antibody (4G12) capable of inhibiting the binding of bovine PD-1 and PD-L1 to the constant region gene of a bovine immunoglobulin (IgG1 with mutations having been introduced into the putative binding sites for Fcγ receptors in CH2 domain to inhibit ADCC activity; see Fig. 48 for amino acid numbers and mutations: 250 E→P, 251 L→V, 252 P→A, 253 G→deletion, 347 A→S, 348 P→S; Ikebuchi R, Konnai S, Okagawa T, Yokoyama K, Nakajima C, Suzuki Y, Murata S, Ohashi K. Immunology 2014 Aug; 142(4):551-561). This chimeric antibody gene was introduced into Chinese hamster ovary cells (CHO cells). By culturing/proliferating the resultant cells, the present inventors have obtained a rat-bovine chimeric anti-bovine PD-L1 antibody (ch4G12) and confirmed its effect *in vitro* and *in vivo.*

### 2. Materials and Methods

### 2.1. Construction of Bovine PD-1 and PD-L1 Expressing Cells

The nucleotide sequences of the full length cDNAs of bovine PD-1 gene (GenBank accession number AB510901; Ikebuchi R, Konnai S, Sunden Y, Onuma M, Ohashi K. Microbiol. Immunol. 2010 May; 54(5):291-298) and bovine PD-L1 gene (GenBank accession number AB510902; Ikebuchi R, Konnai S, Shirai T, Sunden Y, Murata S, Onuma M, Ohashi K. Vet. Res. 2011 Sep. 26; 42:103) were determined. Based on the resultant genetic information, bovine PD-1 and bovine PD-L1 membrane expressing cells were prepared. First, for preparing bovine PD-1 or PD-L1 expressing plasmid, PCR was performed using a synthesized bovine PBMC-derived cDNA as a template and designed primers having *Not*I and *Hind*III (bovine PD-1) recognition sites and *Nhel* and *Xho*I (bovine PD-L1) recognition sites on the 5' side (boPD-1-myc F and R; boPD-L1-EGFP F and R). The PCR products were digested with *Not*I (Takara) and *Hind*III (Takara; bovine PD-1), *Nhel* (Takara) and *Xho*I (Takara; bovine PD-L1), purified with FastGene Gel/PCR Extraction Kit (NIPPON Genetics) and cloned into pCMV-Tag1 vector (Agilent Technologies; bovine PD-1) or pEGFP-N2 vector (Clontech; bovine PD-L1) treated with restriction enzymes in the same manner. The resultant expression plasmid of interest was extracted with QIAGEN Plasmid Midi kit (Qiagen) and stored at -30°C until use in experiments. Hereinafter, the thus prepared expression plasmid is designated as pCMV-Tag1-boPD-1. Primer (boPD-1-myc F): ATATGCGGCCGCATGGGGACCCCGCGGGCGCT (SEQ ID NO: 143) Primer (boPD-1-myc R): GCGCAAGCTTTCAGAGGGGCCAGGAGCAGT (SEQ ID NO: 144) Primer (boPD-L1-EGFP F): CTAGCTAGCACCATGAGGATATATAGTGTCTTAAC (SEQ ID NO: 145) Primer (boPD-L1-EGFP R): CAATCTCGAGTTACAGACAGAAGATGACTGC (SEQ ID NO: 146)

Bovine PD-1 membrane expressing cells were prepared by the procedures described below. First, 2.5 µg of pCMV-Tag1-boPD-1 was introduced into 4x10⁶ CHO-DG44 cells using Lipofectamine LTX (Invitrogen). Forty-eight hours later, the medium was exchanged with CD DG44 medium (Life Technologies) containing 800 µg/ml G418 (Enzo Life Science), 20 ml/L GlutaMAX supplement (Life Technologies), and 18 ml/L 10% Pluronic F-68 (Life Technologies), followed by selection. The resultant expression cells were reacted with rat anti-bovine PD-1 antibody 5D2 at room temperature. After washing, the cells were further reacted with anti-rat IgG microbeads-labeled antibody (Miltenyi Biotec) at room temperature. Cells expressing bovine PD-1 at high levels were isolated with Auto MACS (Miltenyi Biotec). Subsequently, re-isolation was performed in the same manner to obtain still higher purity. The resultant expression cells were subjected to cloning by limiting dilution to thereby obtain a CHO DG44 cell clone expressing bovine PD-1 at high level (bovine PD-1 expressing cells).

Bovine PD-L1 membrane expressing cells were prepared by the procedures described below. First, 2.5 µg ofpEGFP-N2-boPD-L1 or pEGFP-N2 (negative control) was introduced into 4x10⁶ CHO-DG44 cells using Lipofectamine LTX (Invitrogen). Forty-eight hours later, the medium was exchanged with CD DG44 medium (Life Technologies) containing G418 (Enzo Life Science) 800 µg/ml, GlutaMAX supplement (Life Technologies) 20 ml/L, and 10% Pluronic F-68 (Life Technologies) 18 ml/L, followed by selection and cloning by limiting dilution (bovine PD-L1 expressing cell clone). In order to confirm the expression of bovine PD-L1 in the thus prepared expressing cell clone, intracellular localization of EGFP was visualized with an inverted confocal laser microscope LSM700 (ZEISS).

### 2.2. Construction of Soluble Bovine PD-1 and PD-L1

Bovine PD-1-Ig expressing plasmid was constructed by the procedures described below. Briefly, the signal peptide and the extracellular region of bovine PD-1 (GenBank accession number AB510901) were linked to the Fc domain of the constant region of a known bovine IgG1 (GenBank accession number X62916) to prepare a gene sequence. After codons were optimized for CHO cells, gene synthesis was performed in such a manner that *Not*I recognition sequence, KOZAK sequence, bovine PD-1 signal peptide sequence, bovine PD-1 gene extracellular region sequence, bovine IgG1 Fc region sequence, and *Xba*I recognition sequence would be located in the gene in this order. It should be noted here that bovine IgG1 was mutated to inhibit ADCC activity; more specifically, mutations were introduced into the putative binding sites for Fcγ receptors of CH2 domain (sites of mutation: 185 E→P, 186 L→V, 187 P→A, 189 G→deletion, 281 A→S, 282 P→S; Ikebuchi R, Konnai S, Okagawa T, Yokoyama K, Nakajima C, Suzuki Y, Murata S, Ohashi K. Immunology 2014 Aug; 142(4):551-561; the amino acid sequence of PD-1-Ig and the sites of mutation are disclosed in Figure 2 of this article). The synthesized gene strand was digested with *Not*I (Takara) and *Xbal* (Takara), purified with FastGene Gel/PCR Extraction Kit (NIPPON Genetics), and incorporated into the cloning site (*Not*I and *Xbal* restriction enzyme recognition sequences downstream of PCMV and between INRBG and PABGH) of expression vector pDN11 (kindly provided by Prof. S. Suzuki, Hokkaido University Research Center for Zoonosis Control) treated with restriction enzymes in the same manner, whereby bovine PD-1-Ig expressing vector was constructed. The expression plasmid was purified with QIAGEN Plasmid Midi kit (Qiagen) and stored at -30°C until use in experiments. Hereinafter, the thus prepared expression plasmid is designated as pDN11-boPD-1-Ig.

Bovine PD-L1-Ig expressing plasmid was constructed by the procedures described below. In order to amplify the signal peptide and the extracellular region of bovine PD-L1 (GenBank accession number AB510902), primers were designed that had *Nhel* and *Eco*RV recognition sites added on the 5' side (boPD-L1-Ig F and R). PCR was performed using a synthesized bovine PBMC-derived cDNA as a template. The PCR products were digested with NheI (Takara) and EcoRV (Takara), purified with FastGene Gel/PCR Extraction Kit (NIPPON Genetics) and cloned into pCXN2.1-Rabbit IgG1 Fc vector (Niwa et al., 1991; Zettlmeissl et al., 1990; kindly provided by Dr. T. Yokomizo, Juntendo University Graduate School of Medicine, and modified in the inventors' laboratory) treated with restriction enzymes in the same manner. The expression plasmid was purified with QIAGEN Plasmid Midi kit (Qiagen) or FastGene Xpress Plasmid PLUS Kit (NIPPON Genetics) and stored at -30°C until use in experiments. Hereinafter, the thus prepared expression plasmid is designated as pCXN2.1-boPD-L1-Ig. Primer (boPD-L1-Ig F): GCTAGCATGAGGATATATAGTGTCTTAAC (SEQ ID NO: 147) Primer (boPD-L1-Ig R): GATATCATTCCTCTTTTTTGCTGGAT (SEQ ID NO: 148)

Soluble bovine PD-1-Ig expressing cells were prepared by the procedures described below. Briefly, 2.5 µg of pDN11-boPD-1-Ig was introduced into 4x10⁶ CHO-DG44 cells using Lipofectamine LTX (Invitrogen). Forty-eight hours later, the medium was exchanged with OptiCHO AGT medium (Life Technologies) containing 800 µg/ml G418 (Enzo Life Science) and 20 ml/L GlutaMAX supplement (Life Technologies). After cultured for 3 weeks, the cells were subjected to selection. Briefly, the concentrations of the Fc fusion recombinant protein in the culture supernatants of the resultant cell clones were measured by ELISA using anti-bovine IgG F(c) rabbit polyclonal antibody (Rockland) to thereby select those cell clones that express the Fc fusion recombinant protein at high levels. The resultant highly expressing cell clone was transferred to a G418-free medium and cultured under shaking for 14 days, followed by collection of a culture supernatant. The culture supernatant containing the Fc fusion recombinants protein was ultrafiltered with Centricon Plus-70 (Millipore). Then, the Fc fusion recombinant protein was purified with Ab-Capcher Extra (ProteNova). After purification, the buffer was exchanged with phosphate-buffered physiological saline (PBS; pH 7.4) using PD-10 Desalting Column (GE Healthcare). The resultant protein was stored at -30°C until use in experiments (bovine PD-1-Ig). The concentration of the purified bovine PD-1-Ig was measured by ELISA using IgG F(c) rabbit polyclonal antibody (Rockland). For each washing operation in ELISA, Auto Plate Washer BIO WASHER 50 (DS Pharma Biomedical) was used. Absorbance was measured with Microplate Reader MTP-650FA (Corona Electric).

Soluble bovine PD-L1-Ig expressing cells were prepared by the procedures described below. Briefly, 30 µg of pCXN2.1-boPD-L1-Ig was introduced into 7.5x10⁷ Expi293F cells (Life Technologies) using Expifectamine (Life Technologies). After 7-day culture under shaking, the culture supernatant was collected. The recombinant protein was purified from the supernatant using Ab-Capcher Extra (ProteNova; bovine PD-L1-Ig). After purification, the buffer was exchanged with PBS (pH 7.4) using PD MiniTrap G-25 (GE Healthcare). The resultant protein was stored at -30°C until use in experiments (bovine PD-L1-Ig). The concentration of the purified bovine PD-L1-Ig was measured using Rabbit IgG ELISA Quantitation Set (Bethyl). For each washing operation in ELISA, Auto Plate Washer BIO WASHER 50 (DS Pharma Biomedical) was used. Absorbance was measured with Microplate Reader MTP-650FA (Corona Electric).

### 2.3. Preparation of Rat Anti-Bovine PD-L1 Monoclonal Antibody Producing Cells

Rat was immunized in the footpad with bovine PD-L1-Ig (Ikebuchi R, Konnai S, Okagawa T, Yokoyama K, Nakajima C, Suzuki Y, Murata S, Ohashi K. Immunology 2014 Aug.; 142(4):551-561; bovine PD-L1-Ig was prepared by the method disclosed in this article and used for immunization). Hybridomas were established by the iliac lymph node method to thereby obtain rat anti-bovine PD-L1 monoclonal antibody producing hybridoma 4G12. With respect to the method of establishment of rat anti-bovine PD-L1 monoclonal antibody, details are disclosed in the following non-patent document (Ikebuchi R, Konnai S, Okagawa T, Yokoyama K, Nakajima C, Suzuki Y, Murata S, Ohashi K. Vet. Res. 2013 Jul. 22; 44:59; Ikebuchi R, Konnai S, Okagawa T, Yokoyama K, Nakajima C, Suzuki Y, Murata S, Ohashi K. Immunology 2014 Aug.; 142(4):551-561).

### 2.4. Preparation of Rat-Bovine Chimeric Anti-Bovine PD-L1 Antibody Expressing Vector

Rat-bovine chimeric anti-bovine PD-L1 antibody ch4G12 was established by fusing the antibody constant regions of bovine IgG1 and Igλ with rat anti-bovine PD-L1 antibody 4G12 being used as an antibody variable region.

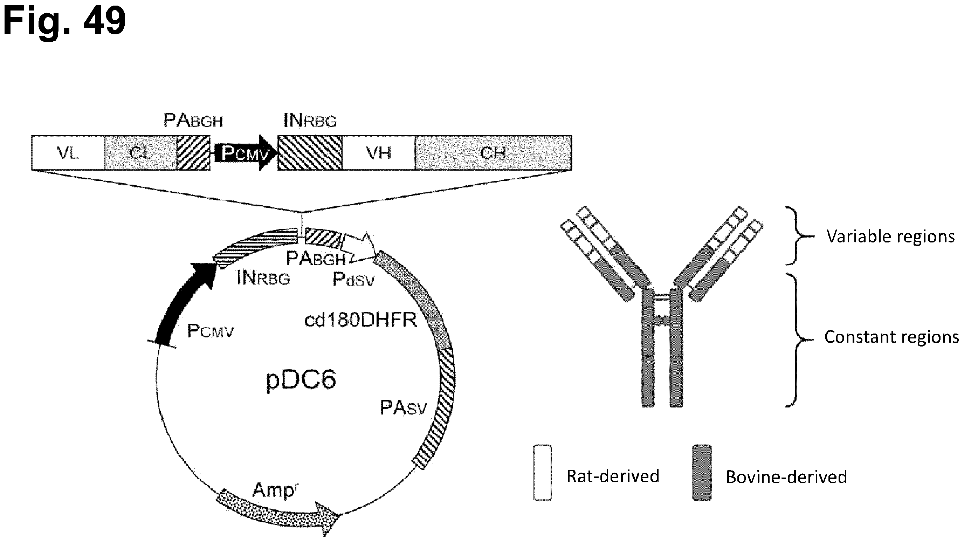

First, the genes of heavy chain and light chain variable regions were identified from a hybridoma that would produce rat anti-bovine PD-L1 antibody 4G12. Subsequently, a gene sequence was prepared in which the heavy chain and the light chain variable regions of the antibody 4G12 were linked to known constant regions of bovine IgG1 (heavy chain; modified from GenBank Accession number X62916) and bovine Igλ (light chain; GenBank Accession number X62917), respectively, and codon optimization was carried out [rat-bovine chimeric anti-bovine PD-L1 antibody ch4G12: SEQ ID NOS: 115 and 116 (amino acid sequences), SEQ ID NOS: 117 and 118 (nucleotide sequences after codon optimization)]. It should be noted that in order to suppress the ADCC activity of bovine IgG1, mutations were added to the putative binding sites of Fcγ receptors in CH2 domain (See Fig. 48 for amino acid numbers and mutations: 250 E→P, 251 L→V, 252 P→A, 253 G→deletion, 347 A→S, 348 P→S; Ikebuchi R, Konnai S, Okagawa T, Yokoyama K, Nakajima C, Suzuki Y, Murata S, Ohashi K. Immunology 2014 Aug; 142(4):551-561). Then, the gene was artificially synthesized in such a manner that *Not*I recognition sequence, KOZAK sequence, chimeric antibody light chain sequence, poly-A addition signal sequence (PABGH), promoter sequence (PCMV), *Sac*I recognition sequence, intron sequence (INRBG), KOZAK sequence, chimeric antibody heavy chain sequence and *Xbal* recognition sequence would be located in this order. The synthesized gene strand was digested with *Not*I (Takara) and *Xbal* (Takara), purified with FastGene Gel/PCR Extraction Kit (NIPPON Genetics) and cloned into the cloning site (*Not*I and *Xbal* restriction enzyme recognition sequences downstream of PCMV and between INRBG and PABGH) of expression plasmid pDC6 (kindly provided by Prof. S. Suzuki, Hokkaido University Research Center for Zoonosis Control) treated with restriction enzymes in the same manner (Fig. 49). The resultant plasmid was extracted with QIAGEN Plasmid Midi kit (Qiagen) and stored at -30°C until use in experiments. Hereinafter, the thus prepared expression plasmid is designated as pDC6-boPD-L1ch4G12.

### 2.5. Expression of Rat-Bovine Chimeric Anti-Bovine PD-L1 Antibody

The pDC6-boPD-L1ch4G12 was transfected into CHO-DG44 cells (CHO-DG44 (dfhr⁻/⁻)) which were a dihydrofolate reductase deficient cell. Forty-eight hours later, the medium was exchanged with OptiCHO AGT medium (Life Technologies) containing 20 ml/L GlutaMAX supplement (Life Technologies). After cultured for 3 weeks, the cells were subjected to selection and cloning by limiting dilution. Subsequently, the concentrations of the chimeric antibody in the culture supernatants were measured by dot blotting and ELISA using anti-bovine IgG F(c) rabbit polyclonal antibody (Rockland) to thereby select high expression clones. Further, the selected clones expressing rat-bovine chimeric anti-bovine PD-L1 antibody at high levels were subjected to gene amplification treatment by adding a load with 60 nM methotrexate (Mtx)-containing medium. The thus established cell clone stably expressing rat-bovine chimeric anti-bovine PD-L1 antibody was transferred into Mtx-free Opti-CHO AGT medium and cultured under shaking for 14 days (125 rpm, 37°C, 5% CO₂). Chimeric antibody production in the culture supernatant was measured by ELISA using anti-bovine IgG F(c) rabbit polyclonal antibody (Rockland). For each washing operation in ELISA, Auto Plate Washer BIO WASHER 50 (DS Pharma Biomedical) was used. Absorbance was measured with Microplate Reader MTP-650FA (Corona Electric). The culture supernatant at day 14 was centrifuged at 10,000 g for 10 min to remove cells, and the centrifugal supernatant was passed through a Steritop-GP 0.22 µm filter (Millipore) for sterilization and then stored at 4°C until it was subjected to purification.

### 2.6. Purification of Rat-Bovine Chimeric Anti-Bovine PD-L1Antibody

From the culture supernatant prepared as described above, each chimeric antibody was purified using Ab Capcher Extra (ProteNova). An open column method was used for binding to resin; PBS pH 7.4 was used as an equilibration buffer and a wash buffer. As an elution buffer, IgG Elution Buffer (Thermo Fisher Scientific) was used. As a neutralization buffer, 1M Tris (pH 9.0) was used. The purified antibody was subjected to buffer exchange with PBS (pH 7.4) using PD-10 Desalting Column (GE Healthcare) and concentrated using Amicon Ultra-15 (50 kDa, Millipore). The thus purified chimeric antibody was passed through a 0.22 µm syringe filter (Millipore) for sterilization and stored at 4°C until use in experiments.

### 2.7. Confirmation of the Purity of Purified Rat-Bovine Chimeric Anti-Bovine PD-L1 Antibody

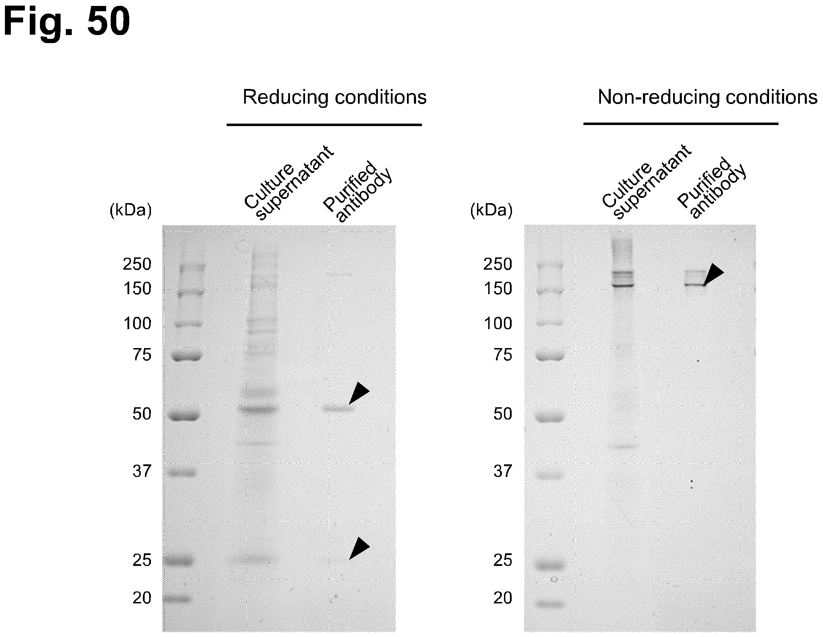

In order to confirm the purity of purified rat-bovine chimeric anti-bovine PD-L1 antibody, antibody proteins were detected by SDS-PAGE and CBB staining. Using 10% acrylamide gel, the purified rat-bovine chimeric antibody was electrophoresed under reducing conditions (reduction with 2-mercaptoethanol from Sigma-Aldrich) and non-reducing conditions. Bands were stained with Quick-CBB kit (Wako) and decolored in distilled water. The results are shown in Fig. 50. Bands were observed at predicted positions, that is, at 25 kDa and 50 kDa under reducing conditions and at 150 kDa under non-reducing conditions.

### 2.8. Binding Specificity of Rat-Bovine Chimeric Anti-Bovine PD-L1 Antibody

It was confirmed by flow cytometry that the rat-bovine chimeric anti-bovine PD-L1 antibody specifically binds to the bovine PD-L1 expressing cells (described above). First, rat anti-bovine PD-L1 antibody 4G12 or rat-bovine chimeric anti-bovine PD-L1 antibody ch4G12 was reacted with bovine PD-L1 expressing cells at room temperature for 30 min. After washing, APC-labeled anti-rat Ig goat antibody (Southern Biotech) or Alexa Fluor 647-labeled anti-bovine IgG (H+L) goat F(ab')2 (Jackson ImmunoResearch) was reacted at room temperature for 30 min. As negative control antibody, rat IgG2a (κ) isotype control (BD Biosciences) or bovine IgG1 antibody (Bethyl) was used. After washing, each rat antibody or rat-bovine chimeric antibody bound to cell surfaces was detected by FACS Verse (BD Biosciences). For every washing operation and dilution of antibody, PBS supplemented with 1% bovine serum albumin (Sigma-Aldrich) was used.

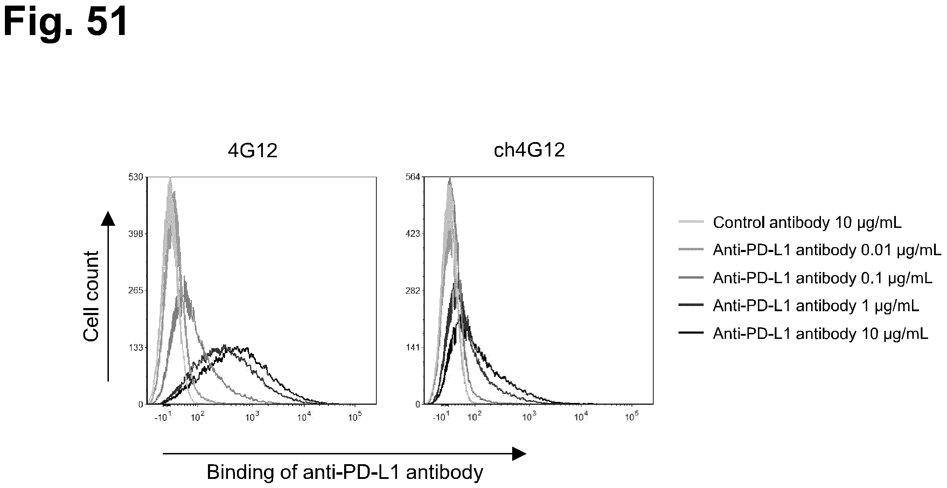

The experimental results are shown in Fig. 51. It was revealed that rat-bovine chimeric anti-bovine PD-L1 antibody ch4G12 binds to bovine PD-L1 expressing cells in the same manner as rat anti-bovine PD-L1 antibody 4G12.

### 2.9. Inhibitory Activity of Rat-Bovine Chimeric Anti-PD-Ll Antibody against Bovine PD-1/PD-L1 Binding

### (1) Binding Inhibition Test on Bovine PD-L1 Expressing Cells and Soluble Bovine PD-1

Using bovine PD-L1 expressing cells (described above) and bovine PD-1-Ig (described above), bovine PD-1/PD-L1 binding inhibition by anti-bovine PD-L1 antibody was tested. First, 2x10⁵ bovine PD-L1 expressing cells were reacted with various concentrations (0, 0.32, 0.63, 1.25, 2.5, 5 or 10 µg/ml) of rat anti-bovine PD-L1 antibody 4G12 or rat-bovine chimeric anti-bovine PD-L1 antibody ch4G12 at room temperature for 30 min. As negative control antibody, rat IgG2a (κ) isotype control (BD Biosciences) or bovine IgG1 antibody (Bethyl) was used. After washing, bovine PD-1-Ig labeled with biotin using Lightning-Link Type A Biotin Labeling Kit (Innova Bioscience) was added to a final concentration of 2 µg/ml, followed by reaction for another 30 min at room temperature. Subsequently, after washing, bovine PD-1-Ig bound to cell surfaces was detected with APC-labeled streptavidin (BioLegend). For analysis, FACS Verse (BD Biosciences) was used. For every washing operation and dilution of antibody, PBS supplemented with 1% bovine serum albumin (Sigma-Aldrich) was used. Taking the proportion of PD-1-Ig bound cells without antibody addition as 100%, the proportion of PD-1-Ig bound cells at each antibody concentration was shown as relative value.

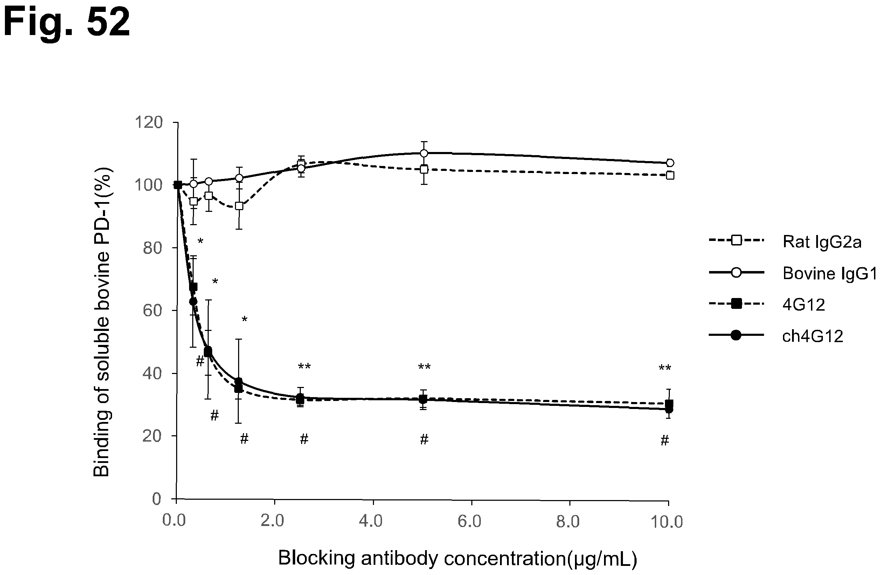

The experimental results are shown in Fig. 52. It was revealed that like rat anti-bovine PD-L1 antibody 4G12, rat-bovine chimeric anti-bovine PD-L1 antibody ch4G12 is capable of inhibiting bovine PD-1/PD-L1 binding in a concentration dependent manner.

### (2) Binding Inhibition Test on Bovine PD-1 Expressing Cells and Soluble Bovine PD-L1

Using bovine PD-1 expressing cells (described above) and bovine PD-L1-Ig (described above), bovine pD-1/PD-L1 binding inhibition by anti-bovine PD-L1 antibody was tested. First, rat anti-bovine PD-L1 antibody 4G12 or rat-bovine chimeric anti-bovine PD-L1 antibody ch4G12 at a final concentration of 0, 0.32, 0.63, 1.25, 2.5, 5 or 10 µg/ml and bovine PD-L1-Ig at a final concentration of 1 µg/ml were placed in 96-well plates, where they were reacted at room temperature for 30 min. The resultant mixture was reacted with 2x10⁵ bovine PD-1 expressing cells at room temperature for 30 min. As negative control antibody, rat IgG2a (κ) isotype control (BD Biosciences) or bovine IgG1 antibody (Bethyl) was used. After washing, Alexa Fluor 647-labeled anti-rabbit IgG (H+L) goat F(ab')2 (Life Technologies) was reacted at room temperature for 30 min to thereby detect bovine PD-L1-Ig bound to cell surfaces. For analysis, FACS Verse (BD Biosciences) was used. For every washing operation and dilution of antibody, PBS supplemented with 1% bovine serum albumin (Sigma-Aldrich) was used. Taking the proportion of PD-L1-Ig bound cells without antibody addition as 100%, the proportion of PD-L1-Ig bound cells at each antibody concentration was shown as relative value.

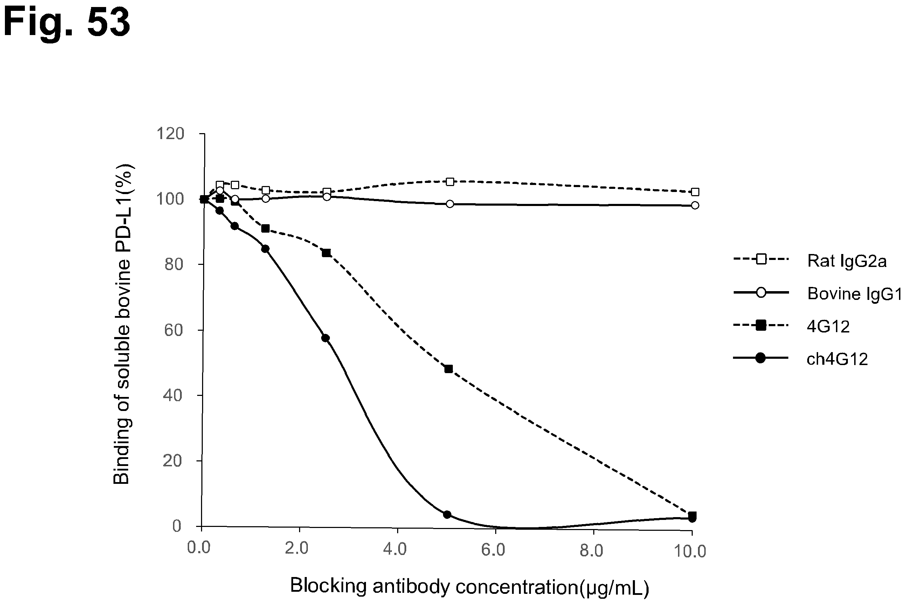

The experimental results are shown in Fig. 53. It was revealed that like rat anti-bovine PD-L1 antibody 4G12, rat-bovine chimeric anti-bovine PD-L1 antibody ch4G12 is capable of inhibiting bovine PD-1/PD-L1 binding in a concentration dependent manner.

### 2.10. Biological Activity Test Using Rat-Bovine Chimeric Anti-Bovine PD-L1 Antibody (1) Effect on Cell Proliferation

In order to confirm that bovine PD-1/PD-L1 binding inhibition by rat-bovine chimeric anti-PD-L1 antibody activates lymphocytes, a biological activity test was performed using cell proliferation as an indicator. Briefly, bovine PBMCs isolated from peripheral blood of healthy cattle were suspended in PBS to give a concentration of 10x10⁶ cells/ml, and reacted with carboxyfluorescein succinimidyl ester (CFSE) at room temperature for 20 min. After washing twice with RPMI 1640 medium (Sigma-Aldrich) containing 10% inactivated fetal bovine serum (Cell Culture Technologies), antibiotics (streptomycin 200 µg/ml, penicillin 200 U/ml) (Life Technologies) and 0.01% L-glutamine (Life Technologies), the PBMCs were reacted with anti-bovine CD3 mouse antibody (WSU Monoclonal Antibody Center) at 4°C for 30 min. After washing, the PBMCs were reacted with anti-mouse IgG1 microbeads (Miltenyi Biotec) at 4°C for 15 min, followed by isolation of CD3-positive T cells using autoMACS™ Pro(Miltenyi Biotec). To the isolated CD3-positive T cells, anti-bovine CD3 mouse antibody (WSU Monoclonal Antibody Center) and anti-bovine CD28 mouse antibody (Bio-Rad) were added. Then, the cells were co-cultured with bovine PD-L1 expressing cells (CD3-positive T cells: bovine PD-L1 expressing cells = 10:1) in the presence or absence of 10 µg/ml of rat-bovine chimeric anti-bovine PD-L1 antibody ch4G12. As a control for antibodies, serum-derived bovine IgG (Sigma-Aldrich) was used; as a control for PD-L1 expressing cells, EGFP expressing cells transfected with pEGFP-N2 were used. After a 6-day coculture, cells were harvested and reacted with anti-bovine CD4 mouse antibody and anti-bovine CD8 mouse antibody (Bio-Rad) at room temperature for 30 min. For the labeling of antibodies, Zenon Mouse IgG1 Labeling Kits (Life Technologies) or Lightning-Link Kit (Innova Biosciences) was used. For analysis, FACS Verse (BD Biosciences) was used. For washing operation after culturing and dilution of antibody, PBS supplemented with 1% bovine serum albumin (Sigma-Aldrich) was used.

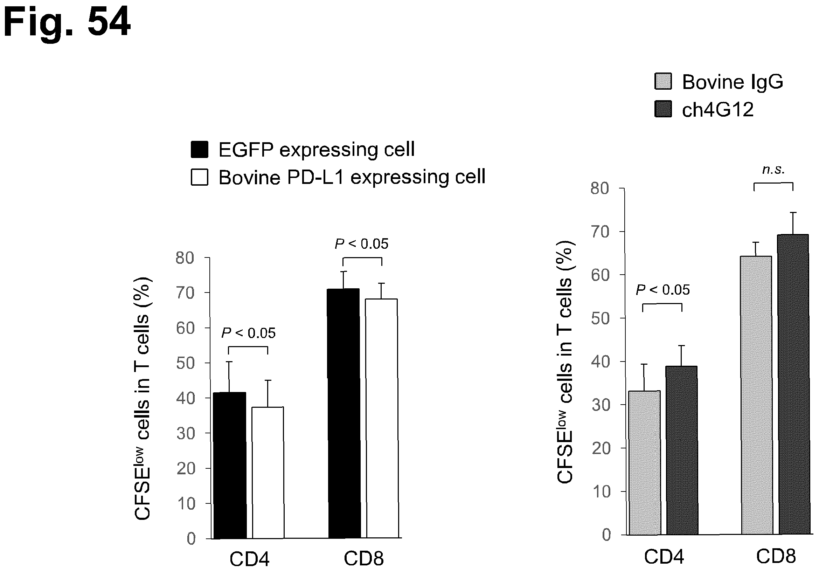

The experimental results are shown in Fig. 54. Proliferation of CD4-positive and CD8-positive T cells was significantly suppressed by co-culture with bovine PD-L1 expressing cells. It was revealed that rat-bovine chimeric anti-bovine PD-L1 antibody ch4G12 inhibits this suppression in CD4-positive T cells.

### (2) Effect on IFN-γ Production

In order to confirm that bovine PD-1/PD-L1 binding inhibition by rat-bovine chimeric anti-PD-L1 antibody activates lymphocytes, a biological activity test was performed using IFN-γ production as an indicator. Briefly, PBMCs isolated from peripheral blood of BLV-infected cattle were suspended in RPMI medium (Sigma-Aldrich) containing 10% inactivated fetal bovine serum (Cell Culture Technologies), antibiotics (streptomycin 200 µg/ml, penicillin 200 U/ml) (Life Technologies) and 0.01% L-glutamine (Life Technologies) to give a concentration of 4x10⁶ cells/ml. To the PBMCs, 10 µg/ml of rat anti-bovine PD-L1 antibody 4G12 or rat-bovine chimeric anti-bovine PD-L1 antibody ch4G12, and 2% BLV-infected fetal lamp kidney cell (FLK-BLV) culture supernatant were added; culturing was then performed at 37 °C under 5% CO₂ for 6 days. As control antibodies, serum-derived rat IgG (Sigma-Aldrich) and serum-derived bovine IgG (Sigma-Aldrich) were used. After a 6-day culture, a culture supernatant was collected, and IFN-γ production was measured with Bovine IFN-γ ELISA Kit (BETYL). For each washing operation in ELISA, Auto Plate Washer BIO WASHER 50 (DS Pharma Biomedical) was used. Absorbance was measured with Microplate Reader MTP-650FA (Corona Electric).

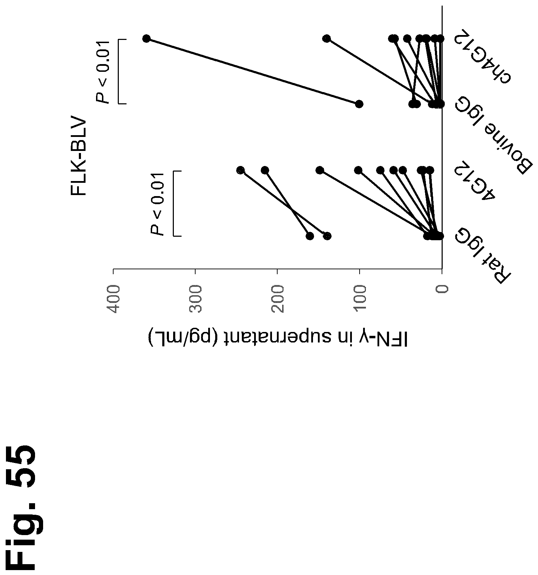

The experimental results are shown in Fig. 55. It was revealed that rat-bovine chimeric anti-bovine PD-L1 antibody ch4G12 increases bovine PBMCs' IFN-γ response to BLV antigen in the same manner as rat anti-bovine PD-L1 antibody 4G12 (n=10).

### 2.11. Inoculation Test on Cattle

Established rat-bovine chimeric anti-bovine PD-L1 antibody ch4G12 (about 260 mg; 1 mg/kg) was intravenously administered into experimentally BLV-infected calf (Holstein, male, 7 months old, 267 kg). Blood samples were collected chronologically from the infected calf, followed by isolation of PBMCs by density gradient centrifugation.

### (1) Cell Proliferation Response of T Cells to BLV Antigen

Bovine PBMCs were suspended in PBS and reacted with CFSE at room temperature for 20 min. After washing twice with RPMI 1640 medium (Sigma-Aldrich) containing 10% inactivated fetal bovine serum (Cell Culture Technologies), antibiotics (streptomycin 200 µg/ml, penicillin 200 U/ml) (Life Technologies) and 0.01% L-glutamine (Life Technologies), the cell concentration was adjusted to 4x10⁶ cells/ml using the same medium. Culture supernatant of 2% BLV-infected fetal lamp kidney cells (FLK-BLV) was added to the PBMCs, which were then cultured at 37°C under 5% CO₂ for 6 days. As a control, culture supernatant of 2% BLV-not-infected fetal lamp kidney cells (FLK) was used. After a 6-day culture, PBMCs were collected and reacted with anti-bovine CD4 mouse antibody, anti-bovine CD8 mouse antibody and anti-bovine IgM mouse antibody (Bio-Rad) at 4°C for 20 min. For the labeling of antibodies, Zenon Mouse IgG1 Labeling Kits (Life Technologies) or Lightning-Link Kit (Innova Biosciences) was used. For analysis, FACS Verse (BD Biosciences) was used. For every washing operation and dilution of antibody, PBS supplemented with 1% bovine serum albumin (Sigma-Aldrich) was used.

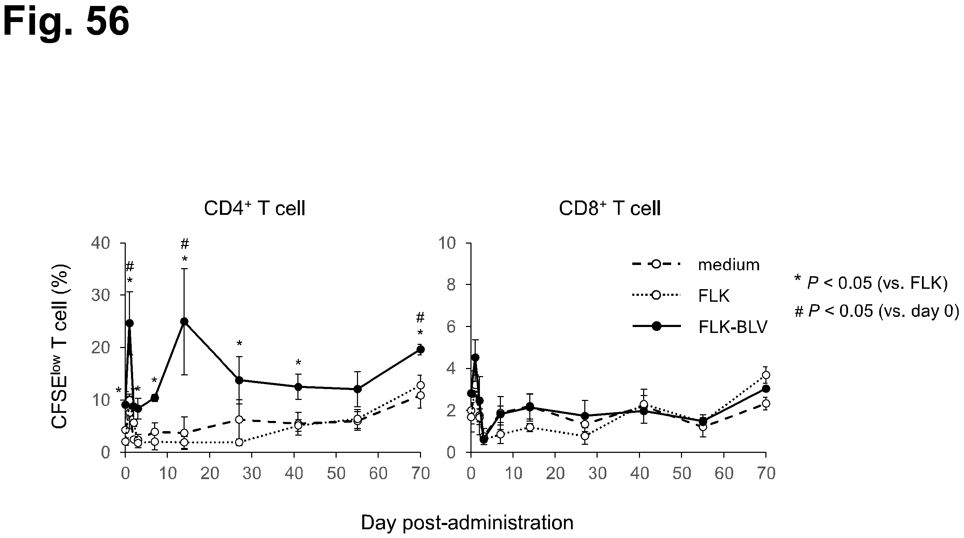

The experimental results are shown in Fig. 56. As a result of antibody administration, BLV-specific cell proliferation response of CD4-positive T cells increased compared to the response before administration.

### (2) Changes in the BLV Provirus Load

DNA was extracted from isolated bovine PBMCs using Wizard DNA Purification kit (Promega). The concentration of the extracted DNA was quantitatively determined, taking the absorbance (260 nm) measured with Nanodrop 8000 Spectrophotometer (Thermo Fisher Scientific) as a reference. In order to measure the BLV provirus load in PBMCs, real time PCR was performed using Cycleave PCR Reaction Mix SP (TaKaRa) and Probe/Primer/Positive control for bovine leukemia virus detection (TaKaRa). Light Cycler 480 System II (Roche Diagnosis) was used for measurement.

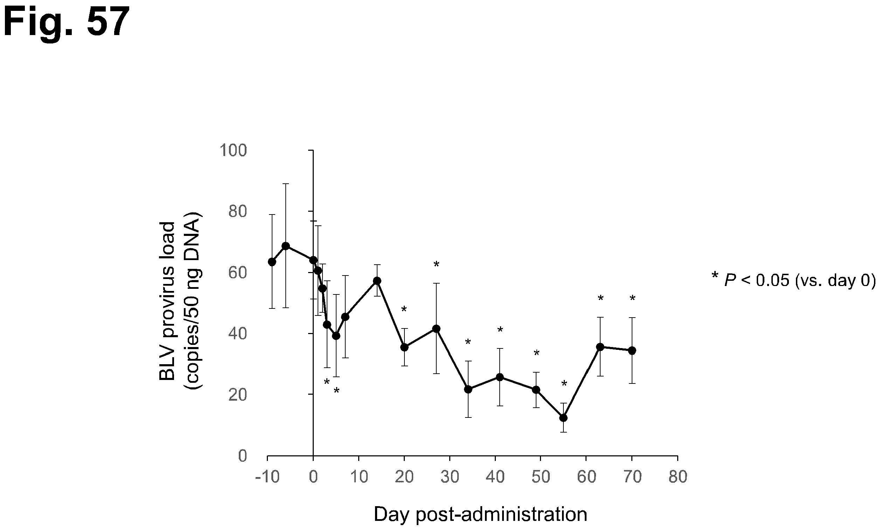

The experimental results are shown in Fig. 57. The BLV provirus load significantly decreased until the end of test period compared to the load before administration.

All publications, patents and patent applications cited herein are incorporated herein by reference in their entirety.

### INDUSTRIAL APPLICABILITY

The pharmaceutical composition of the present invention is applicable to prevention and/or treatment of cancer and/or infection.

### SEQUENCE LISTING FREE TEXT

<SEQ ID NO: 1>
SEQ ID NO: 1 shows the amino acid sequence of the light chain variable region (VL) of rat
anti-bovine PD-L1antibody.
MESQTHVLISLLLSVSGTYGDIAITQSPSSVAVSVGETVTLSCKSSQSLLYSENQKDYL
GWYQQKPGQTPKPLIYWATNRHTGVPDRFTGSGSGTDFTLIISSVQAEDLADYYCGQ
YLVYPFTFGPGTKLELK

<SEQ ID NO: 2>
SEQ ID NO: 2 shows the amino acid sequence of the heavy chain variable region (VH) of rat
anti-bovine PD-L1antibody.
MGWSQIILFLVAAATCVHSQVQLQQSGAELVKPGSSVKISCKASGYTFTSNFMHWVK
QQPGNGLEWIGWIYPEYGNTKYNQKFDGKATLTADKSSSTAYMQLSSLTSEDSAVYF
CASEEAVISLVYWGQGTLVTVSS

<SEQ ID NO: 3>
SEQ ID NO: 3 shows the amino acid sequence of the light chain constant region (CL) of a
canine antibody.
QPKASPSVTLFPPSSEELGANKATLVCLISDFYPSGVTVAWKASGSPVTQGVETTKPSK
QSNNKYAASSYLSLTPDKWKSHSSFSCLVTHEGSTVEKKVAPAECS

<SEQ ID NO: 4>
SEQ ID NO: 4 shows the amino acid sequence of the heavy chain constant region (CH) of a
canine antibody.
ASTTAPSVFPLAPSCGSTSGSTVALACLVSGYFPEPVTVSWNSGSLTSGVHTFPSVLQS

SGLYSLSSTVTVPSSRWPSETFTCNVVHPASNTKVDKPVPKESTCKCISPCPVPESLGG
PSVFIFPPKPKDILRITRTPEITCVVLDLGREDPEVQISWFVDGKEVHTAKTQPREQQFN
STYRVVSVLPIEHQDWLTGKEFKCRVNHIGLPSPIERTISKARGQAHQPSVYVLPPSPK
ELSSSDTVTLTCLIKDFFPPEIDVEWQSNGQPEPESKYHTTAPQLDEDGSYFLYSKLSV
DKSRWQQGDTFTCAVMHEALQNHYTDLSLSHSPGK

<SEQ ID NO: 5>
SEQ ID NO: 5 shows the nucleotide sequence of the VL of rat anti-bovine PD-L1 antibody.
ATGGAATCACAGACGCATGTCCTCATTTCCCTTCTGCTCTCGGTATCTGGTACCTAT
GGGGACATTGCGATAACCCAGTCTCCATCCTCTGTGGCTGTGTCAGTAGGAGAGA
CGGTCACTCTGAGCTGCAAGTCCAGTCAGAGTCTTTTATACAGTGAAAACCAAAA
GGACTATTTGGGCTGGTACCAGCAGAAACCAGGGCAGACTCCTAAACCCCTTATCT
ACTGGGCAACCAACCGGCACACTGGGGTCCCTGATCGCTTCACAGGTAGTGGATC
CGGGACAGACTTCACTCTGATCATCAGCAGTGTGCAGGCTGAAGACCTGGCTGAT
TATTACTGTGGGCAGTACCTTGTCTATCCGTTCACGTTTGGACCTGGGACCAAGCT
GGAACTGAAA

A nucleotide sequence of SEQ ID NO: 5 after codon optimization is shown in <SEQ ID NO: 15>.

ATGGAATCTCAAACTCATGTTTTGATTTCATTACTTCTGAGTGTTTCCGGAACCTAC
GGTGATATCGCTATCACTCAATCTCCCTCCTCTGTTGCTGTGTCTGTGGGCGAAAC
CGTTACCCTGTCCTGCAAGTCCAGTCAGTCTCTTCTCTACTCCGAGAATCAAAAGG
ACTACCTGGGCTGGTACCAACAGAAGCCCGGCCAGACCCCAAAGCCACTGATATA
CTGGGCAACCAACAGGCACACCGGAGTGCCCGACAGGTTCACAGGCAGTGGATC
TGGCACCGACTTTACCTTGATCATTTCAAGCGTGCAGGCTGAAGATCTGGCCGACT
ACTACTGTGGTCAGTATCTGGTGTATCCTTTCACTTTCGGGCCAGGGACAAAATTG
GAATTGAAG

A nucleotide sequence of SEQ ID NO: 5 after codon optimization is shown in <SEQ ID NO: 112>.

ATGGAATCTCAAACTCATGTTTTGATTTCATTACTTCTGAGTGTTTCCGGAACCTAC
GGTGATATCGCTATCACTCAATCTCCCTCCTCTGTTGCTGTGTCTGTGGGCGAAAC

CGTTACCCTGTCCTGCAAGTCCAGTCAGTCTCTTCTCTACTCCGAGAATCAAAAGG
ACTACCTGGGCTGGTACCAACAGAAGCCCGGCCAGACCCCAAAGCCACTGATATA
CTGGGCAACCAACAGGCACACCGGAGTGCCCGACAGGTTCACAGGCAGTGGATC
TGGCACCGACTTTACCTTGATCATTTCAAGCGTGCAGGCTGAAGATCTGGCCGACT
ACTACTGTGGTCAGTATCTGGTGTATCCTTTCACTTTCGGGCCAGGGACAAAACTC
GAGCTCAAA

<SEQ ID NO: 6>
SEQ ID NO: 6 shows the nucleotide sequence of the VH of rat anti-bovine PD-L1 antibody.
ATGGGATGGAGCCAGATCATCCTCTTTCTGGTGGCAGCAGCTACATGTGTTCACTC
CCAGGTACAGCTGCAGCAATCTGGGGCTGAATTAGTGAAGCCTGGGTCCTCAGTG
AAAATTTCCTGCAAGGCTTCTGGCTACACCTTCACCAGTAACTTTATGCACTGGGT
AAAGCAGCAGCCTGGAAATGGCCTTGAGTGGATTGGGTGGATTTATCCTGAATATG
GTAATACTAAGTACAATCAAAAGTTCGATGGGAAGGCAACACTCACTGCAGACAA
ATCCTCCAGCACAGCCTATATGCAGCTCAGCAGCCTGACATCTGAGGACTCTGCAG
TCTATTTCTGTGCAAGTGAGGAGGCAGTTATATCCCTTGTTTACTGGGGCCAAGGC
ACTCTGGTCACTGTCTCTTCA

A nucleotide sequence of SEQ ID NO: 6 after codon optimization is shown in <SEQ ID NO: 16>.

ATGGGTTGGTCTCAAATTATCTTGTTTTTGGTTGCTGCAGCCACTTGTGTTCATTCT
CAGGTGCAGCTGCAACAAAGCGGCGCAGAACTGGTGAAACCTGGCAGCAGCGTG
AAAATATCTTGTAAGGCCAGCGGATATACTTTCACCTCCAATTTCATGCATTGGGTC
AAACAGCAGCCCGGCAACGGACTCGAGTGGATCGGCTGGATCTACCCCGAGTATG
GCAACACAAAATATAACCAAAAATTTGATGGAAAGGCTACCCTGACTGCCGATAA
GTCCTCCAGCACCGCATACATGCAACTCTCCTCCCTGACCTCCGAGGATAGCGCTG
TCTACTTCTGTGCTTCCGAAGAGGCTGTCATATCCTTGGTCTATTGGGGCCAAGGA
ACTCTGGTGACCGTCTCATCT

A nucleotide sequence of SEQ ID NO: 6 after codon optimization is shown in <SEQ ID NO: 113>.

ATGGGGTGGTCCCAGATTATATTGTTCCTCGTCGCCGCCGCCACTTGCGTACACAG

CCAAGTGCAACTTCAACAAAGCGGTGCAGAACTGGTAAAGCCCGGTAGCTCTGT
GAAAATATCCTGTAAAGCCAGTGGCTACACATTTACCAGCAACTTTATGCACTGGG
TGAAGCAACAGCCCGGAAATGGCTTGGAGTGGATTGGCTGGATCTATCCCGAATAT
GGTAACACCAAGTATAATCAGAAGTTCGACGGTAAGGCCACCCTCACCGCCGATA
AGTCATCCTCCACCGCCTATATGCAGCTCAGCAGCCTGACCAGCGAGGATTCCGCT
GTGTACTTCTGTGCCAGCGAAGAGGCTGTGATCTCATTGGTGTATTGGGGACAGG
GCACCCTCGTCACCGTGTCCAGC

<SEQ ID NO: 7>
SEQ ID NO: 7 shows the nucleotide sequence of the CL of a canine antibody.
CAGCCCAAGGCCTCCCCCTCGGTCACACTCTTCCCGCCCTCCTCTGAGGAGCTCG
GCGCCAACAAGGCCACCCTGGTGTGCCTCATCAGCGACTTCTACCCCAGCGGCGT
GACGGTGGCCTGGAAGGCAAGCGGCAGCCCCGTCACCCAGGGCGTGGAGACCAC
CAAGCCCTCCAAGCAGAGCAACAACAAGTACGCGGCCAGCAGCTACCTGAGCCT
GACGCCTGACAAGTGGAAATCTCACAGCAGCTTCAGCTGCCTGGTCACGCACGA
GGGGAGCACCGTGGAGAAGAAGGTGGCCCCCGCAGAGTGCTCTTAG

A nucleotide sequence of SEQ ID NO: 7 after codon optimization is shown in <SEQ ID NO: 17>.

CAGCCCAAAGCCTCTCCCAGCGTCACCCTCTTCCCACCTTCCAGTGAGGAGCTGG
GGGCAAACAAAGCCACTTTGGTGTGTCTCATCTCCGATTTTACCCCTCCGGGGTC
ACAGTCGCATGGAAGGCCTCCGGATCCCCTGTGACACAGGGAGTGGAGACAACA
AAACCTAGCAAGCAGAGTAACAATAAGTATGCCGCCTCAAGCTATCTCAGCCTTAC
TCCTGATAAGTGGAAGTCACATAGCAGTTTTAGTTGCCTCGTAACACATGAGGGTT
CAACTGTGGAGAAAAAAGTAGCTCCAGCTGAGTGCTCATGA

<SEQ ID NO: 8>
SEQ ID NO: 8 shows the nucleotide sequence of the CH of a canine antibody.
GCCTCCACCACGGCCCCCTCGGTTTTCCCACTGGCCCCCAGCTGCGGGTCCACTT
CCGGCTCCACGGTGGCCCTGGCCTGCCTGGTGTCAGGCTACTTCCCCGAGCCTGT
AACTGTGTCCTGGAATTCCGGCTCCTTGACCAGCGGTGTGCACACCTTCCCGTCC
GTCCTGCAGTCCTCAGGGCTCTACTCCCTCAGCAGCACGGTGACAGTGCCCTCCA

GCAGGTGGCCCAGCGAGACCTTCACCTGCAACGTGGTCCACCCGGCCAGCAACA
CTAAAGTAGACAAGCCAGTGCCCAAAGAGTCCACCTGCAAGTGTATATCCCCATG
CCCAGTCCCTGAATCACTGGGAGGGCCTTCGGTCTTCATCTTTCCCCCGAAACCCA
AGGACATCCTCAGGATTACCCGAACACCCGAGATCACCTGTGTGGTGTTAGATCTG
GGCCGTGAGGACCCTGAGGTGCAGATCAGCTGGTTCGTGGATGGTAAGGAGGTG
CACACAGCCAAGACGCAGCCTCGTGAGCAGCAGTTCAACAGCACCTACCGTGTG
GTCAGCGTCCTCCCCATTGAGCACCAGGACTGGCTCACCGGAAAGGAGTTCAAGT
GCAGAGTCAACCACATAGGCCTCCCGTCCCCCATCGAGAGGACTATCTCCAAAGC
CAGAGGGCAAGCCCATCAGCCCAGTGTGTATGTCCTGCCACCATCCCCAAAGGAG
TTGTCATCCAGTGACACGGTCACCCTGACCTGCCTGATCAAAGACTTCTTCCCACC
TGAGATTGATGTGGAGTGGCAGAGCAATGGACAGCCGGAGCCCGAGAGCAAGTA
CCACACGACTGCGCCCCAGCTGGACGAGGACGGGTCCTACTTCCTGTACAGCAAG
CTCTCTGTGGACAAGAGCCGCTGGCAGCAGGGAGACACCTTCACATGTGCGGTGA
TGCATGAAGCTCTACAGAACCACTACACAGATCTATCCCTCTCCCATTCTCCGGGT
AAATGA

A nucleotide sequence of SEQ ID NO: 8 after codon optimization is shown in <SEQ ID NO: 18>. 
GCTAGCACAACCGCTCCCTCCGTTTTTCCCCTCGCCCATCCTGCGGGTCAACCAG
CGGATCCACCGTCGCTCTGGCTTGTCTGGTGTCAGGATACTTCCCCGAGCCTGTCA
CCGTTTCTTGGAATAGCGGCAGCCTTACTTCCGGCGTGCATACCTTCCCTAGCGTG
CTTCAGTCCTCCGGTCTGTATTCCCTCAGCTCCACCGTAACTGTCCCAAGCTCAAG
GTGGCCCTCTGAGACATTTACCTGCAATGTGGTCCATCCTGCTTCAAATACCAAAG
TGGACAAGCCCGTCCCAAAAGAGTCTACCTGCAAATGTATCAGTCCTTGTCCCGT
GCCCGAGTCTCTGGGCGGACCCTCAGTCTTTATCTTCCCACCCAAGCCAAAGGAC
ATATTGCGCATTACACGGACACCCGAAATCACCTGTGTTGTGTTGGATCTCGGCCG
GGAAGATCCTGAGGTGCAGATTAGTTGGTTTGTTGATGGCAAGGAGGTGCACACA
GCAAAAACACAGCCCAGAGAACAGCAGTTCAACAGTACTTATAGAGTAGTGAGT
GTGTTGCCTATAGAGCATCAGGACTGGCTGACAGGCAAAGAATTCAAATGTAGGG
TTAACCACATTGGCCTCCCTAGTCCAATCGAGAGGACAATCTCTAAAGCCCGAGG
CCAGGCTCATCAGCCTTCTGTGTACGTTCTGCCTCCTAGTCCTAAGGAACTGTCTT
CTTCAGACACAGTAACACTCACTTGCCTGATTAAGGACTTTTTCCTCCAGAGATT
 
GATGTGGAATGGCAGTCTAACGGGCAGCCAGAGCCAGAATCTAAGTACCACACTA
CTGCACCACAGCTGGATGAGGATGGGTCTTACTTCCTGTACAGTAAGCTGAGTGT
GGACAAGTCTCGATGGCAGCAGGGGGATACTTTTACTTGCGCAGTAATGCACGAA
GCATTGCAGAACCACTACACTGACCTGTCACTTAGTCACTCACCAGGGAAGTAA
 
<SEQ ID NO: 9>
SEQ ID NO: 9 shows the amino acid sequence of a chimeric light chain comprising the VL of
rat anti-bovine PD-L1 antibody and the CL of a canine antibody.
MESQTHVLISLLLSVSGTYGDIAITQSPSSVAVSVGETVTLSCKSSQSLLYSENQKDYL
GWYQQKPGQTPKPLIYWATNRHTGVPDRFTGSGSGTDFTLIISSVQAEDLADYYCGQ
YLVYPFTFGPGTKLELKQPKASPSVTLFPPSSEELGANKATLVCLISDFYPSGVTVAWK
ASGSPVTQGVETTKPSKQSNNKYAASSYLSLTPDKWKSHSSFSCLVTHEGSTVEKKV
APAECS
 
<SEQ ID NO: 10>
SEQ ID NO: 10 shows the amino acid sequence of a chimeric heavy chain comprising the
VH of rat anti-bovine PD-L1 antibody and the CH of a canine antibody.
MGWSQIILFLVAAATCVHSQVQLQQSGAELVKPGSSVKISCKASGYTFTSNFMHWVK
QQPGNGLEWIGWIYPEYGNTKYNQKFDGKATLTADKSSSTAYMQLSSLTSEDSAVYF
CASEEAVISLVYWGQGTLVTVSSASTTAPSVFPLAPSCGSTSGSTVALACLVSGYFPEP
VTVSWNSGSLTSGVHTFPSVLQSSGLYSLSSTVTVPSSRWPSETFTCNVVHPASNTKV
DKPVPKESTCKCISPCPVPESLGGPSVFIFPPKPKDILRITRTPEITCVVLDLGREDPEVQ
ISWFVDGKEVHTAKTQPREQQFNSTYRVVSVLPIEHQDWLTGKEFKCRVNHIGLPSPI
ERTISKARGQAHQPSVYVLPPSPKELSSSDTVTLTCLIKDFFPPEIDVEWQSNGQPEPES
KYHTTAPQLDEDGSYFLYSKLSVDKSRWQQGDTFTCAVMHEALQNHYTDLSLSHSP
GK
 
<SEQ ID NO: 19>
SEQ ID NO: 19 shows the nucleotide sequence (after codon optimization) of a chimeric light
chain comprising the VL of rat anti-bovine PD-L1 antibody and the CL of a canine antibody.

ATGGAATCTCAAACTCATGTTTTGATTTCATTACTTCTGAGTGTTTCCGGAACCTAC
 
GGTGATATCGCTATCACTCAATCTCCCTCCTCTGTTGCTGTGTCTGTGGGCGAAAC
CGTTACCCTGTCCTGCAAGTCCAGTCAGTCTCTTCTCTACTCCGAGAATCAAAAGG
ACTACCTGGGCTGGTACCAACAGAAGCCCGGCCAGACCCCAAAGCCACTGATATA
CTGGGCAACCAACAGGCACACCGGAGTGCCCGACAGGTTCACAGGCAGTGGATC
TGGCACCGACTTTACCTTGATCATTTCAAGCGTGCAGGCTGAAGATCTGGCCGACT
ACTACTGTGGTCAGTATCTGGTGTATCCTTTCACTTTCGGCCAGGGACAAAATTG
GAATTGAAGCAGCCCAAAGCCTCTCCCAGCGTCACCCTCTTCCCACCTTCCAGTG
AGGAGCTGGGGGCAAACAAAGCCACTTTGGTGTGTCTCATCTCCGATTTTACCC
CTCCGGGGTCACAGTCGCATGGAAGGCCTCCGGATCCCCTGTGACACAGGGAGTG
GAGACAACAAAACCTAGCAAGCAGAGTAACAATAAGTATGCCGCCTCAAGCTATC
TCAGCCTTACTCCTGATAAGTGGAAGTCACATAGCAGTTTTAGTTGCCTCGTAACA
CATGAGGGTTCAACTGTGGAGAAAAAAGTAGCTCCAGCTGAGTGCTCATGA
 
<SEQ ID NO: 20>
SEQ ID NO: 20 shows the nucleotide sequence (after codon optimization) of a chimeric
heavy chain comprising the VH of rat anti-bovine PD-L1 antibody and the CH of a canine
antibody.
ATGGGTTGGTCTCAAATTATCTTGTTTTTGGTTGCTGCAGCCACTTGTGTTCATTCT
CAGGTGCAGCTGCAACAAAGCGGCGCAGAACTGGTGAAACCTGGCAGCAGCGTG
AAAATATCTTGTAAGGCCAGCGGATATACTTTCACCTCCAATTTCATGCATTGGGTC
AAACAGCAGCCCGGCAACGGACTCGAGTGGATCGGCTGGATCTACCCCGAGTATG
GCAACACAAAATATAACCAAAAATTTGATGGAAAGGCTACCCTGACTGCCGATAA
GTCCTCCAGCACCGCATACATGCAACTCTCCTCCCTGACCTCCGAGGATAGCGCTG
TCTACTTCTGTGCTTCCGAAGAGGCTGTCATATCCTTGGTCTATTGGGGCCAAGGA
ACTCTGGTGACCGTCTCATCTGCTAGCACAACCGCTCCCTCCGTTTTCCCCTCGC
CCCATCCTGCGGGTCAACCAGCGGATCCACCGTCGCTCTGGCTTGTCTGGTGTCA
GGATACTTCCCCGAGCCTGTCACCGTTTCTTGGAATAGCGGCAGCCTTACTTCCGG
CGTGCATACCTTCCCTAGCGTGCTTCAGTCCTCCGGTCTGTATTCCCTCAGCTCCAC
CGTAACTGTCCCAAGCTCAAGGTGGCCCTCTGAGACATTTACCTGCAATGTGGTCC
ATCCTGCTTCAAATACCAAAGTGGACAAGCCCGTCCCAAAAGAGTCTACCTGCAA
ATGTATCAGTCCTTGTCCCGTGCCCGAGTCTCTGGGCGGACCCTCAGTCTTTATCTT
CCCACCCAAGCCAAAGGACATATTGCGCATTACACGGACACCCGAAATCACCTGT
 
GTTGTGTTGGATCTCGGCCGGGAAGATCCTGAGGTGCAGATTAGTTGGTTTGTTGA
TGGCAAGGAGGTGCACACAGCAAAAACACAGCCCAGAGAACAGCAGTTCAACA
GTACTTATAGAGTAGTGAGTGTGTTGCCTATAGAGCATCAGGACTGGCTGACAGGC
AAAGAATTCAAATGTAGGGTTAACCACATTGGCCTCCCTAGTCCAATCGAGAGGA
CAATCTCTAAAGCCCGAGGCCAGGCTCATCAGCCTTCTGTGTACGTTCTGCCTCCT
AGTCCTAAGGAACTGTCTTCTTCAGACACAGTAACACTCACTTGCCTGATTAAGG
ACTTTTTCCTCCAGAGATTGATGTGGAATGGCAGTCTAACGGGCAGCCAGAGCC
AGAATCTAAGTACCACACTACTGCACCACAGCTGGATGAGGATGGGTCTTACTTCC
TGTACAGTAAGCTGAGTGTGGACAAGTCTCGATGGCAGCAGGGGGATACTTTTAC
TTGCGCAGTAATGCACGAAGCATTGCAGAACCACTACACTGACCTGTCACTTAGT
CACTCACCAGGGAAGTAA
 
<SEQ ID NO: 11>
SEQ ID NO: 11 shows the amino acid sequence of the CL of a human antibody.
TVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
 
<SEQ ID NO: 12>
SEQ ID NO: 12 shows the amino acid sequence of the CH (CH1-CH3) of a human antibody
(IgG4 variant 1).
STKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPSCPAPEFLGGP
SVFLFPPKPKDTLMISRTPEVTCVVVDVSQEDPEVQFNWYVDGVEVHNAKTKPREE
QFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSR
LTVDKSRWQEGNVFSCSVMHEALHNHYTQKSLSLSLGK
 
<SEQ ID NO: 13>
SEQ ID NO: 13 shows the nucleotide sequence of the CL of a human antibody.
ACTGTGGCTGCACCATCTGTCTTCATCTTCCCGCCATCTGATGAGCAGTTGAAATC
TGGAACTGCCTCTGTTGTGTGCCTGCTGAATAACTTCTATCCCAGAGAGGCCAAA
GTACAGTGGAAGGTGGATAACGCCCTCCAATCGGGTAACTCCCAGGAGAGTGTCA
 
CAGAGCAGGACAGCAAGGACAGCACCTACAGCCTCAGCAGCACCCTGACGCTGA
GCAAAGCAGACTACGAGAAACACAAAGTCTACGCCTGCGAAGTCACCCATCAGG
GCCTGAGCTCGCCCGTCACAAAGAGCTTCAACAGGGGAGAGTGTTAG
 
<SEQ ID NO: 14>
SEQ ID NO: 14 shows the nucleotide sequence of the CH (CH1-CH3) of a human antibody
(IgG4 variant 1).
TCCACCAAGGGCCCATCCGTCTTCCCCCTGGCGCCCTGCTCCAGGAGCACCTCCG
AGAGCACAGCCGCCCTGGGCTGCCTGGTCAAGGACTACTTCCCCGAACCGGTGA
CGGTGTCGTGGAACTCAGGCGCCCTGACCAGCGGCGTGCACACCTTCCCGGCTGT
CCTACAGTCCTCAGGACTCTACTCCCTCAGCAGCGTGGTGACCGTGCCCTCCAGC
AGCTTGGGCACGAAGACCTACACCTGCAACGTAGATCACAAGCCCAGCAACACC
AAGGTGGACAAGAGAGTTGAGTCCAAATATGGTCCCCCATGCCCATCATGCCCAG
CACCTGAGTTCCTGGGGGGACCATCAGTCTTCCTGTTCCCCCCAAAACCCAAGGA
CACTCTCATGATCTCCCGGACCCCTGAGGTCACGTGCGTGGTGGTGGACGTGAGC
CAGGAAGACCCCGAGGTCCAGTTCAACTGGTACGTGGATGGCGTGGAGGTGCATA
ATGCCAAGACAAAGCCGCGGGAGGAGCAGTTCAACAGCACGTACCGTGTGGTCA
GCGTCCTCACCGTCCTGCACCAGGACTGGCTGAACGGCAAGGAGTACAAGTGCA
AGGTCTCCAACAAAGGCCTCCCGTCCTCCATCGAGAAAACCATCTCCAAAGCCAA
AGGGCAGCCCCGAGAGCCACAGGTGTACACCCTGCCCCCATCCCAGGAGGAGAT
GACCAAGAACCAGGTCAGCCTGACCTGCCTGGTCAAAGGCTTCTACCCCAGCGA
CATCGCCGTGGAGTGGGAGAGCAATGGGCAGCCGGAGAACAACTACAAGACCAC
GCCTCCCGTGCTGGACTCCGACGGCTCCTTCTTCCTCTACAGCAGGCTAACCGTG
GACAAGAGCAGGTGGCAGGAGGGGAATGTCTTCTCATGCTCCGTGATGCATGAGG
CTCTGCACAACCACTACACACAGAAGAGCCTCTCCCTGTCTCTGGGTAAATGA
<SEQ ID NOS: 21-36>
   SEQ ID NOS: 21-36 show the nucleotide sequences of primers cPD-1 inner F, cPD-1 inner R, cPD-L1 inner F, cPD-L1 inner R, cPD-1 5' GSP, cPD-1 3' GSP, cPD-L1 5' GSP, cPD-L1 3' GSP, cPD-1-EGFP F, cPD-1-EGFP R, cPD-L1-EGFP F, cPD-L1-EGFP R, cPD-1-Ig, cPD-1-Ig R, cPD-L1-Ig F and cPD-L1-Ig R in this order.
<SEQ ID NO: 37>
   SEQ ID NO: 37 shows the amino acid sequence (QSLLYSENQKDY) of CDR1 of the VL of rat anti-bovine PD-L1 antibody 4G12.
<SEQ ID NO: 38>
   SEQ ID NO: 38 shows the amino acid sequence (GQYLVYPFT) of CDR3 of the VL of rat anti-bovine PD-L1 antibody 4G12.
<SEQ ID NO: 39>
   SEQ ID NO: 39 shows the amino acid sequence (GYTFTSNF) of CDR1 of the VH of rat anti-bovine PD-L1 antibody 4G12.
<SEQ ID NO: 40>
   SEQ ID NO: 40 shows the amino acid sequence (IYPEYGNT) of CDR2 of the VH of rat anti-bovine PD-L1 antibody 4G12.
<SEQ ID NO: 41>
   SEQ ID NO: 41 shows the amino acid sequence (ASEEAVISLVY) of CDR3 of the VH of rat anti-bovine PD-L1 antibody 4G12.
<SEQ ID NO: 42>
   SEQ ID NO: 42 shows the amino acid sequence of the CH (CH1-CH3) of ovine antibody (IgG1).
<SEQ ID NO: 43>
   SEQ ID NO: 43 shows the nucleotide sequence of the CH (CH1-CH3) of ovine antibody (IgG1).
<SEQ ID NO: 44>
   SEQ ID NO: 44 he amino acid sequence of the CH (CH1-CH3) of ovine antibody (IgG2).
<SEQ ID NO: 45>
   SEQ ID NO: 45 shows the nucleotide sequence of the CH (CH1-CH3) of ovine antibody (IgG2).
<SEQ ID NO: 46>
   SEQ ID NO: 46 shows the amino acid sequence of the light chain (Ig kappa(CK)) constant region of an ovine antibody.
<SEQ ID NO: 47>
   SEQ ID NO: 47 shows the nucleotide sequence of the light chain (Ig kappa(CK)) constant region of an ovine antibody.
<SEQ ID NO: 48>
   SEQ ID NO: 48 shows the amino acid sequence of the light chain (Ig lambda(CL)) constant region of an ovine antibody.
<SEQ ID NO: 49>
   SEQ ID NO: 49 shows the nucleotide sequence of the light chain (Ig lambda(CL)) constant region of an ovine antibody.
<SEQ ID NO: 50>
   SEQ ID NO: 50 shows the amino acid sequence of the CH (CH1-CH3) of porcine antibody (IgG1^{a}).
<SEQ ID NO: 51>
   SEQ ID NO: 51 shows the nucleotide acid sequence of the CH (CH1-CH3) of porcine antibody (IgG1^{a}).
<SEQ ID NO: 52>
   SEQ ID NO: 52 shows the amino acid sequence of the CH (CH1-CH3) of porcine antibody (IgG1^{b}).
<SEQ ID NO: 53>
   SEQ ID NO: 53 shows the nucleotide sequence of the CH (CH1-CH3) of porcine antibody (IgG1^{b}).
<SEQ ID NO: 54>
   SEQ ID NO: 54 shows the amino acid sequence of the CH (CH1-CH3) of porcine antibody (lgG2^{a}).
<SEQ ID NO: 55>
   SEQ ID NO: 55 shows the nucleotide sequence of the CH (CH1-CH3) of porcine antibody (IgG2^{a}).
<SEQ ID NO: 56>
   SEQ ID NO: 56 shows the amino acid sequence of the CH (CH1-CH3) of porcine antibody (IgG2^{b}).
<SEQ ID NO: 57>
   SEQ ID NO: 57 shows the nucleotide sequence of the CH (CH1-CH3) of porcine antibody (IgG2^{b}).
<SEQ ID NO: 58>
   SEQ ID NO: 58 shows the amino acid sequence of the CH (CH1-CH3) of porcine antibody (IgG3).
<SEQ ID NO: 59>
   SEQ ID NO: 59 shows the nucleotide sequence of the CH (CH1-CH3) of porcine antibody (IgG3).
<SEQ ID NO: 60>
   SEQ ID NO: 60 shows the amino acid sequence of the CH (CH1-CH3) of porcine antibody (IgG4^{a}).
<SEQ ID NO: 61>
   SEQ ID NO: 61 shows the nucleotide sequence of the CH (CH1-CH3) of porcine antibody (IgG4^{a}).
<SEQ ID NO: 62>
   SEQ ID NO: 62 shows the amino acid sequence of the CH (CH1-CH3) of porcine antibody (IgG4^{b}).
<SEQ ID NO: 63>
   SEQ ID NO: 63 shows the nucleotide sequence of the CH (CH1-CH3) of porcine antibody (IgG4^{b}).
<SEQ ID NO: 64>
   SEQ ID NO: 64 shows the amino acid sequence of the CH (CH1-CH3) of porcine antibody (IgG5^{a}).
<SEQ ID NO: 65>
   SEQ ID NO: 65 shows the nucleotide sequence of the CH (CH1-CH3) of porcine antibody (IgG5^{a}).
<SEQ ID NO: 66>
   SEQ ID NO: 66 shows the amino acid sequence of the CH (CH1-CH3) of porcine antibody (IgG5^{b}).
<SEQ ID NO: 67>
   SEQ ID NO: 67 shows the nucleotide sequence of the CH (CH1-CH3) of porcine antibody (IgG5^{b}).
<SEQ ID NO: 68>
   SEQ ID NO: 68 shows the amino acid sequence of the CH (CH1-CH3) of porcine antibody (IgG6^{a}).
<SEQ ID NO: 69>
   SEQ ID NO: 69 shows the nucleotide sequence of the CH (CH1-CH3) of porcine antibody (IgG6^{a}).
<SEQ ID NO: 70>
   SEQ ID NO: 70 shows the amino acid sequence of the CH (CH1-CH3) of porcine antibody (IgG6^{b}).
<SEQ ID NO: 71>
   SEQ ID NO: 71 shows the nucleotide sequence of the CH (CH1-CH3) of porcine antibody (IgG6^{b}).
<SEQ ID NO: 72>
   SEQ ID NO: 72 shows the amino acid sequence of the CH (CH1-CH3) of a water buffalo antibody (estimated to be IgG1).
<SEQ ID NO: 73>
   SEQ ID NO: 73 shows the nucleotide sequence of the CH (CH1-CH3) of a water buffalo antibody (estimated to be IgG1).
<SEQ ID NO: 74>
   SEQ ID NO: 74 shows the amino acid sequence of the CH (CH1-CH3) of a water buffalo antibody (estimated to be IgG2).
<SEQ ID NO: 75>
   SEQ ID NO: 75 shows the nucleotide sequence of the CH (CH1-CH3) of a water buffalo antibody (estimated to be IgG2).
<SEQ ID NO: 76>
   SEQ ID NO: 76 shows the amino acid sequence of the CH (CH1-CH3) of a water buffalo antibody (estimated to be IgG3).
<SEQ ID NO: 77>
   SEQ ID NO: 77 shows the nucleotide sequence of the CH (CH1-CH3) of a water buffalo antibody (estimated to be IgG3).
<SEQ ID NO: 78>
   SEQ ID NO: 78 shows the amino acid sequence of the light chain (estimated to be Ig lambda) constant region (CL) of a water buffalo antibody.
<SEQ ID NO: 79>
   SEQ ID NO: 79 shows the nucleotide sequence of the light chain (estimated to be Ig lambda) constant region (CL) of a water buffalo antibody. <SEQ ID NO: 80>
   SEQ ID NO: 80 shows the amino acid sequence of the CH (CH1-CH3) of human antibody (IgG4 variant 2).
<SEQ ID NO: 81>
   SEQ ID NO: 81 shows the nucleotide sequence of the CH (CH1-CH3) of human antibody (IgG4 variant 2).
<SEQ ID NO: 82>
   SEQ ID NO: 82 shows the amino acid sequence of the CH (CH1-CH3) of human antibody (IgG4 variant 3).
<SEQ ID NO: 83>
   SEQ ID NO: 83 shows the nucleotide sequence of the CH (CH1-CH3) of human antibody (IgG4 variant 3).
<SEQ ID NO: 84>
   SEQ ID NO: 84 shows the amino acid sequence of the CH (CH1-CH3) of bovine antibody (IgG1 variant 1).
<SEQ ID NO: 85>
   SEQ ID NO: 85 shows the amino acid sequence of the CH (CH1-CH3) of bovine antibody (IgG1 variant 2).
<SEQ ID NO: 86>
   SEQ ID NO: 86 shows the amino acid sequence of the CH (CH1-CH3) of bovine antibody (IgG1 variant 3).
<SEQ ID NO: 87>
   SEQ ID NO: 87 shows the amino acid sequence of the CH (CH1-CH3) of bovine antibody (IgG2 variant 1).
<SEQ ID NO: 88>
   SEQ ID NO: 88 shows the amino acid sequence of the CH (CH1-CH3) of bovine antibody (IgG2 variant 2).
<SEQ ID NO: 89>
   SEQ ID NO: 89 shows the amino acid sequence of the CH (CH1-CH3) of bovine antibody (IgG2 variant 3).
<SEQ ID NO: 90>
   SEQ ID NO: 90 shows the amino acid sequence of the CH (CH1-CH3) of bovine antibody (IgG3 variant 1).
<SEQ ID NO: 91>
   SEQ ID NO: 91 shows the amino acid sequence of the CH (CH1-CH3) of bovine antibody (IgG3 variant 2).
<SEQ ID NO: 92>
   SEQ ID NO: 92 shows the nucleotide sequence of the CH (CH1-CH3) of bovine antibody (IgG1 variant 1).
<SEQ ID NO: 93>
   SEQ ID NO: 93 shows the nucleotide sequence of the CH (CH1-CH3) of bovine antibody (IgG1 variant 2).
<SEQ ID NO: 94>
   SEQ ID NO: 94 shows the nucleotide sequence of the CH (CH1-CH3) of bovine antibody (IgG1 variant 3).
<SEQ ID NO: 95>
   SEQ ID NO: 95 shows the nucleotide sequence of the CH (CH1-CH3) of bovine antibody (IgG2 variant 1).
<SEQ ID NO: 96>
   SEQ ID NO: 96 shows the nucleotide sequence of the CH (CH1-CH3) of bovine antibody (IgG2 variant 2).
<SEQ ID NO: 97>
   SEQ ID NO: 97 shows the nucleotide sequence of the CH (CH1-CH3) of bovine antibody (IgG2 variant 3).
<SEQ ID NO: 98>
   SEQ ID NO: 98 shows the nucleotide sequence of the CH (CH1-CH3) of bovine antibody (IgG3 variant 1).
<SEQ ID NO: 99>
   SEQ ID NO: 99 shows the nucleotide sequence of the CH (CH1-CH3) of bovine antibody (IgG3 variant 2).

<SEQ ID NO: 100>
SEQ ID NO: 100 shows the amino acid sequence of the CL of a bovine antibody (bovine Ig
lambda, GenBank: X62917).
QPKSPPSVTLFPPSTEELNGNKATLVCLISDFYPGSVTVVWKADGSTITRNVETTRASK

QSNSKYAASSYLSLTSSDWKSKGSYSCEVTHEGSTVTKTVKPSECS

<SEQ ID NO: 101>
SEQ ID NO: 101 shows the nucleotide sequence of the CL of a bovine antibody (bovine Ig
lambda, GenBank: X62917).
CAGCCCAAGTCCCCACCCTCGGTCACCCTGTTCCCGCCCTCCACGGAGGAGCTCA
ACGGCAACAAGGCCACCCTGGTGTGTCTCATCAGCGACTTCTACCCGGGTAGCGT
GACCGTGGTCTGGAAGGCAGACGGCAGCACCATCACCCGCAACGTGGAGACCAC
CCGGGCCTCCAAACAGAGCAACAGCAAGTACGCGGCCAGCAGCTACCTGAGCCT
GACGAGCAGCGACTGGAAATCGAAAGGCAGTTACAGCTGCGAGGTCACGCACGA
GGGGAGCACCGTGACGAAGACAGTGAAGCCCTCAGAGTGTTCTTAG

<SEQ ID NO: 102>
SEQ ID NO: 102 shows the amino acid sequence of the CH of a bovine antibody (bovine
IgG1, modified from GenBank: X62916). The sites of mutation are underlined. Amino acid
numbers and mutations: 113E→P, 114L→V, 115P→A, 116G→deletion, 209A→S, 210P→S
ASTTAPKVYPLSSCCGDKSSSTVTLGCLVSSYMPEPVTVTWNSGALKSGVHTFPAVL
QSSGLYSLSSMVTVPGSTSGQTFTCNVAHPASSTKVDKAVDPTCKPSPCDCCPPPPVA
GPSVFIFPPKPKDTLTISGTPEVTCVVVDVGHDDPEVKFSWFVDDVEVNTATTKPREE
QFNSTYRVVSALRIQHQDWTGGKEFKCKVHNEGLPSSIVRTISRTKGPAREPQVYVLA
PPQEELSKSTVSLTCMVTSFYPDYIAVEWQRNGQPESEDKYGTTPPQLDADSSYFLYS
KLRVDRNSWQEGDTYTCVVMHEALHNHYTQKSTSKSAGK

<SEQ ID NO: 103>
SEQ ID NO: 103 shows the nucleotide sequence (after codon optimization) of the CH of a
bovine antibody (bovine IgG1, modified from GenBank: X62916).
GCTAGCACAACTGCTCCTAAGGTGTACCCCCTGAGCTCTTGCTGCGGCGACAAGT
CTAGCAGCACCGTGACCCTCGGATGCCTCGTCAGCAGCTATATGCCTGAGCCAGTT
ACAGTGACATGGAATTCTGGTGCCCTTAAGTCCGGCGTCCATACCTTCCCTGCTGT
GCTGCAGTCCTCTGGCCTGTACAGTTTGTCCTCTATGGTGACAGTACCCGGTTCCA
CCTCCGGACAGACCTTTACCTGTAATGTGGCTCATCCCGCCTCCTCCACAAAGGTG
GATAAGGCTGTTGACCCTACCTGTAAACCCAGTCCATGCGACTGCTGTCCCCCCC
TCCAGTTGCCGGACCCTCAGTCTTTATTTTCCCACCCAAACCCAAAGACACCCTGA
CAATCTCTGGAACACCAGAAGTCACCTGCGTCGTCGTGGATGTGGGCCACGACGA
TCCTGAGGTAAAATTCTCATGGTTCGTCGACGATGTGGAAGTGAATACAGCTACTA

CAAAACCTCGCGAAGAGCAGTTTAACTCTACCTATCGAGTGGTTTCTGCTTTGCGG
ATTCAGCATCAGGATTGGACAGGCGGCAAAGAGTTTAAATGTAAAGTCCATAACG
AGGGACTTCCTTCTAGTATCGTGCGCACTATCAGTAGAACTAAAGGGCCTGCTCGG
GAACCTCAGGTGTACGTCCTGGCACCTCCACAGGAAGAGCTGAGTAAGTCTACAG
TTTCTCTGACTTGTATGGTAACATCTTTTATCCAGATTACATCGCAGTTGAATGGC
AGAGGAACGGGCAGCCAGAGAGTGAGGATAAGTACGGGACTACTCCACCACAGC
TGGACGCAGACTCAAGTTACTTCCTGTACTCAAAGCTGAGGGTTGACAGAAACTC
ATGGCAGGAGGGGGACACTTACACTTGCGTAGTTATGCACGAGGCACTTCACAAC
CACTACACTCAGAAGAGTACTTCAAAGAGTGCAGGGAAGTAA

<SEQ ID NOS: 104 to 107>
   SEQ ID NOS: 104 to 107 show the nucleotide sequences of primers cCD80-Ig F, cCD80-Ig R, cPD-L1-His F and cPD-L1-His R in this order.
<SEQ ID NOS: 108 to 111>
   SEQ ID NOS: 108 to 111 show the nucleotide sequences of primers canine COX2 rt F, canine COX2 rt R, canine HPRT1 rt F and canine HPRT1 rt R in this order. 
<SEQ ID NO: 114>
SEQ ID NO: 114 shows the nucleotide sequence (after codon optimization) of the CL of a
bovine antibody (bovine Ig lambda, GenBank: X62917).
CAGCCTAAGAGTCCTCCTTCTGTAACACTCTTTCCCCCTCTACCGAGGAACTCAA
CGGCAATAAAGCTACCTTGGTTTGCCTTATTTCTGATTTCTACCCCGGGTCTGTGAC
CGTGGTGTGGAAAGCTGATGGGTCCACCATTACTCGGAATGTGGAAACCACCCGG
GCTTCTAAGCAGTCCAACTCTAAATACGCAGCATCCTCCTATTTGAGTCTTACTAGT
AGTGACTGGAAGTCAAAGGGTAGTTACAGTTGCGAAGTCACACATGAAGGTTCA
ACAGTGACAAAGACAGTCAAGCCCTCAGAGTGCTCATAG
 
<SEQ ID NO: 115>
SEQ ID NO: 115 shows the amino acid sequence of a chimeric light chain comprising the VL
of rat anti-bovine PD-L1 antibody and the CL of an antibody.
MESQTHVLISLLLSVSGTYGDIAITQSPSSVAVSVGETVTLSCKSSQSLLYSENQKDYL
GWYQQKPGQTPKPLIYWATNRHTGVPDRFTGSGSGTDFTLIISSVQAEDLADYYCGQ
YLVYPFTFGPGTKLELKQPKSPPSVTLFPPSTEELNGNKATLVCLISDFYPGSVTVVWK
 
ADGSTITRNVETTRASKQSNSKYAASSYLSLTSSDWKSKGSYSCEVTHEGSTVTKTV
KPSECS
 
<SEQ ID NO: 116>
SEQ ID NO: 116 shows the amino acid sequence of a chimeric heavy chain comprising the
VH of rat anti-bovine PD-L1 antibody and the CH of a bovine antibody.
MGWSQIILFLVAAATCVHSQVQLQQSGAELVKPGSSVKISCKASGYTFTSNFMHWVK
QQPGNGLEWIGWIYPEYGNTKYNQKFDGKATLTADKSSSTAYMQLSSLTSEDSAVYF
CASEEAVISLVYWGQGTLVTVSSASTTAPKVYPLSSCCGDKSSSTVTLGCLVSSYMPE
PVTVTWNSGALKSGVHTFPAVLQSSGLYSLSSMVTVPGSTSGQTFTCNVAHPASSTKV
DKAVDPTCKPSPCDCCPPPPVAGPSVFIFPPKPKDTLTISGTPEVTCVVVDVGHDDPEV
KFSWFVDDVEVNTATTKPREEQFNSTYRVVSALRIQHQDWTGGKEFKCKVHNEGLP
SSIVRTISRTKGPAREPQVYVLAPPQEELSKSTVSLTCMVTSFYPDYIAVEWQRNGQPE
SEDKYGTTPPQLDADSSYFLYSKLRVDRNSWQEGDTYTCVVMHEALHNHYTQKSTS
KSAGK
 
<SEQ ID NO: 117>
SEQ ID NO: 117 shows the nucleotide sequence (after codon optimization) of a chimeric
light chain comprising the VL of rat anti-bovine PD-L1 antibody and the CL of a canine
antibody.
ATGGAATCTCAAACTCATGTTTTGATTTCATTACTTCTGAGTGTTTCCGGAACCTAC
GGTGATATCGCTATCACTCAATCTCCCTCCTCTGTTGCTGTGTCTGTGGGCGAAAC
CGTTACCCTGTCCTGCAAGTCCAGTCAGTCTCTTCTCTACTCCGAGAATCAAAAGG
ACTACCTGGGCTGGTACCAACAGAAGCCCGGCCAGACCCCAAAGCCACTGATATA
CTGGGCAACCAACAGGCACACCGGAGTGCCCGACAGGTTCACAGGCAGTGGATC
TGGCACCGACTTTACCTTGATCATTTCAAGCGTGCAGGCTGAAGATCTGGCCGACT
ACTACTGTGGTCAGTATCTGGTGTATCCTTTCACTTTCGGGCCAGGGACAAAACTC
GAGCTCAAACAGCCTAAGAGTCCTCCTTCTGTAACACTCTTTCCCCCCTCTACCGA
GGAACTCAACGGCAATAAAGCTACCTTGGTTTGCCTTATTTCTGATTTCTACCCCG
GGTCTGTGACCGTGGTGTGGAAAGCTGATGGGTCCACCATTACTCGGAATGTGGA
AACCACCCGGGCTTCTAAGCAGTCCAACTCTAAATACGCAGCATCCTCCTATTTGA
GTCTTACTAGTAGTGACTGGAAGTCAAAGGGTAGTTACAGTTGCGAAGTCACACA
TGAAGGTTCAACAGTGACAAAGACAGTCAAGCCCTCAGAGTGCTCATAG
 <SEQ ID NO: 118> 
SEQ ID NO: 118 shows the nucleotide sequence (after codon optimization) of a chimeric
heavy chain comprising the VH of rat anti-bovine PD-L1 antibody and the CH of a canine
antibody.
ATGGGGTGGTCCCAGATTATATTGTTCCTCGTCGCCGCCACTTGCGTACACAG
CCAAGTGCAACTTCAACAAAGCGGTGCAGAACTGGTAAAGCCCGGTAGCTCTGT
GAAAATATCCTGTAAAGCCAGTGGCTACACATTTACCAGCAACTTTATGCACTGGG
TGAAGCAACAGCCCGGAAATGGCTTGGAGTGGATTGGCTGGATCTATCCCGAATAT
GGTAACACCAAGTATAATCAGAAGTTCGACGGTAAGGCCACCCTCACCGCCGATA
AGTCATCCTCCACCGCCTATATGCAGCTCAGCAGCCTGACCAGCGAGGATTCCGCT
GTGTACTTCTGTGCCAGCGAAGAGGCTGTGATCTCATTGGTGTATTGGGGACAGG
GCACCCTCGTCACCGTGTCCAGCGCTAGCACAACTGCTCCTAAGGTGTACCCCCT
GAGCTCTTGCTGCGGCGACAAGTCTAGCAGCACCGTGACCCTCGGATGCCTCGTC
AGCAGCTATATGCCTGAGCCAGTTACAGTGACATGGAATTCTGGTGCCCTTAAGTC
CGGCGTCCATACCTTCCCTGCTGTGCTGCAGTCCTCTGGCCTGTACAGTTTGTCCT
CTATGGTGACAGTACCCGGTTCCACCTCCGGACAGACCTTTACCTGTAATGTGGCT
CATCCCGCCTCCTCCACAAAGGTGGATAAGGCTGTTGACCCTACCTGTAAACCCA
GTCCATGCGACTGCTGTCCCCCCCCTCCAGTTGCCGGACCCTCAGTCTTTATTTTC
CCACCCAAACCCAAAGACACCCTGACAATCTCTGGAACACCAGAAGTCACCTGC
GTCGTCGTGGATGTGGGCCACGACGATCCTGAGGTAAAATTCTCATGGTTCGTCGA
CGATGTGGAAGTGAATACAGCTACTACAAAACCTCGCGAAGAGCAGTTTAACTCT
ACCTATCGAGTGGTTTCTGCTTTGCGGATTCAGCATCAGGATTGGACAGGCGGCAA
AGAGTTTAAATGTAAAGTCCATAACGAGGGACTTCCTTCTAGTATCGTGCGCACTA
TCAGTAGAACTAAAGGGCCTGCTCGGGAACCTCAGGTGTACGTCCTGGCACCTCC
ACAGGAAGAGCTGAGTAAGTCTACAGTTTCTCTGACTTGTATGGTAACATCTTTTT
ATCCAGATTACATCGCAGTTGAATGGCAGAGGAACGGGCAGCCAGAGAGTGAGG
ATAAGTACGGGACTACTCCACCACAGCTGGACGCAGACTCAAGTTACTTCCTGTA
CTCAAAGCTGAGGGTTGACAGAAACTCATGGCAGGAGGGGGACACTTACACTTG
CGTAGTTATGCACGAGGCACTTCACAACCACTACACTCAGAAGAGTACTTCAAAG
AGTGCAGGGAAGTAA
<SEQ ID NOS: 119 to 148>
   SEQ ID NOS: 119 to 148 show the nucleotide sequences of primers boIL2 F, boIL2 R, boIL10 F, boIL10 R, boIFNγ F, boIFNγ R, boTNFα F, boTNFα R, boTGFβ1 F, boTGFβ1 R, boFoxp3 F, boFoxp3 R, boSTAT3 F, boSTAT3 R, boACTB F, boACTB R, boGAPDH F, boGAPDH R, boPDL1 F, boPDL1 R, boCOX2 F, boCOX2 R, boEP4 F, boEP4 R, boPD-1-myc F, boPD-1-myc R, boPD-L1-EGFP F, boPD-L1-EGFP R, boPD-L1-Ig F and boPD-L1-Ig R in this order.

## Claims

1. A pharmaceutical composition which comprises a COX-2 inhibitor and is administered before, after or simultaneously with the administration of an inhibitor targeting PD-1/PD-L1.

2. The pharmaceutical composition of claim 1, wherein the inhibitor targeting PD-1/PD-L1 is an antibody.

3. The pharmaceutical composition of claim 1 or 2, wherein the antibody is at least one antibody selected from the group consisting of anti-PD-1 antibody and anti-PD-Ll antibody.

4. The pharmaceutical composition of any one of claims 1 to 3, wherein the COX-2 inhibitor is at least one compound selected from the group consisting of meloxicam, piroxicam, celecoxib, firocoxib, robenacoxib, carprofen and etodolac.

5. The pharmaceutical composition of any one of claims 1 to 4 for use in prevention and/or treatment of cancer and/or infection.

6. The pharmaceutical composition of any one of claims 1 to 5, wherein the inhibitor targeting PD-1/PD-L1 and the COX-2 inhibitor are administered separately.

7. The pharmaceutical composition of any one of claims 1 to 5, which is a combination drug comprising the inhibitor targeting PD-1/PD-L1 and the COX-2 inhibitor.

8. A potentiator for the immunostimulatory effect of an inhibitor targeting PD-1/PD-L1, which comprises a COX-2 inhibitor.

9. A method of preventing and/or treating cancer and/or infection, comprising administering to a human or animal subject a pharmaceutically effective amount of a COX-2 inhibitor before, after or simultaneously with the administration of an inhibitor targeting PD-1/PD-L1.

10. Use of a COX-2 inhibitor for preventing and/or treating cancer and/or infection, wherein the COX-2 inhibitor is administered before, after or simultaneously with the administration of an inhibitor targeting PD-1/PD-L1.

11. Use of a COX-2 inhibitor for use in a method of preventing and/or treating cancer and/or infection, wherein the COX-2 inhibitor is administered before, after or simultaneously with the administration of an inhibitor targeting PD-1/PD-L1.


SEQUENCE LISTING

<110>  NATIONAL UNIVERSITY CORPORATION HOKKAIDO UNIVERSITY
       FUSO PHARMACEUTICAL INDUSTRIES, LTD.

<120>  Combinatorial therapies with PD-1/PD-L1 targeting inhibitors and
       COX-2 inhibitors

<130>  FP-234PCT

<150>  JP2017-140891
<151>  2017-07-20

<150>  JP2018-016074
<151>  2018-02-01

<160>  148

<170>  PatentIn version 3.5

<210>  1
<211>  133
<212>  PRT
<213>  Rattus norvegicus

<400>  1

Met Glu Ser Gln Thr His Val Leu Ile Ser Leu Leu Leu Ser Val Ser
1               5                   10                  15

Gly Thr Tyr Gly Asp Ile Ala Ile Thr Gln Ser Pro Ser Ser Val Ala
            20                  25                  30

Val Ser Val Gly Glu Thr Val Thr Leu Ser Cys Lys Ser Ser Gln Ser
        35                  40                  45

Leu Leu Tyr Ser Glu Asn Gln Lys Asp Tyr Leu Gly Trp Tyr Gln Gln
    50                  55                  60

Lys Pro Gly Gln Thr Pro Lys Pro Leu Ile Tyr Trp Ala Thr Asn Arg
65                  70                  75                  80

His Thr Gly Val Pro Asp Arg Phe Thr Gly Ser Gly Ser Gly Thr Asp
                85                  90                  95

Phe Thr Leu Ile Ile Ser Ser Val Gln Ala Glu Asp Leu Ala Asp Tyr
            100                 105                 110

Tyr Cys Gly Gln Tyr Leu Val Tyr Pro Phe Thr Phe Gly Pro Gly Thr
        115                 120                 125

Lys Leu Glu Leu Lys
    130

<210>  2
<211>  137
<212>  PRT
<213>  Rattus norvegicus

<400>  2

Met Gly Trp Ser Gln Ile Ile Leu Phe Leu Val Ala Ala Ala Thr Cys
1               5                   10                  15

Val His Ser Gln Val Gln Leu Gln Gln Ser Gly Ala Glu Leu Val Lys
            20                  25                  30

Pro Gly Ser Ser Val Lys Ile Ser Cys Lys Ala Ser Gly Tyr Thr Phe
        35                  40                  45

Thr Ser Asn Phe Met His Trp Val Lys Gln Gln Pro Gly Asn Gly Leu
    50                  55                  60

Glu Trp Ile Gly Trp Ile Tyr Pro Glu Tyr Gly Asn Thr Lys Tyr Asn
65                  70                  75                  80

Gln Lys Phe Asp Gly Lys Ala Thr Leu Thr Ala Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Ser Glu Glu Ala Val Ile Ser Leu Val Tyr Trp Gly
        115                 120                 125

Gln Gly Thr Leu Val Thr Val Ser Ser
    130                 135


<210>  3
<211>  105
<212>  PRT
<213>  Canis lupus

<400>  3

Gln Pro Lys Ala Ser Pro Ser Val Thr Leu Phe Pro Pro Ser Ser Glu
1               5                   10                  15

Glu Leu Gly Ala Asn Lys Ala Thr Leu Val Cys Leu Ile Ser Asp Phe
            20                  25                  30

Tyr Pro Ser Gly Val Thr Val Ala Trp Lys Ala Ser Gly Ser Pro Val
        35                  40                  45

Thr Gln Gly Val Glu Thr Thr Lys Pro Ser Lys Gln Ser Asn Asn Lys

50                  55                  60

Tyr Ala Ala Ser Ser Tyr Leu Ser Leu Thr Pro Asp Lys Trp Lys Ser
65                  70                  75                  80

His Ser Ser Phe Ser Cys Leu Val Thr His Glu Gly Ser Thr Val Glu
                85                  90                  95

Lys Lys Val Ala Pro Ala Glu Cys Ser
            100                 105

<210>  4
<211>  331
<212>  PRT
<213>  Canis lupus

<400>  4

Ala Ser Thr Thr Ala Pro Ser Val Phe Pro Leu Ala Pro Ser Cys Gly
1               5                   10                  15

Ser Thr Ser Gly Ser Thr Val Ala Leu Ala Cys Leu Val Ser Gly Tyr
            20                  25                  30

Phe Pro Glu Pro Val Thr Val Ser Trp Asn Ser Gly Ser Leu Thr Ser
        35                  40                  45

Gly Val His Thr Phe Pro Ser Val Leu Gln Ser Ser Gly Leu Tyr Ser
    50                  55                  60

Leu Ser Ser Thr Val Thr Val Pro Ser Ser Arg Trp Pro Ser Glu Thr
65                  70                  75                  80

Phe Thr Cys Asn Val Val His Pro Ala Ser Asn Thr Lys Val Asp Lys
                85                  90                  95

Pro Val Pro Lys Glu Ser Thr Cys Lys Cys Ile Ser Pro Cys Pro Val
            100                 105                 110

Pro Glu Ser Leu Gly Gly Pro Ser Val Phe Ile Phe Pro Pro Lys Pro
        115                 120                 125

Lys Asp Ile Leu Arg Ile Thr Arg Thr Pro Glu Ile Thr Cys Val Val
    130                 135                 140

Leu Asp Leu Gly Arg Glu Asp Pro Glu Val Gln Ile Ser Trp Phe Val
145                 150                 155                 160

Asp Gly Lys Glu Val His Thr Ala Lys Thr Gln Pro Arg Glu Gln Gln

165                 170                 175

Phe Asn Ser Thr Tyr Arg Val Val Ser Val Leu Pro Ile Glu His Gln
            180                 185                 190

Asp Trp Leu Thr Gly Lys Glu Phe Lys Cys Arg Val Asn His Ile Gly
        195                 200                 205

Leu Pro Ser Pro Ile Glu Arg Thr Ile Ser Lys Ala Arg Gly Gln Ala
    210                 215                 220

His Gln Pro Ser Val Tyr Val Leu Pro Pro Ser Pro Lys Glu Leu Ser
225                 230                 235                 240

Ser Ser Asp Thr Val Thr Leu Thr Cys Leu Ile Lys Asp Phe Phe Pro
                245                 250                 255

Pro Glu Ile Asp Val Glu Trp Gln Ser Asn Gly Gln Pro Glu Pro Glu
            260                 265                 270

Ser Lys Tyr His Thr Thr Ala Pro Gln Leu Asp Glu Asp Gly Ser Tyr
        275                 280                 285

Phe Leu Tyr Ser Lys Leu Ser Val Asp Lys Ser Arg Trp Gln Gln Gly
    290                 295                 300

Asp Thr Phe Thr Cys Ala Val Met His Glu Ala Leu Gln Asn His Tyr
305                 310                 315                 320

Thr Asp Leu Ser Leu Ser His Ser Pro Gly Lys
                325                 330

<210>  5
<211>  399
<212>  DNA
<213>  Rattus norvegicus

<400>  5
atggaatcac agacgcatgt cctcatttcc cttctgctct cggtatctgg tacctatggg      60

gacattgcga taacccagtc tccatcctct gtggctgtgt cagtaggaga gacggtcact     120

ctgagctgca agtccagtca gagtctttta tacagtgaaa accaaaagga ctatttgggc     180

tggtaccagc agaaaccagg gcagactcct aaacccctta tctactgggc aaccaaccgg     240

cacactgggg tccctgatcg cttcacaggt agtggatccg ggacagactt cactctgatc     300

atcagcagtg tgcaggctga agacctggct gattattact gtgggcagta ccttgtctat     360

ccgttcacgt ttggacctgg gaccaagctg gaactgaaa                            399

<210>  6
<211>  411
<212>  DNA
<213>  Rattus norvegicus

<400>  6
atgggatgga gccagatcat cctctttctg gtggcagcag ctacatgtgt tcactcccag      60

gtacagctgc agcaatctgg ggctgaatta gtgaagcctg ggtcctcagt gaaaatttcc     120

tgcaaggctt ctggctacac cttcaccagt aactttatgc actgggtaaa gcagcagcct     180

ggaaatggcc ttgagtggat tgggtggatt tatcctgaat atggtaatac taagtacaat     240

caaaagttcg atgggaaggc aacactcact gcagacaaat cctccagcac agcctatatg     300

cagctcagca gcctgacatc tgaggactct gcagtctatt tctgtgcaag tgaggaggca     360

gttatatccc ttgtttactg gggccaaggc actctggtca ctgtctcttc a              411


<210>  7
<211>  318
<212>  DNA
<213>  Canis lupus

<400>  7
cagcccaagg cctccccctc ggtcacactc ttcccgccct cctctgagga gctcggcgcc      60

aacaaggcca ccctggtgtg cctcatcagc gacttctacc ccagcggcgt gacggtggcc     120

tggaaggcaa gcggcagccc cgtcacccag ggcgtggaga ccaccaagcc ctccaagcag     180

agcaacaaca agtacgcggc cagcagctac ctgagcctga cgcctgacaa gtggaaatct     240

cacagcagct tcagctgcct ggtcacgcac gaggggagca ccgtggagaa gaaggtggcc     300

cccgcagagt gctcttag                                                   318


<210>  8
<211>  996
<212>  DNA
<213>  Canis lupus

<400>  8
gcctccacca cggccccctc ggttttccca ctggccccca gctgcgggtc cacttccggc      60

tccacggtgg ccctggcctg cctggtgtca ggctacttcc ccgagcctgt aactgtgtcc     120

tggaattccg gctccttgac cagcggtgtg cacaccttcc cgtccgtcct gcagtcctca     180

gggctctact ccctcagcag cacggtgaca gtgccctcca gcaggtggcc cagcgagacc     240

ttcacctgca acgtggtcca cccggccagc aacactaaag tagacaagcc agtgcccaaa     300

gagtccacct gcaagtgtat atccccatgc ccagtccctg aatcactggg agggccttcg     360

gtcttcatct ttcccccgaa acccaaggac atcctcagga ttacccgaac acccgagatc     420

acctgtgtgg tgttagatct gggccgtgag gaccctgagg tgcagatcag ctggttcgtg     480

gatggtaagg aggtgcacac agccaagacg cagcctcgtg agcagcagtt caacagcacc 540

taccgtgtgg tcagcgtcct ccccattgag caccaggact ggctcaccgg aaaggagttc 600

aagtgcagag tcaaccacat aggcctcccg tcccccatcg agaggactat ctccaaagcc 660

agagggcaag cccatcagcc cagtgtgtat gtcctgccac catccccaaa ggagttgtca 720

tccagtgaca cggtcaccct gacctgcctg atcaaagact tcttcccacc tgagattgat 780

gtggagtggc agagcaatgg acagccggag cccgagagca agtaccacac gactgcgccc 840

cagctggacg aggacgggtc ctacttcctg tacagcaagc tctctgtgga caagagccgc 900

tggcagcagg gagacacctt cacatgtgcg gtgatgcatg aagctctaca gaaccactac 960

acagatctat ccctctccca ttctccgggt aaatga 996

<210> 9
<211> 238
<212> PRT
<213> Artificial Sequence

<220>
<223> chimeric L chain

<400> 9

Met Glu Ser Gln Thr His Val Leu Ile Ser Leu Leu Leu Ser Val Ser
1               5                   10                  15

Gly Thr Tyr Gly Asp Ile Ala Ile Thr Gln Ser Pro Ser Ser Val Ala
            20                  25                  30

Val Ser Val Gly Glu Thr Val Thr Leu Ser Cys Lys Ser Ser Gln Ser
        35                  40                  45

Leu Leu Tyr Ser Glu Asn Gln Lys Asp Tyr Leu Gly Trp Tyr Gln Gln
    50                  55                  60

Lys Pro Gly Gln Thr Pro Lys Pro Leu Ile Tyr Trp Ala Thr Asn Arg
65                  70                  75                  80

His Thr Gly Val Pro Asp Arg Phe Thr Gly Ser Gly Ser Gly Thr Asp
                85                  90                  95

Phe Thr Leu Ile Ile Ser Ser Val Gln Ala Glu Asp Leu Ala Asp Tyr
            100                 105                 110

Tyr Cys Gly Gln Tyr Leu Val Tyr Pro Phe Thr Phe Gly Pro Gly Thr
        115                 120                 125

Lys Leu Glu Leu Lys Gln Pro Lys Ala Ser Pro Ser Val Thr Leu Phe
    130                 135                 140

Pro Pro Ser Ser Glu Glu Leu Gly Ala Asn Lys Ala Thr Leu Val Cys
145                 150                 155                 160

Leu Ile Ser Asp Phe Tyr Pro Ser Gly Val Thr Val Ala Trp Lys Ala
                165                 170                 175

Ser Gly Ser Pro Val Thr Gln Gly Val Glu Thr Thr Lys Pro Ser Lys
            180                 185                 190

Gln Ser Asn Asn Lys Tyr Ala Ala Ser Ser Tyr Leu Ser Leu Thr Pro
        195                 200                 205

Asp Lys Trp Lys Ser His Ser Ser Phe Ser Cys Leu Val Thr His Glu
    210                 215                 220

Gly Ser Thr Val Glu Lys Lys Val Ala Pro Ala Glu Cys Ser
225                 230                 235

<210>  10
<211>  468
<212>  PRT
<213>  Artificial Sequence

<220>
<223>  chimeric H chain

<400>  10

Met Gly Trp Ser Gln Ile Ile Leu Phe Leu Val Ala Ala Ala Thr Cys
1               5                   10                  15

Val His Ser Gln Val Gln Leu Gln Gln Ser Gly Ala Glu Leu Val Lys
            20                  25                  30

Pro Gly Ser Ser Val Lys Ile Ser Cys Lys Ala Ser Gly Tyr Thr Phe
        35                  40                  45

Thr Ser Asn Phe Met His Trp Val Lys Gln Gln Pro Gly Asn Gly Leu
    50                  55                  60

Glu Trp Ile Gly Trp Ile Tyr Pro Glu Tyr Gly Asn Thr Lys Tyr Asn
65                  70                  75                  80

Gln Lys Phe Asp Gly Lys Ala Thr Leu Thr Ala Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Ser Glu Glu Ala Val Ile Ser Leu Val Tyr Trp Gly
        115                 120                 125

Gln Gly Thr Leu Val Thr Val Ser Ser Ala Ser Thr Thr Ala Pro Ser
    130                 135                 140

Val Phe Pro Leu Ala Pro Ser Cys Gly Ser Thr Ser Gly Ser Thr Val
145                 150                 155                 160

Ala Leu Ala Cys Leu Val Ser Gly Tyr Phe Pro Glu Pro Val Thr Val
                165                 170                 175

Ser Trp Asn Ser Gly Ser Leu Thr Ser Gly Val His Thr Phe Pro Ser
            180                 185                 190

Val Leu Gln Ser Ser Gly Leu Tyr Ser Leu Ser Ser Thr Val Thr Val
        195                 200                 205

Pro Ser Ser Arg Trp Pro Ser Glu Thr Phe Thr Cys Asn Val Val His
    210                 215                 220

Pro Ala Ser Asn Thr Lys Val Asp Lys Pro Val Pro Lys Glu Ser Thr
225                 230                 235                 240

Cys Lys Cys Ile Ser Pro Cys Pro Val Pro Glu Ser Leu Gly Gly Pro
                245                 250                 255

Ser Val Phe Ile Phe Pro Pro Lys Pro Lys Asp Ile Leu Arg Ile Thr
            260                 265                 270

Arg Thr Pro Glu Ile Thr Cys Val Val Leu Asp Leu Gly Arg Glu Asp
        275                 280                 285

Pro Glu Val Gln Ile Ser Trp Phe Val Asp Gly Lys Glu Val His Thr
    290                 295                 300

Ala Lys Thr Gln Pro Arg Glu Gln Gln Phe Asn Ser Thr Tyr Arg Val
305                 310                 315                 320

Val Ser Val Leu Pro Ile Glu His Gln Asp Trp Leu Thr Gly Lys Glu
                325                 330                 335

Phe Lys Cys Arg Val Asn His Ile Gly Leu Pro Ser Pro Ile Glu Arg
            340                 345                 350

Thr Ile Ser Lys Ala Arg Gly Gln Ala His Gln Pro Ser Val Tyr Val
        355                 360                 365

Leu Pro Pro Ser Pro Lys Glu Leu Ser Ser Ser Asp Thr Val Thr Leu
    370                 375                 380

Thr Cys Leu Ile Lys Asp Phe Phe Pro Pro Glu Ile Asp Val Glu Trp
385                 390                 395                 400

Gln Ser Asn Gly Gln Pro Glu Pro Glu Ser Lys Tyr His Thr Thr Ala
                405                 410                 415

Pro Gln Leu Asp Glu Asp Gly Ser Tyr Phe Leu Tyr Ser Lys Leu Ser
            420                 425                 430

Val Asp Lys Ser Arg Trp Gln Gln Gly Asp Thr Phe Thr Cys Ala Val
        435                 440                 445

Met His Glu Ala Leu Gln Asn His Tyr Thr Asp Leu Ser Leu Ser His
    450                 455                 460

Ser Pro Gly Lys
465

<210>  11
<211>  106
<212>  PRT
<213>  Homo sapiens

<400>  11

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210>  12
<211>  326
<212>  PRT
<213>  Homo sapiens

<400>  12

Ser Thr Lys Gly Pro Ser Val Phe Pro Leu Ala Pro Cys Ser Arg Ser
1               5                   10                  15

Thr Ser Glu Ser Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Ser Leu Gly Thr Lys Thr Tyr
65                  70                  75                  80

Thr Cys Asn Val Asp His Lys Pro Ser Asn Thr Lys Val Asp Lys Arg
                85                  90                  95

Val Glu Ser Lys Tyr Gly Pro Pro Cys Pro Ser Cys Pro Ala Pro Glu
            100                 105                 110

Phe Leu Gly Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser Gln Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Tyr Arg Val Val Ser Val Leu Thr Val Leu His Gln Asp Trp
            180                 185                 190

Leu Asn Gly Lys Glu Tyr Lys Cys Lys Val Ser Asn Lys Gly Leu Pro
        195                 200                 205

Ser Ser Ile Glu Lys Thr Ile Ser Lys Ala Lys Gly Gln Pro Arg Glu
    210                 215                 220

Pro Gln Val Tyr Thr Leu Pro Pro Ser Gln Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Val Leu Asp Ser Asp Gly Ser Phe Phe Leu Tyr Ser Arg
        275                 280                 285

Leu Thr Val Asp Lys Ser Arg Trp Gln Glu Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Leu Gly Lys
                325

<210>  13
<211>  321
<212>  DNA
<213>  Homo sapiens

<400>  13
actgtggctg caccatctgt cttcatcttc ccgccatctg atgagcagtt gaaatctgga      60

actgcctctg ttgtgtgcct gctgaataac ttctatccca gagaggccaa agtacagtgg     120

aaggtggata acgccctcca atcgggtaac tcccaggaga gtgtcacaga gcaggacagc     180

aaggacagca cctacagcct cagcagcacc ctgacgctga gcaaagcaga ctacgagaaa     240

cacaaagtct acgcctgcga agtcacccat cagggcctga gctcgcccgt cacaaagagc     300

ttcaacaggg gagagtgtta g                                               321

<210>  14
<211>  981
<212>  DNA
<213>  Homo sapiens

<400>  14
tccaccaagg gcccatccgt cttccccctg gcgccctgct ccaggagcac ctccgagagc      60

acagccgccc tgggctgcct ggtcaaggac tacttccccg aaccggtgac ggtgtcgtgg     120

aactcaggcg ccctgaccag cggcgtgcac accttcccgg ctgtcctaca gtcctcagga     180

ctctactccc tcagcagcgt ggtgaccgtg ccctccagca gcttgggcac gaagacctac     240

acctgcaacg tagatcacaa gcccagcaac accaaggtgg acaagagagt tgagtccaaa 300

tatggtcccc catgcccatc atgcccagca cctgagttcc tggggggacc atcagtcttc 360

ctgttccccc caaaacccaa ggacactctc atgatctccc ggacccctga ggtcacgtgc 420

gtggtggtgg acgtgagcca ggaagacccc gaggtccagt tcaactggta cgtggatggc 480

gtggaggtgc ataatgccaa gacaaagccg cgggaggagc agttcaacag cacgtaccgt 540

gtggtcagcg tcctcaccgt cctgcaccag gactggctga acggcaagga gtacaagtgc 600

aaggtctcca acaaaggcct cccgtcctcc atcgagaaaa ccatctccaa agccaaaggg 660

cagccccgag agccacaggt gtacaccctg cccccatccc aggaggagat gaccaagaac 720

caggtcagcc tgacctgcct ggtcaaaggc ttctacccca gcgacatcgc cgtggagtgg 780

gagagcaatg ggcagccgga gaacaactac aagaccacgc ctcccgtgct ggactccgac 840

ggctccttct tcctctacag caggctaacc gtggacaaga gcaggtggca ggaggggaat 900

gtcttctcat gctccgtgat gcatgaggct ctgcacaacc actacacaca gaagagcctc 960

tccctgtctc tgggtaaatg a 981

<210> 15
<211> 399
<212> DNA
<213> Artificial Sequence

<220>
<223> codon-optimized sequence

<400> 15
atggaatctc aaactcatgt tttgatttca ttacttctga gtgtttccgg aacctacggt 60

gatatcgcta tcactcaatc tccctcctct gttgctgtgt ctgtgggcga aaccgttacc 120

ctgtcctgca agtccagtca gtctcttctc tactccgaga atcaaaagga ctacctgggc 180

tggtaccaac agaagcccgg ccagacccca aagccactga tatactgggc aaccaacagg 240

cacaccggag tgcccgacag gttcacaggc agtggatctg gcaccgactt taccttgatc 300

atttcaagcg tgcaggctga agatctggcc gactactact gtggtcagta tctggtgtat 360

cctttcactt tcgggccagg gacaaaattg gaattgaag 399

<210> 16
<211> 411
<212> DNA
<213> Artificial Sequence

<220>
<223> codon-optimized sequence

<400> 16
atgggttggt ctcaaattat cttgtttttg gttgctgcag ccacttgtgt tcattctcag 60

gtgcagctgc aacaaagcgg cgcagaactg gtgaaacctg gcagcagcgt gaaaatatct 120

tgtaaggcca gcggatatac tttcacctcc aatttcatgc attgggtcaa acagcagccc 180

ggcaacggac tcgagtggat cggctggatc taccccgagt atggcaacac aaaatataac 240

caaaaatttg atggaaaggc taccctgact gccgataagt cctccagcac cgcatacatg 300

caactctcct ccctgacctc cgaggatagc gctgtctact tctgtgcttc cgaagaggct 360

gtcatatcct tggtctattg gggccaagga actctggtga ccgtctcatc t 411

<210> 17
<211> 318
<212> DNA
<213> Artificial Sequence

<220>
<223> codon-optimized sequence

<400> 17
cagcccaaag cctctcccag cgtcaccctc ttcccacctt ccagtgagga gctgggggca 60

aacaaagcca ctttggtgtg tctcatctcc gatttttacc cctccggggt cacagtcgca 120

tggaaggcct ccggatcccc tgtgacacag ggagtggaga caacaaaacc tagcaagcag 180

agtaacaata agtatgccgc ctcaagctat ctcagcctta ctcctgataa gtggaagtca 240

catagcagtt ttagttgcct cgtaacacat gagggttcaa ctgtggagaa aaaagtagct 300

ccagctgagt gctcatga 318

<210> 18
<211> 996
<212> DNA
<213> Artificial Sequence

<220>
<223> codon-optimized sequence

<400> 18
   gctagcacaa ccgctccctc cgtttttccc ctcgccccat cctgcgggtc aaccagcgga 60
   tccaccgtcg ctctggcttg tctggtgtca ggatacttcc ccgagcctgt caccgtttct 120
   tggaatagcg gcagccttac ttccggcgtg cataccttcc ctagcgtgct tcagtcctcc 180
   ggtctgtatt ccctcagctc caccgtaact gtcccaagct caaggtggcc ctctgagaca 240
   tttacctgca atgtggtcca tcctgcttca aataccaaag tggacaagcc cgtcccaaaa 300
   gagtctacct gcaaatgtat cagtccttgt cccgtgcccg agtctctggg cggaccctca 360
   gtctttatct tcccacccaa gccaaaggac atattgcgca ttacacggac acccgaaatc 420
   acctgtgttg tgttggatct cggccgggaa gatcctgagg tgcagattag ttggtttgtt 480
   gatggcaagg aggtgcacac agcaaaaaca cagcccagag aacagcagtt caacagtact 540
   tatagagtag tgagtgtgtt gcctatagag catcaggact ggctgacagg caaagaattc 600
   aaatgtaggg ttaaccacat tggcctccct agtccaatcg agaggacaat ctctaaagcc 660
   cgaggccagg ctcatcagcc ttctgtgtac gttctgcctc ctagtcctaa ggaactgtct 720
   tcttcagaca cagtaacact cacttgcctg attaaggact tttttcctcc agagattgat 780
   gtggaatggc agtctaacgg gcagccagag ccagaatcta agtaccacac tactgcacca 840
   cagctggatg aggatgggtc ttacttcctg tacagtaagc tgagtgtgga caagtctcga 900
   tggcagcagg gggatacttt tacttgcgca gtaatgcacg aagcattgca gaaccactac 960
   actgacctgt cacttagtca ctcaccaggg aagtaa 996

<210> 19
<211> 717
<212> DNA
<213> Artificial Sequence

<220>
<223> codon-optimized sequence

<400> 19
atggaatctc aaactcatgt tttgatttca ttacttctga gtgtttccgg aacctacggt 60

gatatcgcta tcactcaatc tccctcctct gttgctgtgt ctgtgggcga aaccgttacc 120

ctgtcctgca agtccagtca gtctcttctc tactccgaga atcaaaagga ctacctgggc 180

tggtaccaac agaagcccgg ccagacccca aagccactga tatactgggc aaccaacagg 240

cacaccggag tgcccgacag gttcacaggc agtggatctg gcaccgactt taccttgatc 300

atttcaagcg tgcaggctga agatctggcc gactactact gtggtcagta tctggtgtat 360

cctttcactt tcgggccagg gacaaaattg gaattgaagc agcccaaagc ctctcccagc 420

gtcaccctct tcccaccttc cagtgaggag ctgggggcaa acaaagccac tttggtgtgt 480

ctcatctccg atttttaccc ctccggggtc acagtcgcat ggaaggcctc cggatcccct 540

gtgacacagg gagtggagac aacaaaacct agcaagcaga gtaacaataa gtatgccgcc 600

tcaagctatc tcagccttac tcctgataag tggaagtcac atagcagttt tagttgcctc 660

gtaacacatg agggttcaac tgtggagaaa aaagtagctc cagctgagtg ctcatga 717

<210> 20
<211> 1407
<212> DNA
<213> Artificial Sequence

<220>
<223> codon-optimized sequence

<400> 20
atgggttggt ctcaaattat cttgtttttg gttgctgcag ccacttgtgt tcattctcag 60

gtgcagctgc aacaaagcgg cgcagaactg gtgaaacctg gcagcagcgt gaaaatatct 120

tgtaaggcca gcggatatac tttcacctcc aatttcatgc attgggtcaa acagcagccc 180

ggcaacggac tcgagtggat cggctggatc taccccgagt atggcaacac aaaatataac 240

caaaaatttg atggaaaggc taccctgact gccgataagt cctccagcac cgcatacatg 300

caactctcct ccctgacctc cgaggatagc gctgtctact tctgtgcttc cgaagaggct 360

gtcatatcct tggtctattg gggccaagga actctggtga ccgtctcatc tgctagcaca 420

accgctccct ccgtttttcc cctcgcccca tcctgcgggt caaccagcgg atccaccgtc 480

gctctggctt gtctggtgtc aggatacttc cccgagcctg tcaccgtttc ttggaatagc 540

ggcagcctta cttccggcgt gcataccttc cctagcgtgc ttcagtcctc cggtctgtat 600

tccctcagct ccaccgtaac tgtcccaagc tcaaggtggc cctctgagac atttacctgc 660

aatgtggtcc atcctgcttc aaataccaaa gtggacaagc ccgtcccaaa agagtctacc 720

tgcaaatgta tcagtccttg tcccgtgccc gagtctctgg gcggaccctc agtctttatc 780

ttcccaccca agccaaagga catattgcgc attacacgga cacccgaaat cacctgtgtt 840

gtgttggatc tcggccggga agatcctgag gtgcagatta gttggtttgt tgatggcaag 900

gaggtgcaca cagcaaaaac acagcccaga gaacagcagt tcaacagtac ttatagagta 960

gtgagtgtgt tgcctataga gcatcaggac tggctgacag gcaaagaatt caaatgtagg 1020

gttaaccaca ttggcctccc tagtccaatc gagaggacaa tctctaaagc ccgaggccag 1080

gctcatcagc cttctgtgta cgttctgcct cctagtccta aggaactgtc ttcttcagac 1140

acagtaacac tcacttgcct gattaaggac ttttttcctc cagagattga tgtggaatgg 1200

cagtctaacg ggcagccaga gccagaatct aagtaccaca ctactgcacc acagctggat 1260

gaggatgggt cttacttcct gtacagtaag ctgagtgtgg acaagtctcg atggcagcag 1320

ggggatactt ttacttgcgc agtaatgcac gaagcattgc agaaccacta cactgacctg 1380

tcacttagtc actcaccagg gaagtaa 1407

<210> 21
<211> 20
<212> DNA
<213> Artificial Sequence

<220>
<223> primer

<400> 21
aggatggctc ctagactccc 20

<210> 22
<211> 20
<212> DNA
<213> Artificial Sequence

<220>
<223> primer

<400>  22
agacgatggt ggcatactcg                                                   20


<210>  23
<211>  20
<212>  DNA
<213>  Artificial Sequence

<220>
<223>  primer

<400>  23
atgagaatgt ttagtgtctt                                                   20


<210>  24
<211>  24
<212>  DNA
<213>  Artificial Sequence

<220>
<223>  primer

<400>  24
ttatgtctct tcaaattgta tatc                                              24


<210>  25
<211>  16
<212>  DNA
<213>  Artificial Sequence

<220>
<223>  primer

<400>  25
gttgatctgt gtgttg                                                       16


<210>  26
<211>  20
<212>  DNA
<213>  Artificial Sequence

<220>
<223>  primer

<400>  26
cgggacttcc acatgagcat                                                   20


<210>  27
<211>  17
<212>  DNA
<213>  Artificial Sequence

<220>
<223>  primer

<400>  27
ttttagacag aaagtga                                                      17

<210>  28
<211>  20
<212>  DNA
<213>  Artificial Sequence

<220>
<223>  primer

<400>  28
gaccagctct tcttggggaa                                                   20


<210>  29
<211>  29
<212>  DNA
<213>  Artificial Sequence

<220>
<223>  primer

<400>  29
ccgctcgaga tggggagccg gcgggggcc                                         29


<210>  30
<211>  30
<212>  DNA
<213>  Artificial Sequence

<220>
<223>  primer

<400>  30
cgcggatcct gaggggccac aggccgggtc                                        30


<210>  31
<211>  26
<212>  DNA
<213>  Artificial Sequence

<220>
<223>  primer

<400>  31
gaagatctat gagaatgttt agtgtc                                            26


<210>  32
<211>  28
<212>  DNA
<213>  Artificial Sequence

<220>
<223>  primer

<400>  32
ggaattctgt ctcttcaaat tgtatatc                                          28


<210>  33

<211>  30
<212>  DNA
<213>  Artificial Sequence

<220>
<223>  primer

<400>  33
cgcggctagc atggggagcc ggcgggggcc                                        30


<210>  34
<211>  30
<212>  DNA
<213>  Artificial Sequence

<220>
<223>  primer

<400>  34
cgcggatatc cagcccctgc aactggccgc                                        30


<210>  35
<211>  30
<212>  DNA
<213>  Artificial Sequence

<220>
<223>  primer

<400>  35
cgcggctagc atgagaatgt ttagtgtctt                                        30


<210>  36
<211>  30
<212>  DNA
<213>  Artificial Sequence

<220>
<223>  primer

<400>  36
cgcggatatc agtcctctca cttgctggaa                                        30


<210>  37
<211>  12
<212>  PRT
<213>  Rattus norvegicus

<400>  37

Gln Ser Leu Leu Tyr Ser Glu Asn Gln Lys Asp Tyr
1               5                   10


<210>  38
<211>  9
<212>  PRT
<213>  Rattus norvegicus

<400>  38

Gly Gln Tyr Leu Val Tyr Pro Phe Thr
1               5


<210>  39
<211>  8
<212>  PRT
<213>  Rattus norvegicus

<400>  39

Gly Tyr Thr Phe Thr Ser Asn Phe
1               5


<210>  40
<211>  8
<212>  PRT
<213>  Rattus norvegicus

<400>  40

Ile Tyr Pro Glu Tyr Gly Asn Thr
1               5


<210>  41
<211>  11
<212>  PRT
<213>  Rattus norvegicus

<400>  41

Ala Ser Glu Glu Ala Val Ile Ser Leu Val Tyr
1               5                   10


<210>  42
<211>  331
<212>  PRT
<213>  Ovis aries

<400>  42

Ala Ser Thr Thr Pro Pro Lys Val Tyr Pro Leu Thr Ser Cys Cys Gly
1               5                   10                  15


Asp Thr Ser Ser Ser Ile Val Thr Leu Gly Cys Leu Val Ser Ser Tyr
            20                  25                  30


Met Pro Glu Pro Val Thr Val Thr Trp Asn Ser Gly Ala Leu Thr Ser
        35                  40                  45


Gly Val His Thr Phe Pro Ala Ile Leu Gln Ser Ser Gly Leu Tyr Ser
    50                  55                  60


Leu Ser Ser Val Val Thr Val Pro Ala Ser Thr Ser Gly Ala Gln Thr

65                  70                  75                  80

Phe Ile Cys Asn Val Ala His Pro Ala Ser Ser Thr Lys Val Asp Lys
                85                  90                  95

Arg Val Glu Pro Gly Cys Pro Asp Pro Cys Lys His Cys Arg Cys Pro
            100                 105                 110

Pro Pro Glu Leu Pro Gly Gly Pro Ser Val Phe Ile Phe Pro Pro Lys
        115                 120                 125

Pro Lys Asp Thr Leu Thr Ile Ser Gly Thr Pro Glu Val Thr Cys Val
    130                 135                 140

Val Val Asp Val Gly Gln Asp Asp Pro Glu Val Gln Phe Ser Trp Phe
145                 150                 155                 160

Val Asp Asn Val Glu Val Arg Thr Ala Arg Thr Lys Pro Arg Glu Glu
                165                 170                 175

Gln Phe Asn Ser Thr Phe Arg Val Val Ser Ala Leu Pro Ile Gln His
            180                 185                 190

Gln Asp Trp Thr Gly Gly Lys Glu Phe Lys Cys Lys Val His Asn Glu
        195                 200                 205

Ala Leu Pro Ala Pro Ile Val Arg Thr Ile Ser Arg Thr Lys Gly Gln
    210                 215                 220

Ala Arg Glu Pro Gln Val Tyr Val Leu Ala Pro Pro Gln Glu Glu Leu
225                 230                 235                 240

Ser Lys Ser Thr Leu Ser Val Thr Cys Leu Val Thr Gly Phe Tyr Pro
                245                 250                 255

Asp Tyr Ile Ala Val Glu Trp Gln Lys Asn Gly Gln Pro Glu Ser Glu
            260                 265                 270

Asp Lys Tyr Gly Thr Thr Thr Ser Gln Leu Asp Ala Asp Gly Ser Tyr
        275                 280                 285

Phe Leu Tyr Ser Arg Leu Arg Val Asp Lys Asn Ser Trp Gln Glu Gly
    290                 295                 300

Asp Thr Tyr Ala Cys Val Val Met His Glu Ala Leu His Asn His Tyr
305                 310                 315                 320

Thr Gln Lys Ser Ile Ser Lys Pro Pro Gly Lys
                325                 330

<210>  43
<211>  996
<212>  DNA
<213>  Ovis aries

<400>  43
gcctcaacaa cacccccgaa agtctaccct ctgacttctt gctgcgggga cacgtccagc      60

tccatcgtga ccctgggctg cctggtctcc agctatatgc ccgagccggt gaccgtgacc     120

tggaactctg gtgccctgac cagcggcgtg cacaccttcc cggccatcct gcagtcctcc     180

gggctctact ctctcagcag cgtggtgacc gtgccggcca gcacctcagg agcccagacc     240

ttcatctgca acgtagccca cccggccagc agcaccaagg tggacaagcg tgttgagccc     300

ggatgcccgg acccatgcaa acattgccga tgcccacccc ctgagctccc cggaggaccg     360

tctgtcttca tcttcccacc gaaacccaag gacaccctta caatctctgg aacgcccgag     420

gtcacgtgtg tggtggtgga cgtgggccag gatgaccccg aggtgcagtt ctcctggttc     480

gtggacaacg tggaggtgcg cacggccagg acaaagccga gagaggagca gttcaacagc     540

accttccgcg tggtcagcgc cctgcccatc cagcaccaag actggactgg aggaaaggag     600

ttcaagtgca aggtccacaa cgaagccctc ccggccccca tcgtgaggac catctccagg     660

accaaagggc aggcccggga gccgcaggtg tacgtcctgg ccccacccca ggaagagctc     720

agcaaaagca cgctcagcgt cacctgcctg gtcaccggct tctacccaga ctacatcgcc     780

gtggagtggc agaaaaatgg gcagcctgag tcggaggaca agtacggcac gaccacatcc     840

cagctggacg ccgacggctc ctacttcctg tacagcaggc tcagggtgga caagaacagc     900

tggcaagaag gagacaccta cgcgtgtgtg gtgatgcacg aggctctgca caaccactac     960

acacagaagt cgatctctaa gcctccgggt aaatga                               996

<210>  44
<211>  329
<212>  PRT
<213>  Ovis aries

<400>  44

Ala Ser Thr Thr Ala Pro Lys Val Tyr Pro Leu Thr Ser Cys Cys Gly
1               5                   10                  15

Asp Thr Ser Ser Ser Ser Ser Ile Val Thr Leu Gly Cys Leu Val Ser
            20                  25                  30

Ser Tyr Met Pro Glu Pro Val Thr Val Thr Trp Asn Ser Gly Ala Leu
        35                  40                  45

Thr Ser Gly Val His Thr Phe Pro Ala Ile Leu Gln Ser Ser Gly Leu
    50                  55                  60

Tyr Ser Leu Ser Ser Val Val Thr Val Pro Ala Ser Thr Ser Gly Ala
65                  70                  75                  80

Gln Thr Phe Ile Cys Asn Val Ala His Pro Ala Ser Ser Ala Lys Val
                85                  90                  95

Asp Lys Arg Val Gly Ile Ser Ser Asp Tyr Ser Lys Cys Ser Lys Pro
            100                 105                 110

Pro Cys Val Ser Arg Pro Ser Val Phe Ile Phe Pro Pro Lys Pro Lys
        115                 120                 125

Asp Ser Leu Met Ile Thr Gly Thr Pro Glu Val Thr Cys Val Val Val
    130                 135                 140

Asp Val Gly Gln Gly Asp Pro Glu Val Gln Phe Ser Trp Phe Val Asp
145                 150                 155                 160

Asn Val Glu Val Arg Thr Ala Arg Thr Lys Pro Arg Glu Glu Gln Phe
                165                 170                 175

Asn Ser Thr Phe Arg Val Val Ser Ala Leu Pro Ile Gln His Asp His
            180                 185                 190

Trp Thr Gly Gly Lys Glu Phe Lys Cys Lys Val His Ser Lys Gly Leu
        195                 200                 205

Pro Ala Pro Ile Val Arg Thr Ile Ser Arg Ala Lys Gly Gln Ala Arg
    210                 215                 220

Glu Pro Gln Val Tyr Val Leu Ala Pro Pro Gln Glu Glu Leu Ser Lys
225                 230                 235                 240

Ser Thr Leu Ser Val Thr Cys Leu Val Thr Gly Phe Tyr Pro Asp Tyr
                245                 250                 255

Ile Ala Val Glu Trp Gln Arg Ala Arg Gln Pro Glu Ser Glu Asp Lys
            260                 265                 270

Tyr Gly Thr Thr Thr Ser Gln Leu Asp Ala Asp Gly Ser Tyr Phe Leu
        275                 280                 285

Tyr Ser Arg Leu Arg Val Asp Lys Ser Ser Trp Gln Arg Gly Asp Thr
    290                 295                 300

Tyr Ala Cys Val Val Met His Glu Ala Leu His Asn His Tyr Thr Gln
305                 310                 315                 320

Lys Ser Ile Ser Lys Pro Pro Gly Lys
                325

<210>  45
<211>  990
<212>  DNA
<213>  Ovis aries

<400>  45
gcctccacca cagccccgaa agtctaccct ctgacttctt gctgcgggga cacgtccagc      60

tccagctcca tcgtgaccct gggctgcctg gtctccagct atatgcccga gccggtgacc     120

gtgacctgga actctggtgc cctgaccagc ggcgtgcaca ccttcccggc catcctgcag     180

tcctccgggc tctactctct cagcagcgtg gtgaccgtgc cggccagcac ctcaggagcc     240

cagaccttca tctgcaacgt agcccacccg gccagcagcg ccaaggtgga caagcgtgtt     300

gggatctcca gtgactactc caagtgttct aaaccgcctt gcgtgagccg accgtctgtc     360

ttcatcttcc ccccgaaacc caaggacagc ctcatgatca caggaacgcc cgaggtcacg     420

tgtgtggtgg tggacgtggg ccagggtgac cccgaggtgc agttctcctg gttcgtggac     480

aacgtggagg tgcgcacggc caggacaaag ccgagagagg agcagttcaa cagcaccttc     540

cgcgtggtca gcgccctgcc catccagcac gaccactgga ctggaggaaa ggagttcaag     600

tgcaaggtcc acagcaaagg cctcccggcc cccatcgtga ggaccatctc cagggccaaa     660

gggcaggccc gggagccgca ggtgtacgtc ctggccccac cccaggaaga gctcagcaaa     720

agcacgctca gcgtcacctg cctggtcacc ggcttctacc cagactacat cgccgtggag     780

tggcagagag cgcggcagcc tgagtcggag gacaagtacg gcacgaccac atcccagctg     840

gacgccgacg gctcctactt cctgtacagc aggctcaggg tggacaagag cagctggcaa     900

agaggagaca cctacgcgtg tgtggtgatg cacgaggctc tgcacaacca ctacacacag     960

aagtcgatct ctaagcctcc gggtaaatga                                      990

<210>  46
<211>  102
<212>  PRT
<213>  Ovis aries

<400>  46

Pro Ser Val Phe Leu Phe Lys Pro Ser Glu Glu Gln Leu Arg Thr Gly
1               5                   10                  15

Thr Val Ser Val Val Cys Leu Val Asn Asp Phe Tyr Pro Lys Asp Ile

20                  25                  30

Asn Val Lys Val Lys Val Asp Gly Val Thr Gln Asn Ser Asn Phe Gln
        35                  40                  45

Asn Ser Phe Thr Asp Gln Asp Ser Lys Lys Ser Thr Tyr Ser Leu Ser
    50                  55                  60

Ser Thr Leu Thr Leu Ser Ser Ser Glu Tyr Gln Ser His Asn Ala Tyr
65                  70                  75                  80

Ala Cys Glu Val Ser His Lys Ser Leu Pro Thr Ala Leu Val Lys Ser
                85                  90                  95

Phe Asn Lys Asn Glu Cys
            100

<210>  47
<211>  309
<212>  DNA
<213>  Ovis aries

<400>  47
ccatccgtct tcctcttcaa accatctgag gaacagctga ggaccggaac tgtctctgtc      60

gtgtgcttgg tgaatgattt ctaccccaaa gatatcaatg tcaaggtgaa agtggatggg     120

gttacccaga acagcaactt ccagaacagc ttcacagacc aggacagcaa gaaaagcacc     180

tacagcctca gcagcaccct gacactgtcc agctcagagt accagagcca taacgcctat     240

gcgtgtgagg tcagccacaa gagcctgccc accgccctcg tcaagagctt caataagaat     300

gaatgttag                                                             309

<210>  48
<211>  106
<212>  PRT
<213>  Ovis aries

<400>  48

Gly Gln Pro Lys Ser Ala Pro Ser Val Thr Leu Phe Pro Pro Ser Thr
1               5                   10                  15

Glu Glu Leu Ser Thr Asn Lys Ala Thr Val Val Cys Leu Ile Asn Asp
            20                  25                  30

Phe Tyr Pro Gly Ser Val Asn Val Val Trp Lys Ala Asp Gly Ser Thr
        35                  40                  45

Ile Asn Gln Asn Val Lys Thr Thr Gln Ala Ser Lys Gln Ser Asn Ser
    50                  55                  60

Lys Tyr Ala Ala Ser Ser Tyr Leu Thr Leu Thr Gly Ser Glu Trp Lys
65                  70                  75                  80

Ser Lys Ser Ser Tyr Thr Cys Glu Val Thr His Glu Gly Ser Thr Val
                85                  90                  95

Thr Lys Thr Val Lys Pro Ser Glu Cys Ser
            100                 105

<210>  49
<211>  321
<212>  DNA
<213>  Ovis aries

<400>  49
ggtcagccca agtccgcacc ctcggtcacc ctgttcccgc cttccacgga ggagctcagt      60

accaacaagg ccaccgtggt gtgtctcatc aacgacttct acccgggtag cgtgaacgtg     120

gtctggaagg cagatggcag caccatcaat cagaacgtga agaccaccca ggcctccaaa     180

cagagcaaca gcaagtacgc ggccagcagc tacctgaccc tgacgggcag cgagtggaag     240

tctaagagca gttacacctg cgaggtcacg cacgagggga gcaccgtgac gaagacagtg     300

aagccctcag agtgttctta g                                               321

<210>  50
<211>  328
<212>  PRT
<213>  Sus scrofa

<400>  50

Ala Pro Lys Thr Ala Pro Ser Val Tyr Pro Leu Ala Pro Cys Gly Arg
1               5                   10                  15

Asp Thr Ser Gly Pro Asn Val Ala Leu Gly Cys Leu Ala Ser Ser Tyr
            20                  25                  30

Phe Pro Glu Pro Val Thr Met Thr Trp Asn Ser Gly Ala Leu Thr Ser
        35                  40                  45

Gly Val His Thr Phe Pro Ser Val Leu Gln Pro Ser Gly Leu Tyr Ser
    50                  55                  60

Leu Ser Ser Met Val Thr Val Pro Ala Ser Ser Leu Ser Ser Lys Ser
65                  70                  75                  80

Tyr Thr Cys Asn Val Asn His Pro Ala Thr Thr Thr Lys Val Asp Lys
                85                  90                  95

Arg Val Gly Thr Lys Thr Lys Pro Pro Cys Pro Ile Cys Pro Gly Cys
        100                 105                 110

Glu Val Ala Gly Pro Ser Val Phe Ile Phe Pro Pro Lys Pro Lys Asp
    115                 120                 125

Thr Leu Met Ile Ser Gln Thr Pro Glu Val Thr Cys Val Val Val Asp
    130                 135                 140

Val Ser Lys Glu His Ala Glu Val Gln Phe Ser Trp Tyr Val Asp Gly
145                 150                 155                 160

Val Glu Val His Thr Ala Glu Thr Arg Pro Lys Glu Glu Gln Phe Asn
                165                 170                 175

Ser Thr Tyr Arg Val Val Ser Val Leu Pro Ile Gln His Gln Asp Trp
            180                 185                 190

Leu Lys Gly Lys Glu Phe Lys Cys Lys Val Asn Asn Val Asp Leu Pro
        195                 200                 205

Ala Pro Ile Thr Arg Thr Ile Ser Lys Ala Ile Gly Gln Ser Arg Glu
    210                 215                 220

Pro Gln Val Tyr Thr Leu Pro Pro Pro Ala Glu Glu Leu Ser Arg Ser
225                 230                 235                 240

Lys Val Thr Val Thr Cys Leu Val Ile Gly Phe Tyr Pro Pro Asp Ile
                245                 250                 255

His Val Glu Trp Lys Ser Asn Gly Gln Pro Glu Pro Glu Gly Asn Tyr
            260                 265                 270

Arg Thr Thr Pro Pro Gln Gln Asp Val Asp Gly Thr Phe Phe Leu Tyr
        275                 280                 285

Ser Lys Leu Ala Val Asp Lys Ala Arg Trp Asp His Gly Glu Thr Phe
    290                 295                 300

Glu Cys Ala Val Met His Glu Ala Leu His Asn His Tyr Thr Gln Lys
305                 310                 315                 320

Ser Ile Ser Lys Thr Gln Gly Lys
                325

<210>  51
<211>  987

<212>  DNA
<213>  Sus scrofa

<400>  51
   gcccccaaga cggccccatc ggtctaccct ctggccccct gcggcaggga cacgtctggc      60
   cctaacgtgg ccttgggctg cctggcctca agctacttcc ccgagccagt gaccatgacc      120
   tggaactcgg gcgccctgac cagtggcgtg cataccttcc catccgtcct gcagccgtca      180
   gggctctact ccctcagcag catggtgacc gtgccggcca gcagcctgtc cagcaagagc     240
   tacacctgca atgtcaacca cccggccacc accaccaagg tggacaagcg tgttggaaca     300
   aagaccaaac caccatgtcc catatgccca ggctgtgaag tggccgggcc ctcggtcttc     360
   atcttccctc caaaacccaa ggacaccctc atgatctccc agacccccga ggtcacgtgc     420
   gtggtggtgg acgtcagcaa ggagcacgcc gaggtccagt tctcctggta cgtggacggc     480
   gtagaggtgc acacggccga gacgagacca aaggaggagc agttcaacag cacctaccgt     540
   gtggtcagcg tcctgcccat ccagcaccag gactggctga aggggaagga gttcaagtgc     600
   aaggtcaaca acgtagacct cccagccccc atcacgagga ccatctccaa ggctataggg     660
   cagagccggg agccgcaggt gtacaccctg cccccacccg ccgaggagct gtccaggagc     720
   aaagtcaccg taacctgcct ggtcattggc ttctacccac ctgacatcca tgttgagtgg     780
   aagagcaacg gacagccgga gccagagggc aattaccgca ccaccccgcc ccagcaggac     840
   gtggacggga ccttcttcct gtacagcaag ctcgcggtgg acaaggcaag atgggaccat     900
   ggagaaacat ttgagtgtgc ggtgatgcac gaggctctgc acaaccacta cacccagaag     960
   tccatctcca agactcaggg taaatga                                         987


<210>  52
<211>  328
<212>  PRT
<213>  Sus scrofa

<400>  52

Ala Pro Lys Thr Ala Pro Ser Val Tyr Pro Leu Ala Pro Cys Gly Arg
1               5                   10                  15


Asp Val Ser Gly Pro Asn Val Ala Leu Gly Cys Leu Ala Ser Ser Tyr
            20                  25                  30


Phe Pro Glu Pro Val Thr Val Thr Trp Asn Ser Gly Ala Leu Thr Ser
        35                  40                  45


Gly Val His Thr Phe Pro Ser Val Leu Gln Pro Ser Gly Leu Tyr Ser
    50                  55                  60


Leu Ser Ser Met Val Thr Val Pro Ala Ser Ser Leu Ser Ser Lys Ser

65                  70                  75                  80

Tyr Thr Cys Asn Val Asn His Pro Ala Thr Thr Thr Lys Val Asp Lys
                85                  90                  95

Arg Val Gly Ile His Gln Pro Gln Thr Cys Pro Ile Cys Pro Gly Cys
            100                 105                 110

Glu Val Ala Gly Pro Ser Val Phe Ile Phe Pro Pro Lys Pro Lys Asp
        115                 120                 125

Thr Leu Met Ile Ser Gln Thr Pro Glu Val Thr Cys Val Val Val Asp
    130                 135                 140

Val Ser Lys Glu His Ala Glu Val Gln Phe Ser Trp Tyr Val Asp Gly
145                 150                 155                 160

Val Glu Val His Thr Ala Glu Thr Arg Pro Lys Glu Glu Gln Phe Asn
                165                 170                 175

Ser Thr Tyr Arg Val Val Ser Val Leu Pro Ile Gln His Gln Asp Trp
            180                 185                 190

Leu Lys Gly Lys Glu Phe Lys Cys Lys Val Asn Asn Val Asp Leu Pro
        195                 200                 205

Ala Pro Ile Thr Arg Thr Ile Ser Lys Ala Ile Gly Gln Ser Arg Glu
    210                 215                 220

Pro Gln Val Tyr Thr Leu Pro Pro Pro Ala Glu Glu Leu Ser Arg Ser
225                 230                 235                 240

Lys Val Thr Leu Thr Cys Leu Val Ile Gly Phe Tyr Pro Pro Asp Ile
                245                 250                 255

His Val Glu Trp Lys Ser Asn Gly Gln Pro Glu Pro Glu Asn Thr Tyr
            260                 265                 270

Arg Thr Thr Pro Pro Gln Gln Asp Val Asp Gly Thr Phe Phe Leu Tyr
        275                 280                 285

Ser Lys Leu Ala Val Asp Lys Ala Arg Trp Asp His Gly Asp Lys Phe
    290                 295                 300

Glu Cys Ala Val Met His Glu Ala Leu His Asn His Tyr Thr Gln Lys
305                 310                 315                 320

Ser Ile Ser Lys Thr Gln Gly Lys
                325

<210>  53
<211>  987
<212>  DNA
<213>  Sus scrofa

<400>  53
gcccccaaga cggccccatc ggtctaccct ctggccccct gcggcaggga cgtgtctggc        60

cctaacgtgg ccttgggctg cctggcctca agctacttcc ccgagccagt gaccgtgacc       120

tggaactcgg gcgccctgac cagtggcgtg cacaccttcc catccgtcct gcagccgtca       180

gggctctact ccctcagcag catggtgacc gtgccggcca gcagcctgtc cagcaagagc       240

tacacctgca atgtcaacca cccggccacc accaccaagg tggacaagcg tgttggaata       300

caccagccgc aaacatgtcc catatgccca ggctgtgaag tggccgggcc ctcggtcttc       360

atcttccctc caaaacccaa ggacaccctc atgatctccc agacccccga ggtcacgtgc       420

gtggtggtgg acgtcagcaa ggagcacgcc gaggtccagt tctcctggta cgtggacggc       480

gtagaggtgc acacggccga gacgagacca aaggaggagc agttcaacag cacctaccgt       540

gtggtcagcg tcctgcccat ccagcaccag gactggctga aggggaagga gttcaagtgc       600

aaggtcaaca acgtagacct cccagccccc atcacgagga ccatctccaa ggctataggg       660

cagagccggg agccgcaggt gtacaccctg cccccacccg ccgaggagct gtccaggagc       720

aaagtcacgc taacctgcct ggtcattggc ttctacccac ctgacatcca tgttgagtgg       780

aagagcaacg gacagccgga gccagagaac acataccgca ccaccccgcc ccagcaggac       840

gtggacggga ccttcttcct gtacagcaaa ctcgcggtgg acaaggcaag atgggaccat       900

ggagacaaat ttgagtgtgc ggtgatgcac gaggctctgc acaaccacta cacccagaag       960

tccatctcca agactcaggg taaatga                                           987

<210>  54
<211>  328
<212>  PRT
<213>  Sus scrofa

<400>  54

Ala Pro Lys Thr Ala Pro Ser Val Tyr Pro Leu Ala Pro Cys Ser Arg
1               5                   10                  15

Asp Thr Ser Gly Pro Asn Val Ala Leu Gly Cys Leu Ala Ser Ser Tyr
            20                  25                  30

Phe Pro Glu Pro Val Thr Val Thr Trp Asn Ser Gly Ala Leu Ser Ser
        35                  40                  45

Gly Val His Thr Phe Pro Ser Val Leu Gln Pro Ser Gly Leu Tyr Ser
    50                  55                  60

Leu Ser Ser Met Val Thr Val Pro Ala Ser Ser Leu Ser Ser Lys Ser
65                  70                  75                  80

Tyr Thr Cys Asn Val Asn His Pro Ala Thr Thr Thr Lys Val Asp Lys
                85                  90                  95

Arg Val Gly Thr Lys Thr Lys Pro Pro Cys Pro Ile Cys Pro Ala Cys
            100                 105                 110

Glu Ser Pro Gly Pro Ser Val Phe Ile Phe Pro Pro Lys Pro Lys Asp
        115                 120                 125

Thr Leu Met Ile Ser Arg Thr Pro Gln Val Thr Cys Val Val Val Asp
    130                 135                 140

Val Ser Gln Glu Asn Pro Glu Val Gln Phe Ser Trp Tyr Val Asp Gly
145                 150                 155                 160

Val Glu Val His Thr Ala Gln Thr Arg Pro Lys Glu Glu Gln Phe Asn
                165                 170                 175

Ser Thr Tyr Arg Val Val Ser Val Leu Pro Ile Gln His Gln Asp Trp
            180                 185                 190

Leu Asn Gly Lys Glu Phe Lys Cys Lys Val Asn Asn Lys Asp Leu Pro
        195                 200                 205

Ala Pro Ile Thr Arg Ile Ile Ser Lys Ala Lys Gly Gln Thr Arg Glu
    210                 215                 220

Pro Gln Val Tyr Thr Leu Pro Pro His Ala Glu Glu Leu Ser Arg Ser
225                 230                 235                 240

Lys Val Ser Ile Thr Cys Leu Val Ile Gly Phe Tyr Pro Pro Asp Ile
                245                 250                 255

Asp Val Glu Trp Gln Arg Asn Gly Gln Pro Glu Pro Glu Gly Asn Tyr
            260                 265                 270

Arg Thr Thr Pro Pro Gln Gln Asp Val Asp Gly Thr Tyr Phe Leu Tyr
        275                 280                 285

Ser Lys Phe Ser Val Asp Lys Ala Ser Trp Gln Gly Gly Gly Ile Phe
    290                 295                 300

Gln Cys Ala Val Met His Glu Ala Leu His Asn His Tyr Thr Gln Lys
305                 310                 315                 320

Ser Ile Ser Lys Thr Pro Gly Lys
                325

<210>  55
<211>  987
<212>  DNA
<213>  Sus scrofa

<400>  55
   gcccccaaga cggccccatc ggtctaccct ctggccccct gcagcaggga cacgtctggc      60
   cctaacgtgg ccttgggctg cctggcctca agctacttcc ccgagccagt gaccgtgacc      120
   tggaactcgg gcgccctgtc cagtggcgtg cataccttcc catccgtcct gcagccgtca      180
   gggctctact ccctcagcag catggtgacc gtgccggcca gcagcctgtc cagcaagagc     240
   tacacctgca atgtcaacca cccggccacc accaccaagg tggacaagcg tgttggaaca     300
   aagaccaaac caccatgtcc catatgccca gcctgtgaat caccagggcc ctcggtcttc     360
   atcttccctc caaaacccaa ggacaccctc atgatctccc ggacacccca ggtcacgtgc     420
   gtggtggttg atgtgagcca ggagaacccg gaggtccagt tctcctggta cgtggacggc     480
   gtagaggtgc acacggccca gacgaggcca aaggaggagc agttcaacag cacctaccgc     540
   gtggtcagcg tcctacccat ccagcaccag gactggctga acgggaagga gttcaagtgc     600
   aaggtcaaca acaaagacct cccagccccc atcacaagga tcatctccaa ggccaaaggg     660
   cagacccggg agccgcaggt gtacaccctg cccccacacg ccgaggagct gtccaggagc     720
   aaagtcagca taacctgcct ggtcattggc ttctacccac ctgacatcga tgtcgagtgg     780
   caaagaaacg gacagccgga gccagagggc aattaccgca ccaccccgcc ccagcaggac     840
   gtggacggga cctacttcct gtacagcaag ttctcggtgg acaaggccag ctggcagggt     900
   ggaggcatat tccagtgtgc ggtgatgcac gaggctctgc acaaccacta cacccagaag     960
   tctatctcca agactccggg taaatga                                         987

<210>  56
<211>  328
<212>  PRT
<213>  Sus scrofa

<400>  56

Ala Pro Lys Thr Ala Pro Leu Val Tyr Pro Leu Ala Pro Cys Gly Arg
1               5                   10                  15

Asp Thr Ser Gly Pro Asn Val Ala Leu Gly Cys Leu Ala Ser Ser Tyr

            20                  25                  30

Phe Pro Glu Pro Val Thr Val Thr Trp Asn Ser Gly Ala Leu Thr Ser
        35                  40                  45

Gly Val His Thr Phe Pro Ser Val Leu Gln Pro Ser Gly Leu Tyr Ser
    50                  55                  60

Leu Ser Ser Met Val Thr Val Pro Ala Ser Ser Leu Ser Ser Lys Ser
65                  70                  75                  80

Tyr Thr Cys Asn Val Asn His Pro Ala Thr Thr Thr Lys Val Asp Lys
                85                  90                  95

Arg Val Gly Thr Lys Thr Lys Pro Pro Cys Pro Ile Cys Pro Ala Cys
            100                 105                 110

Glu Ser Pro Gly Pro Ser Val Phe Ile Phe Pro Pro Lys Pro Lys Asp
        115                 120                 125

Thr Leu Met Ile Ser Arg Thr Pro Gln Val Thr Cys Val Val Val Asp
    130                 135                 140

Val Ser Gln Glu Asn Pro Glu Val Gln Phe Ser Trp Tyr Val Asp Gly
145                 150                 155                 160

Val Glu Val His Thr Ala Gln Thr Arg Pro Lys Glu Glu Gln Phe Asn
                165                 170                 175

Ser Thr Tyr Arg Val Val Ser Val Leu Pro Ile Gln His Gln Asp Trp
            180                 185                 190

Leu Asn Gly Lys Glu Phe Lys Cys Lys Val Asn Asn Lys Asp Leu Pro
        195                 200                 205

Ala Pro Ile Thr Arg Ile Ile Ser Lys Ala Lys Gly Gln Thr Arg Glu
    210                 215                 220

Pro Gln Val Tyr Thr Leu Pro Pro His Ala Glu Glu Leu Ser Arg Ser
225                 230                 235                 240

Lys Val Ser Ile Thr Cys Leu Val Ile Gly Phe Tyr Pro Pro Asp Ile
                245                 250                 255

Asp Val Glu Trp Gln Arg Asn Gly Gln Pro Glu Pro Glu Gly Asn Tyr
            260                 265                 270

Arg Thr Thr Pro Pro Gln Gln Asp Val Asp Gly Thr Tyr Phe Leu Tyr
        275                 280                 285

Ser Lys Phe Ser Val Asp Lys Ala Ser Trp Gln Gly Gly Gly Ile Phe
    290                 295                 300

Gln Cys Ala Val Met His Glu Ala Leu His Asn His Tyr Thr Gln Lys
305                 310                 315                 320

Ser Ile Ser Lys Thr Pro Gly Lys
                325

<210>  57
<211>  987
<212>  DNA
<213>  Sus scrofa

<400>  57
   gcccccaaga cggccccatt ggtctaccct ctggccccct gcggcaggga cacgtctggc      60
   cctaacgtgg ccttgggctg cctggcctca agctacttcc ccgagccagt gaccgtgacc     120
   tggaactcgg gcgccctgac cagtggcgtg cataccttcc catccgtcct gcagccgtca     180
   gggctctact ccctcagcag catggtgacc gtgccggcca gcagcctgtc cagcaagagc     240
   tacacctgca atgtcaacca cccggccacc accaccaagg tggacaagcg tgttggaaca     300
   aagaccaaac caccatgtcc catatgccca gcctgtgaat cgccagggcc ctcggtcttc     360
   atcttccctc caaaacccaa ggacaccctc atgatctccc ggacacccca ggtcacgtgc     420
   gtggtagttg atgtgagcca ggagaacccg gaggtccagt tctcctggta cgtggacggc     480
   gtagaggtgc acacggccca gacgaggcca aaggaggagc agttcaacag cacctaccgc     540
   gtggtcagcg tcctgcccat ccagcaccag gactggctga acgggaagga gttcaagtgc     600
   aaggtcaaca acaaagacct cccagccccc atcacaagga tcatctccaa ggccaaaggg     660
   cagacccggg agccgcaggt gtacaccctg cccccacacg ccgaggagct gtccaggagc     720
   aaagtcagca taacctgcct ggtcattggc ttctacccac ctgacatcga tgtcgagtgg     780
   caaagaaacg gacagccgga gccagagggc aattaccgca ccaccccgcc ccagcaggac     840
   gtggacggga cctacttcct gtacagcaag ttctcggtgg acaaggccag ctggcagggt     900
   ggaggcatat tccagtgtgc ggtgatgcac gaggctctgc acaaccacta cacccagaag     960
   tctatctcca agactccggg taaatga                                         987

<210>  58
<211>  333
<212>  PRT
<213>  Sus scrofa

<400>  58

Ala Tyr Asn Thr Ala Pro Ser Val Tyr Pro Leu Ala Pro Cys Gly Arg
1               5                   10                  15

Asp Val Ser Asp His Asn Val Ala Leu Gly Cys Leu Val Ser Ser Tyr
            20                  25                  30

Phe Pro Glu Pro Val Thr Val Thr Trp Asn Ser Gly Ala Leu Ser Arg
        35                  40                  45

Val Val His Thr Phe Pro Ser Val Leu Gln Pro Ser Gly Leu Tyr Ser
    50                  55                  60

Leu Ser Ser Met Val Ile Val Ala Ala Ser Ser Leu Ser Thr Leu Ser
65                  70                  75                  80

Tyr Thr Cys Asn Val Tyr His Pro Ala Thr Asn Thr Lys Val Asp Lys
                85                  90                  95

Arg Val Asp Ile Glu Pro Pro Thr Pro Ile Cys Pro Glu Ile Cys Ser
            100                 105                 110

Cys Pro Ala Ala Glu Val Leu Gly Ala Pro Ser Val Phe Leu Phe Pro
        115                 120                 125

Pro Lys Pro Lys Asp Ile Leu Met Ile Ser Arg Thr Pro Lys Val Thr
    130                 135                 140

Cys Val Val Val Asp Val Ser Gln Glu Glu Ala Glu Val Gln Phe Ser
145                 150                 155                 160

Trp Tyr Val Asp Gly Val Gln Leu Tyr Thr Ala Gln Thr Arg Pro Met
                165                 170                 175

Glu Glu Gln Phe Asn Ser Thr Tyr Arg Val Val Ser Val Leu Pro Ile
            180                 185                 190

Gln His Gln Asp Trp Leu Lys Gly Lys Glu Phe Lys Cys Lys Val Asn
        195                 200                 205

Asn Lys Asp Leu Leu Ser Pro Ile Thr Arg Thr Ile Ser Lys Ala Thr
    210                 215                 220

Gly Pro Ser Arg Val Pro Gln Val Tyr Thr Leu Pro Pro Ala Trp Glu
225                 230                 235                 240

Glu Leu Ser Lys Ser Lys Val Ser Ile Thr Cys Leu Val Thr Gly Phe
                245                 250                 255

Tyr Pro Pro Asp Ile Asp Val Glu Trp Gln Ser Asn Gly Gln Gln Glu
            260                 265                 270

Pro Glu Gly Asn Tyr Arg Thr Thr Pro Pro Gln Gln Asp Val Asp Gly
        275                 280                 285

Thr Tyr Phe Leu Tyr Ser Lys Leu Ala Val Asp Lys Val Arg Trp Gln
    290                 295                 300

Arg Gly Asp Leu Phe Gln Cys Ala Val Met His Glu Ala Leu His Asn
305                 310                 315                 320

His Tyr Thr Gln Lys Ser Ile Ser Lys Thr Gln Gly Lys
                325                 330

<210>  59
<211>  1002
<212>  DNA
<213>  Sus scrofa

<400>  59
gcctacaaca cagctccatc ggtctaccct ctggccccct gtggcaggga cgtgtctgat      60

cataacgtgg ccttgggctg ccttgtctca agctacttcc ccgagccagt gaccgtgacc     120

tggaactcgg gtgccctgtc cagagtcgtg cataccttcc catccgtcct gcagccgtca     180

gggctctact ccctcagcag catggtgatc gtggcggcca gcagcctgtc caccctgagc     240

tacacgtgca acgtctacca cccggccacc aacaccaagg tggacaagcg tgttgacatc     300

gaacccccca cacccatctg tcccgaaatt tgctcatgcc cagctgcaga ggtcctggga     360

gcaccgtcgg tcttcctctt ccctccaaaa cccaaggaca tcctcatgat ctcccggaca     420

cccaaggtca cgtgcgtggt ggtggacgtg agccaggagg aggctgaagt ccagttctcc     480

tggtacgtgg acggcgtaca gttgtacacg gcccagacga ggccaatgga ggagcagttc     540

aacagcacct accgcgtggt cagcgtcctg cccatccagc accaggactg gctgaagggg     600

aaggagttca agtgcaaggt caacaacaaa gacctccttt cccccatcac gaggaccatc     660

tccaaggcta cagggccgag ccgggtgccg caggtgtaca ccctgccccc agcctgggaa     720

gagctgtcca agagcaaagt cagcataacc tgcctggtca ctggcttcta cccacctgac     780

atcgatgtcg agtggcagag caacggacaa caagagccag agggcaatta ccgcaccacc     840

ccgccccagc aggacgtgga tgggacctac ttcctgtaca gcaagctcgc ggtggacaag     900

gtcaggtggc agcgtggaga cctattccag tgtgcggtga tgcacgaggc tctgcacaac     960

cactacaccc agaagtccat ctccaagact cagggtaaat ga                       1002

<210>  60
<211>  277
<212>  PRT
<213>  Sus scrofa

<400>  60

Thr Phe Pro Ser Val Leu Gln Pro Ser Gly Leu Tyr Ser Leu Ser Ser
1               5                   10                  15

Met Val Thr Val Pro Ala Ser Ser Leu Ser Ser Lys Ser Tyr Thr Cys
            20                  25                  30

Asn Val Asn His Pro Ala Thr Thr Thr Lys Val Asp Lys Arg Val Gly
        35                  40                  45

Thr Lys Thr Lys Pro Pro Cys Pro Ile Cys Pro Ala Cys Glu Gly Pro
    50                  55                  60

Gly Pro Ser Ala Phe Ile Phe Pro Pro Lys Pro Lys Asp Thr Leu Met
65                  70                  75                  80

Ile Ser Arg Thr Pro Lys Val Thr Cys Val Val Val Asp Val Ser Gln
                85                  90                  95

Glu Asn Pro Glu Val Gln Phe Ser Trp Tyr Val Asp Gly Val Glu Val
            100                 105                 110

His Thr Ala Gln Thr Arg Pro Lys Glu Glu Gln Phe Asn Ser Thr Tyr
        115                 120                 125

Arg Val Val Ser Val Leu Pro Ile Gln His Gln Asp Trp Leu Asn Gly
    130                 135                 140

Lys Glu Phe Lys Cys Lys Val Asn Asn Lys Asp Leu Pro Ala Pro Ile
145                 150                 155                 160

Thr Arg Ile Ile Ser Lys Ala Lys Gly Gln Thr Arg Glu Pro Gln Val
                165                 170                 175

Tyr Thr Leu Pro Pro Pro Thr Glu Glu Leu Ser Arg Ser Lys Val Thr
            180                 185                 190

Leu Thr Cys Leu Val Thr Gly Phe Tyr Pro Pro Asp Ile Asp Val Glu
        195                 200                 205

Trp Gln Arg Asn Gly Gln Pro Glu Pro Glu Gly Asn Tyr Arg Thr Thr
    210                 215                 220

Pro Pro Gln Gln Asp Val Asp Gly Thr Tyr Phe Leu Tyr Ser Lys Leu
225                 230                 235                 240

Ala Val Asp Lys Ala Ser Trp Gln Arg Gly Asp Thr Phe Gln Cys Ala
                245                 250                 255

Val Met His Glu Ala Leu His Asn His Tyr Thr Gln Lys Ser Ile Phe
            260                 265                 270

Lys Thr Pro Gly Lys
        275

<210>  61
<211>  834
<212>  DNA
<213>  Sus scrofa

<400>  61
accttcccat ccgtcctgca gccgtcaggg ctctactccc tcagcagcat ggtgaccgtg      60

ccggccagca gcctgtccag caagagctac acctgcaatg tcaaccaccc ggccaccacc     120

accaaggtgg acaagcgtgt tggaacaaag accaaaccac catgtcccat atgcccagcc     180

tgtgaagggc ccgggccctc ggccttcatc ttccctccaa aacccaagga caccctcatg     240

atctcccgga cccccaaggt cacgtgcgtg gtggtagatg tgagccagga gaacccggag     300

gtccagttct cctggtacgt ggacggcgta gaggtgcaca cggcccagac gaggccaaag     360

gaggagcagt tcaacagcac ctaccgcgtg gtcagcgtcc tgcccatcca gcaccaggac     420

tggctgaacg ggaaggagtt caagtgcaag gtcaacaaca aagacctccc agcccccatc     480

acaaggatca tctccaaggc caaagggcag acccgggagc cgcaggtgta caccctgccc     540

ccacccaccg aggagctgtc caggagcaaa gtcacgctaa cctgcctggt cactggcttc     600

tacccacctg acatcgatgt cgagtggcaa agaaacggac agccggagcc agagggcaat     660

taccgcacca ccccgcccca gcaggacgtg gacgggacct acttcctgta cagcaagctc     720

gcggtggaca aggccagctg gcagcgtgga gacacattcc agtgtgcggt gatgcacgag     780

gctctgcaca accactacac ccagaagtcc atcttcaaga ctccgggtaa atga           834

<210>  62
<211>  318
<212>  PRT
<213>  Sus scrofa

<400>  62

Ala Pro Lys Thr Ala Pro Ser Val Tyr Pro Leu Ala Pro Cys Gly Arg
1               5                   10                  15

Asp Val Ser Gly Pro Asn Val Ala Leu Gly Cys Leu Ala Ser Ser Tyr

            20                  25                  30

Phe Pro Glu Pro Val Thr Val Thr Trp Asn Ser Gly Ala Leu Thr Ser
        35                  40                  45

Gly Val His Thr Phe Pro Ser Val Leu Gln Pro Ser Gly Leu Tyr Ser
    50                  55                  60

Leu Ser Ser Met Val Thr Val Pro Ala Ser Ser Leu Ser Ser Lys Ser
65                  70                  75                  80

Tyr Thr Cys Asn Val Asn His Pro Ala Thr Thr Thr Lys Val Asp Lys
                85                  90                  95

Arg Val Gly Ile His Gln Pro Gln Thr Cys Pro Ile Cys Pro Ala Cys
            100                 105                 110

Glu Gly Pro Gly Pro Ser Ala Phe Ile Phe Pro Pro Lys Pro Lys Asp
        115                 120                 125

Thr Leu Met Ile Ser Arg Thr Pro Lys Val Thr Cys Val Val Val Asp
    130                 135                 140

Val Ser Gln Glu Asn Pro Glu Val Gln Phe Ser Trp Tyr Val Asp Gly
145                 150                 155                 160

Val Glu Val His Thr Ala Gln Thr Arg Pro Lys Glu Glu Gln Phe Asn
                165                 170                 175

Ser Thr Tyr Arg Val Val Ser Val Leu Leu Ile Gln His Gln Asp Trp
            180                 185                 190

Leu Asn Gly Lys Glu Phe Lys Cys Lys Val Asn Asn Lys Asp Leu Pro
        195                 200                 205

Ala Pro Ile Thr Arg Ile Ile Ser Lys Ala Lys Gly Gln Thr Arg Glu
    210                 215                 220

Pro Gln Val Tyr Thr Leu Pro Pro Pro Thr Glu Glu Leu Ser Arg Ser
225                 230                 235                 240

Lys Val Thr Leu Thr Cys Leu Val Thr Gly Phe Tyr Pro Pro Asp Ile
                245                 250                 255

Asp Val Glu Trp Gln Arg Asn Gly Gln Pro Glu Pro Glu Gly Asn Tyr
            260                 265                 270

Arg Thr Thr Pro Pro Gln Gln Asp Val Asp Gly Thr Tyr Phe Leu Tyr
        275                 280                 285

Ser Lys Leu Ala Val Asp Lys Ala Ser Trp Gln Arg Gly Asp Thr Phe
    290                 295                 300

Gln Cys Ala Val Met His Glu Ala Leu His Asn His Tyr Thr
305                 310                 315

<210>  63
<211>  955
<212>  DNA
<213>  Sus scrofa

<400>  63
gcccccaaga cggccccatc ggtctaccct ctggccccct gcggcaggga cgtgtctggc      60

cctaacgtgg ccttgggctg cctggcctca agctacttcc ccgagccagt gaccgtgacc     120

tggaactcgg gcgccctgac cagtggcgtg cacaccttcc catccgtcct gcagccgtca     180

gggctctact ccctcagcag catggtgacc gtgccggcca gcagcctgtc cagcaagagc     240

tacacctgca atgtcaacca cccggccacc accaccaagg tggacaagcg tgttggaata     300

caccagccgc aaacatgtcc catatgccca gcctgtgaag ggcccgggcc ctcggccttc     360

atcttccctc caaaacccaa ggacaccctc atgatctccc ggacccccaa ggtcacgtgc     420

gtggtggttg atgtgagcca ggagaacccg gaggtccagt tctcctggta cgtggacggc     480

gtagaggtgc acacggccca gacgaggcca aaggaggagc agttcaacag cacctaccgc     540

gtggtcagcg tcctgctcat ccagcaccag gactggctga acgggaagga gttcaagtgc     600

aaggtcaaca acaaagacct cccagccccc atcacaagga tcatctccaa ggccaaaggg     660

cagacccggg agccgcaggt gtacaccctg cccccaccca ccgaggagct gtccaggagc     720

aaagtcacgc taacctgcct ggtcactggc ttctacccac ctgacatcga tgtcgagtgg     780

caaagaaacg gacagccgga gccagagggc aattaccgca ccaccccgcc ccagcaggac     840

gtggacggga cctacttcct gtacagcaag ctcgcggtgg acaaggccag ctggcagcgt     900

ggagacacat tccagtgtgc ggtgatgcac gaggctctgc acaaccacta caccc          955

<210>  64
<211>  323
<212>  PRT
<213>  Sus scrofa

<400>  64

Ala Pro Lys Thr Ala Pro Ser Val Tyr Pro Leu Ala Pro Cys Ser Arg
1               5                   10                  15

Asp Thr Ser Gly Pro Asn Val Ala Leu Gly Cys Leu Val Ser Ser Tyr

20                  25                  30

Phe Pro Glu Pro Val Thr Val Thr Trp Asn Ser Gly Ala Leu Thr Ser
        35                  40                  45

Gly Val His Thr Phe Pro Ser Val Leu Gln Pro Ser Gly Leu Tyr Ser
    50                  55                  60

Leu Ser Ser Met Val Thr Val Pro Ala His Ser Leu Ser Ser Lys Arg
65                  70                  75                  80

Tyr Thr Cys Asn Val Asn His Pro Ala Thr Lys Thr Lys Val Asp Leu
                85                  90                  95

Cys Val Gly Arg Pro Cys Pro Ile Cys Pro Gly Cys Glu Val Ala Gly
            100                 105                 110

Pro Ser Val Phe Ile Phe Pro Pro Lys Pro Lys Asp Ile Leu Met Ile
        115                 120                 125

Ser Arg Thr Pro Glu Val Thr Cys Val Val Val Asp Val Ser Lys Glu
    130                 135                 140

His Ala Glu Val Gln Phe Ser Trp Tyr Val Asp Gly Glu Glu Val His
145                 150                 155                 160

Thr Ala Glu Thr Arg Pro Lys Glu Glu Gln Phe Asn Ser Thr Tyr Arg
                165                 170                 175

Val Val Ser Val Leu Pro Ile Gln His Glu Asp Trp Leu Lys Gly Lys
            180                 185                 190

Glu Phe Glu Cys Lys Val Asn Asn Glu Asp Leu Pro Gly Pro Ile Thr
        195                 200                 205

Arg Thr Ile Ser Lys Ala Lys Gly Val Val Arg Ser Pro Glu Val Tyr
    210                 215                 220

Thr Leu Pro Pro Pro Ala Glu Glu Leu Ser Lys Ser Ile Val Thr Leu
225                 230                 235                 240

Thr Cys Leu Val Lys Ser Ile Phe Pro Phe Ile His Val Glu Trp Lys
                245                 250                 255

Ile Asn Gly Lys Pro Glu Pro Glu Asn Ala Tyr Arg Thr Thr Pro Pro
            260                 265                 270

Gln Glu Asp Glu Asp Arg Thr Tyr Phe Leu Tyr Ser Lys Leu Ala Val
        275                 280                 285

Asp Lys Ala Arg Trp Asp His Gly Glu Thr Phe Glu Cys Ala Val Met
    290                 295                 300

His Glu Ala Leu His Asn His Tyr Thr Gln Lys Ser Ile Ser Lys Thr
305                 310                 315                 320

Gln Gly Lys

<210>  65
<211>  975
<212>  DNA
<213>  Sus scrofa

<220>
<221>  misc_feature
<222>  (748)..(748)
<223>  n is a, c, g, or t

<400>  65
   gcccccaaga cggccccatc ggtctaccct ctggccccct gcagcaggga cacgtctggc      60
   cctaacgtgg ccttgggctg cctggtctca agctacttcc ccgagccagt gaccgtgacc     120
   tggaactcgg gcgccctgac cagtggcgtg cacaccttcc catccgtcct gcagccgtca     180
   gggctctact ccctcagcag catggtgacc gtgccggccc acagcttgtc cagcaagcgc     240
   tatacgtgca atgtcaacca cccagccacc aaaaccaagg tggacctgtg tgttggacga     300
   ccatgtccca tatgcccagg ctgtgaagtg gccgggccct cggtcttcat cttccctcca     360
   aaacccaagg acatcctcat gatctcccgg acccccgagg tcacgtgcgt ggtggtggac     420
   gtcagcaagg agcacgccga ggtccagttc tcctggtacg tggacggcga agaggtgcac     480
   acggccgaga cgaggccaaa ggaggagcag ttcaacagca cctaccgcgt ggtcagcgtc     540
   ctgcccatcc agcacgagga ctggctgaag gggaaggagt tcgagtgcaa ggtcaacaac     600
   gaagacctcc caggccccat cacgaggacc atctccaagg ccaaaggggt ggtacggagc     660
   ccggaggtgt acaccctgcc cccacccgcc gaggagctgt ccaagagcat agtcacgcta     720
   acctgcctgg tcaaaagcat cttcccgnct ttcatccatg ttgagtggaa aatcaacgga     780
   aaaccagagc cagagaacgc atatcgcacc accccgcctc aggaggacga ggacaggacc     840
   tacttcctgt acagcaagct cgcggtggac aaggcaagat gggaccatgg agaaacattt     900
   gagtgtgcgg tgatgcacga ggctctgcac aaccactaca cccagaagtc catctccaag     960
   actcagggta aatga                                                      975

<210>  66
<211>  317
<212>  PRT
<213>  Sus scrofa

<400>  66

Ala Tyr Asn Thr Ala Pro Ser Val Tyr Pro Leu Ala Pro Cys Gly Arg
1               5                   10                  15

Asp Val Ser Asp His Asn Val Ala Leu Gly Cys Leu Val Ser Ser Tyr
            20                  25                  30

Phe Pro Glu Pro Val Thr Val Thr Trp Asn Trp Gly Ala Gln Thr Ser
        35                  40                  45

Gly Val His Thr Phe Pro Ser Val Leu Gln Pro Ser Gly Leu Tyr Ser
    50                  55                  60

Leu Ser Ser Thr Val Thr Val Pro Ala His Ser Leu Ser Ser Lys Cys
65                  70                  75                  80

Phe Thr Cys Asn Val Asn His Pro Ala Thr Thr Thr Lys Val Asp Leu
                85                  90                  95

Cys Val Gly Lys Lys Thr Lys Pro Arg Cys Pro Ile Cys Pro Gly Cys
            100                 105                 110

Glu Val Ala Gly Pro Ser Val Phe Ile Phe Pro Pro Lys Pro Lys Asp
        115                 120                 125

Ile Leu Met Ile Ser Arg Thr Pro Glu Val Thr Cys Val Val Val Asp
    130                 135                 140

Val Ser Lys Glu His Ala Glu Val Gln Phe Ser Trp Tyr Val Asp Gly
145                 150                 155                 160

Glu Glu Val His Thr Ala Glu Thr Arg Pro Lys Glu Glu Gln Phe Asn
                165                 170                 175

Ser Thr Tyr Arg Val Val Ser Val Leu Pro Ile Gln His Glu Asp Trp
            180                 185                 190

Leu Lys Gly Lys Glu Phe Glu Cys Lys Val Asn Asn Glu Asp Leu Pro
        195                 200                 205

Gly Pro Ile Thr Arg Thr Ile Ser Lys Ala Lys Gly Val Val Arg Ser
    210                 215                 220

Pro Glu Val Tyr Thr Leu Pro Pro Pro Ala Glu Glu Leu Ser Lys Ser
225                 230                 235                 240

Ile Val Thr Leu Thr Cys Leu Val Lys Ser Phe Phe Pro Pro Phe Ile
                245                 250                 255

His Val Glu Trp Lys Ile Asn Gly Lys Pro Glu Pro Glu Asn Ala Tyr
            260                 265                 270

Arg Thr Thr Pro Pro Gln Glu Asp Glu Asp Gly Thr Tyr Phe Leu Tyr
        275                 280                 285

Ser Lys Phe Ser Val Glu Lys Phe Arg Trp His Ser Gly Gly Ile His
    290                 295                 300

Cys Ala Val Met His Glu Ala Leu His Asn His Tyr Thr
305                 310                 315

<210>  67
<211>  952
<212>  DNA
<213>  Sus scrofa

<400>  67
gcctacaaca cagctccatc ggtctaccct ctggccccct gtggcaggga cgtgtctgat      60

cataacgtgg ccttgggctg cctggtctca agctacttcc ccgagccagt gaccgtgacc     120

tggaactggg gcgcccagac cagtggcgtg cacaccttcc catccgtcct gcagccgtca     180

gggctctact ccctcagcag cacggtgacc gtgccggccc acagcttgtc cagcaagtgc     240

ttcacgtgca atgtcaacca cccggccacc accaccaagg tggacctgtg tgttggaaaa     300

aagaccaagc ctcgatgtcc catatgccca ggctgtgaag tggccgggcc ctcggtcttc     360

atcttccctc caaaacccaa ggacatcctc atgatctccc ggacccccga ggtcacgtgc     420

gtggtggtgg acgtcagcaa ggagcacgcc gaggtccagt tctcctggta cgtggacggc     480

gaagaggtgc acacggccga gacgagacca aaggaggagc agttcaacag cacttaccgc     540

gtggtcagcg tcctgcccat ccagcacgag gactggctga aggggaagga gttcgagtgc     600

aaggtcaaca acgaagacct cccaggcccc atcacgagga ccatctccaa ggccaaaggg     660

gtggtacgga gcccggaggt gtacaccctg cccccacccg ccgaggagct gtccaagagc     720

atagtcacgc taacctgcct ggtcaaaagc ttcttcccgc ctttcatcca tgttgagtgg     780

aaaatcaacg gaaaaccaga gccagagaac gcataccgca ccaccccgcc ccaggaggac     840

gaggacggga cctacttcct gtacagcaag ttctcggtgg aaaagttcag gtggcacagt     900

ggaggcatcc actgtgcggt gatgcacgag gctctgcaca accactacac cc            952

<210>  68
<211>  314
<212>  PRT
<213>  Sus scrofa

<400>  68

Ala Pro Lys Thr Ala Pro Ser Val Tyr Pro Leu Ala Pro Cys Gly Arg
1               5                   10                  15

Asp Thr Ser Gly Pro Asn Val Ala Leu Gly Cys Leu Ala Ser Ser Tyr
            20                  25                  30

Phe Pro Glu Pro Val Thr Leu Thr Trp Asn Ser Gly Ala Leu Thr Ser
        35                  40                  45

Gly Val His Thr Phe Pro Ser Val Leu Gln Pro Ser Gly Leu Tyr Ser
    50                  55                  60

Leu Ser Ser Met Val Thr Val Pro Ala Ser Ser Leu Ser Ser Lys Ser
65                  70                  75                  80

Tyr Thr Cys Asn Val Asn His Pro Ala Thr Thr Thr Lys Val Asp Leu
                85                  90                  95

Cys Val Gly Arg Pro Cys Pro Ile Cys Pro Ala Cys Glu Gly Pro Gly
            100                 105                 110

Pro Ser Val Phe Ile Phe Pro Pro Lys Pro Lys Asp Thr Leu Met Ile
        115                 120                 125

Ser Arg Thr Pro Gln Val Thr Cys Val Val Val Asp Val Ser Gln Glu
    130                 135                 140

Asn Pro Glu Val Gln Phe Ser Trp Tyr Val Asp Gly Val Glu Val His
145                 150                 155                 160

Thr Ala Gln Thr Arg Pro Lys Glu Ala Gln Phe Asn Ser Thr Tyr Arg
                165                 170                 175

Val Val Ser Val Leu Pro Ile Gln His Glu Asp Trp Leu Lys Gly Lys
            180                 185                 190

Glu Phe Glu Cys Lys Val Asn Asn Lys Asp Leu Pro Ala Pro Ile Thr
        195                 200                 205

Arg Ile Ile Ser Lys Ala Lys Gly Pro Ser Arg Glu Pro Gln Val Tyr
    210                 215                 220

Thr Leu Ser Pro Ser Ala Glu Glu Leu Ser Arg Ser Lys Val Ser Ile
225                 230                 235                 240

Thr Cys Leu Val Thr Gly Phe Tyr Pro Pro Asp Ile Asp Val Glu Trp
                245                 250                 255

Lys Ser Asn Gly Gln Pro Glu Pro Glu Gly Asn Tyr Arg Thr Thr Pro
            260                 265                 270

Pro Gln Gln Asp Val Asp Gly Thr Tyr Phe Leu Tyr Ser Lys Leu Ala
        275                 280                 285

Val Asp Lys Ala Ser Trp Gln Arg Gly Asp Pro Phe Gln Cys Ala Val
    290                 295                 300

Met His Glu Ala Leu His Asn His Tyr Thr
305                 310

<210>  69
<211>  943
<212>  DNA
<213>  Sus scrofa

<400>  69
gcccccaaga cggccccatc ggtctaccct ctggccccct gcggcaggga cacgtctggc      60

cctaacgtgg ccttgggctg cctggcctca agctacttcc ccgagccagt gaccctgacc     120

tggaactcgg gcgccctgac cagtggcgtg cataccttcc catccgtcct gcagccgtca     180

gggctctact ccctcagcag catggtgacc gtgccggcca gcagcctgtc cagcaagagc     240

tacacctgca atgtcaacca cccggccacc accaccaagg tggacctgtg tgttggacga     300

ccatgtccca tatgcccagc ctgtgaaggg cccgggccct cggtcttcat cttccctcca     360

aaacccaagg acaccctcat gatctcccgg acaccccagg tcacgtgcgt ggtggtagat     420

gtgagccagg aaaacccgga ggtccagttc tcctggtatg tggacggtgt agaggtgcac     480

acggcccaga cgaggccaaa ggaggcgcag ttcaacagca cctaccgtgt ggtcagcgtc     540

ctgcccatcc agcacgagga ctggctgaag gggaaggagt tcgagtgcaa ggtcaacaac     600

aaagacctcc cagcccccat cacaaggatc atctccaagg ccaaagggcc gagccgggag     660

ccgcaggtgt acaccctgtc cccatccgcc gaggagctgt ccaggagcaa agtcagcata     720

acctgcctgg tcactggctt ctacccacct gacatcgatg tcgagtggaa gagcaacgga     780

cagccggagc cagagggcaa ttaccgcacc accccgcccc agcaggacgt ggacgggacc     840

tacttcctgt acagcaagct cgcggtggac aaggccagct ggcagcgtgg agacccattc     900

cagtgtgcgg tgatgcacga ggctctgcac aaccactaca ccc                       943

<210>  70
<211>  320
<212>  PRT
<213>  Sus scrofa

<400>  70

Ala Pro Lys Thr Ala Pro Ser Val Tyr Pro Leu Ala Pro Cys Gly Arg
1               5                   10                  15

Asp Thr Ser Gly Pro Asn Val Ala Leu Gly Cys Leu Ala Ser Ser Tyr
            20                  25                  30

Phe Pro Glu Pro Val Thr Val Thr Trp Asn Ser Gly Ala Leu Thr Ser
        35                  40                  45

Gly Val His Thr Phe Pro Ser Val Leu Gln Pro Ser Gly Leu Tyr Ser
    50                  55                  60

Leu Ser Ser Thr Val Thr Val Pro Ala Arg Ser Ser Ser Arg Lys Cys
65                  70                  75                  80

Phe Thr Cys Asn Val Asn His Pro Ala Thr Thr Thr Lys Val Asp Leu
                85                  90                  95

Cys Val Gly Arg Pro Cys Pro Ile Cys Pro Ala Cys Glu Gly Asn Gly
            100                 105                 110

Pro Ser Val Phe Ile Phe Pro Pro Lys Pro Lys Asp Thr Leu Met Ile
        115                 120                 125

Ser Arg Thr Pro Glu Val Thr Cys Val Val Val Asp Val Ser Gln Glu
    130                 135                 140

Asn Pro Glu Val Gln Phe Ser Trp Tyr Val Asp Gly Glu Glu Val His
145                 150                 155                 160

Thr Ala Glu Thr Arg Pro Lys Glu Glu Gln Phe Asn Ser Thr Tyr Arg
                165                 170                 175

Val Val Ser Val Leu Pro Ile Gln His Gln Asp Trp Leu Lys Gly Lys
            180                 185                 190

Glu Phe Glu Cys Lys Val Asn Asn Lys Asp Leu Pro Ala Pro Ile Thr
        195                 200                 205

Arg Ile Ile Ser Lys Ala Lys Gly Pro Ser Arg Glu Pro Gln Val Tyr
    210                 215                 220

Thr Leu Ser Pro Ser Ala Glu Glu Leu Ser Arg Ser Lys Val Ser Ile
225                 230                 235                 240

Thr Cys Leu Val Thr Gly Phe Tyr Pro Pro Asp Ile Asp Val Glu Trp
                245                 250                 255

Lys Ser Asn Gly Gln Pro Glu Pro Glu Gly Asn Tyr Arg Ser Thr Pro
            260                 265                 270

Pro Gln Glu Asp Glu Asp Gly Thr Tyr Phe Leu Tyr Ser Lys Leu Ala
        275                 280                 285

Val Asp Lys Ala Arg Leu Gln Ser Gly Gly Ile His Cys Ala Val Met
    290                 295                 300

His Glu Ala Leu His Asn His Tyr Thr Gln Lys Ser Ile Ser Lys Thr
305                 310                 315                 320

<210>  71
<211>  960
<212>  DNA
<213>  Sus scrofa

<400>  71
gcccccaaga cggccccatc ggtctaccct ctggccccct gcggcaggga cacgtctggc      60

cctaacgtgg ccttgggctg cctggcctca agctacttcc ccgagccagt gaccgtgacc     120

tggaactcgg gcgccctgac cagtggcgtg cacaccttcc catccgtcct gcagccgtca     180

gggctctact ccctcagcag cacggtgacc gtgccggcca ggagctcgtc cagaaagtgc     240

ttcacgtgca atgtcaacca cccggccacc accaccaagg tggacctgtg tgttggacga     300

ccatgtccca tatgcccagc ctgtgaaggg aacgggccct cggtcttcat cttccctcca     360

aaacccaagg acaccctcat gatctcccgg acccccgagg tcacgtgcgt ggtggtagat     420

gtgagccagg aaaacccgga ggtccagttc tcctggtacg tggacggcga agaggtgcac     480

acggccgaga cgaggccaaa ggaggagcag ttcaacagca cctaccgtgt ggtcagcgtc     540

ctgcccatcc agcaccagga ctggctgaag ggaaaggagt tcgagtgcaa ggtcaacaac     600

aaagacctcc cagcccccat cacaaggatc atctccaagg ccaaagggcc gagccgggag     660

ccgcaggtgt acaccctgtc cccatccgcc gaggagctgt ccaggagcaa agtcagcata     720

acctgcctgg tcactggctt ctacccacct gacatcgatg tcgagtggaa gagcaacgga     780

cagccggagc cagagggcaa ttaccgctcc accccgcccc aggaggacga ggacgggacc     840

tacttcctgt acagcaaact cgcggtggac aaggcgaggt tgcagagtgg aggcatccac     900

tgtgcggtga tgcacgaggc tctgcacaac cactacaccc agaagtccat ctccaagact     960

<210>  72
<211>  266
<212>  PRT
<213>  Bubalus bubalis

<400>  72

Ser Gly Val His Thr Phe Pro Ala Val Leu Gln Ser Ser Gly Leu Tyr
1               5                   10                  15

Ser Leu Ser Ser Thr Val Thr Ala Pro Ala Ser Ala Thr Lys Ser Gln
            20                  25                  30

Thr Phe Thr Cys Asn Val Ala His Pro Ala Ser Ser Thr Lys Val Asp
        35                  40                  45

Lys Ala Val Val Pro Pro Cys Arg Pro Lys Pro Cys Asp Cys Cys Pro
    50                  55                  60

Pro Pro Glu Leu Pro Gly Gly Pro Ser Val Phe Ile Phe Pro Pro Lys
65                  70                  75                  80

Pro Lys Asp Thr Leu Thr Ile Ser Gly Thr Pro Glu Val Thr Cys Val
                85                  90                  95

Val Val Asp Val Gly His Asp Asp Pro Glu Val Lys Phe Ser Trp Phe
            100                 105                 110

Val Asp Asp Val Glu Val Asn Thr Ala Arg Thr Lys Pro Arg Glu Glu
        115                 120                 125

Gln Phe Asn Ser Thr Tyr Arg Val Val Ser Ala Leu Pro Ile Gln His
    130                 135                 140

Asn Asp Trp Thr Gly Gly Lys Glu Phe Lys Cys Lys Val Tyr Asn Glu
145                 150                 155                 160

Gly Leu Pro Ala Pro Ile Val Arg Thr Ile Ser Arg Thr Lys Gly Gln
                165                 170                 175

Ala Arg Glu Pro Gln Val Tyr Val Leu Ala Pro Pro Gln Asp Glu Leu
            180                 185                 190

Ser Lys Ser Thr Val Ser Ile Thr Cys Met Val Thr Gly Phe Tyr Pro
        195                 200                 205

Asp Tyr Ile Ala Val Glu Trp Gln Lys Asp Gly Gln Pro Glu Ser Glu
    210                 215                 220

Asp Lys Tyr Gly Thr Thr Pro Pro Gln Leu Asp Ser Asp Gly Ser Tyr
225                 230                 235                 240

Phe Leu Tyr Ser Arg Leu Arg Val Asn Lys Asn Ser Trp Gln Glu Gly
                245                 250                 255

Gly Ala Tyr Thr Cys Val Val Met His Glu
            260                 265

<210>  73
<211>  801
<212>  DNA
<213>  Bubalus bubalis

<400>  73
gagcggcgtg cacaccttcc cggccgtcct tcagtcctcc gggctctact ctctcagcag      60

cacggtgacc gcgcccgcca gcgccacaaa aagccagacc ttcacctgca acgtagccca     120

cccggccagc agcaccaagg tggacaaggc tgttgttccc ccatgcagac cgaaaccctg     180

tgattgctgc ccaccccctg agctccccgg aggaccctct gtcttcatct tcccaccaaa     240

acccaaggac accctcacaa tctctggaac tcctgaggtc acgtgtgtgg tggtggacgt     300

gggccacgat gaccccgagg tgaagttctc ctggttcgtg gacgatgtgg aggtaaacac     360

agccaggacg aagccaagag aggagcagtt caacagcacc taccgcgtgg tcagcgccct     420

gcccatccag cacaacgact ggactggagg aaaggagttc aagtgcaagg tctacaatga     480

aggcctccca gcccccatcg tgaggaccat ctccaggacc aaagggcagg cccgggagcc     540

gcaggtgtac gtcctggccc caccccagga cgagctcagc aaaagcacgg tcagcatcac     600

ttgcatggtc actggcttct acccagacta catcgccgta gagtggcaga aagatgggca     660

gcctgagtca gaggacaaat atggcacgac cccgccccag ctggacagcg atggctccta     720

cttcctgtac agcaggctca gggtgaacaa gaacagctgg caagaaggag gcgcctacac     780

gtgtgtagtg atgcatgagg c                                               801

<210>  74
<211>  309
<212>  PRT
<213>  Bulalus bubalis

<400>  74

Ala Ser Ile Thr Ala Pro Lys Val Tyr Pro Leu Thr Ser Cys Arg Gly
1               5                   10                  15

Glu Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser

35                  40                  45

Gly Val His Thr Phe Pro Ala Val Leu Gln Ser Ser Gly Leu Tyr Ser
    50                  55                  60

Leu Ser Ser Thr Val Thr Ala Pro Ala Ser Ala Thr Lys Ser Gln Thr
65                  70                  75                  80

Phe Thr Cys Asn Val Ala His Pro Ala Ser Ser Thr Lys Val Asp Thr
                85                  90                  95

Ala Val Gly Phe Ser Ser Asp Cys Cys Lys Phe Pro Lys Pro Cys Val
            100                 105                 110

Arg Gly Pro Ser Val Phe Ile Phe Pro Pro Lys Pro Lys Asp Thr Leu
        115                 120                 125

Met Ile Thr Gly Asn Pro Glu Val Thr Cys Val Val Val Asp Val Gly
    130                 135                 140

Arg Asp Asn Pro Glu Val Gln Phe Ser Trp Phe Val Gly Asp Val Glu
145                 150                 155                 160

Val His Thr Gly Arg Ser Lys Pro Arg Glu Glu Gln Phe Asn Ser Thr
                165                 170                 175

Tyr Arg Val Val Ser Thr Leu Pro Ile Gln His Asn Asp Trp Thr Gly
            180                 185                 190

Gly Lys Glu Phe Lys Cys Lys Val Asn Asn Lys Gly Leu Pro Ala Pro
        195                 200                 205

Ile Val Arg Thr Ile Ser Arg Thr Lys Gly Gln Ala Arg Glu Pro Gln
    210                 215                 220

Val Tyr Val Leu Ala Pro Pro Gln Glu Glu Leu Ser Lys Ser Thr Val
225                 230                 235                 240

Ser Val Thr Cys Met Val Thr Gly Phe Tyr Pro Asp Tyr Ile Ala Val
                245                 250                 255

Glu Trp His Arg Asp Arg Gln Ala Glu Ser Glu Asp Lys Tyr Arg Thr
            260                 265                 270

Thr Pro Pro Gln Leu Asp Ser Asp Gly Ser Tyr Phe Leu Tyr Ser Arg
        275                 280                 285

Leu Lys Val Asn Lys Asn Ser Trp Gln Glu Gly Gly Ala Tyr Thr Cys
    290                 295                 300

Val Val Met His Glu
305

<210>  75
<211>  929
<212>  DNA
<213>  Bubalus bubalis

<400>  75
gcctccatca cagccccgaa agtctaccct ctgacttctt gccgcgggga aacgtccagc      60

tccaccgtga ccctgggctg cctggtctcc agctacatgc ccgagccggt gaccgtgacc     120

tggaactcgg gtgccctgaa gagcggcgtg cacaccttcc cggccgtcct tcagtcctct     180

gggctctact ctctcagcag cacggtgacc gcgcccgcca gcgccacaaa aagccagacc     240

ttcacctgca acgtagccca cccggccagc agcaccaagg tggacacggc tgttgggttc     300

tccagtgact gctgcaagtt tcctaagcct tgtgtgaggg gaccatctgt cttcatcttc     360

ccgccgaaac ccaaagacac cctgatgatc acaggaaatc ccgaggtcac atgtgtggtg     420

gtggacgtgg gccgggataa ccccgaggtg cagttctcct ggttcgtggg tgatgtggag     480

gtgcacacgg gcaggtcgaa gccgagagag gagcagttca acagcaccta ccgcgtggtc     540

agcaccctgc ccatccagca caatgactgg actggaggaa aggagttcaa gtgcaaggtc     600

aacaacaaag gcctcccagc ccccatcgtg aggaccatct ccaggaccaa agggcaggcc     660

cgggagccgc aggtgtacgt cctggcccca ccccaggaag agctcagcaa aagcacggtc     720

agcgtcactt gcatggtcac tggcttctac ccagactaca tcgccgtaga gtggcataga     780

gaccggcagg ctgagtcgga ggacaagtac cgcacgaccc cgccccagct ggacagcgat     840

ggctcctact tcctgtacag caggctcaag gtgaacaaga acagctggca agaaggaggc     900

gcctacacgt gtgtagtgat gcatgaggc                                       929

<210>  76
<211>  352
<212>  PRT
<213>  Bubalus bubalis

<400>  76

Ala Ser Thr Thr Ala Pro Lys Val Tyr Pro Leu Ala Ser Ser Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Asn

35                  40                  45

Gly Val His Thr Phe Pro Ala Val Arg Gln Ser Ser Gly Leu Tyr Ser
    50                  55                  60

Leu Ser Ser Met Val Thr Met Pro Thr Ser Thr Ala Gly Thr Gln Thr
65                  70                  75                  80

Phe Thr Cys Asn Val Ala His Pro Ala Ser Ser Thr Lys Val Asp Thr
                85                  90                  95

Ala Val Thr Ala Arg His Pro Val Pro Lys Thr Pro Glu Thr Pro Ile
            100                 105                 110

His Pro Val Lys Pro Pro Thr Gln Glu Pro Arg Asp Glu Lys Thr Pro
        115                 120                 125

Cys Gln Cys Pro Lys Cys Pro Glu Pro Leu Gly Gly Leu Ser Val Phe
    130                 135                 140

Ile Phe Pro Pro Lys Pro Lys Asp Thr Leu Thr Ile Ser Gly Thr Pro
145                 150                 155                 160

Glu Val Thr Cys Val Val Val Asp Val Gly Gln Asp Asp Pro Glu Val
                165                 170                 175

Gln Phe Ser Trp Phe Val Asp Asp Val Glu Val His Thr Ala Arg Met
            180                 185                 190

Lys Pro Arg Glu Glu Gln Phe Asn Ser Thr Tyr Arg Val Val Ser Ala
        195                 200                 205

Leu Pro Ile Gln His Gln Asp Trp Leu Arg Glu Lys Glu Phe Lys Cys
    210                 215                 220

Lys Val Asn Asn Lys Gly Leu Pro Ala Pro Ile Val Arg Thr Ile Ser
225                 230                 235                 240

Arg Thr Lys Gly Gln Ala Arg Glu Pro Gln Val Tyr Val Leu Ala Pro
                245                 250                 255

Pro Arg Glu Glu Leu Ser Lys Ser Thr Leu Ser Leu Thr Cys Leu Ile
            260                 265                 270

Thr Gly Phe Tyr Pro Glu Glu Val Asp Val Glu Trp Gln Arg Asn Gly
        275                 280                 285

Gln Pro Glu Ser Glu Asp Lys Tyr His Thr Thr Pro Pro Gln Leu Asp
    290                 295                 300

Ala Asp Gly Ser Tyr Phe Leu Tyr Ser Arg Leu Arg Val Asn Arg Ser
305                 310                 315                 320

Ser Trp Gln Glu Gly Asp His Tyr Thr Cys Ala Val Met His Glu Ala
                325                 330                 335

Leu Arg Asn His Tyr Lys Glu Lys Pro Ile Ser Arg Ser Pro Gly Lys
            340                 345                 350

<210>  77
<211>  1059
<212>  DNA
<213>  Bubalus bubalis

<400>  77
gcctccacca cagccccgaa agtctaccct ctggcatcca gctgcgggga cacgtccagc      60

tccaccgtga ccctgggctg cctggtctcc agctacatgc ccgagccggt gaccgtgacc     120

tggaactcgg gtgccctgaa gaacggcgtg cacaccttcc cggccgtccg gcagtcctcc     180

gggctctact ctctcagcag catggtgacc atgcccacca gcaccgcagg aacccagacc     240

ttcacctgca acgtagccca cccggccagc agcaccaagg tggacacggc tgtcactgca     300

aggcatccgg tcccgaagac accagagaca cctatccatc ctgtaaaacc cccaacccag     360

gagcccagag atgaaaagac accctgccag tgtcccaaat gcccagaacc tctgggagga     420

ctgtctgtct tcatcttccc accgaaaccc aaggacaccc tcacaatctc tggaacgccc     480

gaggtcacgt gtgtggtggt ggacgtgggc caggatgacc ccgaagtgca gttctcctgg     540

ttcgtggatg acgtggaggt gcacacagcc aggatgaagc caagagagga gcagttcaac     600

agcacctacc gcgtggtcag cgccctgccc atccagcacc aggactggct gcgggaaaag     660

gagttcaagt gcaaggtcaa caacaaaggc ctcccggccc ccatcgtgag gaccatctcc     720

aggaccaaag ggcaggcccg ggagccacag gtgtatgtcc tggccccacc ccgggaagag     780

ctcagcaaaa gcacgctcag cctcacctgc ctaatcaccg gcttctaccc agaagaggta     840

gacgtggagt ggcagagaaa tgggcagcct gagtcagagg acaagtacca cacgacccca     900

ccccagctgg acgctgacgg ctcctacttc ctgtacagca ggctcagggt gaacaggagc     960

agctggcagg aaggagacca ctacacgtgt gcagtgatgc atgaagcttt acggaatcac    1020

tacaaagaga agcccatctc gaggtctccg ggtaaatga                           1059

<210>  78
<211>  105
<212>  PRT
<213>  Bubalus bubalis

<400>  78

Gln Pro Lys Ser Ala Pro Ser Val Thr Leu Phe Pro Pro Ser Thr Glu
1               5                   10                  15

Glu Leu Ser Ala Asn Lys Ala Thr Leu Val Cys Leu Ile Ser Asp Phe
            20                  25                  30

Tyr Pro Gly Ser Met Thr Val Ala Arg Lys Ala Asp Gly Ser Thr Ile
        35                  40                  45

Thr Arg Asn Val Glu Thr Thr Arg Ala Ser Lys Gln Ser Asn Ser Lys
    50                  55                  60

Tyr Ala Ala Ser Ser Tyr Leu Ser Leu Thr Gly Ser Glu Trp Lys Ser
65                  70                  75                  80

Lys Gly Ser Tyr Ser Cys Glu Val Thr His Glu Gly Ser Thr Val Thr
                85                  90                  95

Lys Thr Val Lys Pro Ser Glu Cys Ser
            100                 105

<210>  79
<211>  318
<212>  DNA
<213>  Bubalus buballis

<400>  79
cagcccaagt ccgcaccctc agtcaccctg ttcccaccct ccacggagga gctcagcgcc      60

aacaaggcca ccctggtgtg tctcatcagc gacttctacc cgggtagcat gaccgtggcc     120

aggaaggcag acggcagcac catcacccgg aacgtggaga ccacccgggc ctccaaacag     180

agcaacagca agtacgcggc cagcagctac ctgagcctga cgggcagcga gtggaaatcg     240

aaaggcagtt acagctgcga ggtcacgcac gaggggagca ccgtgacaaa gacagtgaag     300

ccctcagagt gttcttag                                                   318

<210>  80
<211>  229
<212>  PRT
<213>  Homo sapiens

<400>  80

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Val His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210> 81
<211> 690
<212> DNA
<213> Homo sapiens

<400> 81
gagtccaaat atggtccccc gtgcccatca tgcccagcac ctgagttcct ggggggacca 60

tcagtcttcc tgttcccccc aaaacccaag gacactctca tgatctcccg gacccctgag 120

gtcacgtgcg tggtggtgga cgtgagccag gaagaccccg aggtccagtt caactggtac 180

gtggatggcg tggaggtgca taatgccaag acaaagccgc gggaggagca gttcaacagc 240

acgtaccgtg tggtcagcgt cctcaccgtc gtgcaccagg actggctgaa cggcaaggag 300

tacaagtgca aggtctccaa caaaggcctc ccgtcctcca tcgagaaaac catctccaaa 360

gccaaagggc agccccgaga gccacaggtg tacaccctgc ccccatccca ggaggagatg 420

accaagaacc aggtcagcct gacctgcctg gtcaaaggct tctaccccag cgacatcgcc 480

gtggagtggg agagcaatgg gcagccggag aacaactaca agaccacgcc tcccgtgctg 540

gactccgacg gctccttctt cctctacagc aggctaaccg tggacaagag caggtggcag 600

gaggggaatg tcttctcatg ctccgtgatg catgaggctc tgcacaacca ctacacgcag 660

aagagcctct ccctgtctct gggtaaatga 690

<210> 82
<211> 217
<212> PRT
<213> Homo sapiens

<400> 82

Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Glu Gly Asn Val
            180                 185                 190

Phe Ser Cys Ser Val Met His Glu Ala Leu His Asn His Tyr Thr Gln
        195                 200                 205

Lys Ser Leu Ser Leu Ser Leu Gly Lys
    210                 215

<210>  83
<211>  654
<212>  DNA
<213>  Homo sapiens

<400>  83
gcacctgagt tcctgggggg accatcagtc ttcctgttcc ccccaaaacc caaggacact      60

ctcatgatct cccggacccc tgaggtcacg tgcgtggtgg tggacgtgag ccaggaagac     120

cccgaggtcc agttcaactg gtacgtggat ggcgtggagg tgcataatgc caagacaaag     180

ccgcgggagg agcagttcaa cagcacgtac cgtgtggtca gcgtcctcac cgtcctgcac     240

caggactggc tgaacggcaa ggagtacaag tgcaaggtct ccaacaaagg cctcccgtcc     300

tccatcgaga aaaccatctc caaagccaaa gggcagcccc gagagccaca ggtgtacacc     360

ctgcccccat cccaggagga gatgaccaag aaccaggtca gcctgacctg cctggtcaaa     420

ggcttctacc ccagcgacat cgccgtggag tgggagagca atgggcagcc ggagaacaac     480

tacaagacca cgcctcccgt gctggactcc gacggctcct tcttcctcta cagcaagctc     540

accgtggaca agagcaggtg gcaggagggg aacgtcttct catgctccgt gatgcatgag     600

gctctgcaca accactacac gcagaagagc ctctccctgt ctctgggtaa atga           654

<210>  84
<211>  329
<212>  PRT
<213>  Bos taurus

<400>  84

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr

                20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe
65                  70                  75                  80

Thr Cys Asn Val Ala His Pro Ala Ser Ser Thr Lys Val Asp Lys Ala
                85                  90                  95

Val Asp Pro Thr Cys Lys Pro Ser Pro Cys Asp Cys Cys Pro Pro Pro
            100                 105                 110

Glu Leu Pro Gly Gly Pro Ser Val Phe Ile Phe Pro Pro Lys Pro Lys
        115                 120                 125

Asp Thr Leu Thr Ile Ser Gly Thr Pro Glu Val Thr Cys Val Val Val
    130                 135                 140

Asp Val Gly His Asp Asp Pro Glu Val Lys Phe Ser Trp Phe Val Asp
145                 150                 155                 160

Asp Val Glu Val Asn Thr Ala Thr Thr Lys Pro Arg Glu Glu Gln Phe
                165                 170                 175

Asn Ser Thr Tyr Arg Val Val Ser Ala Leu Arg Ile Gln His Gln Asp
            180                 185                 190

Trp Thr Gly Gly Lys Glu Phe Lys Cys Lys Val His Asn Glu Gly Leu
        195                 200                 205

Pro Ala Pro Ile Val Arg Thr Ile Ser Arg Thr Lys Gly Pro Ala Arg
    210                 215                 220

Glu Pro Gln Val Tyr Val Leu Ala Pro Pro Gln Glu Glu Leu Ser Lys
225                 230                 235                 240

Ser Thr Val Ser Leu Thr Cys Met Val Thr Ser Phe Tyr Pro Asp Tyr
                245                 250                 255

Ile Ala Val Glu Trp Gln Arg Asn Gly Gln Pro Glu Ser Glu Asp Lys
            260                 265                 270

Tyr Gly Thr Thr Pro Pro Gln Leu Asp Ala Asp Ser Ser Tyr Phe Leu
        275                 280                 285

Tyr Ser Lys Leu Arg Val Asp Arg Asn Ser Trp Gln Glu Gly Asp Thr
    290                 295                 300

Tyr Thr Cys Val Val Met His Glu Ala Leu His Asn His Tyr Thr Gln
305                 310                 315                 320

Lys Ser Thr Ser Lys Ser Ala Gly Lys
                325

<210>  85
<211>  329
<212>  PRT
<213>  Bos taurus

<400>  85

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe
65                  70                  75                  80

Thr Cys Asn Val Ala His Pro Ala Ser Ser Thr Lys Val Asp Lys Ala
                85                  90                  95

Val Asp Pro Thr Cys Lys Pro Ser Pro Cys Asp Cys Cys Pro Pro Pro
            100                 105                 110

Glu Leu Pro Gly Gly Pro Ser Val Phe Ile Phe Pro Pro Lys Pro Lys
        115                 120                 125

Asp Thr Leu Thr Ile Ser Gly Thr Pro Glu Val Thr Cys Val Val Val
    130                 135                 140

Asp Val Gly His Asp Asp Pro Glu Val Lys Phe Ser Trp Phe Val Asp
145                 150                 155                 160

Asp Val Glu Val Asn Thr Ala Thr Thr Lys Pro Arg Glu Glu Gln Phe
                165                 170                 175

Asn Ser Thr Tyr Arg Val Val Ser Ala Leu Arg Ile Gln His Gln Asp
            180                 185                 190

Trp Thr Gly Gly Lys Glu Phe Lys Cys Lys Val His Asn Glu Gly Leu
        195                 200                 205

Pro Ala Pro Ile Val Arg Thr Ile Ser Arg Thr Lys Gly Pro Ala Arg
    210                 215                 220

Glu Pro Gln Val Tyr Val Leu Ala Pro Pro Gln Glu Glu Leu Ser Lys
225                 230                 235                 240

Ser Thr Val Ser Leu Thr Cys Met Val Thr Ser Phe Tyr Pro Asp Tyr
                245                 250                 255

Ile Ala Val Glu Trp Gln Arg Asn Gly Gln Pro Glu Ser Glu Asp Lys
            260                 265                 270

Tyr Gly Thr Thr Pro Pro Gln Leu Asp Ala Asp Ser Ser Tyr Phe Leu
        275                 280                 285

Tyr Ser Lys Leu Arg Val Asp Arg Asn Ser Trp Gln Glu Gly Asp Thr
    290                 295                 300

Tyr Thr Cys Val Val Met His Glu Ala Leu His Asn His Tyr Thr Gln
305                 310                 315                 320

Lys Ser Thr Ser Lys Ser Ala Gly Lys
                325

<210>  86
<211>  329
<212>  PRT
<213>  Bos taurus

<400>  86

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Thr Gln Thr
65                  70                  75                  80

Phe Thr Cys Asn Val Ala His Pro Ala Ser Ser Thr Lys Val Asp Lys
                85                  90                  95

Ala Val Asp Pro Arg Cys Lys Thr Thr Cys Asp Cys Cys Pro Pro Pro
            100                 105                 110

Glu Leu Pro Gly Gly Pro Ser Val Phe Ile Phe Pro Pro Lys Pro Lys
        115                 120                 125

Asp Thr Leu Thr Ile Ser Gly Thr Pro Glu Val Thr Cys Val Val Val
    130                 135                 140

Asp Val Gly His Asp Asp Pro Glu Val Lys Phe Ser Trp Phe Val Asp
145                 150                 155                 160

Asp Val Glu Val Asn Thr Ala Thr Thr Lys Pro Arg Glu Glu Gln Phe
                165                 170                 175

Asn Ser Thr Tyr Arg Val Val Ser Ala Leu Arg Ile Gln His Gln Asp
            180                 185                 190

Trp Thr Gly Gly Lys Glu Phe Lys Cys Lys Val His Asn Glu Gly Leu
        195                 200                 205

Pro Ala Pro Ile Val Arg Thr Ile Ser Arg Thr Lys Gly Pro Ala Arg
    210                 215                 220

Glu Pro Gln Val Tyr Val Leu Ala Pro Pro Gln Glu Glu Leu Ser Lys
225                 230                 235                 240

Ser Thr Val Ser Leu Thr Cys Met Val Thr Ser Phe Tyr Pro Asp Tyr
                245                 250                 255

Ile Ala Val Glu Trp Gln Arg Asn Gly Gln Pro Glu Ser Glu Asp Lys
            260                 265                 270

Tyr Gly Thr Thr Pro Pro Gln Leu Asp Ala Asp Gly Ser Tyr Phe Leu
        275                 280                 285

Tyr Ser Arg Leu Arg Val Asp Arg Asn Ser Trp Gln Glu Gly Asp Thr
    290                 295                 300

Tyr Thr Cys Val Val Met His Glu Ala Leu His Asn His Tyr Thr Gln
305                 310                 315                 320

Lys Ser Thr Ser Lys Ser Ala Gly Lys
                325

<210>  87
<211>  326
<212>  PRT
<213>  Bos taurus

<400>  87

Ala Ser Thr Thr Ala Pro Lys Val Tyr Pro Leu Ala Ser Ser Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Ala Ser Ser Ser Gly Gln Thr Phe
65                  70                  75                  80

Thr Cys Asn Val Ala His Pro Ala Ser Ser Thr Lys Val Asp Lys Ala
                85                  90                  95

Val Gly Val Ser Ile Asp Cys Ser Lys Cys His Asn Gln Pro Cys Val
            100                 105                 110

Arg Glu Pro Ser Val Phe Ile Phe Pro Pro Lys Pro Lys Asp Thr Leu
        115                 120                 125

Met Ile Thr Gly Thr Pro Glu Val Thr Cys Val Val Val Asn Val Gly
    130                 135                 140

His Asp Asn Pro Glu Val Gln Phe Ser Trp Phe Val Asp Asp Val Glu
145                 150                 155                 160

Val His Thr Ala Arg Ser Lys Pro Arg Glu Glu Gln Phe Asn Ser Thr
                165                 170                 175

Tyr Arg Val Val Ser Ala Leu Pro Ile Gln His Gln Asp Trp Thr Gly
            180                 185                 190

Gly Lys Glu Phe Lys Cys Lys Val Asn Asn Lys Gly Leu Ser Ala Pro
        195                 200                 205

Ile Val Arg Ile Ile Ser Arg Ser Lys Gly Pro Ala Arg Glu Pro Gln
    210                 215                 220

Val Tyr Val Leu Asp Pro Pro Lys Glu Glu Leu Ser Lys Ser Thr Leu
225                 230                 235                 240

Ser Val Thr Cys Met Val Thr Gly Phe Tyr Pro Glu Asp Val Ala Val
                245                 250                 255

Glu Trp Gln Arg Asn Arg Gln Thr Glu Ser Glu Asp Lys Tyr Arg Thr
            260                 265                 270

Thr Pro Pro Gln Leu Asp Thr Asp Arg Ser Tyr Phe Leu Tyr Ser Lys
        275                 280                 285

Leu Arg Val Asp Arg Asn Ser Trp Gln Glu Gly Asp Ala Tyr Thr Cys
    290                 295                 300

Val Val Met His Glu Ala Leu His Asn His Tyr Met Gln Lys Ser Thr
305                 310                 315                 320

Ser Lys Ser Ala Gly Lys
                325

<210>  88
<211>  326
<212>  PRT
<213>  Bos taurus

<400>  88

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe
65                  70                  75                  80

Thr Cys Asn Val Ala His Pro Ala Ser Ser Thr Lys Val Asp Lys Ala
                85                  90                  95

Val Gly Val Ser Ser Asp Cys Ser Lys Pro Asn Asn Gln His Cys Val
            100                 105                 110

Arg Glu Pro Ser Val Phe Ile Phe Pro Pro Lys Pro Lys Asp Thr Leu
        115                 120                 125

Met Ile Thr Gly Thr Pro Glu Val Thr Cys Val Val Val Asn Val Gly
    130                 135                 140

His Asp Asn Pro Glu Val Gln Phe Ser Trp Phe Val Asp Asp Val Glu
145                 150                 155                 160

Val His Thr Ala Arg Thr Lys Pro Arg Glu Glu Gln Phe Asn Ser Thr
                165                 170                 175

Tyr Arg Val Val Ser Ala Leu Pro Ile Gln His Gln Asp Trp Thr Gly
            180                 185                 190

Gly Lys Glu Phe Lys Cys Lys Val Asn Ile Lys Gly Leu Ser Ala Ser
        195                 200                 205

Ile Val Arg Ile Ile Ser Arg Ser Lys Gly Pro Ala Arg Glu Pro Gln
    210                 215                 220

Val Tyr Val Leu Asp Pro Pro Lys Glu Glu Leu Ser Lys Ser Thr Val
225                 230                 235                 240

Ser Val Thr Cys Met Val Ile Gly Phe Tyr Pro Glu Asp Val Asp Val
                245                 250                 255

Glu Trp Gln Arg Asp Arg Gln Thr Glu Ser Glu Asp Lys Tyr Arg Thr
            260                 265                 270

Thr Pro Pro Gln Leu Asp Ala Asp Arg Ser Tyr Phe Leu Tyr Ser Lys
        275                 280                 285

Leu Arg Val Asp Arg Asn Ser Trp Gln Arg Gly Asp Thr Tyr Thr Cys
    290                 295                 300

Val Val Met His Glu Ala Leu His Asn His Tyr Met Gln Lys Ser Thr
305                 310                 315                 320

Ser Lys Ser Ala Gly Lys
                325

<210>  89
<211>  327
<212>  PRT
<213>  Bos taurus

<400>  89

Ala Ser Thr Thr Ala Pro Lys Val Tyr Pro Leu Ser Ser Cys Cys Gly
1               5                   10                  15

Asp Lys Ser Ser Ser Gly Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Ala Ser Ser Ser Gly Thr Gln Thr
65                  70                  75                  80

Phe Thr Cys Asn Val Ala His Pro Ala Ser Ser Thr Lys Val Asp Lys
                85                  90                  95

Ala Val Gly Val Ser Ser Asp Cys Ser Lys Pro Asn Asn Gln His Cys
            100                 105                 110

Val Arg Glu Pro Ser Val Phe Ile Phe Pro Pro Lys Pro Lys Asp Thr
        115                 120                 125

Leu Met Ile Thr Gly Thr Pro Glu Val Thr Cys Val Val Val Asn Val
    130                 135                 140

Gly His Asp Asn Pro Glu Val Gln Phe Ser Trp Phe Val Asp Asp Val
145                 150                 155                 160

Glu Val His Thr Ala Arg Thr Lys Pro Arg Glu Glu Gln Phe Asn Ser
                165                 170                 175

Thr Tyr Arg Val Val Ser Ala Leu Pro Ile Gln His Gln Asp Trp Thr
            180                 185                 190

Gly Gly Lys Glu Phe Lys Cys Lys Val Asn Ile Lys Gly Leu Ser Ala
        195                 200                 205

Ser Ile Val Arg Ile Ile Ser Arg Ser Lys Gly Pro Ala Arg Glu Pro
    210                 215                 220

Gln Val Tyr Val Leu Asp Pro Pro Lys Glu Glu Leu Ser Lys Ser Thr
225                 230                 235                 240

Val Ser Leu Thr Cys Met Val Ile Gly Phe Tyr Pro Glu Asp Val Asp
                245                 250                 255

Val Glu Trp Gln Arg Asp Arg Gln Thr Glu Ser Glu Asp Lys Tyr Arg
            260                 265                 270

Thr Thr Pro Pro Gln Leu Asp Ala Asp Arg Ser Tyr Phe Leu Tyr Ser
        275                 280                 285

Lys Leu Arg Val Asp Arg Asn Ser Trp Gln Arg Gly Asp Thr Tyr Thr
    290                 295                 300

Cys Val Val Met His Glu Ala Leu His Asn His Tyr Met Gln Lys Ser
305                 310                 315                 320

Thr Ser Lys Ser Ala Gly Lys
                325

<210>  90
<211>  352
<212>  PRT
<213>  Bos taurus

<400>  90

Ala Ser Thr Thr Ala Pro Lys Val Tyr Pro Leu Ala Ser Ser Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Arg Gln Ser Ser Gly Leu Tyr Ser
    50                  55                  60

Leu Ser Ser Met Val Thr Val Pro Ala Ser Ser Ser Glu Thr Gln Thr
65                  70                  75                  80

Phe Thr Cys Asn Val Ala His Pro Ala Ser Ser Thr Lys Val Asp Lys
                85                  90                  95

Ala Val Thr Ala Arg Arg Pro Val Pro Thr Thr Pro Lys Thr Thr Ile
            100                 105                 110

Pro Pro Gly Lys Pro Thr Thr Pro Lys Ser Glu Val Glu Lys Thr Pro
        115                 120                 125

Cys Gln Cys Ser Lys Cys Pro Glu Pro Leu Gly Gly Leu Ser Val Phe
    130                 135                 140

Ile Phe Pro Pro Lys Pro Lys Asp Thr Leu Thr Ile Ser Gly Thr Pro
145                 150                 155                 160

Glu Val Thr Cys Val Val Val Asp Val Gly Gln Asp Asp Pro Glu Val
                165                 170                 175

Gln Phe Ser Trp Phe Val Asp Asp Val Glu Val His Thr Ala Arg Thr
            180                 185                 190

Lys Pro Arg Glu Glu Gln Phe Asn Ser Thr Tyr Arg Val Val Ser Ala
        195                 200                 205

Leu Arg Ile Gln His Gln Asp Trp Leu Gln Gly Lys Glu Phe Lys Cys
    210                 215                 220

Lys Val Asn Asn Lys Gly Leu Pro Ala Pro Ile Val Arg Thr Ile Ser
225                 230                 235                 240

Arg Thr Lys Gly Gln Ala Arg Glu Pro Gln Val Tyr Val Leu Ala Pro
                245                 250                 255

Pro Arg Glu Glu Leu Ser Lys Ser Thr Leu Ser Leu Thr Cys Leu Ile
            260                 265                 270

Thr Gly Phe Tyr Pro Glu Glu Ile Asp Val Glu Trp Gln Arg Asn Gly
        275                 280                 285

Gln Pro Glu Ser Glu Asp Lys Tyr His Thr Thr Ala Pro Gln Leu Asp
    290                 295                 300

Ala Asp Gly Ser Tyr Phe Leu Tyr Ser Lys Leu Arg Val Asn Lys Ser
305                 310                 315                 320

Ser Trp Gln Glu Gly Asp His Tyr Thr Cys Ala Val Met His Glu Ala
                325                 330                 335

Leu Arg Asn His Tyr Lys Glu Lys Ser Ile Ser Arg Ser Pro Gly Lys
            340                 345                 350

<210>  91

<211>  352
<212>  PRT
<213>  Bos taurus

<400>  91

Ala Ser Thr Thr Ala Pro Lys Val Tyr Pro Leu Ala Ser Arg Cys Gly
1               5                   10                  15

Asp Thr Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Ala Ser Thr Ser Glu Thr Gln Thr
65                  70                  75                  80

Phe Thr Cys Asn Val Ala His Pro Ala Ser Ser Thr Lys Val Asp Lys
                85                  90                  95

Ala Val Thr Ala Arg Arg Pro Val Pro Thr Thr Pro Lys Thr Thr Ile
            100                 105                 110

Pro Pro Gly Lys Pro Thr Thr Gln Glu Ser Glu Val Glu Lys Thr Pro
        115                 120                 125

Cys Gln Cys Ser Lys Cys Pro Glu Pro Leu Gly Gly Leu Ser Val Phe
    130                 135                 140

Ile Phe Pro Pro Lys Pro Lys Asp Thr Leu Thr Ile Ser Gly Thr Pro
145                 150                 155                 160

Glu Val Thr Cys Val Val Val Asp Val Gly Gln Asp Asp Pro Glu Val
                165                 170                 175

Gln Phe Ser Trp Phe Val Asp Asp Val Glu Val His Thr Ala Arg Thr
            180                 185                 190

Lys Pro Arg Glu Glu Gln Phe Asn Ser Thr Tyr Arg Val Val Ser Ala
        195                 200                 205

Leu Arg Ile Gln His Gln Asp Trp Leu Gln Gly Lys Glu Phe Lys Cys
    210                 215                 220

Lys Val Asn Asn Lys Gly Leu Pro Ala Pro Ile Val Arg Thr Ile Ser

225                 230                 235                 240

Arg Thr Lys Gly Gln Ala Arg Glu Pro Gln Val Tyr Val Leu Ala Pro
                245                 250                 255

Pro Arg Glu Glu Leu Ser Lys Ser Thr Leu Ser Leu Thr Cys Leu Ile
            260                 265                 270

Thr Gly Phe Tyr Pro Glu Glu Ile Asp Val Glu Trp Gln Arg Asn Gly
        275                 280                 285

Gln Pro Glu Ser Glu Asp Lys Tyr His Thr Thr Ala Pro Gln Leu Asp
    290                 295                 300

Ala Asp Gly Ser Tyr Phe Leu Tyr Ser Arg Leu Arg Val Asn Lys Ser
305                 310                 315                 320

Ser Trp Gln Glu Gly Asp His Tyr Thr Cys Ala Val Met His Glu Ala
                325                 330                 335

Leu Arg Asn His Tyr Lys Glu Lys Ser Ile Ser Arg Ser Pro Gly Lys
            340                 345                 350

<210>  92
<211>  990
<212>  DNA
<213>  Bos taurus

<400>  92
gcctccacca cagccccgaa agtctaccct ctgagttctt gctgcgggga caagtccagc      60

tccaccgtga ccctgggctg cctggtctcc agctacatgc ccgagccggt gaccgtgacc     120

tggaactcgg gtgccctgaa gagcggcgtg cacaccttcc cggctgtcct tcagtcctcc     180

gggctgtact ctctcagcag catggtgacc gtgcccggca gcacctcagg acagaccttc     240

acctgcaacg tagcccaccc ggccagcagc accaaggtgg acaaggctgt tgatcccaca     300

tgcaaaccat caccctgtga ctgttgccca ccccctgagc tccccggagg accctctgtc     360

ttcatcttcc caccgaaacc caaggacacc ctcacaatct cgggaacgcc cgaggtcacg     420

tgtgtggtgg tggacgtggg ccacgatgac cccgaggtga agttctcctg gttcgtggac     480

gacgtggagg taaacacagc cacgacgaag ccgagagagg agcagttcaa cagcacctac     540

cgcgtggtca gcgccctgcg catccagcac caggactgga ctggaggaaa ggagttcaag     600

tgcaaggtcc acaacgaagg cctcccggcc cccatcgtga ggaccatctc caggaccaaa     660

gggccggccc gggagccgca ggtgtatgtc ctggccccac cccaggaaga gctcagcaaa     720

agcacggtca gcctcacctg catggtcacc agcttctacc cagactacat cgccgtggag     780

tggcagagaa acgggcagcc tgagtcggag gacaagtacg gcacgacccc gccccagctg 840

gacgccgaca gctcctactt cctgtacagc aagctcaggg tggacaggaa cagctggcag 900

gaaggagaca cctacacgtg tgtggtgatg cacgaggccc tgcacaatca ctacacgcag 960

aagtccacct ctaagtctgc gggtaaatga 990

<210> 93
<211> 990
<212> DNA
<213> Bos taurus

<400> 93
gcctccacca cagccccgaa agtctaccct ctgagttctt gctgcgggga caagtccagc 60

tccaccgtga ccctgggctg cctggtctcc agctacatgc ccgagccggt gaccgtgacc 120

tggaactcgg gtgccctgaa gagcggcgtg cacaccttcc cggccgtcct tcagtcctcc 180

gggctgtact ctctcagcag catggtgacc gtgcccggca gcacctcagg acagaccttc 240

acctgcaacg tagcccaccc ggccagcagc accaaggtgg acaaggctgt tgatcccaca 300

tgcaaaccat caccctgtga ctgttgccca ccccctgagc tccccggagg accctctgtc 360

ttcatcttcc caccgaaacc caaggacacc ctcacaatct cgggaacgcc cgaggtcacg 420

tgtgtggtgg tggacgtggg ccacgatgac cccgaggtga agttctcctg gttcgtggac 480

gacgtggagg taaacacagc cacgacgaag ccgagagagg agcagttcaa cagcacctac 540

cgcgtggtca gcgccctgcg catccagcac caggactgga ctggaggaaa ggagttcaag 600

tgcaaggtcc acaacgaagg cctcccggcc cccatcgtga ggaccatctc caggaccaaa 660

gggccggccc gggagccgca ggtgtatgtc ctggccccac cccaggaaga gctcagcaaa 720

agcacggtca gcctcacctg catggtcacc agcttctacc cagactacat cgccgtggag 780

tggcagagaa acgggcagcc tgagtcggag gacaagtacg gcacgacccc gccccagctg 840

gacgccgaca gctcctactt cctgtacagc aagctcaggg tggacaggaa cagctggcag 900

gaaggagaca cctacacgtg tgtggtgatg cacgaggccc tgcacaatca ctacacgcag 960

aagtccacct ctaagtctgc gggtaaatga 990

<210> 94
<211> 990
<212> DNA
<213> Bos taurus

<400> 94
gcctccacca cagccccgaa agtctaccct ctgagttctt gctgcgggga caagtccagc 60

tccaccgtga ccctgggctg cctggtctcc agctacatgc ccgagccggt gaccgtgacc 120

tggaactcgg gtgccctgaa gagcggcgtg cacaccttcc cggccgtcct tcagtcctcc 180

gggctctact ctctcagcag catggtgacc gtgcccggca gcacctcagg aacccagacc 240

ttcacctgca acgtagccca cccggccagc agcaccaagg tggacaaggc tgttgatccc 300

agatgcaaaa caacctgtga ctgttgccca ccgcctgagc tccctggagg accctctgtc 360

ttcatcttcc caccgaaacc caaggacacc ctcacaatct cgggaacgcc cgaggtcacg 420

tgtgtggtgg tggacgtggg ccacgatgac cccgaggtga agttctcctg gttcgtggac 480

gacgtggagg taaacacagc cacgacgaag ccgagagagg agcagttcaa cagcacctac 540

cgcgtggtca gcgccctgcg catccagcac caggactgga ctggaggaaa ggagttcaag 600

tgcaaggtcc acaacgaagg cctcccagcc cccatcgtga ggaccatctc caggaccaaa 660

gggccggccc gggagccgca ggtgtatgtc ctggccccac cccaggaaga gctcagcaaa 720

agcacggtca gcctcacctg catggtcacc agcttctacc cagactacat cgccgtggag 780

tggcagagaa atgggcagcc tgagtcagag gacaagtacg gcacgacccc tccccagctg 840

gacgccgacg gctcctactt cctgtacagc aggctcaggg tggacaggaa cagctggcag 900

gaaggagaca cctacacgtg tgtggtgatg cacgaggccc tgcacaatca ctacacgcag 960

aagtccacct ctaagtctgc gggtaaatga 990

<210> 95
<211> 981
<212> DNA
<213> Bos taurus

<400> 95
gcctccacca cagccccgaa agtctaccct ctggcatcca gctgcggaga cacatccagc 60

tccaccgtga ccctgggctg cctggtgtcc agctacatgc ccgagccggt gaccgtgacc 120

tggaactcgg gtgccctgaa gagcggcgtg cacaccttcc cggctgtcct tcagtcctcc 180

gggctctact ctctcagcag catggtgacc gtgcccgcca gcagctcagg acagaccttc 240

acctgcaacg tagcccaccc ggccagcagc accaaggtgg acaaggctgt tggggtctcc 300

attgactgct ccaagtgtca taaccagcct tgcgtgaggg aaccatctgt cttcatcttc 360

ccaccgaaac ccaaagacac cctgatgatc acaggaacgc ccgaggtcac gtgtgtggtg 420

gtgaacgtgg gccacgataa ccccgaggtg cagttctcct ggttcgtgga tgacgtggag 480

gtgcacacgg ccaggtcgaa gccaagagag gagcagttca acagcacgta ccgcgtggtc 540

agcgccctgc ccatccagca ccaggactgg actggaggaa aggagttcaa gtgcaaggtc 600

aacaacaaag gcctctcggc ccccatcgtg aggatcatct ccaggagcaa agggccggcc 660

cgggagccgc aggtgtatgt cctggaccca cccaaggaag agctcagcaa aagcacgctc 720

agcgtcacct gcatggtcac cggcttctac ccagaagatg tagccgtgga gtggcagaga 780

aaccggcaga ctgagtcgga ggacaagtac cgcacgaccc cgccccagct ggacaccgac 840

cgctcctact tcctgtacag caagctcagg gtggacagga acagctggca ggaaggagac 900

gcctacacgt gtgtggtgat gcacgaggcc ctgcacaatc actacatgca gaagtccacc 960

tctaagtctg cgggtaaatg a 981

<210> 96
<211> 981
<212> DNA
<213> Bos taurus

<400> 96
gcctccacca cagccccgaa agtctaccct ctgagttctt gctgcgggga caagtccagc 60

tccaccgtga ccctgggctg cctggtgtcc agctacatgc ccgagccggt gaccgtgacc 120

tggaactcgg gtgccctgaa gagcggcgtg cacaccttcc cggccgtcct tcagtcctcc 180

gggctctact ctctcagcag catggtgacc gtgcccggca gcacctcagg acagaccttc 240

acctgcaacg tagcccaccc ggccagcagc accaaggtgg acaaggctgt tggggtctcc 300

agtgactgct ccaagcctaa taaccagcat tgcgtgaggg aaccatctgt cttcatcttc 360

ccaccgaaac ccaaagacac cctgatgatc acaggaacgc ccgaggtcac gtgtgtggtg 420

gtgaacgtgg gccacgataa ccccgaggtg cagttctcct ggttcgtgga cgacgtggag 480

gtgcacacgg ccaggacgaa gccgagagag gagcagttca acagcacgta ccgcgtggtc 540

agcgccctgc ccatccagca ccaggactgg actggaggaa aggagttcaa gtgcaaggtc 600

aacatcaaag gcctctcggc ctccatcgtg aggatcatct ccaggagcaa agggccggcc 660

cgggagccgc aggtgtatgt cctggaccca cccaaggaag agctcagcaa aagcacggtc 720

agcgtcacct gcatggtcat cggcttctac ccagaagatg tagacgtgga gtggcagaga 780

gaccggcaga ctgagtcgga ggacaagtac cgcacgaccc cgccccagct ggacgccgac 840

cgctcctact tcctgtacag caagctcagg gtggacagga acagctggca gagaggagac 900

acctacacgt gtgtggtgat gcacgaggcc ctgcacaatc actacatgca gaagtccacc 960

tctaagtctg cgggtaaatg a 981

<210> 97
<211> 984
<212> DNA
<213> Bos taurus

<400> 97
gcctccacca cagccccgaa agtctaccct ctgagttctt gctgcgggga caagtccagc 60

tcgggggtga ccctgggctg cctggtctcc agctacatgc ccgagccggt gaccgtgacc 120

tggaactcgg gtgccctgaa gagcggcgtg cacaccttcc cggccgtcct tcagtcctcc 180

gggctctact ctctcagcag catggtgacc gtgcccgcca gcagctcagg aacccagacc 240

ttcacctgca acgtagccca cccggccagc agcaccaagg tggacaaggc tgttggggtc 300

tccagtgact gctccaagcc taataaccag cattgcgtga gggaaccatc tgtcttcatc 360

ttcccaccga aacccaaaga caccctgatg atcacaggaa cgcccgaggt cacgtgtgtg 420

gtggtgaacg tgggccacga taaccccgag gtgcagttct cctggttcgt ggacgacgtg 480

gaggtgcaca cggccaggac gaagccgaga gaggagcagt tcaacagcac gtaccgcgtg 540

gtcagcgccc tgcccatcca gcaccaggac tggactggag gaaaggagtt caagtgcaag 600

gtcaacatca aaggcctctc ggcctccatc gtgaggatca tctccaggag caaagggccg 660

gcccgggagc cgcaggtgta tgtcctggac ccacccaagg aagagctcag caaaagcacg 720

gtcagcctca cctgcatggt catcggcttc tacccagaag atgtagacgt ggagtggcag 780

agagaccggc agactgagtc ggaggacaag taccgcacga ccccgcccca gctggacgcc 840

gaccgctcct acttcctgta cagcaagctc agggtggaca ggaacagctg gcagagagga 900

gacacctaca cgtgtgtggt gatgcacgag gccctgcaca atcactacat gcagaagtcc 960

acctctaagt ctgcgggtaa atga 984

<210> 98
<211> 1059
<212> DNA
<213> Bos taurus

<400> 98
gcctccacca cagccccgaa agtctaccct ctggcatcca gctgcggaga cacatccagc 60

tccaccgtga ccctgggctg cctggtctcc agctacatgc ccgagccggt gaccgtgacc 120

tggaactcgg gtgccctgaa gagcggcgtg cacaccttcc cggccgtccg gcagtcctct 180

gggctgtact ctctcagcag catggtgact gtgcccgcca gcagctcaga aacccagacc 240

ttcacctgca acgtagccca cccggccagc agcaccaagg tggacaaggc tgtcactgca 300

aggcgtccag tcccgacgac gccaaagaca actatccctc ctggaaaacc cacaacccca 360

aagtctgaag ttgaaaagac accctgccag tgttccaaat gcccagaacc tctgggagga 420

ctgtctgtct tcatcttccc accgaaaccc aaggacaccc tcacaatctc gggaacgccc 480

gaggtcacgt gtgtggtggt ggacgtgggc caggatgacc ccgaggtgca gttctcctgg 540

ttcgtggacg acgtggaggt gcacacggcc aggacgaagc cgagagagga gcagttcaac 600

agcacctacc gcgtggtcag cgccctgcgc atccagcacc aggactggct gcagggaaag 660

gagttcaagt gcaaggtcaa caacaaaggc ctcccggccc ccattgtgag gaccatctcc 720

aggaccaaag ggcaggcccg ggagccgcag gtgtatgtcc tggccccacc ccgggaagag 780

ctcagcaaaa gcacgctcag cctcacctgc ctgatcaccg gtttctaccc agaagagata 840

gacgtggagt ggcagagaaa tgggcagcct gagtcggagg acaagtacca cacgaccgca 900

ccccagctgg atgctgacgg ctcctacttc ctgtacagca agctcagggt gaacaagagc 960

agctggcagg aaggagacca ctacacgtgt gcagtgatgc acgaagcttt acggaatcac 1020

tacaaagaga agtccatctc gaggtctccg ggtaaatga 1059

<210> 99
<211> 1059
<212> DNA
<213> Bos taurus

<400> 99
gcctccacca cagccccgaa agtctaccct ctggcatccc gctgcggaga cacatccagc 60

tccaccgtga ccctgggctg cctggtctcc agctacatgc ccgagccggt gaccgtgacc 120

tggaactcgg gtgccctgaa gagtggcgtg cacaccttcc cggccgtcct tcagtcctcc 180

gggctgtact ctctcagcag catggtgacc gtgcccgcca gcacctcaga aacccagacc 240

ttcacctgca acgtagccca cccggccagc agcaccaagg tggacaaggc tgtcactgca 300

aggcgtccag tcccgacgac gccaaagaca accatccctc ctggaaaacc cacaacccag 360

gagtctgaag ttgaaaagac accctgccag tgttccaaat gcccagaacc tctgggagga 420

ctgtctgtct tcatcttccc accgaaaccc aaggacaccc tcacaatctc gggaacgccc 480

gaggtcacgt gtgtggtggt ggacgtgggc caggatgacc ccgaggtgca gttctcctgg 540

ttcgtggacg acgtggaggt gcacacggcc aggacgaagc cgagagagga gcagttcaac 600

agcacctacc gcgtggtcag cgccctgcgc atccagcacc aggactggct gcagggaaag 660

gagttcaagt gcaaggtcaa caacaaaggc ctcccggccc ccattgtgag gaccatctcc 720

aggaccaaag ggcaggcccg ggagccgcag gtgtatgtcc tggccccacc ccgggaagag 780

ctcagcaaaa gcacgctcag cctcacctgc ctgatcaccg gtttctaccc agaagagata 840

gacgtggagt ggcagagaaa tgggcagcct gagtcggagg acaagtacca cacgaccgca 900

ccccagctgg atgctgacgg ctcctacttc ctgtacagca ggctcagggt gaacaagagc 960

agctggcagg aaggagacca ctacacgtgt gcagtgatgc atgaagcttt acggaatcac 1020

tacaaagaga agtccatctc gaggtctccg ggtaaatga 1059

<210> 100
<211> 105
<212> PRT
<213> Bos taurus

<400> 100

Gln Pro Lys Ser Pro Pro Ser Val Thr Leu Phe Pro Pro Ser Thr Glu
1               5                   10                  15

Glu Leu Asn Gly Asn Lys Ala Thr Leu Val Cys Leu Ile Ser Asp Phe
            20                  25                  30

Tyr Pro Gly Ser Val Thr Val Val Trp Lys Ala Asp Gly Ser Thr Ile
        35                  40                  45

Thr Arg Asn Val Glu Thr Thr Arg Ala Ser Lys Gln Ser Asn Ser Lys
    50                  55                  60

Tyr Ala Ala Ser Ser Tyr Leu Ser Leu Thr Ser Ser Asp Trp Lys Ser
65                  70                  75                  80

Lys Gly Ser Tyr Ser Cys Glu Val Thr His Glu Gly Ser Thr Val Thr
                85                  90                  95

Lys Thr Val Lys Pro Ser Glu Cys Ser
            100                 105

<210>  101
<211>  318
<212>  DNA
<213>  Bos taurus

<400>  101
cagcccaagt ccccaccctc ggtcaccctg ttcccgccct ccacggagga gctcaacggc      60

aacaaggcca ccctggtgtg tctcatcagc gacttctacc cgggtagcgt gaccgtggtc     120

tggaaggcag acggcagcac catcacccgc aacgtggaga ccacccgggc ctccaaacag     180

agcaacagca agtacgcggc cagcagctac ctgagcctga cgagcagcga ctggaaatcg     240

aaaggcagtt acagctgcga ggtcacgcac gaggggagca ccgtgacgaa gacagtgaag     300

ccctcagagt gttcttag                                                   318

<210>  102
<211>  328
<212>  PRT
<213>  Bos taurus

<400>  102

Ala Ser Thr Thr Ala Pro Lys Val Tyr Pro Leu Ser Ser Cys Cys Gly
1               5                   10                  15

Asp Lys Ser Ser Ser Thr Val Thr Leu Gly Cys Leu Val Ser Ser Tyr
            20                  25                  30

Met Pro Glu Pro Val Thr Val Thr Trp Asn Ser Gly Ala Leu Lys Ser
        35                  40                  45

Gly Val His Thr Phe Pro Ala Val Leu Gln Ser Ser Gly Leu Tyr Ser
    50                  55                  60

Leu Ser Ser Met Val Thr Val Pro Gly Ser Thr Ser Gly Gln Thr Phe

65                  70                  75                  80

Thr Cys Asn Val Ala His Pro Ala Ser Ser Thr Lys Val Asp Lys Ala
                85                  90                  95

Val Asp Pro Thr Cys Lys Pro Ser Pro Cys Asp Cys Cys Pro Pro Pro
            100                 105                 110

Pro Val Ala Gly Pro Ser Val Phe Ile Phe Pro Pro Lys Pro Lys Asp
        115                 120                 125

Thr Leu Thr Ile Ser Gly Thr Pro Glu Val Thr Cys Val Val Val Asp
    130                 135                 140

Val Gly His Asp Asp Pro Glu Val Lys Phe Ser Trp Phe Val Asp Asp
145                 150                 155                 160

Val Glu Val Asn Thr Ala Thr Thr Lys Pro Arg Glu Glu Gln Phe Asn
                165                 170                 175

Ser Thr Tyr Arg Val Val Ser Ala Leu Arg Ile Gln His Gln Asp Trp
            180                 185                 190

Thr Gly Gly Lys Glu Phe Lys Cys Lys Val His Asn Glu Gly Leu Pro
        195                 200                 205

Ser Ser Ile Val Arg Thr Ile Ser Arg Thr Lys Gly Pro Ala Arg Glu
    210                 215                 220

Pro Gln Val Tyr Val Leu Ala Pro Pro Gln Glu Glu Leu Ser Lys Ser
225                 230                 235                 240

Thr Val Ser Leu Thr Cys Met Val Thr Ser Phe Tyr Pro Asp Tyr Ile
                245                 250                 255

Ala Val Glu Trp Gln Arg Asn Gly Gln Pro Glu Ser Glu Asp Lys Tyr
            260                 265                 270

Gly Thr Thr Pro Pro Gln Leu Asp Ala Asp Ser Ser Tyr Phe Leu Tyr
        275                 280                 285

Ser Lys Leu Arg Val Asp Arg Asn Ser Trp Gln Glu Gly Asp Thr Tyr
    290                 295                 300

Thr Cys Val Val Met His Glu Ala Leu His Asn His Tyr Thr Gln Lys
305                 310                 315                 320

Ser Thr Ser Lys Ser Ala Gly Lys
                325

<210>  103
<211>  987
<212>  DNA
<213>  Bos taurus

<400>  103
gctagcacaa ctgctcctaa ggtgtacccc ctgagctctt gctgcggcga caagtctagc      60

agcaccgtga ccctcggatg cctcgtcagc agctatatgc ctgagccagt tacagtgaca     120

tggaattctg gtgcccttaa gtccggcgtc cataccttcc ctgctgtgct gcagtcctct     180

ggcctgtaca gtttgtcctc tatggtgaca gtacccggtt ccacctccgg acagaccttt     240

acctgtaatg tggctcatcc cgcctcctcc acaaaggtgg ataaggctgt tgaccctacc     300

tgtaaaccca gtccatgcga ctgctgtccc ccccctccag ttgccggacc ctcagtcttt     360

attttcccac ccaaacccaa agacaccctg acaatctctg gaacaccaga agtcacctgc     420

gtcgtcgtgg atgtgggcca cgacgatcct gaggtaaaat tctcatggtt cgtcgacgat     480

gtggaagtga atacagctac tacaaaacct cgcgaagagc agtttaactc tacctatcga     540

gtggtttctg ctttgcggat tcagcatcag gattggacag gcggcaaaga gtttaaatgt     600

aaagtccata acgagggact tccttctagt atcgtgcgca ctatcagtag aactaaaggg     660

cctgctcggg aacctcaggt gtacgtcctg gcacctccac aggaagagct gagtaagtct     720

acagtttctc tgacttgtat ggtaacatct ttttatccag attacatcgc agttgaatgg     780

cagaggaacg ggcagccaga gagtgaggat aagtacggga ctactccacc acagctggac     840

gcagactcaa gttacttcct gtactcaaag ctgagggttg acagaaactc atggcaggag     900

ggggacactt acacttgcgt agttatgcac gaggcacttc acaaccacta cactcagaag     960

agtacttcaa agagtgcagg gaagtaa                                         987

<210>  104
<211>  30
<212>  DNA
<213>  Artificial Sequence

<220>
<223>  primer

<400>  104
cgcggatatc atggattaca cagcgaagtg                                       30

<210>  105
<211>  29
<212>  DNA
<213>  Artificial Sequence

<220>

<223>  primer

<400>  105
cggggtaccc cagagctgtt gctggttat                                          29


<210>  106
<211>  30
<212>  DNA
<213>  Artificial Sequence

<220>
<223>  primer

<400>  106
cgcggctagc atgagaatgt ttagtgtctt                                         30


<210>  107
<211>  49
<212>  DNA
<213>  Artificial Sequence

<220>
<223>  primer

<400>  107
cgcggatatc ttaatggtga tggtgatggt gagtcctctc acttgctgg                    49


<210>  108
<211>  22
<212>  DNA
<213>  Artificial Sequence

<220>
<223>  primer

<400>  108
aagcttcgat tgaccagagc ag                                                 22


<210>  109
<211>  21
<212>  DNA
<213>  Artificial Sequence

<220>
<223>  primer

<400>  109
tcaccataaa gggcctccaa c                                                  21


<210>  110
<211>  21
<212>  DNA
<213>  Artificial Sequence

<220>
<223>  primer

<400>  110

tggcgtcgtg attagtgatg a                                                21

<210>  111
<211>  21
<212>  DNA
<213>  Artificial Sequence

<220>
<223>  primer

<400>  111
cagagggcta cgatgtgatg g                                                21

<210>  112
<211>  399
<212>  DNA
<213>  Artificial Sequence

<220>
<223>  codon-optimized sequence

<400>  112
atggaatctc aaactcatgt tttgatttca ttacttctga gtgtttccgg aacctacggt      60

gatatcgcta tcactcaatc tccctcctct gttgctgtgt ctgtgggcga aaccgttacc     120

ctgtcctgca agtccagtca gtctcttctc tactccgaga atcaaaagga ctacctgggc     180

tggtaccaac agaagcccgg ccagacccca aagccactga tatactgggc aaccaacagg     240

cacaccggag tgcccgacag gttcacaggc agtggatctg gcaccgactt taccttgatc     300

atttcaagcg tgcaggctga agatctggcc gactactact gtggtcagta tctggtgtat     360

cctttcactt tcgggccagg gacaaaactc gagctcaaa                            399

<210>  113
<211>  411
<212>  DNA
<213>  Artificial Sequence

<220>
<223>  codon-optimized sequence

<400>  113
atggggtggt cccagattat attgttcctc gtcgccgccg ccacttgcgt acacagccaa      60

gtgcaacttc aacaaagcgg tgcagaactg gtaaagcccg gtagctctgt gaaaatatcc     120

tgtaaagcca gtggctacac atttaccagc aactttatgc actgggtgaa gcaacagccc     180

ggaaatggct tggagtggat tggctggatc tatcccgaat atggtaacac caagtataat     240

cagaagttcg acggtaaggc caccctcacc gccgataagt catcctccac cgcctatatg     300

cagctcagca gcctgaccag cgaggattcc gctgtgtact tctgtgccag cgaagaggct     360

gtgatctcat tggtgtattg gggacagggc accctcgtca ccgtgtccag c               411

<210>  114
<211>  318
<212>  DNA
<213>  Artificial Sequence

<220>
<223>  codon-optimized sequence

<400>  114
cagcctaaga gtcctccttc tgtaacactc tttcccccct ctaccgagga actcaacggc        60

aataaagcta ccttggtttg ccttatttct gatttctacc ccgggtctgt gaccgtggtg       120

tggaaagctg atgggtccac cattactcgg aatgtggaaa ccacccgggc ttctaagcag       180

tccaactcta aatacgcagc atcctcctat ttgagtctta ctagtagtga ctggaagtca       240

aagggtagtt acagttgcga agtcacacat gaaggttcaa cagtgacaaa gacagtcaag       300

ccctcagagt gctcatag                                                     318


<210>  115
<211>  238
<212>  PRT
<213>  Artificial Sequence

<220>
<223>  chimeric  L chain

<400>  115

Met Glu Ser Gln Thr His Val Leu Ile Ser Leu Leu Leu Ser Val Ser
1               5                   10                  15

Gly Thr Tyr Gly Asp Ile Ala Ile Thr Gln Ser Pro Ser Ser Val Ala
            20                  25                  30

Val Ser Val Gly Glu Thr Val Thr Leu Ser Cys Lys Ser Ser Gln Ser
        35                  40                  45

Leu Leu Tyr Ser Glu Asn Gln Lys Asp Tyr Leu Gly Trp Tyr Gln Gln
    50                  55                  60

Lys Pro Gly Gln Thr Pro Lys Pro Leu Ile Tyr Trp Ala Thr Asn Arg
65                  70                  75                  80

His Thr Gly Val Pro Asp Arg Phe Thr Gly Ser Gly Ser Gly Thr Asp
                85                  90                  95

Phe Thr Leu Ile Ile Ser Ser Val Gln Ala Glu Asp Leu Ala Asp Tyr
            100                 105                 110

Tyr Cys Gly Gln Tyr Leu Val Tyr Pro Phe Thr Phe Gly Pro Gly Thr
        115                 120                 125

Lys Leu Glu Leu Lys Gln Pro Lys Ser Pro Pro Ser Val Thr Leu Phe
    130                 135                 140

Pro Pro Ser Thr Glu Glu Leu Asn Gly Asn Lys Ala Thr Leu Val Cys
145                 150                 155                 160

Leu Ile Ser Asp Phe Tyr Pro Gly Ser Val Thr Val Val Trp Lys Ala
                165                 170                 175

Asp Gly Ser Thr Ile Thr Arg Asn Val Glu Thr Thr Arg Ala Ser Lys
            180                 185                 190

Gln Ser Asn Ser Lys Tyr Ala Ala Ser Ser Tyr Leu Ser Leu Thr Ser
        195                 200                 205

Ser Asp Trp Lys Ser Lys Gly Ser Tyr Ser Cys Glu Val Thr His Glu
    210                 215                 220

Gly Ser Thr Val Thr Lys Thr Val Lys Pro Ser Glu Cys Ser
225                 230                 235

<210>  116
<211>  465
<212>  PRT
<213>  Artificial Sequence

<220>
<223>  chimeric H chain

<400>  116

Met Gly Trp Ser Gln Ile Ile Leu Phe Leu Val Ala Ala Ala Thr Cys
1               5                   10                  15

Val His Ser Gln Val Gln Leu Gln Gln Ser Gly Ala Glu Leu Val Lys
            20                  25                  30

Pro Gly Ser Ser Val Lys Ile Ser Cys Lys Ala Ser Gly Tyr Thr Phe
        35                  40                  45

Thr Ser Asn Phe Met His Trp Val Lys Gln Gln Pro Gly Asn Gly Leu
    50                  55                  60

Glu Trp Ile Gly Trp Ile Tyr Pro Glu Tyr Gly Asn Thr Lys Tyr Asn
65                  70                  75                  80

Gln Lys Phe Asp Gly Lys Ala Thr Leu Thr Ala Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Ser Glu Glu Ala Val Ile Ser Leu Val Tyr Trp Gly
        115                 120                 125

Gln Gly Thr Leu Val Thr Val Ser Ser Ala Ser Thr Thr Ala Pro Lys
    130                 135                 140

Val Tyr Pro Leu Ser Ser Cys Cys Gly Asp Lys Ser Ser Ser Thr Val
145                 150                 155                 160

Thr Leu Gly Cys Leu Val Ser Ser Tyr Met Pro Glu Pro Val Thr Val
                165                 170                 175

Thr Trp Asn Ser Gly Ala Leu Lys Ser Gly Val His Thr Phe Pro Ala
            180                 185                 190

Val Leu Gln Ser Ser Gly Leu Tyr Ser Leu Ser Ser Met Val Thr Val
        195                 200                 205

Pro Gly Ser Thr Ser Gly Gln Thr Phe Thr Cys Asn Val Ala His Pro
    210                 215                 220

Ala Ser Ser Thr Lys Val Asp Lys Ala Val Asp Pro Thr Cys Lys Pro
225                 230                 235                 240

Ser Pro Cys Asp Cys Cys Pro Pro Pro Pro Val Ala Gly Pro Ser Val
                245                 250                 255

Phe Ile Phe Pro Pro Lys Pro Lys Asp Thr Leu Thr Ile Ser Gly Thr
            260                 265                 270

Pro Glu Val Thr Cys Val Val Val Asp Val Gly His Asp Asp Pro Glu
        275                 280                 285

Val Lys Phe Ser Trp Phe Val Asp Asp Val Glu Val Asn Thr Ala Thr
    290                 295                 300

Thr Lys Pro Arg Glu Glu Gln Phe Asn Ser Thr Tyr Arg Val Val Ser
305                 310                 315                 320

Ala Leu Arg Ile Gln His Gln Asp Trp Thr Gly Gly Lys Glu Phe Lys
                325                 330                 335

Cys Lys Val His Asn Glu Gly Leu Pro Ser Ser Ile Val Arg Thr Ile
            340                 345                 350

Ser Arg Thr Lys Gly Pro Ala Arg Glu Pro Gln Val Tyr Val Leu Ala
        355                 360                 365

Pro Pro Gln Glu Glu Leu Ser Lys Ser Thr Val Ser Leu Thr Cys Met
    370                 375                 380

Val Thr Ser Phe Tyr Pro Asp Tyr Ile Ala Val Glu Trp Gln Arg Asn
385                 390                 395                 400

Gly Gln Pro Glu Ser Glu Asp Lys Tyr Gly Thr Thr Pro Pro Gln Leu
                405                 410                 415

Asp Ala Asp Ser Ser Tyr Phe Leu Tyr Ser Lys Leu Arg Val Asp Arg
            420                 425                 430

Asn Ser Trp Gln Glu Gly Asp Thr Tyr Thr Cys Val Val Met His Glu
        435                 440                 445

Ala Leu His Asn His Tyr Thr Gln Lys Ser Thr Ser Lys Ser Ala Gly
    450                 455                 460

Lys
465

<210>  117
<211>  717
<212>  DNA
<213>  Artificial Sequence

<220>
<223>  codon-optimized sequence

<400>  117
atggaatctc aaactcatgt tttgatttca ttacttctga gtgtttccgg aacctacggt      60

gatatcgcta tcactcaatc tccctcctct gttgctgtgt ctgtgggcga aaccgttacc     120

ctgtcctgca agtccagtca gtctcttctc tactccgaga atcaaaagga ctacctgggc     180

tggtaccaac agaagcccgg ccagacccca aagccactga tatactgggc aaccaacagg     240

cacaccggag tgcccgacag gttcacaggc agtggatctg gcaccgactt taccttgatc     300

atttcaagcg tgcaggctga agatctggcc gactactact gtggtcagta tctggtgtat     360

cctttcactt tcgggccagg gacaaaactc gagctcaaac agcctaagag tcctccttct     420

gtaacactct ttcccccctc taccgaggaa ctcaacggca ataaagctac cttggtttgc     480

cttatttctg atttctaccc cgggtctgtg accgtggtgt ggaaagctga tgggtccacc     540

attactcgga atgtggaaac cacccgggct tctaagcagt ccaactctaa atacgcagca     600

tcctcctatt tgagtcttac tagtagtgac tggaagtcaa agggtagtta cagttgcgaa     660

gtcacacatg aaggttcaac agtgacaaag acagtcaagc cctcagagtg ctcatag 717

<210> 118
<211> 1398
<212> DNA
<213> Artificial Sequence

<220>
<223> codon-optimized sequence

<400> 118
   atggggtggt cccagattat attgttcctc gtcgccgccg ccacttgcgt acacagccaa 60
   gtgcaacttc aacaaagcgg tgcagaactg gtaaagcccg gtagctctgt gaaaatatcc 120
   tgtaaagcca gtggctacac atttaccagc aactttatgc actgggtgaa gcaacagccc 180
   ggaaatggct tggagtggat tggctggatc tatcccgaat atggtaacac caagtataat 240
   cagaagttcg acggtaaggc caccctcacc gccgataagt catcctccac cgcctatatg 300
   cagctcagca gcctgaccag cgaggattcc gctgtgtact tctgtgccag cgaagaggct 360
   gtgatctcat tggtgtattg gggacagggc accctcgtca ccgtgtccag cgctagcaca 420
   actgctccta aggtgtaccc cctgagctct tgctgcggcg acaagtctag cagcaccgtg 480
   accctcggat gcctcgtcag cagctatatg cctgagccag ttacagtgac atggaattct 540
   ggtgccctta agtccggcgt ccataccttc cctgctgtgc tgcagtcctc tggcctgtac 600
   agtttgtcct ctatggtgac agtacccggt tccacctccg gacagacctt tacctgtaat 660
   gtggctcatc ccgcctcctc cacaaaggtg gataaggctg ttgaccctac ctgtaaaccc 720
   agtccatgcg actgctgtcc cccccctcca gttgccggac cctcagtctt tatttttcca 780
   cccaaaccca aagacaccct gacaatctct ggaacaccag aagtcacctg cgtcgtcgtg 840
   gatgtgggcc acgacgatcc tgaggtaaaa ttctcatggt tcgtcgacga tgtggaagtg 900
   aatacagcta ctacaaaacc tcgcgaagag cagtttaact ctacctatcg agtggtttct 960
   gctttgcgga ttcagcatca ggattggaca ggcggcaaag agtttaaatg taaagtccat 1020
   aacgagggac ttccttctag tatcgtgcgc actatcagta gaactaaagg gcctgctcgg 1080
   gaacctcagg tgtacgtcct ggcacctcca caggaagagc tgagtaagtc tacagtttct 1140
   ctgacttgta tggtaacatc tttttatcca gattacatcg cagttgaatg gcagaggaac 1200
   gggcagccag agagtgagga taagtacggg actactccac cacagctgga cgcagactca 1260
   agttacttcc tgtactcaaa gctgagggtt gacagaaact catggcagga gggggacact 1320
   tacacttgcg tagttatgca cgaggcactt cacaaccact acactcagaa gagtacttca 1380
   aagagtgcag ggaagtaa 1398

<210> 119

<211>  20
<212>  DNA
<213>  Artificial Sequence

<220>
<223>  primer

<400>  119
ttttacgtgc ccaaggttaa                                                   20


<210>  120
<211>  20
<212>  DNA
<213>  Artificial Sequence

<220>
<223>  primer

<400>  120
cgtttactgt tgcatcatca                                                   20


<210>  121
<211>  19
<212>  DNA
<213>  Artificial Sequence

<220>
<223>  primer

<400>  121
tgctggatga ctttaaggg                                                    19


<210>  122
<211>  19
<212>  DNA
<213>  Artificial Sequence

<220>
<223>  primer

<400>  122
agggcagaaa gcgatgaca                                                    19


<210>  123
<211>  20
<212>  DNA
<213>  Artificial Sequence

<220>
<223>  primer

<400>  123
ataaccaggt cattcaaagg                                                   20


<210>  124
<211>  20
<212>  DNA
<213>  Artificial Sequence

<220>
<223>  primer

<400>  124
attctgactt ctcttccgct                                                    20


<210>  125
<211>  20
<212>  DNA
<213>  Artificial Sequence

<220>
<223>  primer

<400>  125
taacaagcca gtagcccacg                                                    20


<210>  126
<211>  21
<212>  DNA
<213>  Artificial Sequence

<220>
<223>  primer

<400>  126
gcaagggctc ttgatggcag a                                                  21


<210>  127
<211>  24
<212>  DNA
<213>  Artificial Sequence

<220>
<223>  primer

<400>  127
ctgctgaggc tcaagttaaa agtg                                               24


<210>  128
<211>  19
<212>  DNA
<213>  Artificial Sequence

<220>
<223>  primer

<400>  128
cagccggttg ctgaggtag                                                     19


<210>  129
<211>  20
<212>  DNA
<213>  Artificial Sequence

<220>
<223>  primer

<400>  129
cacaacctga gcctgcacaa                                                   20


<210>  130
<211>  22
<212>  DNA
<213>  Artificial Sequence

<220>
<223>  primer

<400>  130
tcttgcggaa ctcaaactca tc                                                22


<210>  131
<211>  21
<212>  DNA
<213>  Artificial Sequence

<220>
<223>  primer

<400>  131
atggaaacaa ccagtcggtg a                                                 21


<210>  132
<211>  22
<212>  DNA
<213>  Artificial Sequence

<220>
<223>  primer

<400>  132
tttctgcaca tactccatcg ct                                                22


<210>  133
<211>  20
<212>  DNA
<213>  Artificial Sequence

<220>
<223>  primer

<400>  133
tcttccagcc ttccttcctg                                                   20


<210>  134
<211>  20
<212>  DNA
<213>  Artificial Sequence

<220>
<223>  primer

<400>  134
accgtgttgg cgtagaggtc                                                   20

<210>  135
<211>  23
<212>  DNA
<213>  Artificial Sequence

<220>
<223>  primer

<400>  135
ggcgtgaacc acgagaagta taa                                               23


<210>  136
<211>  19
<212>  DNA
<213>  Artificial Sequence

<220>
<223>  primer

<400>  136
ccctccacga tgccaaagt                                                    19


<210>  137
<211>  20
<212>  DNA
<213>  Artificial Sequence

<220>
<223>  primer

<400>  137
gggggtttac tgttgcttga                                                   20


<210>  138
<211>  20
<212>  DNA
<213>  Artificial Sequence

<220>
<223>  primer

<400>  138
gccactcagg acttggtgat                                                   20


<210>  139
<211>  20
<212>  DNA
<213>  Artificial Sequence

<220>
<223>  primer

<400>  139
acgttttctc gtgaagccct                                                   20


<210>  140

<211>  20
<212>  DNA
<213>  Artificial Sequence

<220>
<223>  primer

<400>  140
tctaccagaa gggcgggata                                                    20


<210>  141
<211>  20
<212>  DNA
<213>  Artificial Sequence

<220>
<223>  primer

<400>  141
gtgaccatcg ccacctactt                                                    20


<210>  142
<211>  20
<212>  DNA
<213>  Artificial Sequence

<220>
<223>  primer

<400>  142
ctcatcgcac sgatgatgct                                                    20


<210>  143
<211>  32
<212>  DNA
<213>  Artificial Sequence

<220>
<223>  primer

<400>  143
atatgcggcc gcatggggac cccgcgggcg ct                                      32


<210>  144
<211>  30
<212>  DNA
<213>  Artificial Sequence

<220>
<223>  primer

<400>  144
gcgcaagctt tcagaggggc caggagcagt                                         30


<210>  145
<211>  35
<212>  DNA
<213>  Artificial Sequence

<220>
<223>  primer

<400>  145
ctagctagca ccatgaggat atatagtgtc ttaac                                35


<210>  146
<211>  31
<212>  DNA
<213>  Artificial Sequence

<220>
<223>  primer

<400>  146
caatctcgag ttacagacag aagatgactg c                                    31


<210>  147
<211>  29
<212>  DNA
<213>  Artificial Sequence

<220>
<223>  primer

<400>  147
gctagcatga ggatatatag tgtcttaac                                       29


<210>  148
<211>  26
<212>  DNA
<213>  Artificial Sequence

<220>
<223>  primer

<400>  148
gatatcattc ctctttttg ctggat                                           26